# EUROPEAN PATENT APPLICATION

(11) **EP 4 722 209 A1**
(43) Date of publication of application: **08.04.2026**
(21) Application number: 24814366.1
(22) Date of filing: 26.05.2024
(51) Int. Cl.: C07D 405/14, C07D 417/14, A61K 31/44, A61K 31/497, A61K 31/501, A61P 5/32

(54) **PROTAC COMPOUND TARGETING ESTROGEN RECEPTOR, PREPARATION METHOD THEREFOR, AND USE THEREOF**

(30) Priority: 26.05.2023 CN 202310610037
(71) Applicant: Beijing Shenogen Pharma Group, Beijing 102206 (CN)
(72) Inventor: MENG, Kun, Beijing 102206 (CN); CHEN, Feng, Beijing 102206 (CN); ZHANG, Lei, Beijing 102206 (CN); WANG, Jing, Beijing 102206 (CN); GUO, Chan, Beijing 102206 (CN); JIN, Mingji, Beijing 102206 (CN); WANG, Yonggang, Beijing 102206 (CN); FAN, Lixin, Beijing 102206 (CN); LU, Xin, Beijing 102206 (CN); RUAN, Ningning, Beijing 102206 (CN); LV, Kesi, Beijing 102206 (CN); PENG, Yong, Beijing 102206 (CN)
(74) Representative: Lavoix
(86) International application number: PCT/CN2024/095380
(87) International publication number: WO 2024/245169

(57) **Abstract**

Provided are a PROTAC compound targeting the hydrolysis of an estrogen receptor (ER) protein and a use thereof.

## Description

### TECHNICAL FIELD

The present application relates to a PROTAC compound targeting the hydrolysis of estrogen receptor (ER) protein, its preparation method and treatment or prevention of diseases mediated by estrogen receptor protein, such as breast cancer, ovarian cancer, etc., belonging to the field of biomedicine.

### BACKGROUND

PROTAC, also known as proteolysis targeting chimera, is a novel engineering technology for targeted protein degradation that utilizes the natural protein cleaning system - ubiquitin (UPS) - proteasome system in human cells to induce degradation of target proteins, selectively and efficiently degrading and removing pathogenic proteins. Ubiquitin is a small protein existing in all eukaryotes, consisting of 76 amino acids with a molecular weight of approximately 8.45 kDa. The main function of ubiquitin is to label proteins that need to be degraded. When ubiquitin attaches to proteins and marks them as defective or damaged, these proteins will be recognized and transported to the proteasome for degradation, to achieve protein balance within the cell.

Most of the small molecule drugs currently used in clinical practice utilize a "occupancy driven" mode of action to inhibit protein function and exert therapeutic effects on diseases. Unlike traditional small molecule inhibitors and antagonists, PROTAC has rapidly developed into a new approach for drug development in recent years attributed to its ability to induce the degradation of pathogenic target proteins. PROTAC is a bifunctional molecule composed of two small molecule ligands, typically consisting of three parts: a targeting protein ligand, an E3 ubiquitin ligase ligand, and a suitable length of linker, which are connected together. Once the PROTAC molecule enters the cell, the protein-targeting ligand at one end of its structure its structure can specifically bind to the target protein, while the E3 ubiquitin ligase (such as cereblon, CRBN) ligand at the other end can recruit E3 ligase, thereby forming a stable target protein-PROTAC-E3 ubiquitin ligase ternary complex. After labeling the target protein with ubiquitination, the ubiquitinated protein can be recognized and degraded by the proteasome 26S inside the cell. Traditional small molecules and antibodies exert their therapeutic effects by inhibiting target protein function through a "occupancy driven" mode of action. This mode of action requires inhibitors or monoclonal antibodies to achieve higher concentrations to occupy the active site of the target, thereby blocking downstream signal transduction pathways. PROTACs operate in an "event driven" mode, capable of binding to any site of the target protein and inducing protein degradation without requiring high binding affinity. Therefore, PROTACs can target proteins with smooth surfaces lacking small-molecule binding regions. 80% of pathogenic proteins in human cells lack active sites accessible to traditional small molecules, which means that many target proteins are undruggable by small molecules may be suitable for PROTAC technology. As long as PROTAC mediates the formation of ternary complexes and tags the target protein for ubiquitination, theoretically it can be reused in a cyclical manner. Therefore, catalytic doses can be effective, and the lower administering concentration reduces toxicity and side effects, making PROTAC drugs relatively safe. Moreover, PROTACs can target proteins without active sites, such as scaffold proteins, by simply forming binding interactions. This significantly expands the range of targetable proteins. To date, over 100 proteins have been successfully degraded, including those previously deemed "undruggable.". Since ubiquitin-mediated degradation occurs intracellularly, small-molecule PROTACs currently focus primarily on attacking targets within the cell, including cytoplasmic and nuclear proteins.

With the extensive and in-depth research on PROTACs, an increasing number of reports on the widespread use of PROTACs in anti-tumor research have emerged in recent years, as disclosed in the following journals (such as : Crews et al, Curr Opin Chem Biol.2019; 50: 111-119;Xu et al, Eur J Med Chem. 2022; 235: 114290;Zou et al, Eur J Med Chem. 2021; 226: 113849;Qin et al, Front Pharmacol, 2021; 12: 692574;Song et al, J Hematol Oncol. 2020;13(1):50; Crews et al, Cancer Res. 2019; 79(1): 251-262; Rao et al, ACS Med Chem Lett. 2020; 11(3): 237-240; Wang et al , J Med Chem. 2019; 62(21): 9471-9487;Crews et al, Pharmacol Ther. 2017 Jun;174:138-144.) and patents and CN113248473, CN112812100, CN109761970, US2019019419, US20190151295, US20200129627, US20180125821, US20160045607, CN107428734, WO2017197046, WO2002020740, WO2017197056, WO2017117474, WO2017197055).

ER⁺and HER⁻ breast cancer refer to estrogen receptor (ER) positive and human epidermal growth factor receptor 2 (HER2) negative breast cancer. This type of breast cancer is the most common type of breast cancer, accounting for 65% of breast cancer cases in women under 50 years old and 75% of breast cancer cases in women aged 50 and above. Estrogen (E2) is a class of steroid compounds with broad biological activities, including estradiol, estriol, estrone, etc. These compounds play important roles in maintaining physiological activities such as reproductive system development and bone maturation. The estrogen receptor (ER) a member of the steroid hormone receptor superfamily, functions as a ligand-dependent transcription factor. Estrogen binds to ER and stimulates ER-regulated transcription, thereby promoting tumor cell growth and proliferation. Hormone therapy for ER positive breast cancer can reduce estrogen production, interrupt ER signaling, degrade ER, or change ER regulated signal transduction or proliferation pathways. Estrogen production and ER signal transduction drive the initiation, growth, proliferation, and metastasis of breast cancer, serving as targets for effective drugs against early-stage disease. Novel targeted therapy combined with endocrine therapy can improve the outcomes in advanced breast cancer and inhibit key pathways involved in cell growth, proliferation and metastasis. Mutations in the ER gene ESR1 (ESR1 mut), or epigenetic alterations in c-myc, cyclin D, and epidermal growth factor receptor (EGFR), are associated with resistance to endocrine therapy.

Epidemiological studies have revealed that ERα expression is higher in breast tissue from breast cancer patients than in breast tissue from non-breast cancer patients. Furthermore, there is more ERα expression in breast epithelial cells of high-incidence populations of breast cancer than that of low-incidence populations. During the development and progression of breast cancer, the most critical factor may not be the levels of ERα or ERβ individually, but rather the ratio of ERα to ERβ.Breast cancer is the most common malignant tumor affecting women. According tothe latest data from the American Cancer Society, over 250,000 new cases of breast cancer were diagnosed annually in the United States, nearly 80% of which were estrogen receptor positive (ER+). Endocrine therapy for breast cancer mainly exerts its antitumor effects by blocking the binding of estrogen to tumor cells and inhibiting estrogen production. Estrogen acts as an endocrine growth factor for breast cancer, and estrogen deprivation is an established treatment for advanced breast cancer in premenopausal women. The main treatment methods currently include: 1. Oophorectomy is the most economical surgical castration approach, which is the most thorough treatment, but has certain trauma. Pharmacological ovarian function suppression is also an alternative method, though complete suppression cannot be achieved in a small proportion of patients. 2. Aromatase inhibitors can block the estrogen receptor conversion pathway and reduce estrogen levels. 3. Estrogen receptor (ER) antagonists, modulators, or degraders can reduce the ER expression at the transcript or protein level, lower estrogen receptor level, and inhibit cancer cell growth. The existing endocrine therapies still face limitations, including such as restricted applicability, ineffectiveness against mutant variants, development of drug resistance, and significant side effects. Therefore, more effective and safer treatment approaches still need to be developed for clinical treatment. In 2019,Arvinas's ER-targeting PROTAC drug ARV-471entered phase I clinical studies. Preclinical results demonstrated that ARV-471 significantly inhibited tumor growth in multiple ER dependent breast cancer models. Based on the preliminary data of phase I clinical studies, ARV-471 demonstrated favorable antitumor activity and a favorable safety profile in patients who had failed prior endocrine therapy and harbored ER mutations. This is the first PROTAC drug to enter the clinical stage in the field of breast cancer.

### Summary

One objective of the present application is to provide a novel oral estrogen receptor targeted protein degrading agent (PROTAC) compound, or a pharmaceutically acceptable salt, solvate, hydrate, or isomer thereof.

Another objective of this application is to provide the use of the compounds of this application, or their pharmaceutically acceptable salts, solvates, hydrates, or isomers.

Specifically, the present application relates to the following technical solutions:
In one embodiment, the present application provides a PROTAC compound targeting an estrogen receptor represented by formula (I) or a pharmaceutically acceptable salt, solvate, hydrate, or isomer thereof: 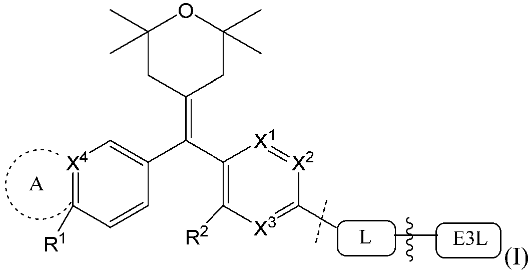
wherein 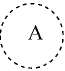 is absent, substituted or unsubstituted 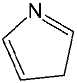 , substituented or unsubstituted 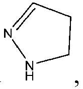 X⁴ is N or CRa, the substituent is selected from the group consisting of halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl;
when A is absent, R¹ is selected from the group consisting of halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl, preferably R₁ is selected from -NH₂ or -OH;
X¹ is N or CR³;
X² or X³ is independently selected from CH,CR³ and N;
R² and R³ are each independently selected from H, halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl;
Ra is selected from the group consisting of H, halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl;
L is any one selected from the group consisting of: 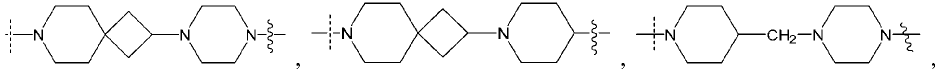 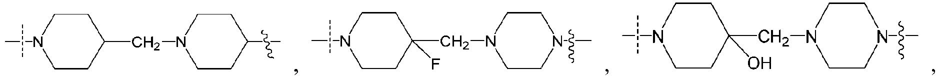 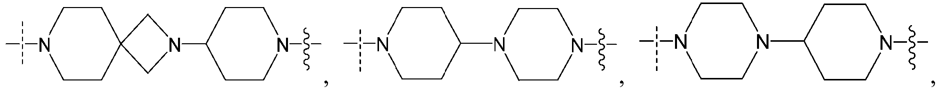 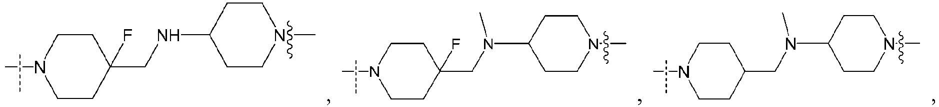 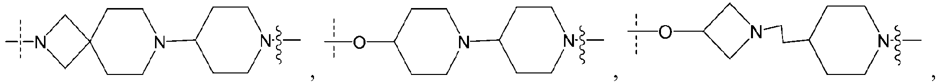 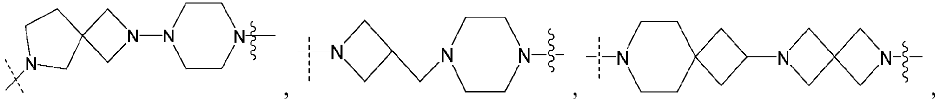 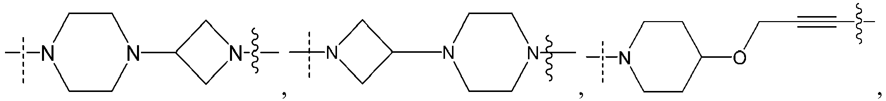 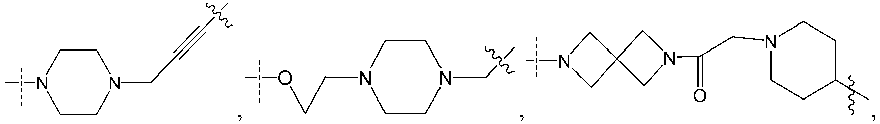 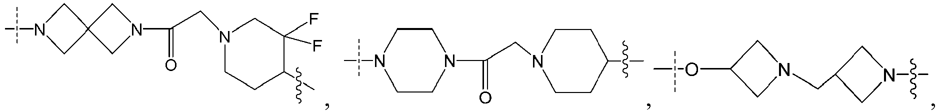 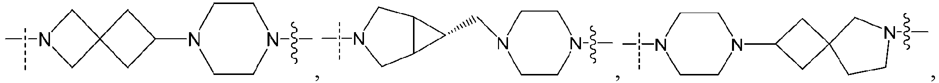 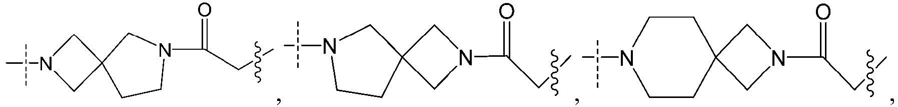 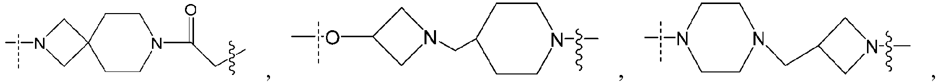 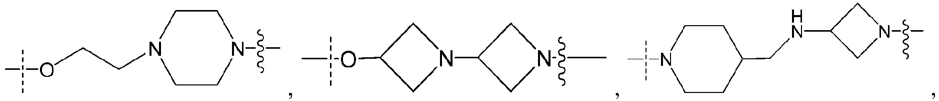 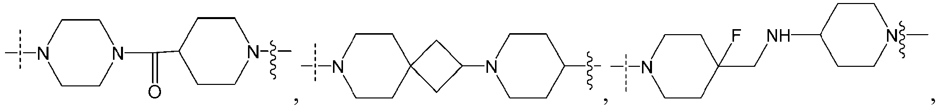 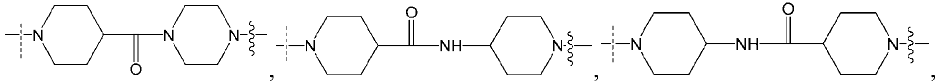 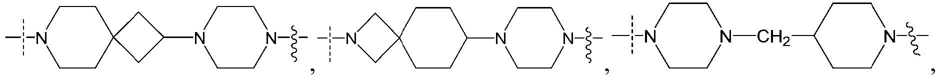 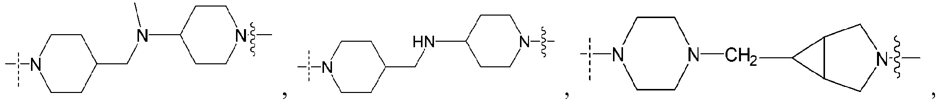 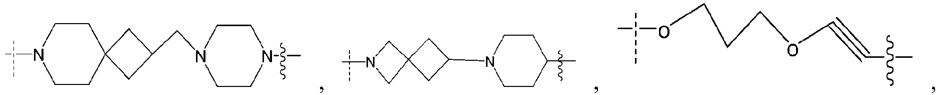 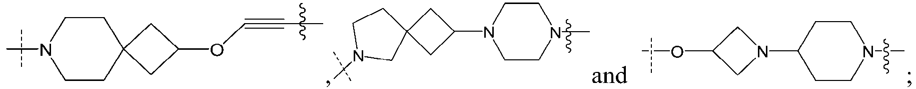
E3L represents a ubiquitin ligase ligand.

Preferably, E3L is selected from:

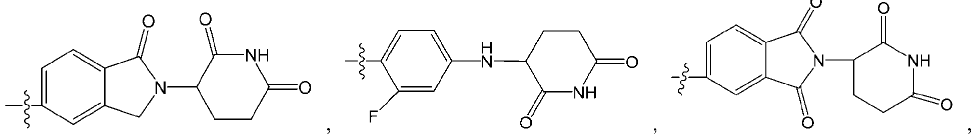

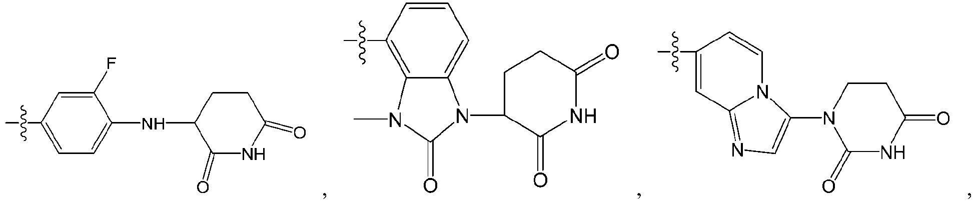

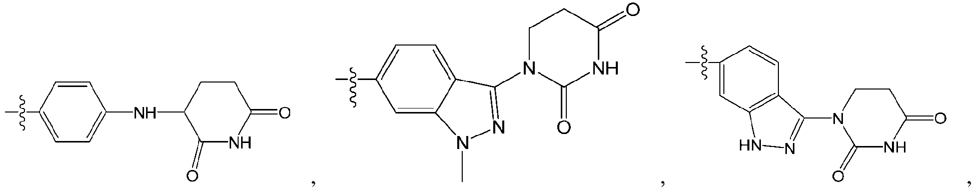

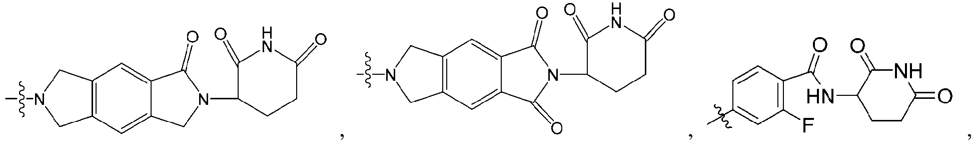

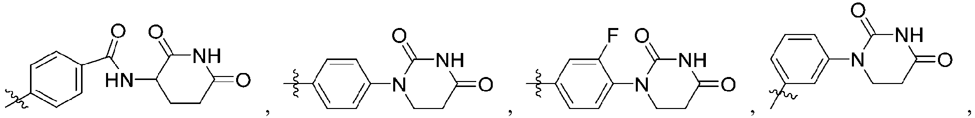

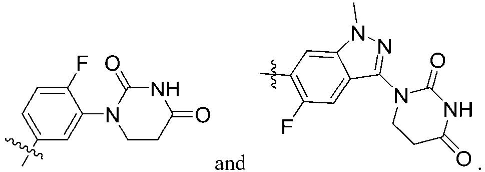

Preferably, the compound of the application is represented by formula (II): 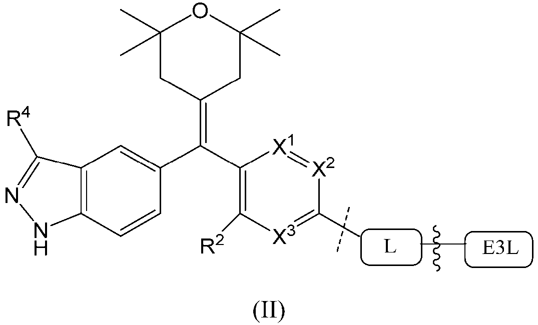 wherein R⁴ is selected from halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl.

Preferably, R⁴ is halogen.

Preferably, the compound of the application is represented by formula (III):

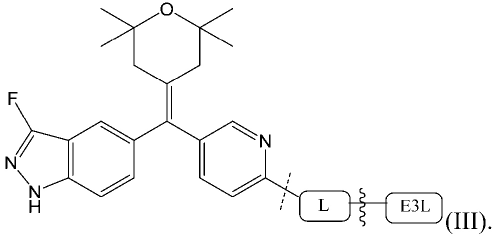

Preferably, the compound of the application is represented by formula (IV):

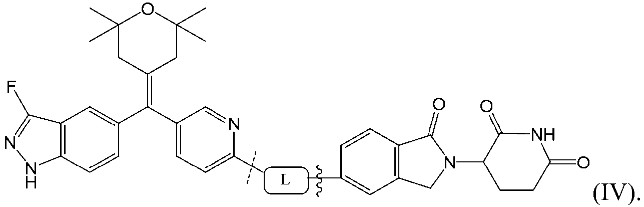

Preferably, L is any one selected from the group consisting of: 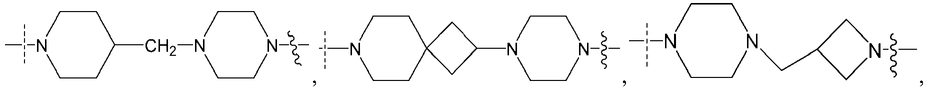 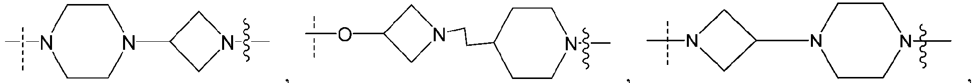 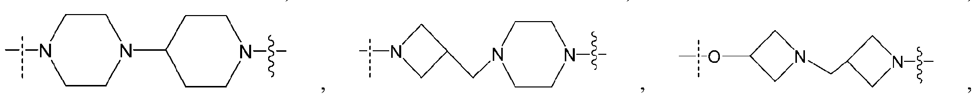 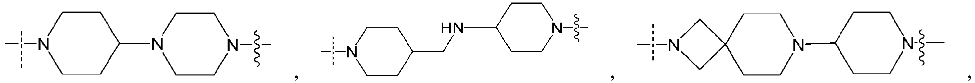 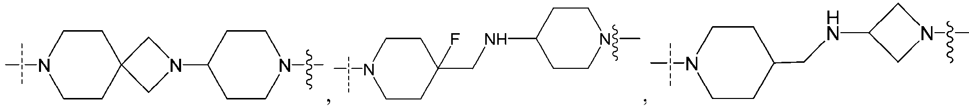 and

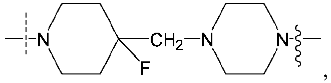

Preferably, X⁴ is N.

Preferably, X⁴ is CRa, and Ra is selected from H,halogen and hydroxyl.

Preferably, Ra is H.

Preferably, X¹ is CR³, and R³ is H,CH₃O or F.

Preferably, R³ is H.

Preferably, X² is CH

Preferably, X² is N.

Preferably, X³ is CH.

Preferably, R² is H.

Preferably, E3L is

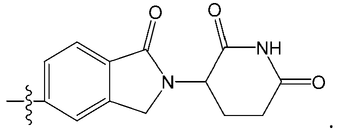

Preferably, the compounds of present application is any one selected from the group consisting of:

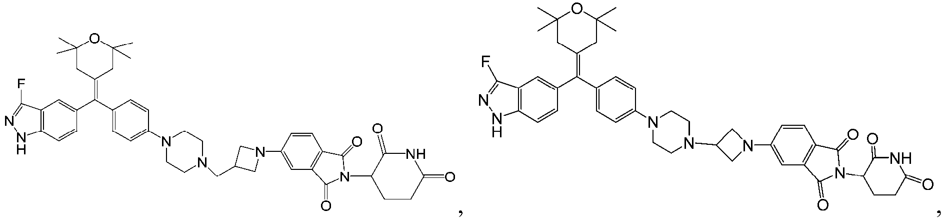

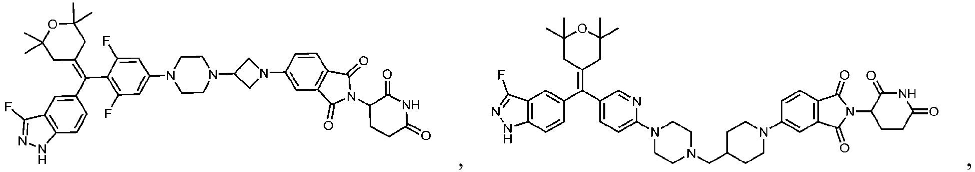

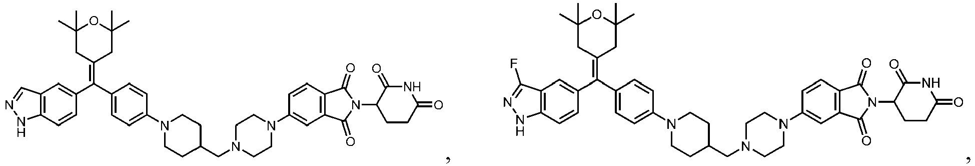

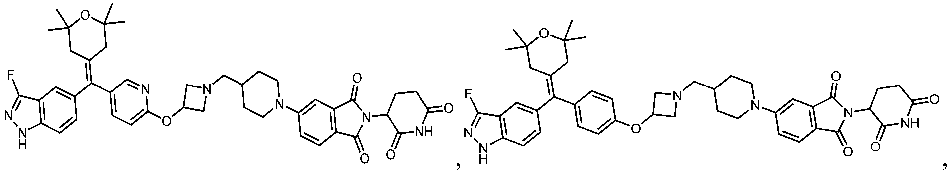

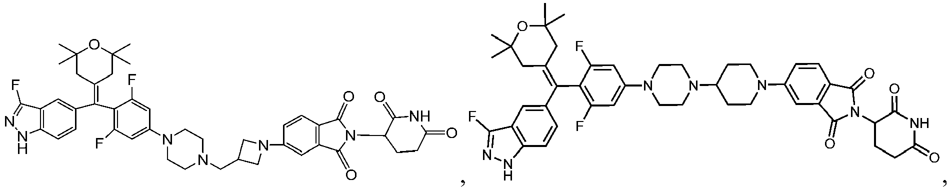

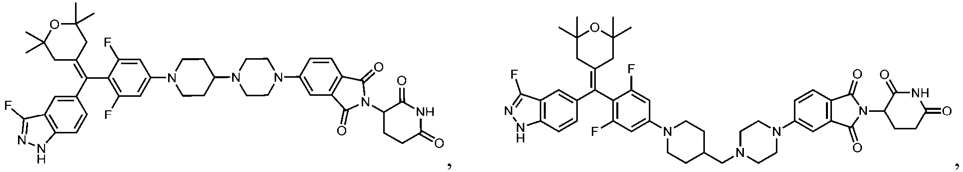

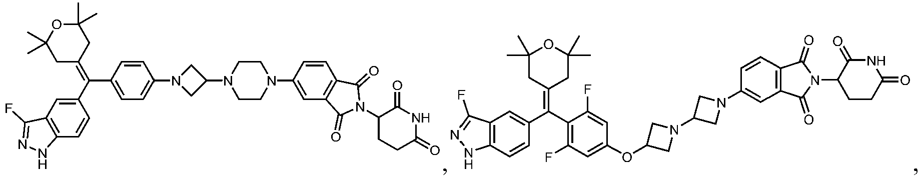

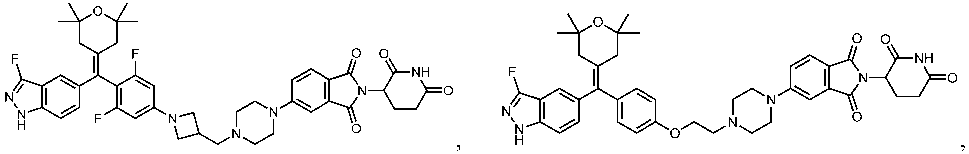

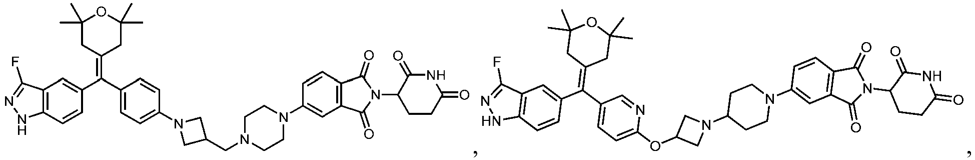

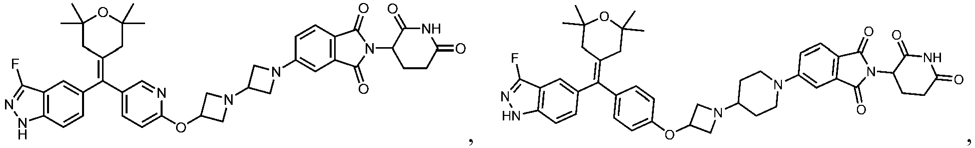

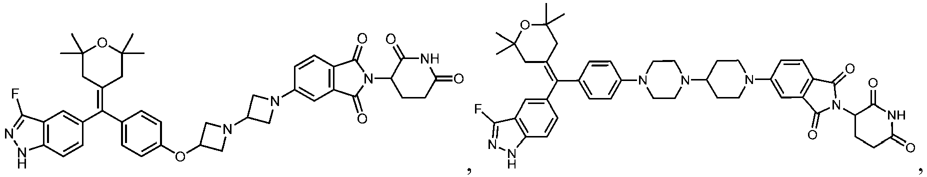

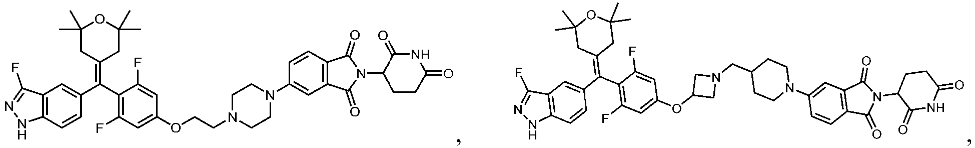

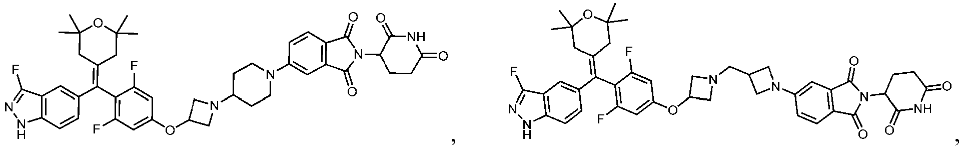

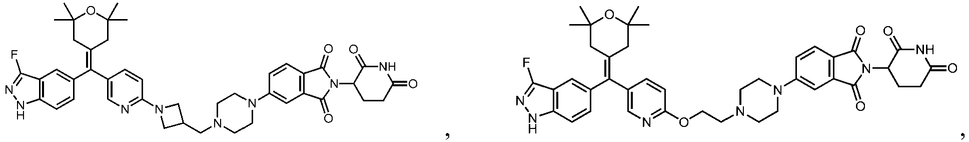

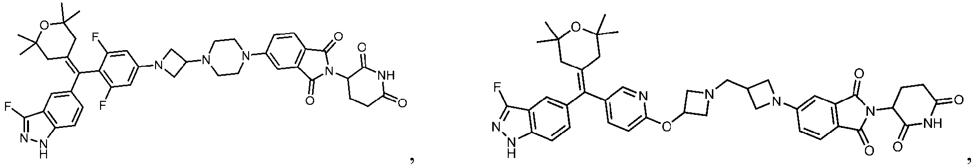

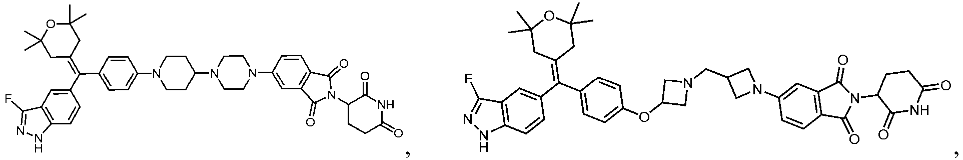

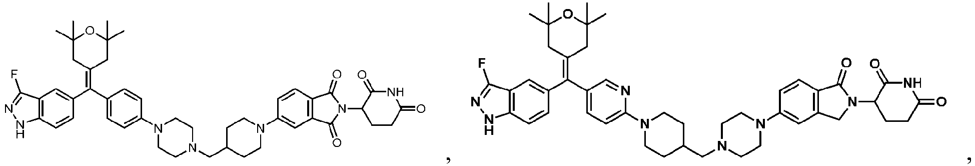

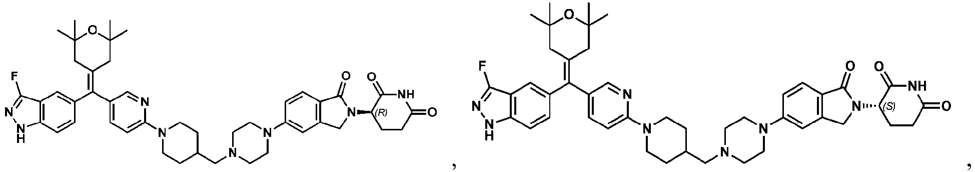

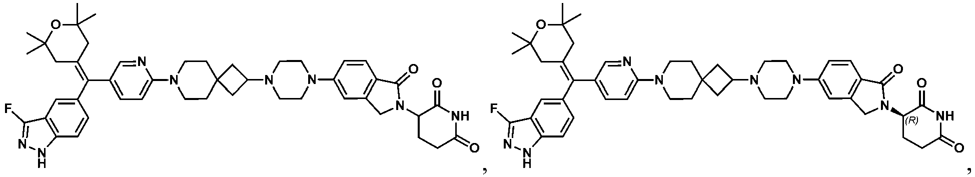

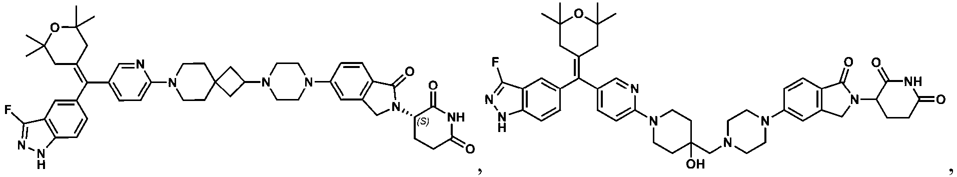

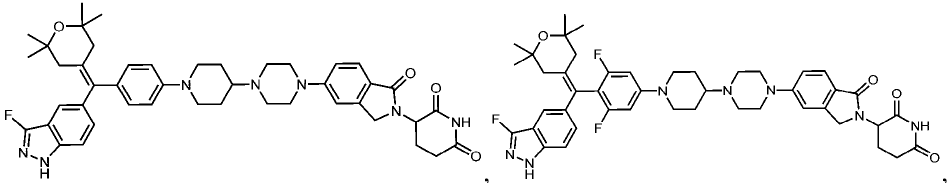

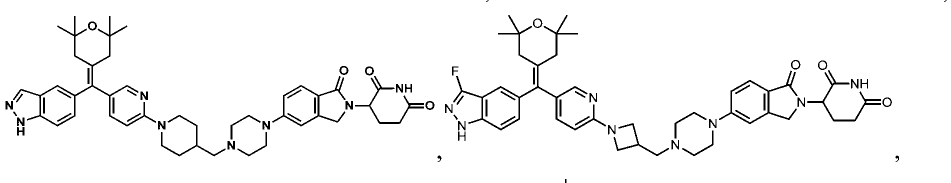

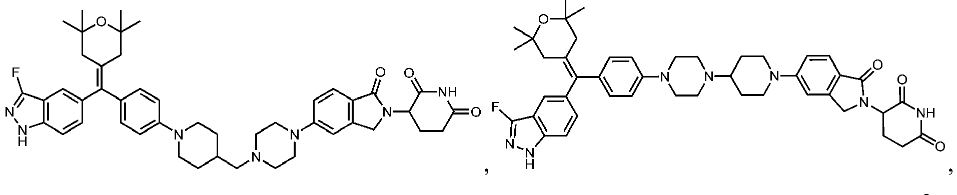

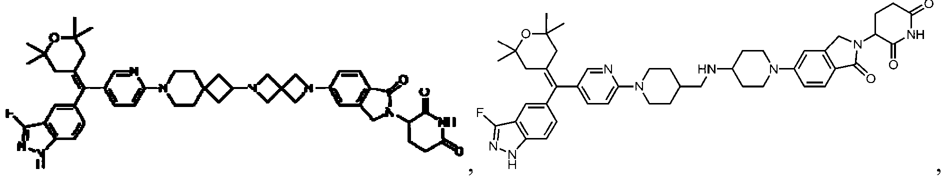

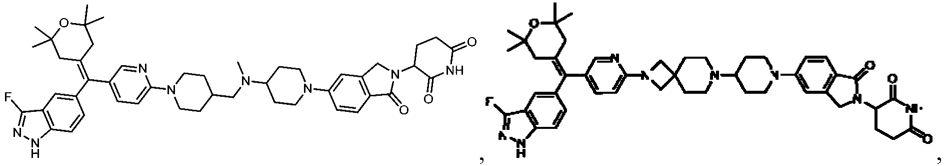

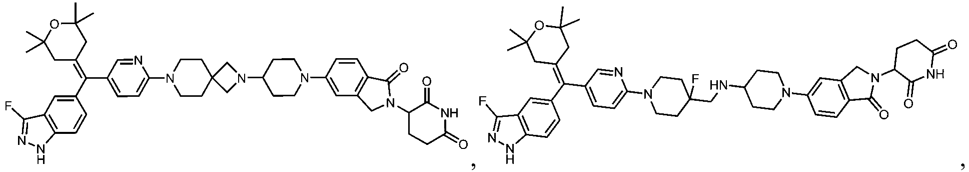

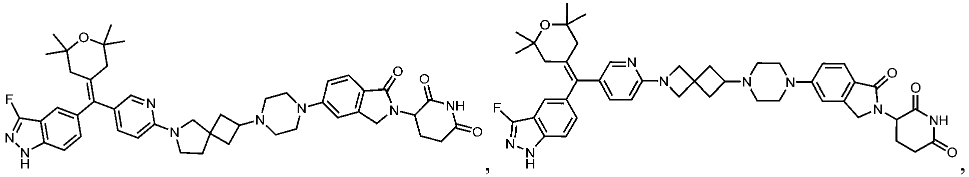

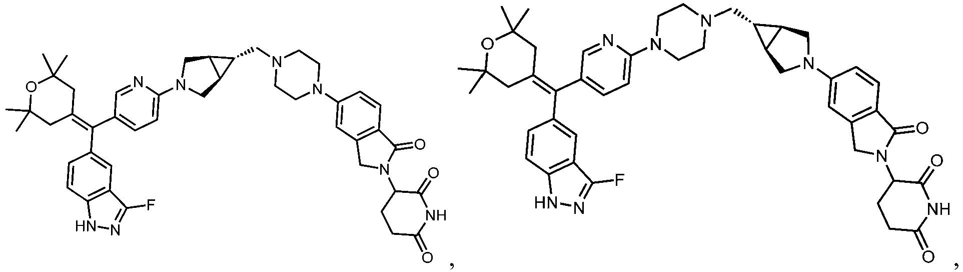

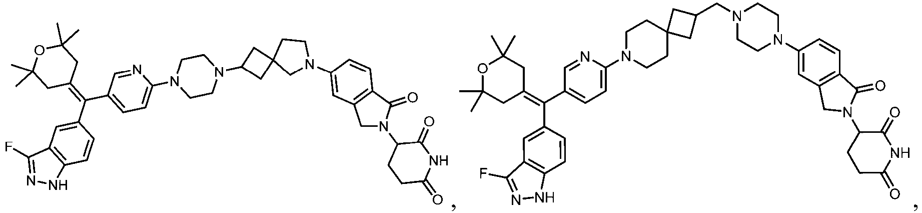

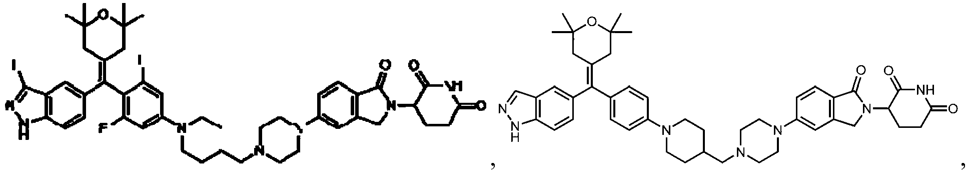

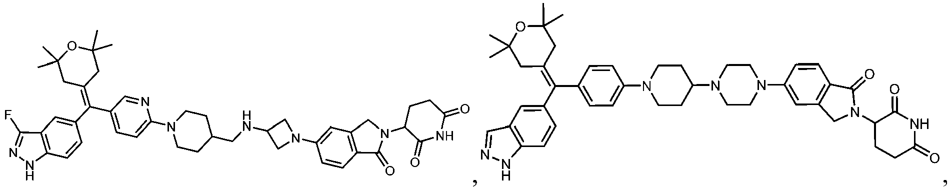

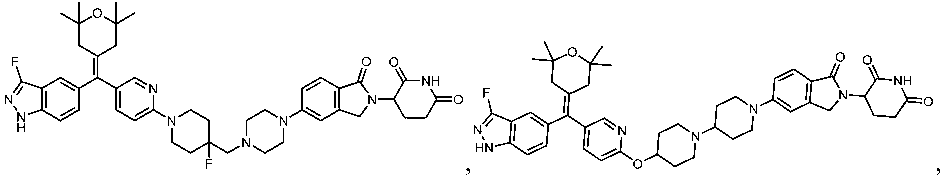

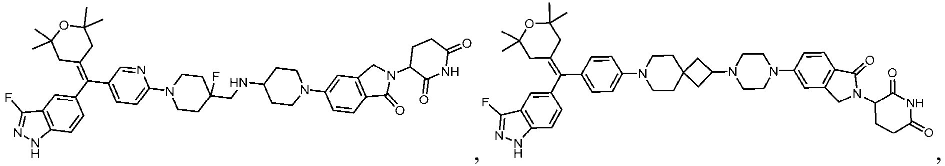

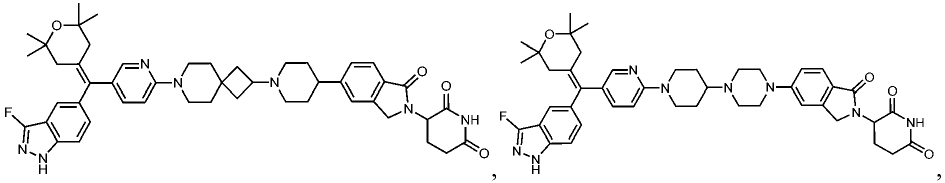

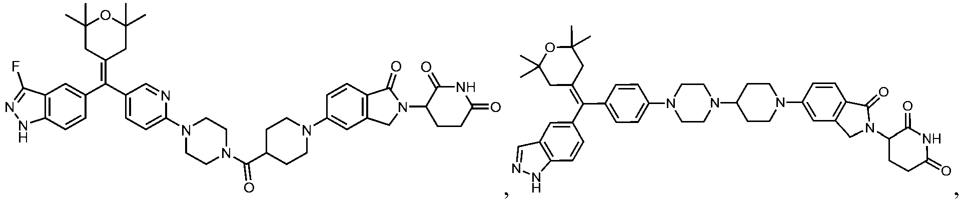

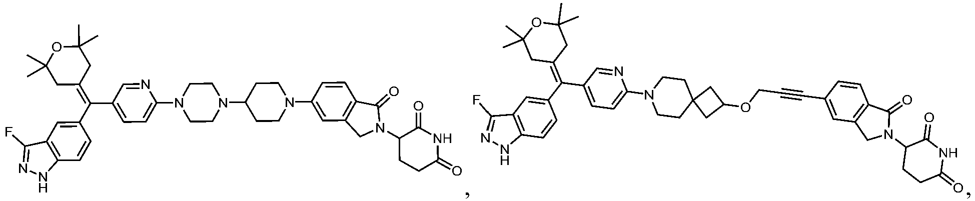

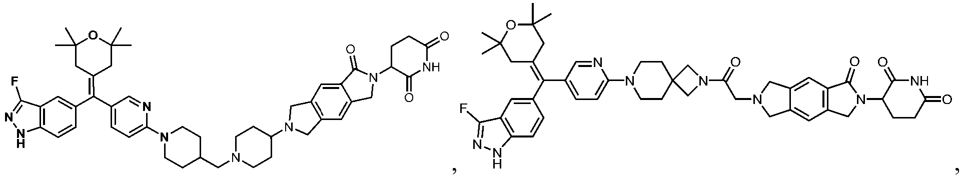

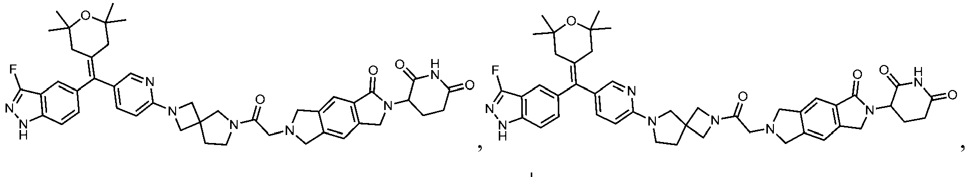

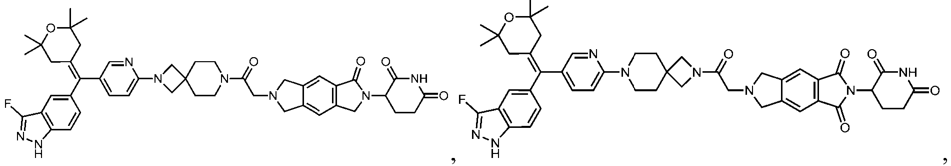

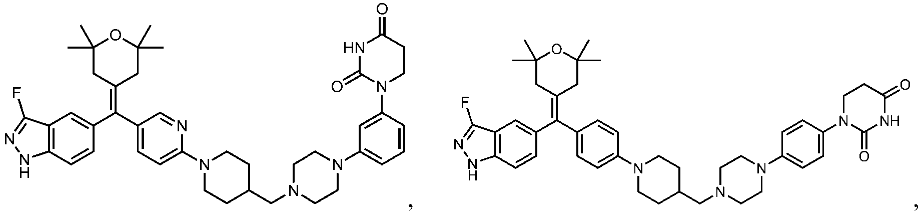

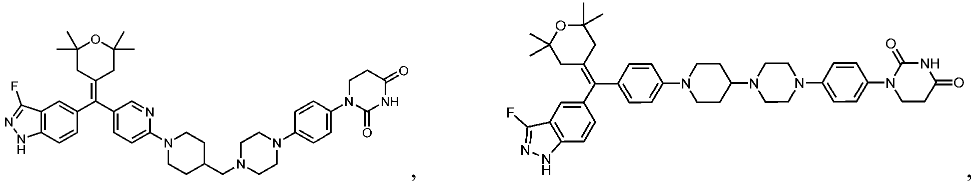

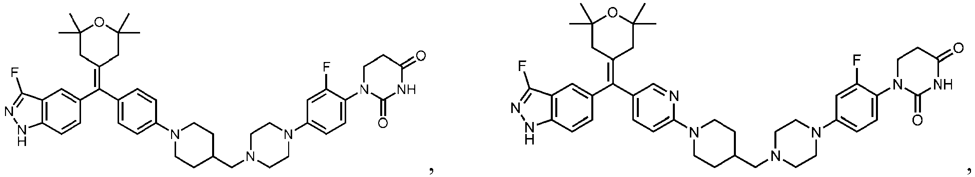

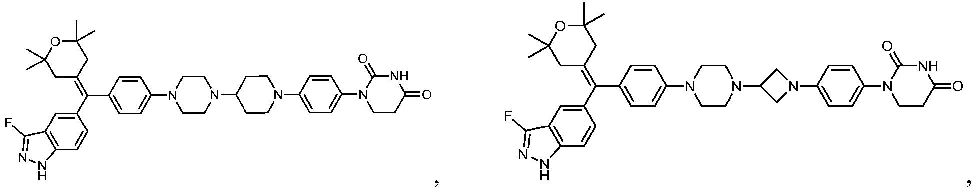

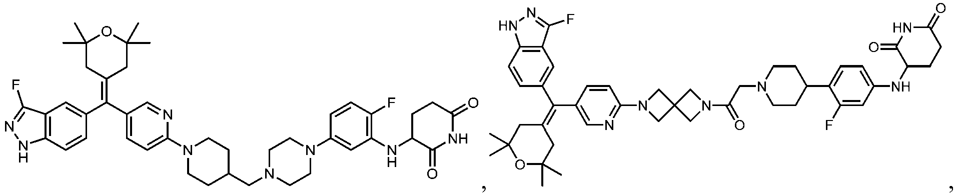

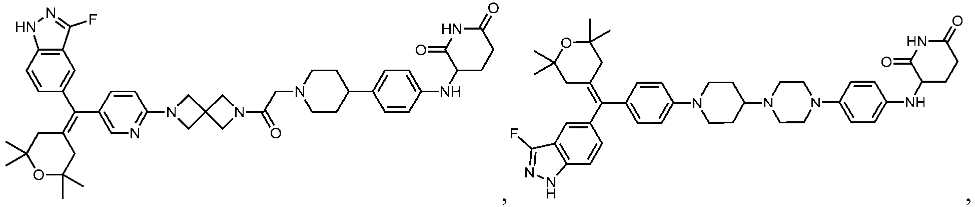

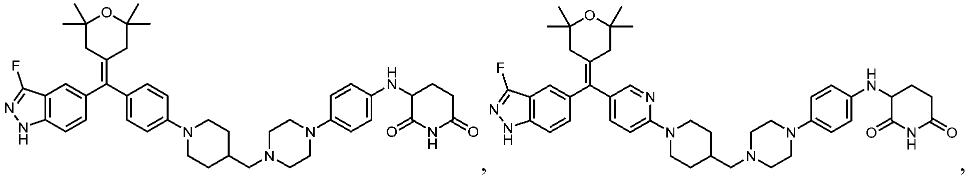

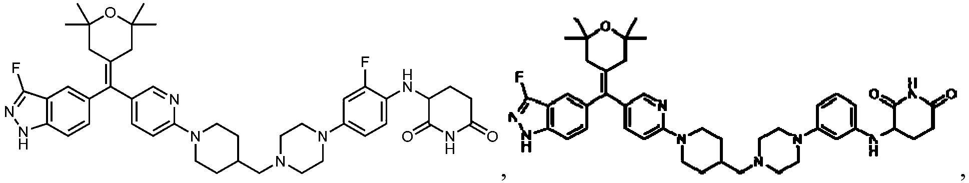

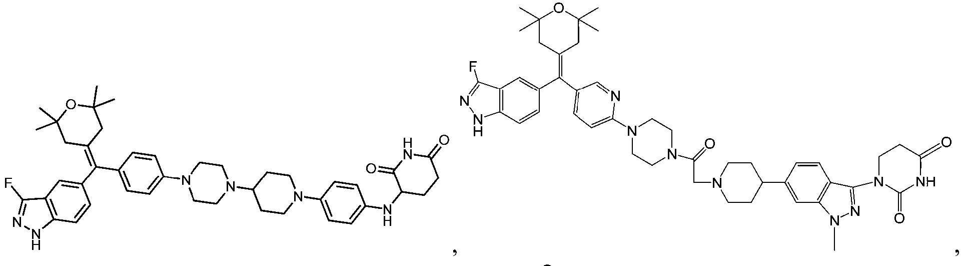

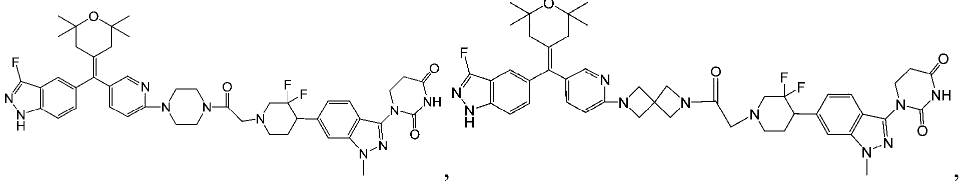

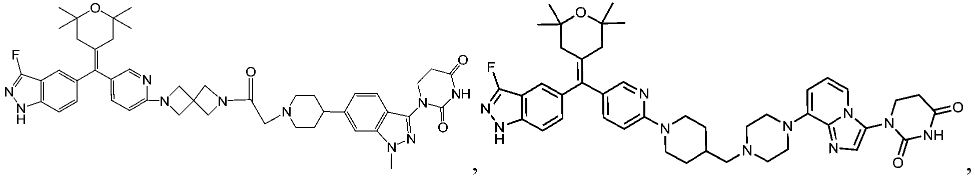

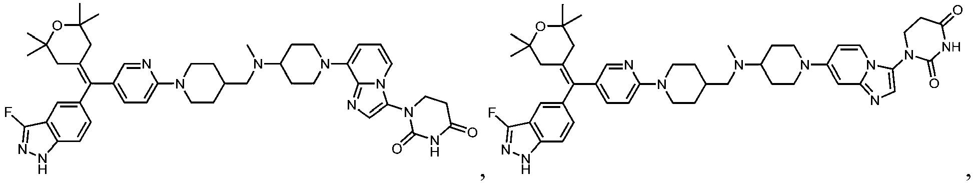

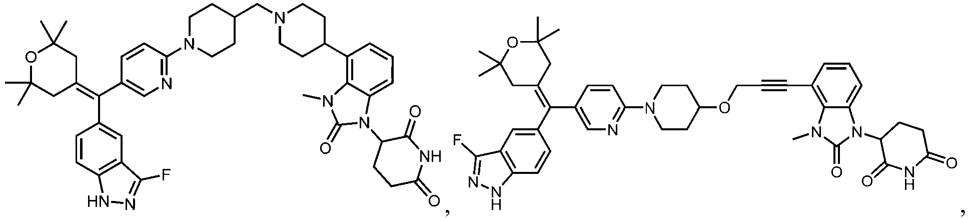

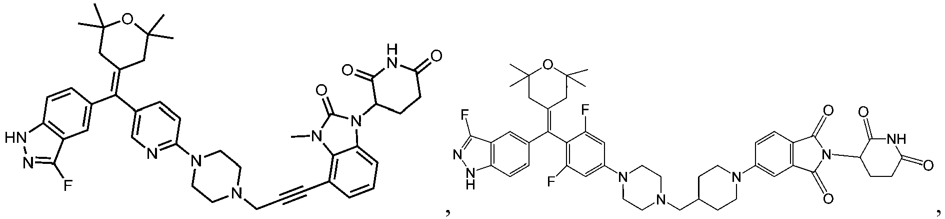

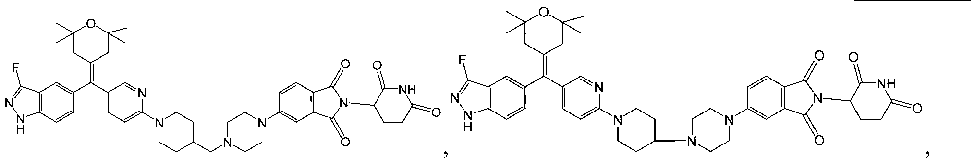

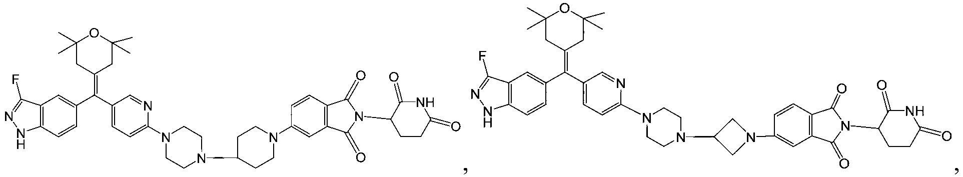

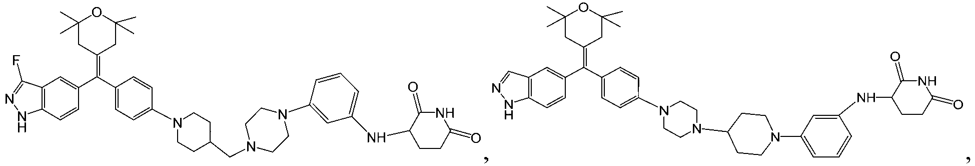

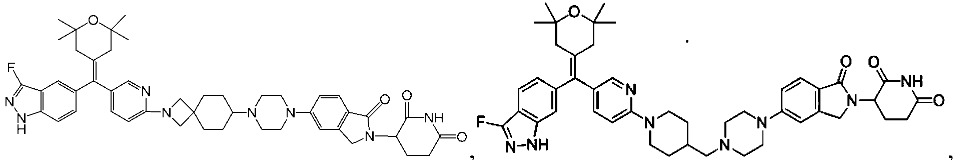

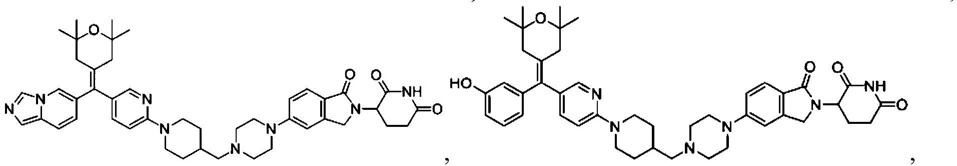

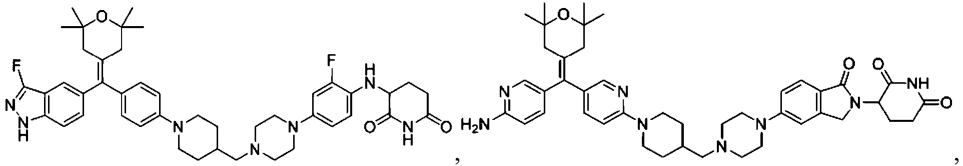

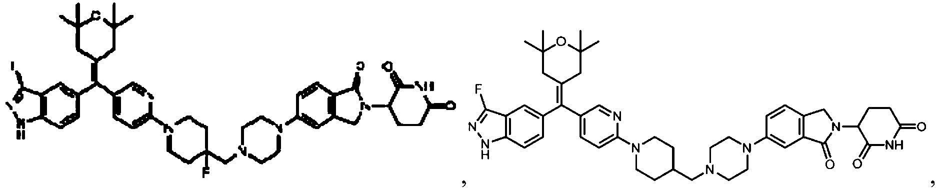

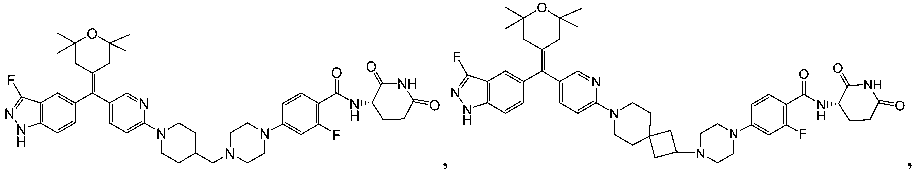

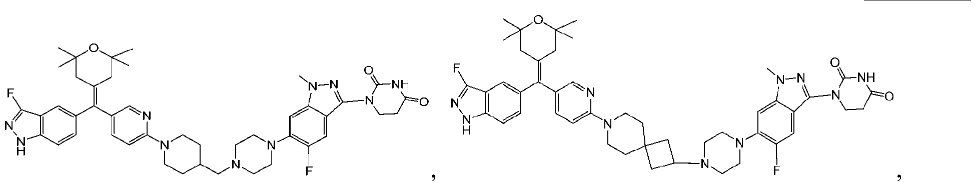

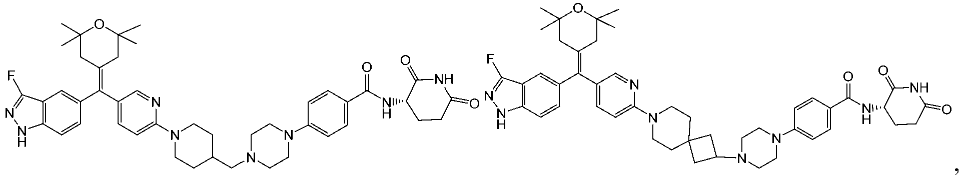

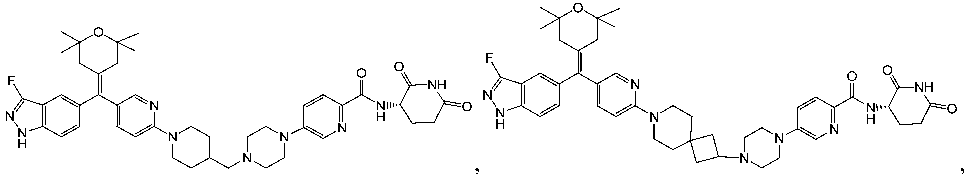

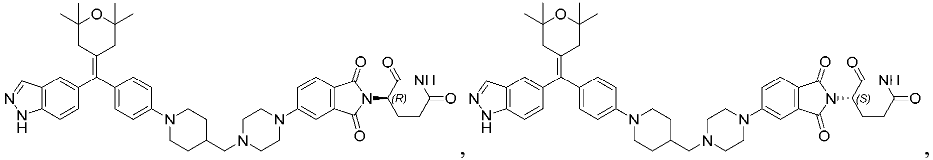

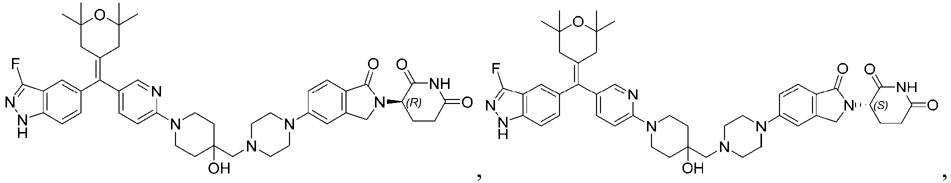

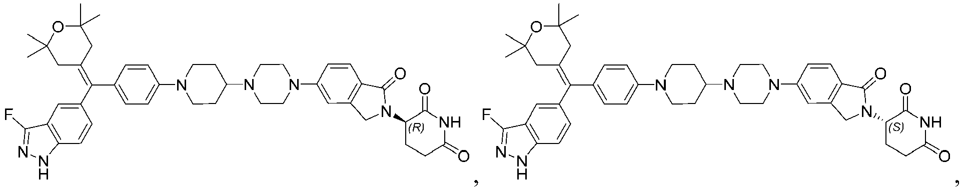

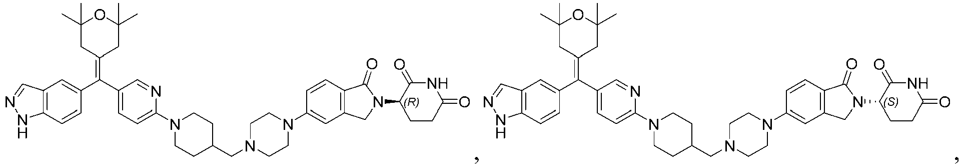

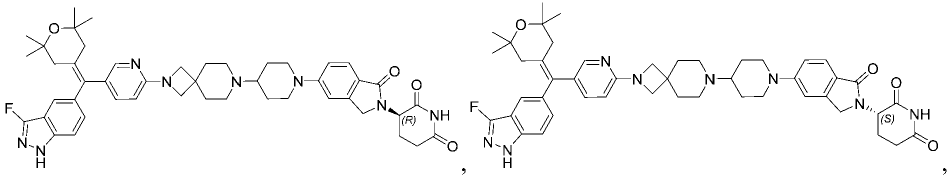

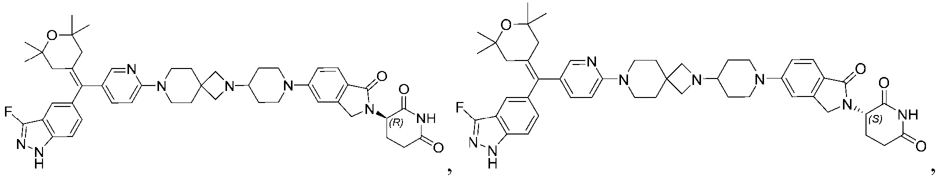

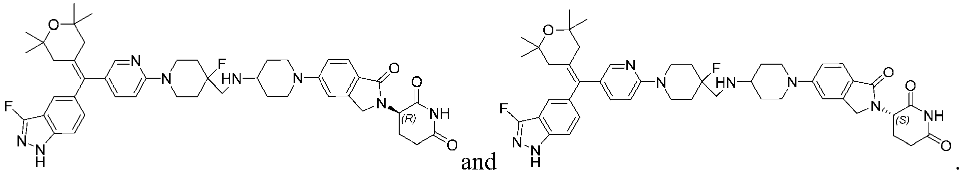

The present application also relates to a pharmaceutical composition comprising above compounds, pharmaceutically acceptable salts, solvates, hydrates, or isomers thereof, and pharmaceutically acceptable carriers.

The present application also relates to the use of compounds of the present application or their pharmaceutically acceptable salts, solvates, hydrates, or isomers, or the pharmaceutical compositions of the present application in the treatment of diseases treated by degrading a target protein that binds to a targeting ligand. Preferably, the disease is selected from abnormal cell proliferation, cancer, immune disease, diabetes, cardiovascular disease, infectious disease and inflammatory disease.

Preferably, the cancer is selected from breast cancer, endometrial cancer, uterine cancer, testicular cancer, cervical cancer, prostate cancer, ovarian cancer, endometriosis, fallopian tube tumors, ovarian tumors, leukemia, skin cancer, squamous cell carcinoma, basal cell carcinoma, adenocarcinoma, renal cell carcinoma, bladder cancer, intestinal cancer, colon cancer, esophageal cancer, head cancer, kidney cancer, liver cancer, lung cancer, neck cancer, pancreatic cancer, stomach cancer, lymphoma, non Hodgkin's lymphoma, melanoma, myeloproliferative disorders, sarcoma, angiosarcoma, peripheral neuroepithelioma, glioma, astrocytoma, oligodendroglioma, ependymoma, glioblastoma, neuroblastoma, gangliocytoma ganglioglioma, medulloblastoma, pineocytoma, meningioma, meningeal sarcoma, neurofibroma, schwannoma, thyroid carcinoma, Hodgkin's lymphoma, Wilms tumor, and teratoma. More preferably, it is selected from breast cancer, endometrial cancer, uterine cancer, testicular cancer, cervical cancer, prostate cancer, ovarian cancer, fallopian tube tumor and ovarian tumor.

Preferably, the cancer is selected from breast cancer, endometrial cancer, ovarian cancer, cervical cancer, prostate cancer, or endometriosis.

Preferably, the infectious disease is selected from viral pneumonia, avian influenza, meningitis, gonorrhea, or diseases caused by infections with HIV, HBV, HCV, HSV, HPV, RSV, CMV, Ebola virus, flavivirus, rhabdovirus, rotavirus, influenza, coronavirus EBV, drug-resistant viruses, RNA viruses, DNA viruses, adenoviruses, poxviruses, small ribonucleic acid viruses, Togaviridae, orthomyxoviruses, retroviruses, Hepadnavirus, Gram negative bacteria, Gram positive bacteria, atypical bacteria, Staphylococcus, Streptococcus, Escherichia coli, Salmonella, Helicobacter pylori, Chlamydiaceae, Mycoplasmataceae, fungi, protozoa, helminths, worms, prions or parasites.

The present application also relates to the use of the compounds of the present application, or a pharmaceutically acceptable salt, solvate, hydrate, or isomer thereof, or the pharmaceutical compositions of the present application, in the manufacture of a medicament for treating a disease treated by degrading a target protein that binds to a targeting ligand.

Preferably, the disease is selected from abnormal cell proliferation, cancer, immune disease, diabetes, cardiovascular disease, infectious disease and inflammatory disease.

Preferably, the cancer is selected from breast cancer, endometrial cancer, uterine cancer, testicular cancer, cervical cancer, prostate cancer, ovarian cancer, endometriosis, fallopian tube tumors, ovarian tumors, leukemia, skin cancer, squamous cell carcinoma, basal cell carcinoma, adenocarcinoma, renal cell carcinoma, bladder cancer, intestinal cancer, colon cancer, esophageal cancer, head cancer, kidney cancer, liver cancer, lung cancer, neck cancer, pancreatic cancer, stomach cancer, lymphoma, non Hodgkin's lymphoma, melanoma, myeloproliferative disorders, sarcoma, angiosarcoma, peripheral neuroepithelioma, glioma, astrocytoma, oligodendroglioma, ependymoma, glioblastoma, neuroblastoma, gangliocytoma ganglioglioma, medulloblastoma, pineocytoma, meningioma, meningeal sarcoma, neurofibroma, schwannoma, thyroid carcinoma, Hodgkin's lymphoma, Wilms tumor, and teratoma. More preferably, it is selected from breast cancer, endometrial cancer, uterine cancer, testicular cancer, cervical cancer, prostate cancer, ovarian cancer, fallopian tube tumor and ovarian tumor.

Preferably, the cancer is selected from breast cancer, endometrial cancer, ovarian cancer, cervical cancer, prostate cancer, or endometriosis.

Preferably, the infectious disease is selected from viral pneumonia, avian influenza, meningitis, gonorrhea, or diseases caused by infections with HIV, HBV, HCV, HSV, HPV, RSV, CMV, Ebola virus, flavivirus, rhabdovirus, rotavirus, influenza, coronavirus EBV, drug-resistant viruses, RNA viruses, DNA viruses, adenoviruses, poxviruses, small ribonucleic acid viruses, Togaviridae, orthomyxoviruses, retroviruses, Hepadnavirus, Gram negative bacteria, Gram positive bacteria, atypical bacteria, Staphylococcus, Streptococcus, Escherichia coli, Salmonella, Helicobacter pylori, Chlamydiaceae, Mycoplasmataceae, fungi, protozoa, helminths, worms, prions or parasites. The present application also relates to a method for treating a disease in a subject by degrading a target protein that binds to a targeting ligand, comprising administering to the subject an effective amount of compounds of the present application, or a pharmaceutically acceptable salt, solvate, hydrate, or isomer thereof, or the pharmaceutical compositions of the present application.Preferably, the disease is selected from abnormal cell proliferation, cancer, immune disease, diabetes, cardiovascular disease, infectious disease and inflammatory disease.

Preferably, the cancer is selected from breast cancer, endometrial cancer, uterine cancer, testicular cancer, cervical cancer, prostate cancer, ovarian cancer, endometriosis, fallopian tube tumors, ovarian tumors, leukemia, skin cancer, squamous cell carcinoma, basal cell carcinoma, adenocarcinoma, renal cell carcinoma, bladder cancer, intestinal cancer, colon cancer, esophageal cancer, head cancer, kidney cancer, liver cancer, lung cancer, neck cancer, pancreatic cancer, stomach cancer, lymphoma, non Hodgkin's lymphoma, melanoma, myeloproliferative disorders, sarcoma, angiosarcoma, peripheral neuroepithelioma, glioma, astrocytoma, oligodendroglioma, ependymoma, glioblastoma, neuroblastoma, gangliocytoma ganglioglioma, medulloblastoma, pineocytoma, meningioma, meningeal sarcoma, neurofibroma, schwannoma, thyroid carcinoma, Hodgkin's lymphoma, Wilms tumor, and teratoma. More preferably, it is selected from breast cancer, endometrial cancer, uterine cancer, testicular cancer, cervical cancer, prostate cancer, ovarian cancer, fallopian tube tumor and ovarian tumor.

Preferably, the cancer is selected from breast cancer, endometrial cancer, ovarian cancer, cervical cancer, prostate cancer, or endometriosis.

Preferably, the infectious disease is selected from viral pneumonia, avian influenza, meningitis, gonorrhea, or diseases caused by infections with HIV, HBV, HCV, HSV, HPV, RSV, CMV, Ebola virus, flavivirus, rhabdovirus, rotavirus, influenza, coronavirus EBV, drug-resistant viruses, RNA viruses, DNA viruses, adenoviruses, poxviruses, small ribonucleic acid viruses, Togaviridae, orthomyxoviruses, retroviruses, Hepadnavirus, Gram negative bacteria, Gram positive bacteria, atypical bacteria, Staphylococcus, Streptococcus, Escherichia coli, Salmonella, Helicobacter pylori, Chlamydiaceae, Mycoplasmataceae, fungi, protozoa, helminths, worms, prions or parasites.

### BRIEF DESCRIPTION OF THE FIGURES

Figure 1A shows the degradation of estrogen receptors in MCF-7 cells determined by Western blotting after treatment of MCF-7 cells with the exemplary compounds 1, 2, 5, 6, 7, and 13 disclosed herein for 6 hours.
Figure 1B shows the degradation of estrogen receptors in MCF-7 cells determined by Western blotting after treatment of MCF-7 cells with the exemplary compounds 27, 30, 31, 34, 35, and 37 disclosed herein for 6 hours.
Figure 1C shows the degradation of estrogen receptors in MCF-7 cells determined by Western blotting after treatment of MCF-7 cells with the exemplary compounds 44, 46, 47, 48, 57, and 59 disclosed herein for 6 hours.
Figure 1D shows the degradation of estrogen receptors in MCF-7 cells determined by Western blotting after treatment of MCF-7 cells with the exemplary compounds 61, 66, 76, 88, 89, and 90 disclosed herein for 6 hours.
Figure 1E shows the degradation of estrogen receptors in MCF-7 cells determined by Western blotting after treatment of MCF-7 cells with the exemplary compounds 103, 104, 110, 119, 120 and 121 disclosed herein for 6 hours.
Figure 2A shows the degradation of estrogen receptors in MCF-7 cells determined by Western blotting after treatment of MCF-7 cells with 10nM exemplary compound 5 disclosed herein for different time periods.
Figure 2B shows the degradation of estrogen receptors in MCF-7 cells determined by Western blotting after treatment of MCF-7 cells with 10nM exemplary compound 31 disclosed herein for different time periods.
Figure 2C shows the degradation of estrogen receptors in MCF-7 cells determined by Western blotting after treatment of MCF-7 cells with 10nM exemplary compound 47 disclosed herein for different time periods.
Figure 2D shows the degradation of estrogen receptors in MCF-7 cells determined by Western blotting after treatment of MCF-7 cells with 10nM exemplary compound 48 disclosed herein for different time periods.
Figure 3A shows the inhibitory effects on MCF-7 cell proliferation after 6 days of treatment with exemplary compounds 2, 5, 6, 22, 31, and 34 disclosed herein.
Figure 3B shows the inhibitory effects on MCF-7 cell proliferation after 6 days of treatment with exemplary compounds 35, 37, 44, 46, 47, and 48 disclosed herein.
Figure 3C shows the inhibitory effects on MCF-7 cell proliferation after 6 days of treatment with exemplary compounds 88, 110, 119, 120, and 121 disclosed herein.
Figure 4A shows the degradation of estrogen receptors in T-47D cells determined by Western blotting after treatment of T-47D cells with the exemplary compound 34 disclosed herein for 4 hours.
Figure 4B shows the degradation of estrogen receptors in T-47D cells determined by Western blotting after treatment of T-47D cells with the exemplary compound 35 disclosed herein for 4 hours.
Figure 5A shows the degradation of estrogen receptors in MCF-7 cells expressing ERα Y537S mutant determined by Western blotting after treatment of MCF-7/ERα Y537S cells with the exemplary compound 34 disclosed herein for 4 hours.
Figure 5B shows the degradation of estrogen receptors in MCF-7 cells expressing ERα Y537S mutant determined by Western blotting after treatment of MCF-7/ERα Y537S cells with the exemplary compound 35 disclosed herein for 4 hours.
Figure 5C shows the degradation of estrogen receptors in MCF-7 cells expressing ERα Y537S mutant determined by Western blotting after treatment of MCF-7/ERα Y537S cells with the exemplary compound 110 disclosed herein for 6 hours.
Figure 5D shows the degradation of estrogen receptors in MCF-7 cells expressing ERα Y537S mutant determined by Western blotting after treatment of MCF-7/ERα Y537S cells with the exemplary compound 120 disclosed herein for 6 hours.
Figure 6A shows the degradation of estrogen receptors in MCF-7 cells expressing ERα D538G mutant determined by Western blotting after treatment of MCF-7/ERα D538G cells with the exemplary compound 110 disclosed herein for 6 hours examined by Western blotting.
Figure 6B shows the degradation of estrogen receptors in MCF-7 cells expressing ERα Y537G mutant determined by Western blotting after treatment of MCF-7/ERα D538G cells with the exemplary compound 120 disclosed herein for 6 hours.
Figure 7A shows the inhibitory effects on MCF-7/ERα Y537S cell proliferation after 6 days of treatment with the exemplary compound 110 disclosed herein.
Figure 7B shows the inhibitory effects on MCF-7/ERα Y537S cell proliferation after 6 days of treatment with the exemplary compound 120 disclosed herein.
Figure 8A shows the dose-dependent growth inhibition ability of exemplary compound 110 disclosed herein in MCF-7/ER α Y537S xenografts.
Figure 8B shows that the exemplary compound 110 disclosed herein has no effect on mouse body weight in MCF-7/ER α Y537S xenografts.
Figure 9A shows the dose-dependent growth inhibition ability of exemplary compound 120 disclosed herein in MCF-7/ER α Y537S xenografts.
Figure 9B that shows the exemplary compound 120 disclosed herein has no effect on mouse body weight in MCF-7/ER α Y537S xenografts.

### DETAILED DESCRIPTION

### DEFINITION

Unless otherwise specified, the definition of certain terms used in the specification and the claims herein are as follows:
The term 'isomer' as used herein encompasses any and all geometric isomers and stereoisomers. For example, "isomers" include cis and trans isomers, E and Z isomers, R and S enantiomers, diastereomers, D-isomers, L-isomers, racemic mixtures as mentioned above, and other mixtures mentioned above. In one embodiment, for example, stereoisomers can be provided in a form that is essentially free of one or more corresponding enantiomers. This forms can also be referred to as "stereochemically enriched".

The term "pharmaceutically acceptable salts" as used herein refers to salts that retain the bioavailability of the specified compound's free acid or free base form, and are not biologically unfriendly deleterious. The anticipated pharmaceutically acceptable salt forms include, but are not limited to monosalts, bisalts, trisalts, tetrasalts, etc. Pharmaceutically acceptable salts are non-toxic at the applied amount and concentrations. Preparing these salts can be beneficial for pharmacological purposes. This is because it can modify the physical properties of the compounds without impeding their physiological effects. Useful physical property changes include reducing the melting point to ease transdermal administration and enhancing solubility to enable the administration of higher drug concentrations. It should also be noted that the compounds of the present application can exist in non-solvate form, solvate forms (such as hydrated form), and solid forms (such as crystalline or polymorphic forms). The present application is intended to encompass all such forms.

The terms "solvate," "solvated," or "solvated form" as used herein refer to solvent addition forms that contain either stoichiometric or non-stoichiometric amounts of solvent. Some compounds have a tendency to incorporate solvent molecules in a fixed molar ratio within crystalline solids, thereby forming solvates. When the solvent is water, the resulting solvate is a hydrate; when the solvent is an alcohol, the formed solvate is an alcoholate. Examples of solvents that can form solvates include but are not limited to water, isopropanol, methanol, ethanol DMSO, ethyl acetate, acetic acid, ethanolamine, acetone, ether, etc.

The term "alkyl" as used herein can either be branched or unbranched and preferably has 1 to 12 carbon atoms. A more preferred class of alkyl groups contain 1 to 6 carbon atoms. Even more preferably alkyl groups have 1, 2, 3, or 4 carbon atoms. Alkyl groups include, but are not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and so on. Alkyl groups can be either substituted or unsubstituted.

The term "substituted" means that to one or more hydrogen atoms in the group are replaced, preferably up to 5 hydrogen atoms, more preferably 1-3 hydrogen atoms are each independently substituted by a corresponding number of substituents. Substitutions are only present at their possible chemical positions, and those skilled in the art can determine whether a substitution is possible or not without undue effort (through experiments or theories). For example, when an amino or hydroxyl group with a free hydrogen combines with a carbon atom having an unsaturated (such as an alkenyl) bond, it may be unstable.

Unless otherwise indicated, the term "substituted" mentioned in this specification, means that the group can be substituted with one or more substituents selected from the following groups: alkyl, alkoxy, alkylthio, alkylamino, halogen, sulphydryl, hydroxy, nitro, cyano, cycloalkyl, heterocyclyl, aryl, heteroaryl, cycloalkoxy, heterocycloalkoxy, cycloalkylthio, heterocycloalkylthio, amino, haloalkyl, hydroxyalkyl; whenever a group is described as "optionally substituted by a substituent", the group can be either unsubstituted or substituted with one or more of the indicated substituents. Similarly, when a group is described as "unsubstituted or substituted", proved that it is substituted, the substituent(s) can be selected from one or more of the indicated substituents. If no substituent is specified, it shall be understood that the indicated "optionally substituted by a substituent" or "substituted" group can be substituted by one or more groups, each of which is individually or independently selected from the group consisting of alkyl, alkenyl, alkynyl, cycloalkyl, cycloalkenyl, cycloalkynyl, aryl, heteroaryl, heteroalicyclic, arylalkyl, heteroarylalkyl, (heteroalicyclic) alkyl, hydroxyl, protective hydroxyl, alkoxy, aryloxy, acyl, sulfhydryl, alkylthio, arylthio, cyano, halogen, thiocarbonyl, O-carbamoyl, N-carbamoyl, O-thiocarbamoyl, N-thiocarbamoyl, C-amido, N-amido, S-sulfonamido, N-sulfonamido, C-carboxyl, protective C-carboxyl, O-carboxyl, isocyanate, thiocyanate, isothiocyanate, nitro, silyl, thio, sulfenyl, sulfonyl, haloalkyl, haloalkoxy, trihalomethanesulfonyl, trihalomethanesulfonamido, amino, monosubstituted amino and disubstituted amino and protected derivatives thereof.

The term "halogen" used herein refers to F, Cl, Br or I.

The term "cyano" used herein refers to -CN.

The term "hydroxyl" used herein refers to -OH.

The term "amino" used herein refers to -NH₂.

The term "oxy" used herein refers to oxygen atom connecting to another atom (such as carbon or sulfur) by double bond. For example, carbonyl is formed when oxy atom is directly connecting to carbon atom.

The term "acyl" used herein refers to a functional group containing a carbonyl group, such as -C (=O) R', where R' is a hydrogen or hydrocarbon group. In one embodiment, the acyl group is a group represented by the formula alkyl C (O).

The term "alkoxy" used herein, whether used as part of another term or independently, refers to an alkyl group as defined above connected to the parent molecule by oxygen atom.

The term "benzyl" used herein refers to -CH₂- phenyl.

The term "pharmaceutical composition" used herein refers to a mixture containing one or more compounds described herein, or their physiologically acceptable salts or prodrugs, together with other chemical components, as well as other components such as physiologically acceptable carriers and excipients. The purpose of the pharmaceutical composition is to facilitate administration to organisms, promote absorption of the active ingredients and thereby further exert biological activity

The term "pharmaceutically acceptable" used herein refers to compounds, materials, compositions and / or dosage forms that are suitable for contact with human or animal tissues without causing excessive toxicity, irritation, allergic reaction, other problems or complications, and exhibit a reasonable benefit/risk ratio. In one embodiment, pharmaceutically acceptable compounds, materials, compositions, and / or dosage forms refer to those approved by regulatory agencies (such as the U.S. Food and Drug Administration, the National Drug Medical Products Administration of China, and the European Medicines Agency) or listed in recognized Pharmacopoeia (such as the United States Pharmacopoeia, the Chinese Pharmacopoeia, and the European Pharmacopoeia) for use in animals, especially humans.

The term "pharmaceutically acceptable carrier" used herein refers to the pharmaceutically acceptable material, compositions or vehicles involved in carrying or transporting the compounds of the present application or their pharmaceutically acceptable salts from one location, body fluid, tissue, organ (internal or external) or body part to another location, body fluid, tissue, organ or body part, such as liquid or solid fillers, diluents, excipients, solvents or encapsulation materials. Pharmaceutically acceptable carriers can be vehicles, diluents, excipients or other materials that can be used to contact animal tissues without excessive toxicity or adverse reactions. Exemplary pharmaceutically acceptable carriers include but are not limited to sugars, starchs, cellulose, malt, gum tragacanth, gelatin, Ringer's solution, alginic acid, isotonic saline, buffer, etc. Pharmaceutically acceptable carriers suitable for use in the present application include those known in the art, such as those disclosed in "Remington Pharmaceutical Sciences" Mack pub. Co., New Jersey (1991).

The term "subject" used herein refers to an organism, tissue or cell. Subjects may include human subjects for medical purposes (e.g., diagnosis and / or treatment of existing conditions or diseases, or prophylactic treatment to prevent the onset of conditions or diseases), or animal subjects for veterinary purposes or development purposes. Subjects also include sample materials from tissue cultures, cell cultures, organ replication, stem cell production, etc. Suitable animal subjects include mammals and birds. The term "mammal" used herein includes, but is not limited to, primates (such as humans, monkeys, apes, etc.), bovines (such as bulls, etc.), sheep (such as sheep, goats, etc.), swine, horses, cats, dogs, rabbits, rodents (such as mice, rats, etc.). The term "birds" used herein includes, but not limited to, chickens, ducks, goose, quails, turkeys, pheasants, etc. In one embodiment, the subjects are mammals or mammalian cells. In one embodiment, the subjects are humans or human cells. Human subjects include, but are not limited to, fetus, neonatus, infants, adolescents and adult subjects. In addition, "subjects" may include patients with or suspected of having a certain disorder or disease. Therefore, the terms "subject" and "patient" are interchangeable herein. The subject can also refer to the cells in a laboratory or the bioprocessing medium in a test.

The term "effective does" used herein refers to the amount of a drug or pharmaceutical reagent that will elicit a biological or medical response in tissues, systems, animals or humans which is sought by researchers and clinicians. Furthermore, the term "therapeutically effective does" means such an does that, compared to subjects not receiving that does, results in the treatment, cure, prevention, or alleviation of a disease, disorder, or side effect, or a reduction in the rate of progression of the disease or disorder. The term also includes, within its scope, the does that effectively enhances normal physiological functions. The therapeutically effective does of one or more compounds of the present application is known to those skilled in the art, or can be easily determined by standard methods known in the art.

In addition, it should be noted that unless otherwise explicitly stated, the description "...independently selected from" used herein should be broadly understood to mean that the described individuals are selected independently of each other. Therefore, substituents may be the same or different from other substituents. More specifically, the description ". independently selected from" can refer to that the specific options expressed between in different groups and the same symbols do not affect each other; It can also mean that in the same groups, the specific options expressed between the same symbols do not affect each other.

Those skilled in the art can understand that according to the conventions used in the art, the structural formula of the groups described in the application, the examplary " 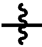 "and " 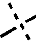 " mean the shown groups are connected with other fragments and groups in the compound through the sp3-sp3 covalent bond at this site. On the basis of not violating common sense in the art, the above preferred conditions can be arbitrarily combined to obtain better examples of the application.

### COMPOUNDS OF THE APPLICATION

The present application provides a PROTAC compound targeting an estrogen receptor represented by formula (I), or a pharmaceutically acceptable salt, solvate, hydrate, or isomer thereof: 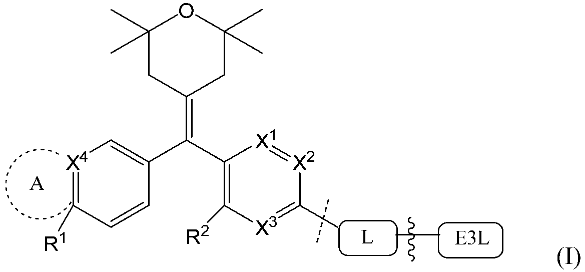 wherein L represents a connecting chain for connecting the ERL fragment and E3L; E3L represents a ubiquitin ligase ligand. Its function is to recruit E3 ligases thereby forming a stable ternary complex consisting of the target protein, PROTAC, and the E3 ubiquitin ligase. After tagging the target protein with ubiquitination, the ubiquitinated protein is recognized and degraded by the 26S proteasome in the cell.

Wherein 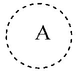 is absent, substituted or unsubstituted 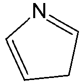 , substituented or unsubstituted 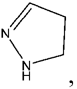 X⁴ is N or CRa, the substituent is selected from the group consisting of halogen, hydroxyl, cyano, oxo, benzyl, substituted or unsubstituted amino, substituted or unsubstituted C₁-C₆ alkyl, substituted or unsubstituted C₁-C₆ alkoxy and C₁-C₆ acyl;
when A is absent, R¹ is selected from the group consisting of halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl.
X¹ is N or CR³;
X² or X³ is independently selected from CH, CR³ and N;
R² and R³ are each independently selected from H, halogen, hydroxyl, cyano, oxo, benzyl, substituted or unsubstituted amino, substituted or unsubstituted C₁-C₆ alkyl, substituted or unsubstituted C₁-C₆ alkoxy, and C₁-C₆ acyl;
Ra is selected from the group consisting of H, halogen, hydroxyl, cyano, oxy, benzyl, substituted or unsubstituted amino, substituted or unsubstituted C₁-C₆ alkyl, substituted or unsubstituted C₁-C₆ alkoxy and C₁-C₆ acyl;
L is any one selected from the group consisting of:

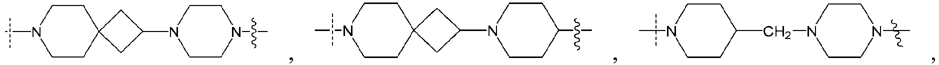

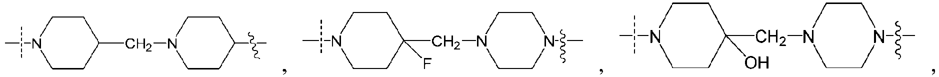

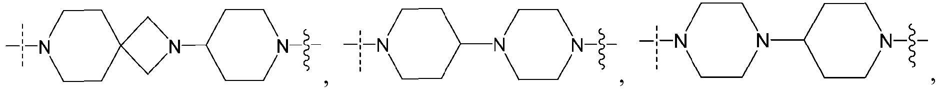

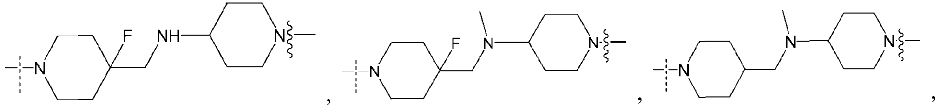

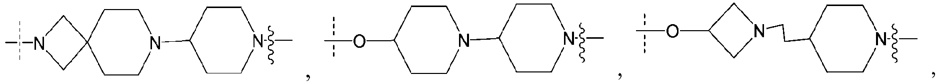

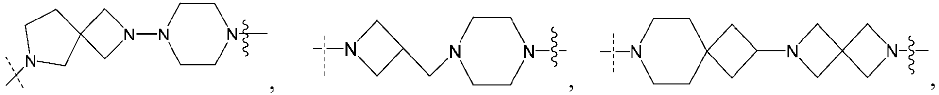

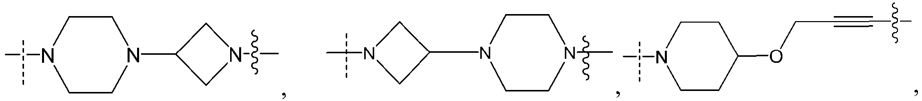

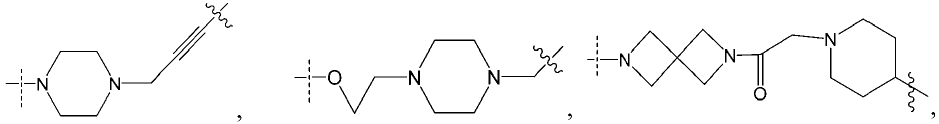

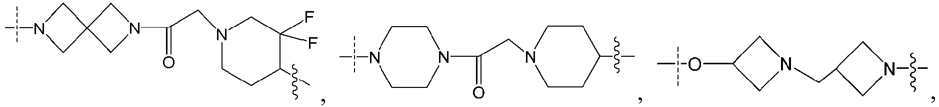

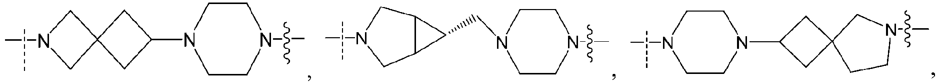

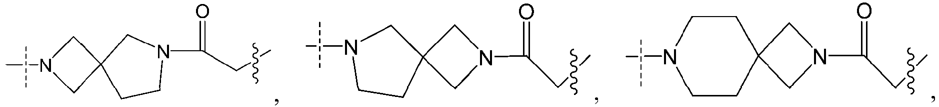

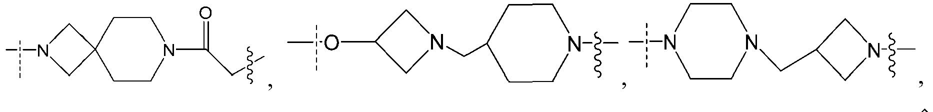

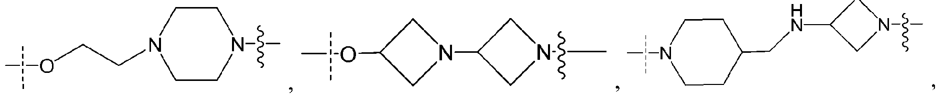

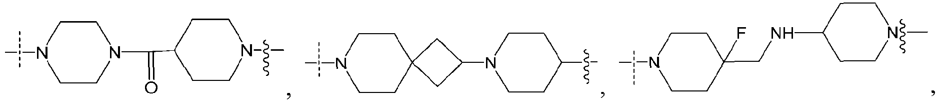

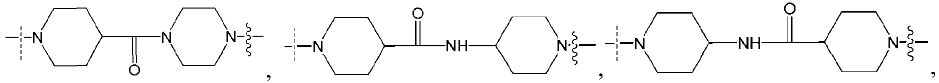

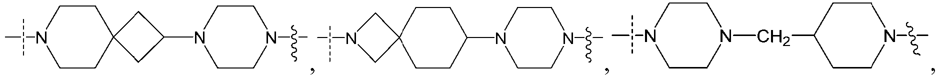

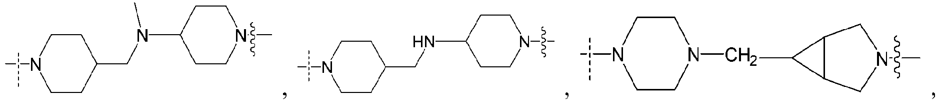

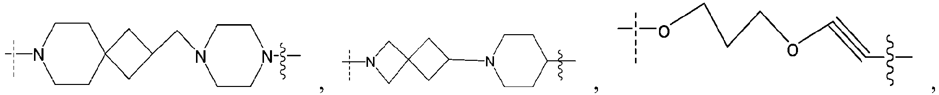

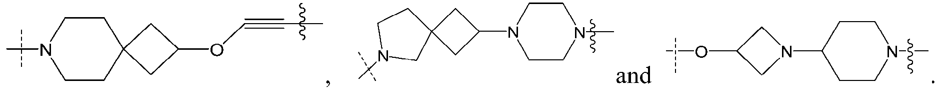

Those skilled in the art can understand that the E3L in this application can be any ubiquitin ligase ligand known in the art.

In one embodiment, E3L is any one selected from the group consisting of:

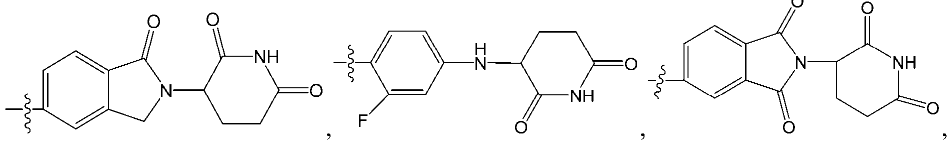

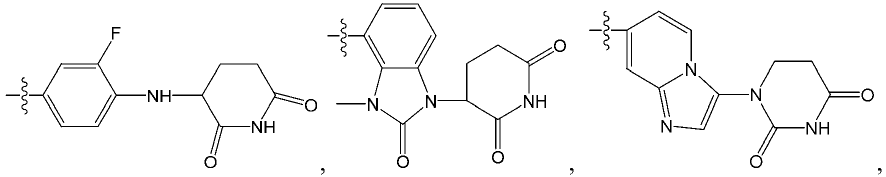

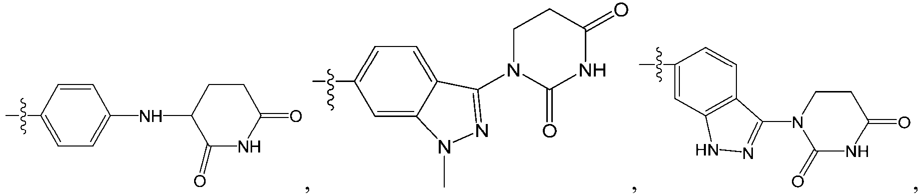

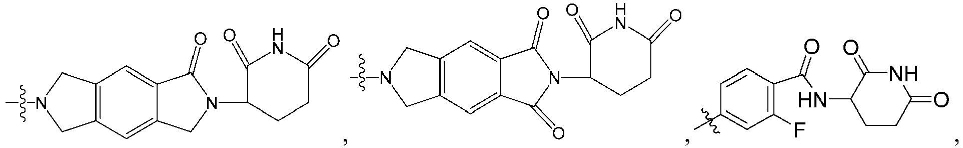

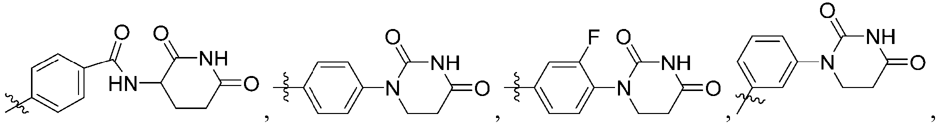

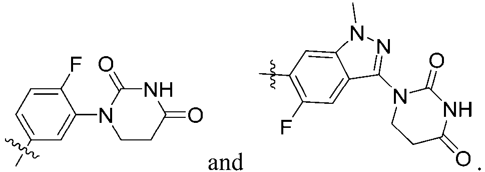

In one embodiment, E3L is

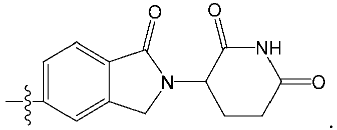

In one embodiment, L is any one selected from the group consisting of: 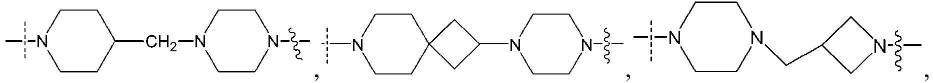 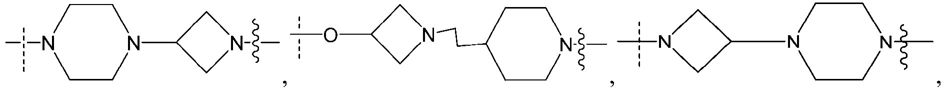 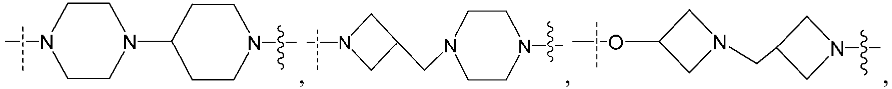 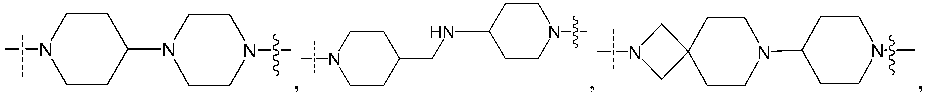 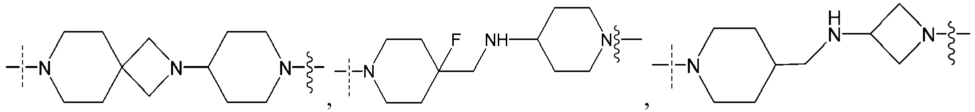 and

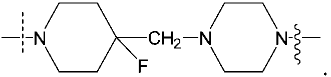

In one embodiment, L is 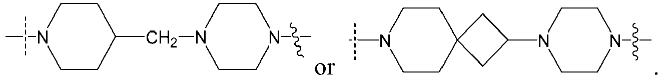 when A is absent, R¹ is selected from -NH₂ or -OH.

In one embodiment, X⁴ is N.

In one embodiment, X⁴ is CRa, and Ra is selected from H,halogen and hydroxyl. In one embodiment, X⁴ is CRa, and Ra is H.

In one embodiment, X¹ is CR³, and R³ is H, CH₃O or F. In one embodiment, X¹ is CR³, and R³ is H.

In one embodiment, X² is CH. In one embodiment, X² is N.

In one embodiment, X³ is CH.

In one embodiment, R² is H.

In one embodiment, the compound is represented by formula (I):
wherein 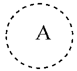 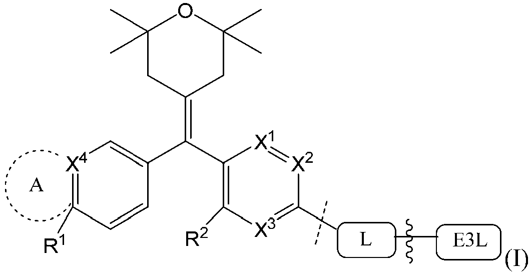 is a substituted or unsubstituted 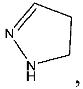 X⁴ is CH; X¹ is CR3, R3 is H, CH3O or F; X² is CH or N; X³ is CH; R² is selected from H, halogen, hydroxyl, cyano, oxo, benzyl, substituted or unsubstituted amino, substituted or unsubstituted C₁-C₆ alkyl, substituted or unsubstituted C₁-C₆ alkoxy and C₁-C₆ acyl;
L is any one selected from the group consisting of: 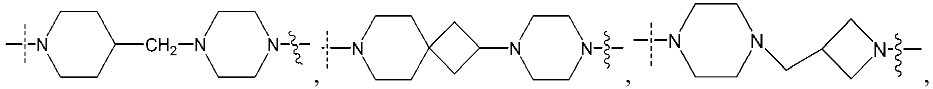 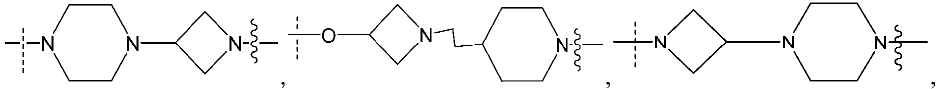 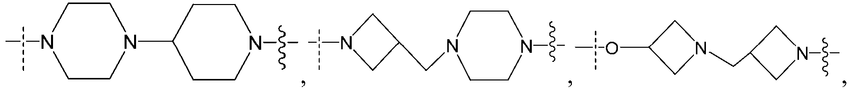 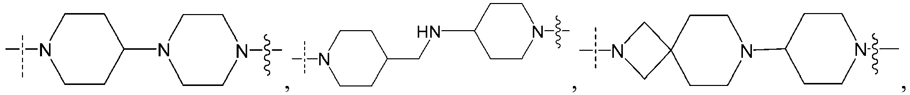 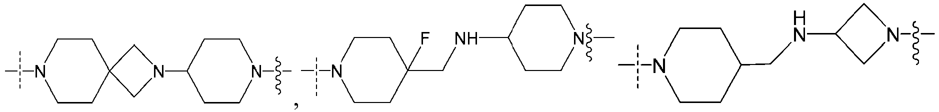 and 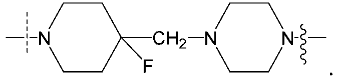
E3L is any one selected from the group consisting of:

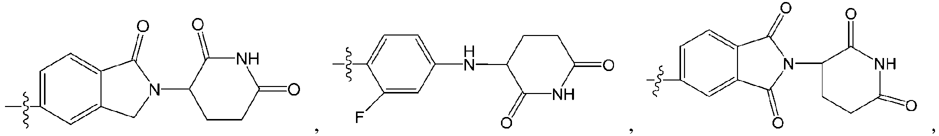

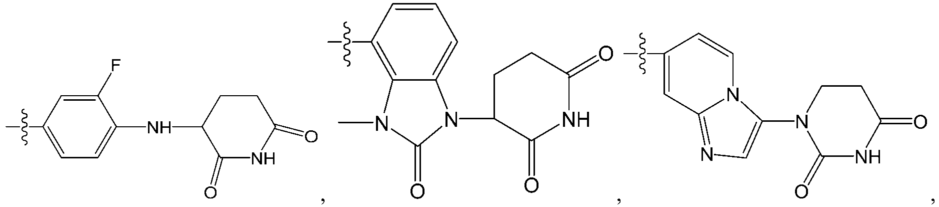

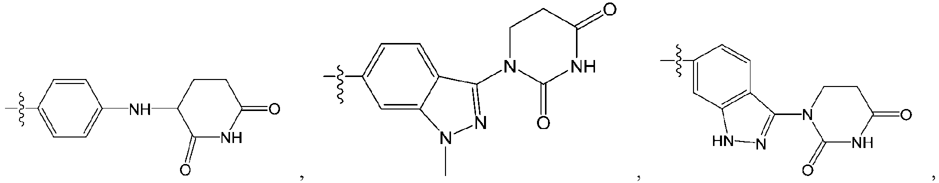

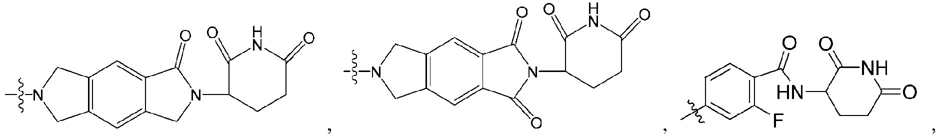

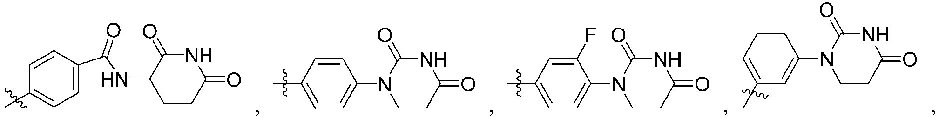

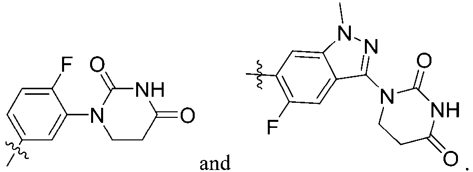

In one embodiment, the compound is represented by formula (I): 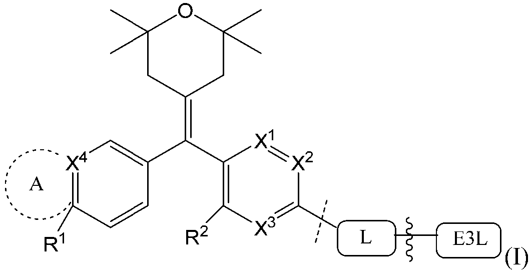 wherein 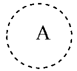 is a substituted or unsubstituted 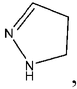 X⁴ is CH; X¹ is CR³, R³ is H, CH3O or F;X² is CH or N; X³ is CH; R² is selected from H, halogen, hydroxyl, cyano, oxo, benzyl, substituted or unsubstituted amino, substituted or unsubstituted C₁-C₆ alkyl, substituted or unsubstituted C₁-C₆ alkoxy and C₁-C₆ acyl;
L is

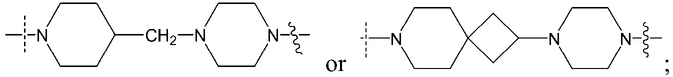

E3L is any one selected from the group consisting of:

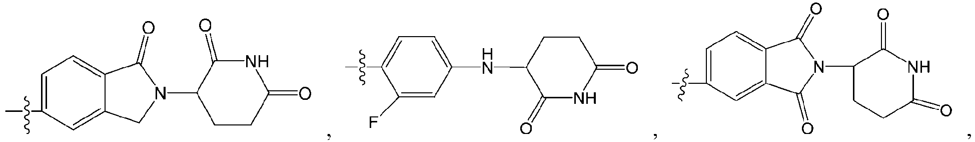

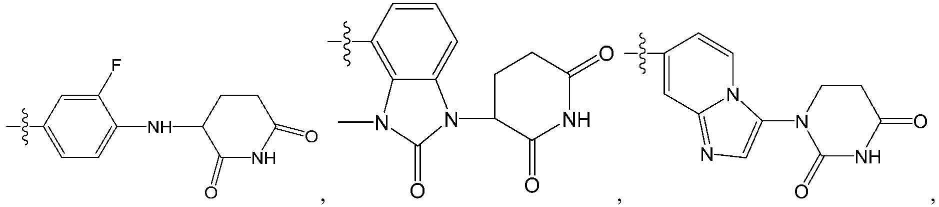

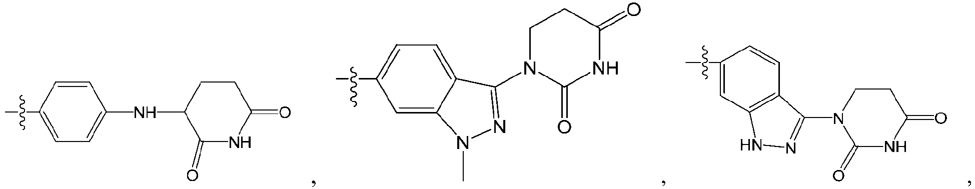

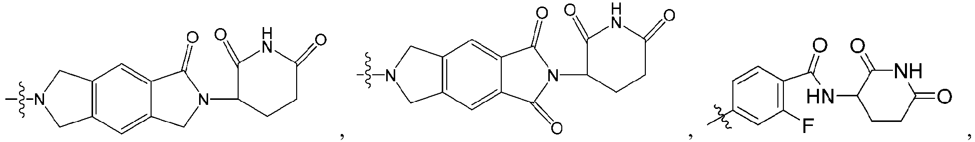

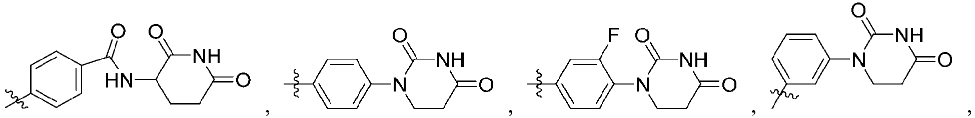

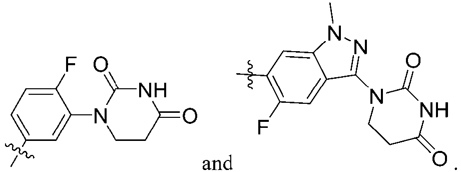

In one embodiment, the compound represented by formula (I) is represented by formula (II) 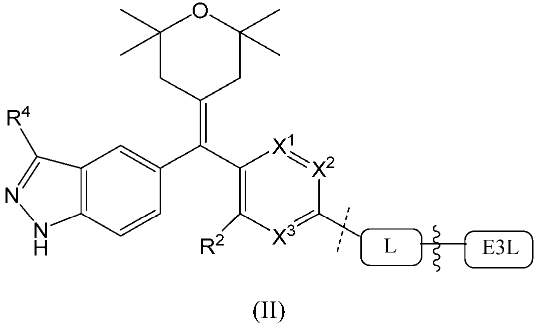 wherein R⁴ is selected from halogen, hydroxyl, cyano, oxo, benzyl, substituted or unsubstituted amine, substituted or unsubstituted C₁-C₆ alkyl, substituted or unsubstituted C₁-C₆ alkoxy and C₁-C₆ acyl.

In one embodiment, R⁴ is halogen. In one embodiment, R⁴ is F.

In one embodiment, the compound is represented by formula (II) 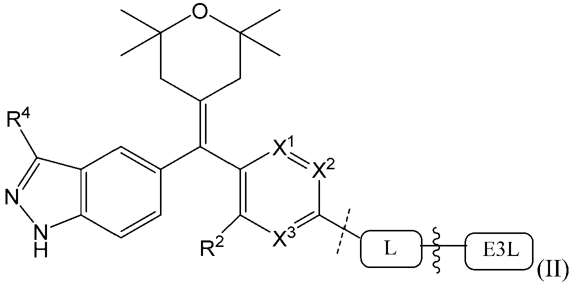 wherein, R⁴ is halogen; X¹ is CR³, R³ is H, CH₃O or F; X² is CH or N; X³ is CH; R² is selected from H, halogen, hydroxyl, cyano, oxy, benzyl, substituted or unsubstituted amino, substituted or unsubstituted C₁-C₆ alkyl, substituted or unsubstituted C₁-C₆ alkoxy and C₁-C₆ acyl;
In one embodiment, L is any one selected from the group consisting of: 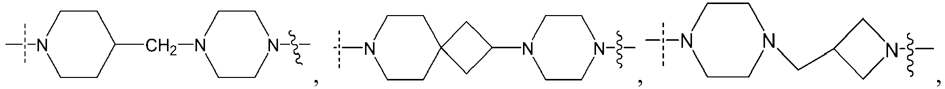 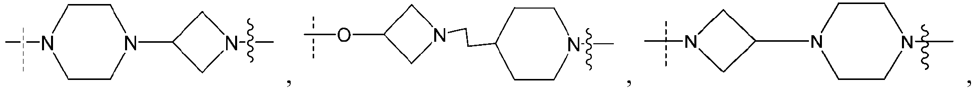 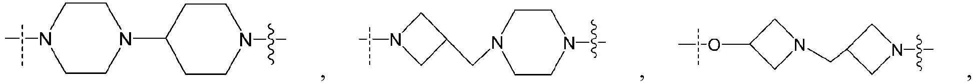 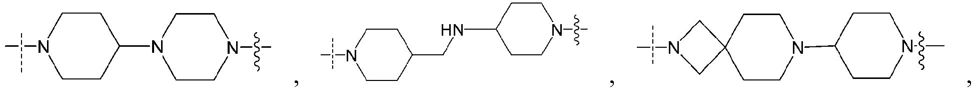 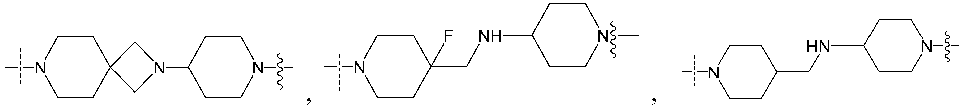 and

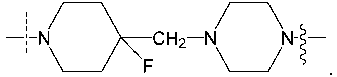

In one embodiment, E3L is any one selected from the group consisting of:

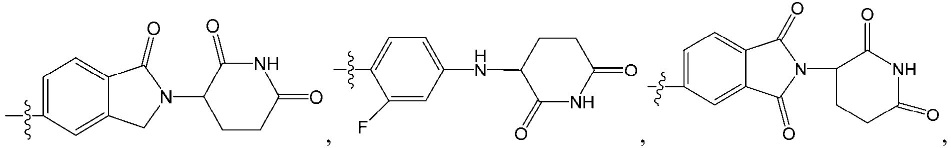

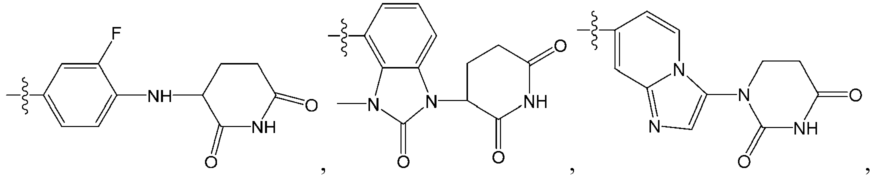

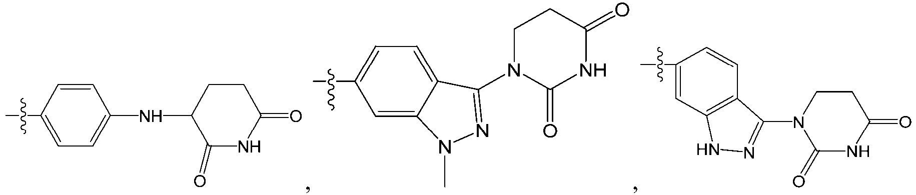

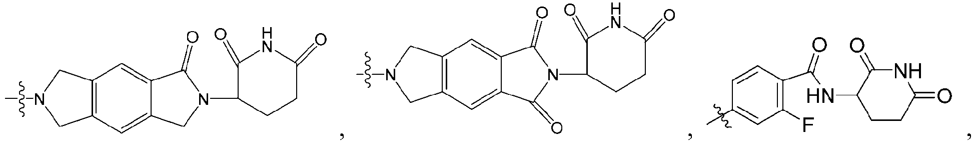

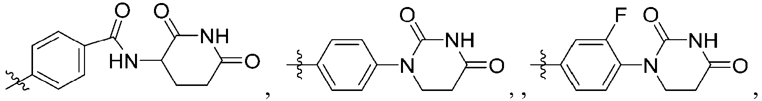

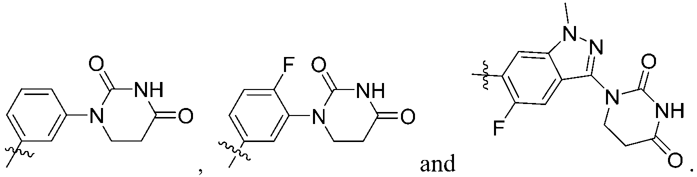

In one embodiment, the compound is represented by formula (II): 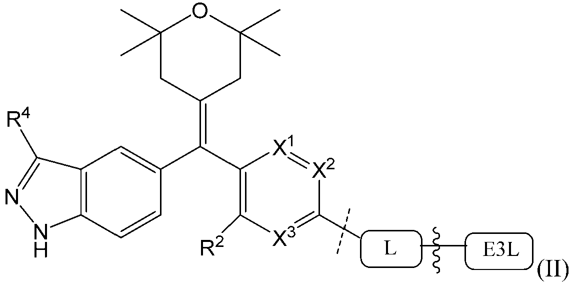
wherein R⁴ is selected from halogen, X¹ is CR³, R³ is H, CH₃O or F; X² is CH or N; R² is selected from H, halogen, hydroxyl, cyano, oxy, benzyl, substituted or unsubstituted amino, substituted or unsubstituted C₁-C₆ alkyl, substituted or unsubstituted C₁-C₆ alkoxy and C₁-C₆ acyl;
L is

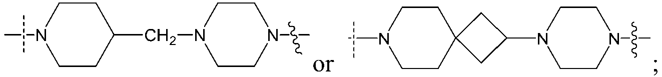

In one embodiment, E3L is any one selected from the group consisting of:

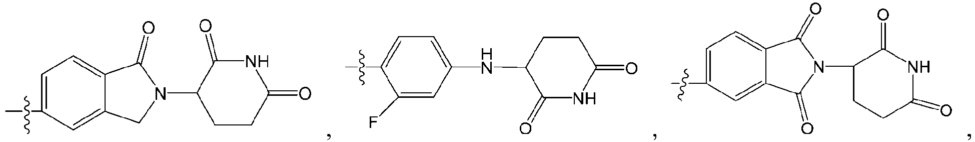

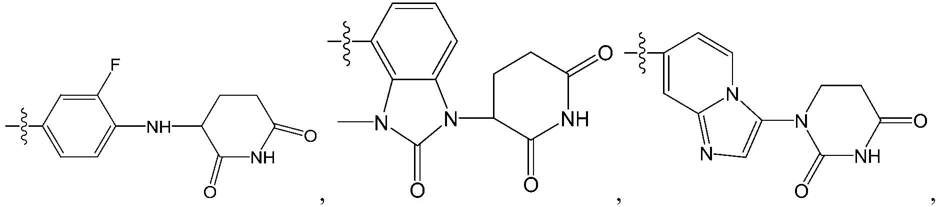

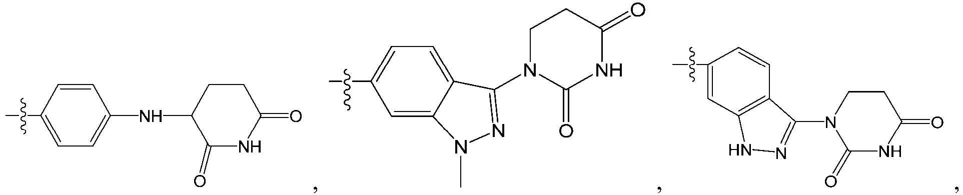

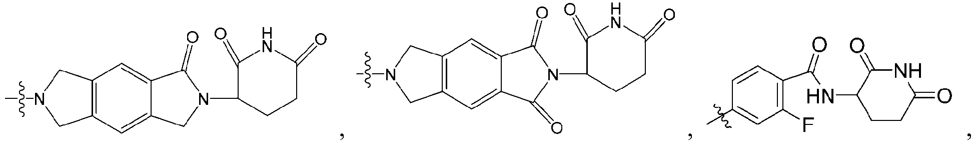

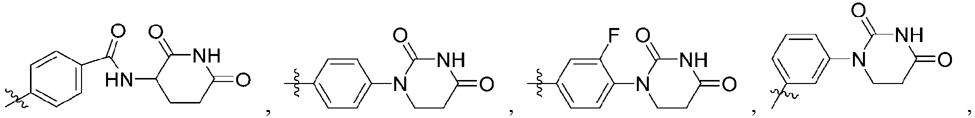

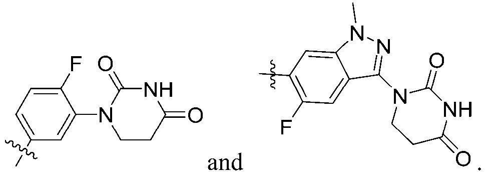

In one embodiment, the compound is represented by formula (III):

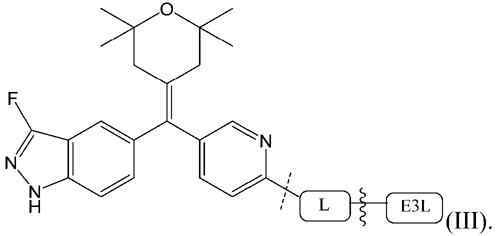

In one embodiment, the compound is represented by formula (III):

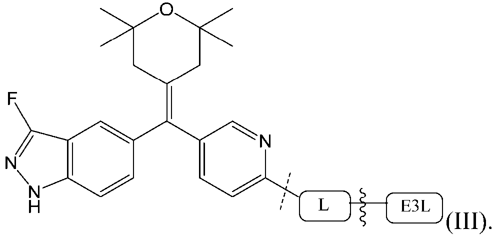

In one embodiment, L is any one selected from the group consisting of: 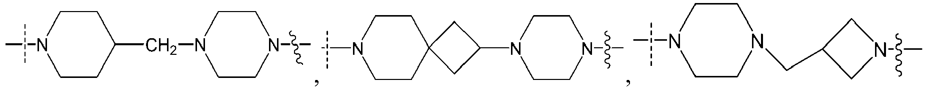 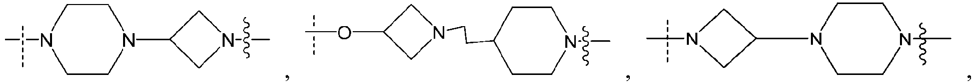 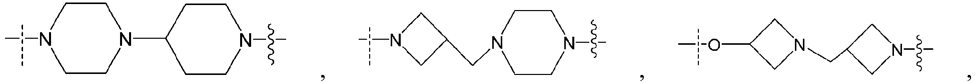 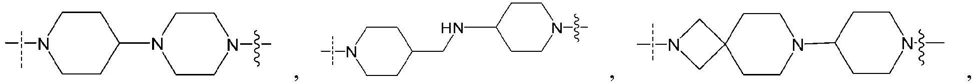 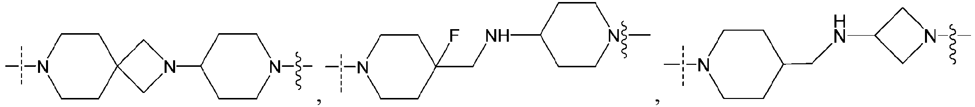 and

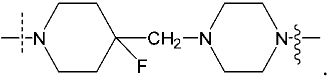

In one embodiment, E3L is any one selected from the group consisting of :

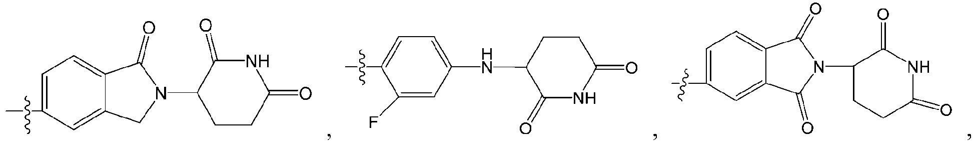

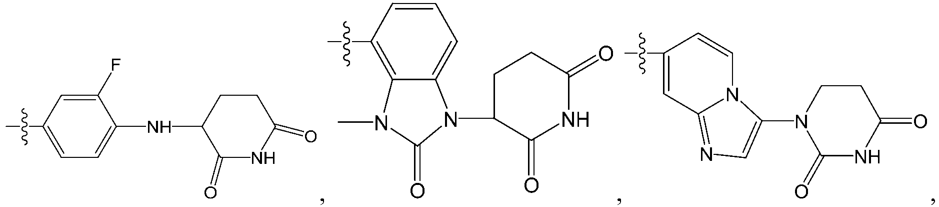

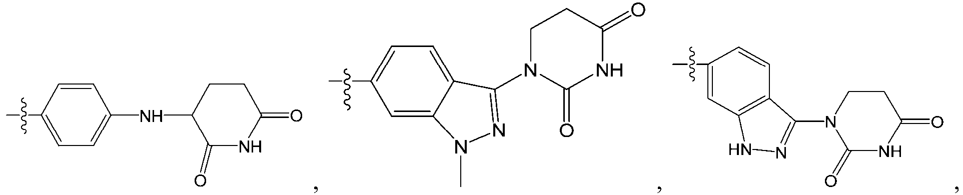

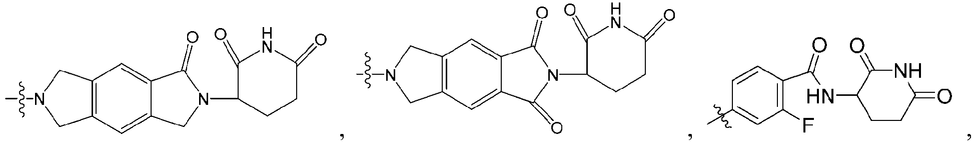

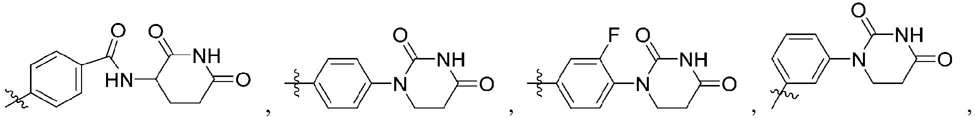

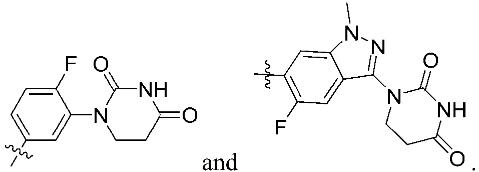

In one embodiment, the compound is represented by formula (III):

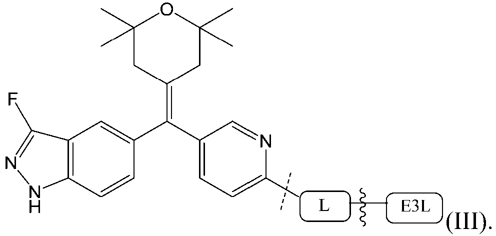

In one embodiment, L is

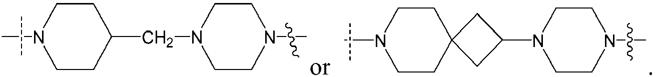

In one embodiment, E3L is any one selected from the group consisting of:

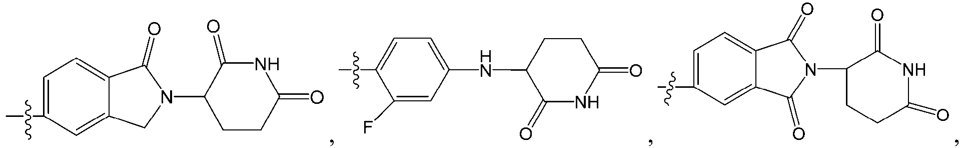

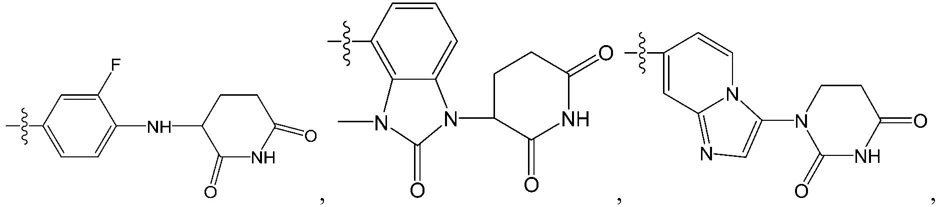

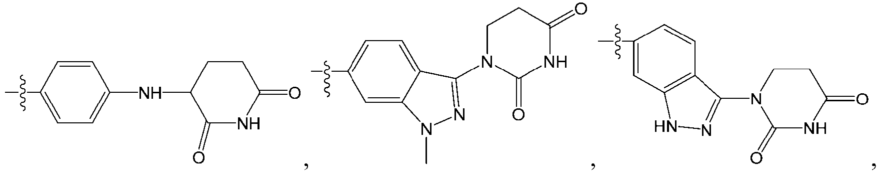

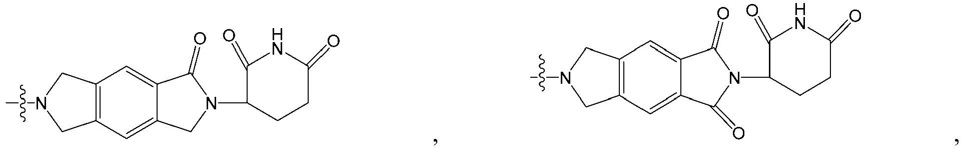

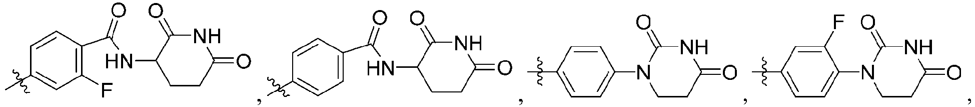

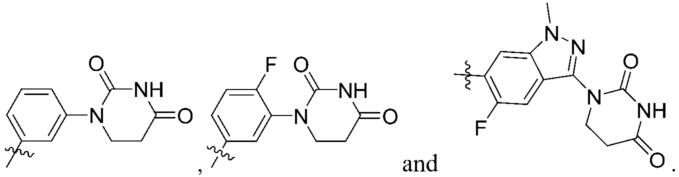

In one embodiment, the compound of formula (I) is represented by formula (IV):

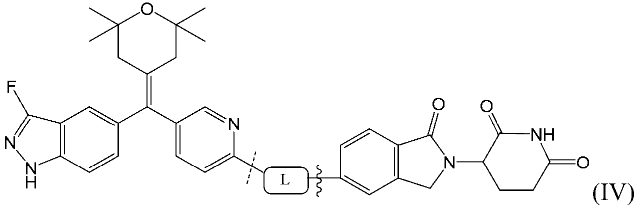

In one embodiment, the compound is represented by formula (IV):

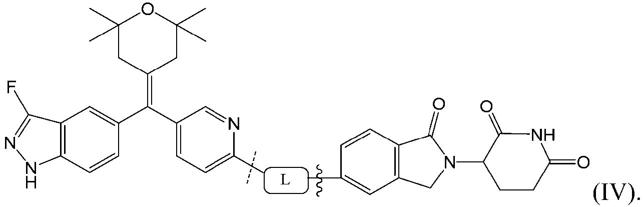

In one embodiment, L is anye one selected from the group consisting of: 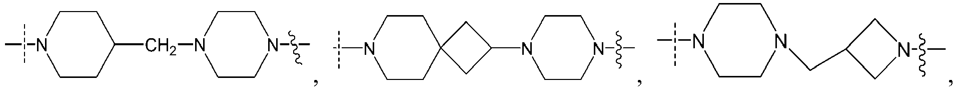 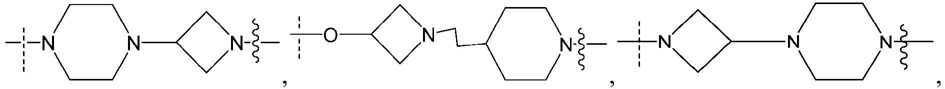 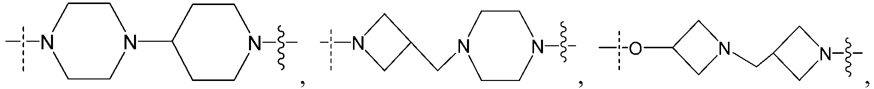 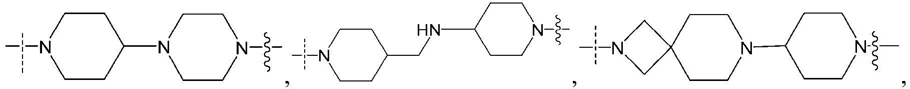 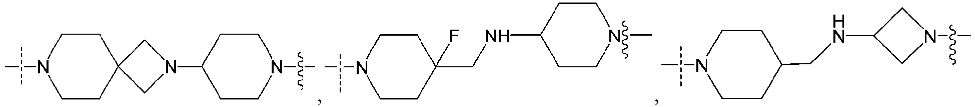 and

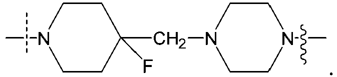

In one embodiment, the compound is represented by formula (IV):

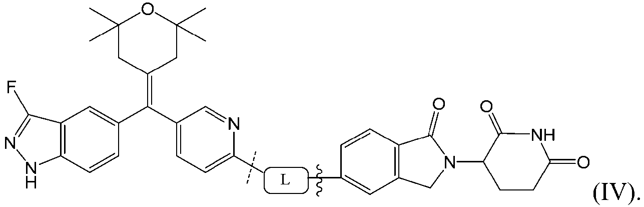

In one embodiment, L is

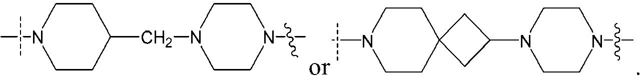

In one embodiment, the compound of the application is one of the compounds 1-143.

### Pharmaceutical Composition

The present application also provides a pharmaceutical composition comprising any of the aforementioned compounds, pharmaceutically acceptable salts, solvates, hydrates or isomers thereof, and a pharmaceutically acceptable carrier.

The form of the pharmaceutical composition depends on multiple factors, including, for example, the route of administration, the severity of the disease, or dosage to be administered, etc.

In one embodiment, the pharmaceutical composition may be formulated for delivery to a subject via appropriate routes, including but not limited to oral administration, injectable routes (e.g., intravenous, intramuscular, subcutaneous, intradermal, intracardiac, intrathecal, intrapleural, intraperitoneal, etc.), mucosal routes (e.g., intranasal, buccal administrationv, etc.), sublingual route, rectal route, transdermal route, intraocular route, and pulmonary route. Depending on the desired administration route, the pharmaceutical composition may be formulated as tablets, capsules, pills, sugar-coated pills, powders, granules, troches, lozenge, suppositories, suspensions, emulsions, syrups, aerosols (as solids or in liquid media), sprays, plaster, pastes, patches, creams, lotions, gels, inhalants, and the like.

### Therapeutic uses and treatment methods

The present application also relates to the uses of compounds of the present application or pharmaceutically acceptable salts, solvates, hydrates, or isomers thereof, or the pharmaceutical compositions of the present application in the treatment of diseases treated by degrading a target protein that binds to the targeting ligands.

The present application also relates to the use of the compounds of the present application, or a pharmaceutically acceptable salt, solvate, hydrate, or isomer thereof, or the pharmaceutical compositions of the present application, in the manufacture of medicaments for treating a disease treated by degrading a target protein that binds to a targeting ligand.

The application also relates to a method for treating a disease in a subject by degrading a target protein that binds to a targeting ligand, comprising administering to the subject an effective amount of compounds of the present application, or a pharmaceutically acceptable salt, solvate, hydrate, or isomer thereof, or the pharmaceutical compositions of the present application.

In one specific embodiment, the disease is selected from abnormal cell proliferation, cancer, immune disease, diabetes, cardiovascular disease, infectious disease and inflammatory disease.

Preferably, the cancer is selected from breast cancer, endometrial cancer, uterine cancer, testicular cancer, cervical cancer, prostate cancer, ovarian cancer, endometriosis, fallopian tube tumors, ovarian tumors, leukemia, skin cancer, squamous cell carcinoma, basal cell carcinoma, adenocarcinoma, renal cell carcinoma, bladder cancer, intestinal cancer, colon cancer, esophageal cancer, head cancer, kidney cancer, liver cancer, lung cancer, neck cancer, pancreatic cancer, stomach cancer, lymphoma, non Hodgkin's lymphoma, melanoma, myeloproliferative disorders, sarcoma, angiosarcoma, peripheral neuroepithelioma, glioma, astrocytoma, oligodendroglioma, ependymoma, glioblastoma, neuroblastoma, gangliocytoma ganglioglioma, medulloblastoma, pineocytoma, meningioma, meningeal sarcoma, neurofibroma, schwannoma, thyroid carcinoma, Hodgkin's lymphoma, Wilms tumor, and teratoma. More preferably, it is selected from breast cancer, endometrial cancer, uterine cancer, testicular cancer, cervical cancer, prostate cancer, ovarian cancer, fallopian tube tumor and ovarian tumor.

In one specific embodiment, the cancer is selected from breast cancer, endometrial cancer, ovarian cancer, cervical cancer, prostate cancer, or endometriosis.

In one specific embodiment, the infectious disease is selected from viral pneumonia, avian influenza, meningitis, gonorrhea, or diseases caused by infections with HIV, HBV, HCV, HSV, HPV, RSV, CMV, Ebola virus, flavivirus, rhabdovirus, rotavirus, influenza, coronavirus EBV, drug-resistant viruses, RNA viruses, DNA viruses, adenoviruses, poxviruses, small ribonucleic acid viruses, Togaviridae, orthomyxoviruses, retroviruses, Hepadnavirus, Gram negative bacteria, Gram positive bacteria, atypical bacteria, Staphylococcus, Streptococcus, Escherichia coli, Salmonella, Helicobacter pylori, Chlamydiaceae, Mycoplasmataceae, fungi, protozoa, helminths, worms, prions or parasites.

### Examples

The following examples illustrates the present application, providing synthetic and biological examples described in the present application to illustrate the compounds, pharmaceutical compositions, and methods provided in this application. The following embodiments are only for illustrative purposes and are not intended to limit the scope of this application. Any modifications, changes, variations, etc. made within the scope of this application are within the scope of protection of this application.

The compounds provided herein can be prepared through the specific synthesis scheme described below, which will be known to those skilled in the art, from readily available starting materials. The experimental methods without specific conditions specified in the following examples can be determined by those skilled in the art through conventional optimized procedures of conventional methods and conditions.

The abbreviations in the examples are shown in the table below:

| English abbreviation | full name | English abbreviation | full name |
|---|---|---|---|
| AcOH | acetic acid | t-BuOK | potassium tert butoxid |
| Cs₂CO₃ | Cesium carbonate | BAST | bis(2-methoxyethyl) amino sulfur trifluoride |
| CDI | Carbonyl diimidazole | CuI | cuprous iodide |
| DIBAL | diisobutylaluminum hydride | Pd (PPh₃)₄ | tetrakis (triphenylphosphine) palladium |
| Pd₂(dba)₃ | Tri(dibenzylideneacetone) dipalladium | Pd(dppf)Cl₂ | 1,1 '- diphenylphosphine Ferrocene Palladium Dichloride |
| Ruphos | 1'-biphenyl; | RuPhos Palladacycle G3 | methanesulfonic (2-Dicyclohexylphosphon-2 ', 6' - diisopropoxy-1,1 '-biphenyl) (2-amino-1,1' - biphenyl-2-yl) palladium (II) |
| | 2-Dicyclohexylphosphon-2 ',6'-diisopropylbiphenyl; | | |
| | 2-Dicyclohexylphosphon-2 ', 6' - diisopropyloxy-1,1 '- biphenyl | | |
| Xantphos | 4,5-diphenylphosphine-9,9-dimethyloxaanthracene | PE | petroleum ether |
| EA | ethyl acetate | Boc | tert butoxycarbonyl |
| DIEA | N. N-diisopropylethylamine | DHP | Dihydro-2H pyran |
| DMA | N. N-dimethylacetamide | DMF | N,N-dimethylformamide |
| DCM | dichloromethane | DMP | Dess-Martin periodinane |
| Selectfluor | N-Fluoro-N'- (Chloromethyl) Triethylenediamine Bis (Tetrafluoroborate) | TFA | Trifluoroacetic acid |
| TfOH | Trifluoromethanesulfonic acid | t-BuXPhos pre-catalyst | Chlorine [2- (di tert butylphosphine) -2 ', 4', 6 '-triisopropyl-1,1'- biphenyl[2-(2-aminoethyl)phenyl)] palladium (II) |
| t-BuXPhos | 2-Di-tert-butylphosphate-3,4, 5,6-tetramethyl-2 ', 4', 6 '-triisopropylbiphenyl | THF | tetrahydrofuran |
| THP | 2- tetrahydropyran | LiBH₄ | Lithium borohydride |
| LiHMDS | bis(trimethylsilyl)amino lithium | MeOH | methanol |
| MeCN | acetonitrile | NaBH₃CN | sodium cyanoborohydride |
| NBS | N-bromosuccinimide | NIS | N-iodobutyrimide |
| nBuLi | N-butyl lithium | PMBCl | 4-methoxybenzyl chloride |
| pTsOH | p-toluenesulfonic acid | d7-DMF | 7 deuterium-N, N-dimethylformamide |
| CDCl₃ | deuterated chloroform | PMBBr | 4-methoxybenzyl bromide |
| TiCl₄ | titanium tetrachloride | PMB | 4-methoxybenzyl |
| 1, 4-dioxane | 1, 4-dioxane | TEA | triethylamine |
| SFC | Supercritical fluid chromatography | STAB | sodium triacetoxyborohydride |
| EtOAc | ethyl acetate | mL | milliliter |
| Tr | triphenylmethyl | MS | mass spectrometry |
| DBU | 1,8-diazabicyclo *[5.4.0]* undec-7-ene. 1,8-diazabicyclo [5.4.0] undec-7-ene | Lac | lactic acid |
| ESI | electrospray ionization | ¹HNMR | nclear magnetic resonance hydrogen spectrum |
| TLC | thin layer chromatography | Chiral HPLC | ciral high-performance liquid chromatography |
| Prep-HPLC | High pressure preparative liquid chromatography | Prep-TLC | peparation of Thin Layer Chromatography |
| ACN | acetonitrile | DCE | dichloroethane |
| Hex | n-hexane | IPA | isopropanol |
| DCM | dichloromethane | Et₂O | Ethyl ether |
| HATU | 2- (7-Azabenzotriazole) - N, N, N ', N' - Tetramethylurea Hexafluorophosphate | STAB | Sodium triacetoxyborohydride |
| IBX | 2-iodoylbenzoic acid | R_{f} | Relative shift value |
| LC-MS | LC-MS iquid chromatography-mass spectrometry | g | gram |
| min | minute | rt | Room temperature |
| mg | milligram | mmol | millimole |
| mol | mole | M | mole/liter |

Unless otherwise specified, all compounds herein include different stereoisomers with the same molecular formula. The term "stereoisomers" herein also includes enantiomers and diastereomers, wherein enantiomers are optical isomers and diastereomers are stereoisomers which can't form chiral enantiomers. Different isomers with the same molecular formula as the compounds herein is also within the scope of protection of this application.

Unless otherwise specified, the term "solvate" herein also called "solvent compound", refers to compounds containing solvents, where solvent molecules can be combined with compound molecules in the ways including coordination bonds, covalent bonds, van der Waals forces, ionic bonds, hydrogen bonds, etc.

Unless otherwise specified, the term 'pharmaceutically acceptable salts' used herein refers to compounds and/or salts formed in the present application that are chemically or physically compatible with other components of
a drug formulation and physiologically compatible with the receptor. Pharmaceutically acceptable salts "can also be referred to acid and/or basic salts formed with inorganic and/or organic acids and bases, as well as zwitterionic salts (internal salts) and quaternary ammonium salts such as alkyl ammonium salts. These salts can be directly obtained during the final separation and purification of the compound. It can also be obtained by appropriately mixing the compound or its stereoisomer or solvate herein with a certain amount of acid or base. These salts may form precipitates in solution and be collected by filtration, or obtained after solvent evaporation, or prepared by cooling and drying after reaction in an aqueous medium.

Unless otherwise specified, the compounds herein identified by traditional methods such as mass spectrometry and nuclear magnetic resonance and each step and condition can be referred to the conventional operating steps and conditions in this field.

Unless otherwise specified, this application adopts standard nomenclature and standard laboratory procedures and techniques for analytical chemistry, organic synthesis chemistry, and optics. In some cases, standard techniques are used for chemical synthesis, chemical analysis, and performance testing of light-emitting devices.

Unless otherwise specified, Minute^{™} Total Protein Extraction Kit herein is purchased from Chengxue Biotechnology (Guangzhou) Co., Ltd, product No. sd-001/sn-002.

Unless otherewise specified, Bio-rad Trans-Blot Turbo is purchased from Shanghai Aibio Biological Technology Co.

Unless otherewise specified, 5×SDS-PAGE sample buffer is purchased from Shanghai LMAI Bio Co.LTD., product No. LM-071.

Unless otherwise specified, the "5-bromo-1-H-indazole" herein is purchased from Shanghai Yuanye Biotechnology Co., Ltd., product number: Y11003.

Unless otherwise specified, the Dress-Martin reagent referred herein is also known as the Dress-Martin oxidant purchased from Hede Chemical (Suzhou) Co., Ltd.

Unless otherwise specified, the 4-bromo-2,6-difluorobenzaldehyde herein is purchased from MackLin Reagent Company with CAS number 537013-51-7.

Unless otherwise specified, the 4-fluorobenzaldehyde herein is purchased from Guide Chemical Technology (Shanghai) Co., Ltd., CAS number: 459-57-4.

Unless otherwise specified, the 5-fluoropyridine-2-aldehyde herein is purchased from Guide Chemical Technology (Shanghai) Co., Ltd., CAS number: 31181-88-1.

Unless otherwise specified, the 5-fluoroisobenzofuran-1,3-dione herein is purchased from Guide Chemical Technology (Shanghai) Co., Ltd., CAS number: 319-03-9.

Unless otherwise stated, the term 'dihydrouracil' herein is purchased from Shanghai Aladdin Technology Co., Ltd., CAS number 504-07-4.

Unless otherwise specified, the "7-bromo-imidazole [1,2-a] pyridine" herein is purchased from Shanghai Tebo Chemical Technology Co., Ltd. with CAS number 808744-34-5.

Unless otherwise stated, the term 'N-iodobutyrimide' herein is from Shanghai Aladdin Biochemical Technology Co., Ltd., CAS number 516-12-1.

Unless otherwise specified, the "(1R, 2R) - N1, N2-dimethylcyclohexane-1,2-diamine" herein is purchased from Shanghai Jixiang Biotechnology Co., Ltd., product number: SH215137.

Unless otherwise specified, the term "5,6-dimethylisoindolin-1-one" herein is purchased from Shanghai Yuanye Biotechnology Co., Ltd., CAS number: 110568-65-5.

Unless otherwise stated, the term 'benzoyl peroxide' herein is purchased from Jinan Huifengda Chemical Co., Ltd., CAS number 94-36-0.

Unless otherwise specified, the "5,6-dimethylisobenzofuran-1,3-dione" herein is purchased from Shanghai Haohong Biomedical Technology Co., Ltd. with CAS number 129-64-6.

Unless otherwise specified, the "3-amino-1- (4-methoxybenzyl) piperidine-2,6-dione" herein is purchased from Chongqing Kalan Pharmaceutical Co., Ltd. with CAS number 1834336-02-5. Unless otherwise specified, the "3-formylazetidine-1-carboxylic tert-butyl ester" herein is purchased from Shanghai Carlo Chemical Co., Ltd. with CAS number 177947-96-5.

Unless otherwise specified, the "di-tert-butyl [2 ', 4', 6 '- tris (propan-2-yl) - [1,1' - biphenyl] [1,1 '-biphenyl] -2-yl] phosphine" herein is purchased from Shaoyuan Technology, CAS number: 1160861-53-9.

Unless otherwise specified, the term '2-methylpropane-2-amine' herein is purchased from Shanghai Macklin Biochemical Technology Co., Ltd., CAS number: 75-64-9.

Unless otherwise specified, the term "Chlorine (2-dicyclohexylphosphon-2',6' - di-isopropoxy-1,1 '-biphenyl) (2-amino-1,1' - biphenyl-2-yl) palladium (II)" herein is purchased from Shanghai Aladdin Biochemical Technology Co., Ltd., CAS number: 1375325-68-0.

Unless otherwise specified, the "TBST" buffer herein was purchased from Shanghai Aladdin Biochemical Technology Co., Ltd.

### Intermediate Synthesis Examples

The present application is further illustrated below through exemplary embodiments, synthetic and biological examples are provided to demonstrate the compounds, pharmaceutical compositions, and methods herein. These embodiments are solely intended for exemplary purposes and should not be construed as limiting the scope of the application. All modifications, alterations, and variations made within the scope of the application are also protected under this application.

### preparation of ligand small molecule fragment ERL for binding estrogen receptor protein:

### Preparation of ERL-1: 3-fluoro-5- ((6-fluoropyridin-3-yl) (2,2,6,6-tetramethyltetrahydro-4H-pyran-4-yllidene) methyl) -1-tetrahydro-2H-pyran-4-yl) -1H-indazole

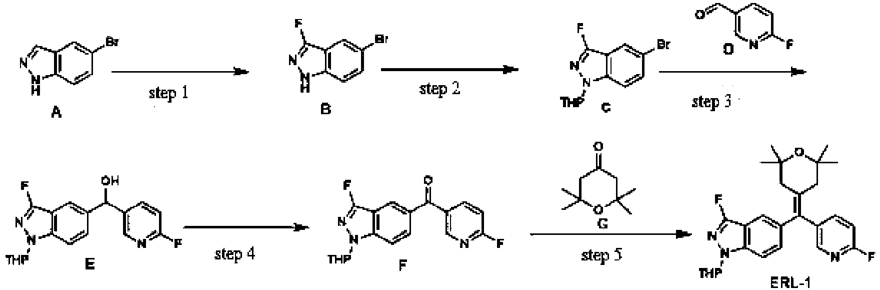

### Step 1: Preparation of 5-bromo-3-fluoro-1H-indazole:

To a stirred solution of 5-bromo-1H-indazole (200 g, 1.01 mol) in DMA (2 L) in an ice bath, Selectfluor (719 g, 2.03 mol) was added batchwise under a nitrogen atmosphere. The resulting mixture was stirred at 60 °C for 12 hours. The mixture was allowed to cool to room temperature. The reaction was quenched by adding water (200 mL). The resulting mixture was extracted with ethyl acetate (3×1500 mL), and the combined organic layers were washed with water (3×500 mL), and then dried over anhydrous sodium sulfate. After filtration, the filtrate was concentrated under reduced pressure. The residue was purified by silica gel column chromatography, and then eluted with CH₂Cl₂/PE (1:1) to obtain 5-bromo-3-fluoro-1H-indazole as a white solid (82g, 37.57%). LCMS (ES, m/z): 215 [M+H]⁺.

### Step 2: preparation of 5-bromo-1-(tetrahydro-2H-pyran-2-yl) -1H-3-fluoroindazole:

Dihydro-2H-pyran (32 g, 380.7 mmol) and pTsOH (8.7 g, 50.8 mmol) were added to a stirred solution of 5-bromo-1H-indazole (50 g, 253.8 mmol) in DCM (800 mL) in an ice bath under a nitrogen atmosphere. The resulting mixture was stirred at room temperature for 4 hours. Adding a saturated aqueous solution of sodium bicarbonate to quench the reaction. The resulting mixture was extracted with DCM (3×1000 mL)×. The combined organic phases were dried over anhydrous sodium sulfate. After filtration, the filtrate was concentrated under reduced pressure. The residue was purified by silica gel column chromatography and eluted with PE/EA (5:1) to obtain 5-bromo-1-(tetrahydro-2H-pyran-2-yl) -1H-3-fluoroindazole as a yellow oily liquid (60 g, 81%). LCMS (ES, m/z): 299 [M+H]⁺.

### Step 3: preparation

### of(4-(6-fluoropyridin-3-yl)(1-(tetrahydro-2H-pyran-2-yl)-1H-3-fluoroindole-5-yl) methanol:

A solution of 5-bromo-1-(tetrahydro-2H-pyran-2-yl)-1H-indazole (10 g, 35.6 mmol) in THF (200 mL) was treated with n-butyl lithium in n-hexane (2.5 M, 17 mL, 42.7 mmol) at -78 °C under nitrogen atmosphere for 5 minutes, followed by the addition of 6-fluoropyridine-3-formaldehyde (6.7g, 53.4 mmol). Stirring the obtained mixture at -78 °C for 1 hour. An saturated ammonium chloride aqueous solution was added to quench the reaction, and the mixture was allowed to warm to room temperature. The obtained mixture was extracted with ethyl acetate (3×50 mL). The organic layers were combined and dried over anhydrous sodium sulfate. After filtration, the filtrate was concentrated under reduced pressure. The residue was purified by silica gel column chromatography, and then eluted with PE/EA to obtain (4-(6-fluoropyridin-3-yl) (1-(tetrahydro-2H-pyran-2-yl) -1H-3-fluoroindole-5-yl) methanol as a white solid (7 g, 58%) . LCMS (ES, m/z): 346 [M+H]⁺.

### Step 4: preparation of (3-fluoro-1-(tetrahydro-2H-pyran-4-yl)-1H-indazol-5-yl) (6-fluoropyridin-3 -yl) ketone:

Dess Martin reagent (13.6 g, 32.1 mmol) was added to a solution of (4-(6-fluoropyridin-3-yl)) (1-(tetrahydro-2H-pyran-2-yl)-1H-3-fluoroindole-5-yl)methanol (7 g, 21.4 mmol) in DCM (150 mL) in an ice bath. The resulting mixture was stirred overnight under a nitrogen atmosphere at room temperature. The obtained mixture was filtered, and the filter cake was washed with DCM. The filtrate was concentrated under reduced pressure. The residue was purified by silica gel column chromatography, and then eluted with PE/EA (10:1) to obtain (3-fluoro-1-(tetrahydro-2H-pyran-4-yl) -1H-indazol-5-yl)(6-fluoropyridin-3-yl) ketone as a gray white solid (4g, 55%). LCMS (ES, m/z): 344 [M+H]⁺.

### Step 5: preparation of 3-fluoro-5-((6-fluoropyridin-3-yl)(2,2,6,6-tetramethyltetrahydro-4H-pyran-4-ylidene)methyl)-1-tetrahydro-2H-pyran-4-yl) -1H-indazole:

Zinc powder (4 g, 61.5 mmol) was added to a stirred solution of TiCl₄ (4.7 g, 24.6 mmol) in THF (200 mL) in an ice bath under a nitrogen atmosphere. Stirring the obtained mixture at 65 °C for 2 hours, then cooling to 0 °C, before adding 2,2,6,6-tetramethyltetrahydro-4H-pyran-4-one (1.5 g, 9.2 mmol) and (3-fluoro-1- (tetrahydro- 2H-pyran-4-yl)-1H-indazol-5-yl)(6-fluoropyridin-3-yl)ketone (2 g, 6.2 mmol). The resulting mixture was stirred at 65 °C for 12 hours. The mixture was cooled to room temperature and the reaction was quenched with a saturated aqueous solution of sodium bicarbonate. The obtained mixture was extracted with ethyl acetate (3x200mL). The combined organic layer was dried over anhydrous sodium sulfate. After filtration, the filtrate was concentrated under reduced pressure. The residue was purified by silica gel column chromatography and eluted with PE/EA (5:1) to obtain 3-fluoro-5- ((6-fluoropyridin-3-yl) (2,2,6,6-tetramethyltetrahydro-4H-pyran-4-ylidene) methyl) -1-tetrahydro-2H-pyran-4-yl)-1H indazole (1.5 g, 52%) as a gray white solid. LCMS (ES, m/z) : 468[M + H]⁺.

Unless otherwise specified, the following intermediates and analogues (not limited to analogues with different substituents, such as halogens) are prepared using corresponding similar starting materials and reagents according to the similar operations described in the ERL-1 embodiment above.

### ERL-2: 5- ((6-fluoropyridin-3-yl) (2,2,6,6-tetramethyltetrahydro-4H-pyran-4-ylidene) methyl)-1-(tetrahydro-2H-pyran-4-yl)-1H-indazole, LCMS: [M+H]⁺=450.2.

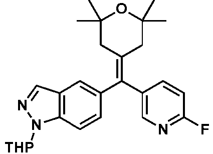

The synthesis method is reffered to Erl-1, and step 1 of fluorination is omitted.

### ERL-3: 5-((4-chlorophenyl) (2,2,6,6-tetramethyltetrahydro-4H-pyran-4-ylidene)methyl)-3-fluoro- 1-(tetrahydro-2H-pyran-4-yl)-1H-indazole, LCMS: [M+H]⁺=483.2

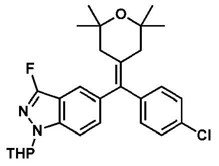

The synthesis method is referred to ERL-1, where 4-fluorobenzaldehyde is replaced by 6-fluoropyridine-3-formaldehyde in step 3.

### ERL-4: 5- ((4-chlorophenyl) (2,2,6,6-tetramethyltetrahydro-4H-pyran-4-ylidene) methyl) -1-(tetrahydro-2H-pyran-2-yl) -1H-indazole, LCMS: [M+H]⁺=465.2.

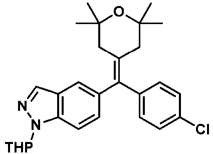

The synthesis method is referred to ERL-1, omitting step 1, where 6-fluoropyridine-3-formaldehyde is replaced by 6-chlorobenzaldehyde in step 3.

### ERL-5: 5- ((4-bromo-2,6-difluorophenyl) (2,2,6,6-tetramethyltetrahydro-4H-pyran-4-methylene) methyl) -3-fluoro-1- (tetrahydro-2H-pyran-4-yl) -1H-indazole, LCMS: [M+H] ⁺=563.1

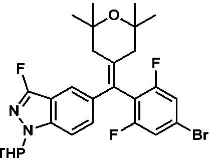

The synthesis method is referred to ERL-1.

### ERL-6: 3-fluoro-5- ((5-fluoropyridin-2-yl) (2,2,6,6-tetramethyltetrahydro-4H-pyran-4-ylidene) methyl) -1- (tetrahydro-2H-pyran-4-yl) -1H-indazole

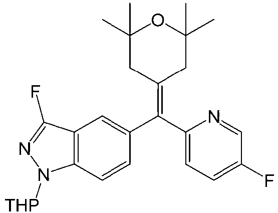

The synthesis method is referred to ERL-1, 6-fluoropyridine-3-formaldehyde is replaced by 5-fluoropyridine-2-formaldehyde in step 3.

Preparation of small molecule fragment E3L that binds to E3 ubiquitin ligase:

### Preparation of E3L-1: 2- (2,6-dioxopiperidin-3-yl) -5-fluoroisoindoline-1,3-dione

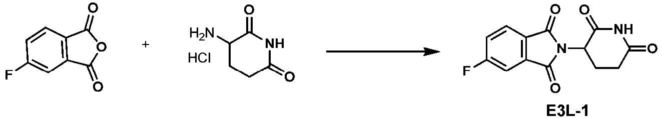

Sodium acetate (14.8 g, 180 mmol) and 3-aminopiperidine-2,6-dione hydrochloride (14.6 g, 90mmol) were added to a solution of 5-fluoroisobenzofuran-1,3-dione (15 g, 90 mmol) in acetic acid (150mL). The mixture was stirred at 120 °C for 12 hours. The reaction mixture was concentrated under reduced pressure to remove most of the acetic acid. The residue was poured into 200 mL of water and stirred for 15 minutes. The mixture was filtered. The filter cake was washed with water (50 mL×2) and dried to obtain 2- (2,6-dioxopiperidin-3-yl) -5- fluoroisoindoline-1,3-dione as a gray white solid (21 g, 90% yield). LC-MS(ESI)m/z: 277 .1[M+1] ⁺;

### Preparation of E3L-2 piperazine hydrochloride: 3- (1-oxo-5-(piperazin-1-yl)isoindole-2-yl) piperidine-2,6-dione hydrochloric acid

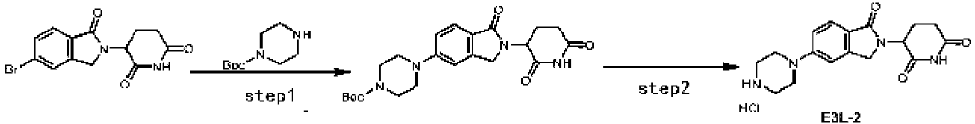

### Step 1: preparation of tert-butyl 4-[2-(2,6-dioxopiperidin-3-yl)-1-oxo-isoindol-5-yl] piperazine-1-carboxylic acid

Tert-butyl piperazine-1-carboxylate (13.8 g, 74.3 mmol), Cs₂CO₃ (24.2 g, 74.3 mmol), and 2,3-di [2,6-bis(pent-3-yl)phenyl]-2,3-dihydro-1H-imidazol-2-yl]dichloro(3-chloropyridine-1-ammonium-1 -yl) palladium (2.0 g, 2.5 mmol) were sequentially added to a solution of 3-(5-bromo-1-oxoindole-2-yl) piperidine-2,6-dione (8 g, 24.8 mmol) in 1,4-dioxane (100 mL) under a nitrogen atmosphere. The resulting mixture was heated to 100 °C and stirred for 4 hours. The reaction was cooled to room temperature and quenched with water. The solid precipitate was collected by filtration and washed with H₂O (2×15 mL). The residue was washed and mashed with ethyl acetate (2×10 mL). The generated solid was dried under infrared light. Tert-butyl 4- [2-(2,6-dioxopiperidin-3-yl)-1-oxo-3H-isoindol-5-yl] piperazine -1-carboxylic acid as a gray white solid *(5* g, 47.13%) was obtained LCMS (ES, m/z) : 429[M+H]⁺.

### Step 2: preparation of 3-(1-oxo-5-(piperazin-1-yl)isoindolin-2-yl)piperidine-2,6-dione

Tert-butyl 4-[2- (2,6-dioxypiperidin-3-yl)-1-oxo-isoindole-5-yl]piperazine-1-carboxylic acid (5 g, 11.7 mmol) and hydrochloric acid (4 M, 10 mL) were stirred in ethyl acetate (30 mL) ove rnight. The resulting mixture was concentrated under reduced pressure. The residue was washed and mashed with ethyl acetate (5×20 mL). 3-(1-oxo-5-(piperazin-1-yl)-3H-isoindole-2-yl] piperidine-2,6-dione hydrochloride as a gray white solid (3 g, 70.47%) was obtained. LCMS (ES, m/z) : 329[M+H]⁺.

### Preparation of E3L-3: 1-(7-bromoimidazo[1,2-α]pyridine-3-yl)dihydropyrimidine-2,4(1H,3H)-dione:

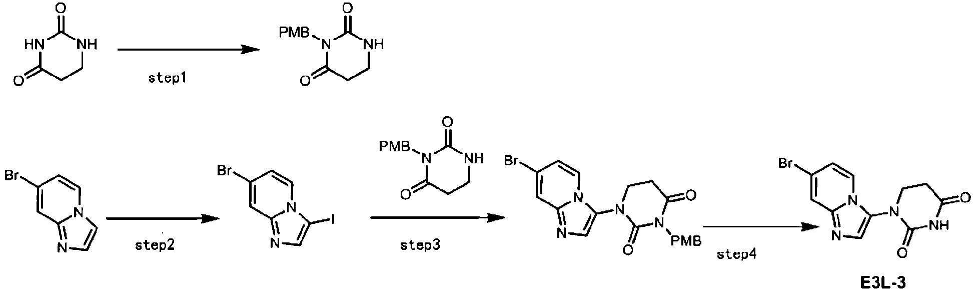

### Step 1: preparation of 3- (4-methoxybenzyl) dihydropyrimidine-2,4 (1H, 3H) - dione:

Dihydrouracil (6.0 g, 52.6 mmol) was dissolved in DMF (100ml), Cs₂CO₃ (34.5 g, 106 mmol) was added at 25 °C, and then PMBCl (7.5 g, 47.5 mmol) was slowly added into the solution at room temperature. After reacting for 2 h at room temperature, the solution was filtered, and the filter cake was washed with EA (50 mL×2). The filtrate was combined and poured into water (300 mL). Then the mixture was extracted with EA (200 mL×2). The organic phases was combined, washed once with water, and washed once with saturated brine. The organic phase was dried over anhydrous sodium sulfate, filtered and concentrated. The crude product was dissolved in EA/PE (1/150 mL), stirred for 30 minutes, filtered and dried. 5.9 g of white solid was obtained, 47% yield. LCMS: [M+H]⁺=235.2

### Step 2: Preparation of 7-bromo-3-iodoimidazole [1,2-a] pyridine:

The raw material of 7-bromo-imidazole [1,2-a] pyridine (11.4 g, 54.9 mmol) was dissolved in DMF (160 mL), and NIS (15.6 g, 69.4 mmol) was added at 25 °C. The resulting mixture was stirred at 100 °C for 1h. After reaction was completed, the solution was poured into water (500 mL), then extracted with EA (200 mL×2). The organic phases were combined, washed once with water (200 mL), and then washed once with saturated brine (200 mL). The organic phase was dried over anhydrous sodium sulfate, filtered and concentrated. The crude product was purified by column chromatography using EA/PE (0~5%) to obtain 7-bromo-3-iodoimidazole [1,2-a] pyridine as a black-brown solid (14.1 g, 75% yield). LCMS: [M+H]⁺=322.9.

### Step 3: preparation of 1-(7-bromoimidazo[1,2-a]pyridin-3-yl)-3-(4-methoxybenzyl) dihydropyrimidine-2,4 (1H, 3H) - dione

The raw materials 3-(4-methoxybenzyl)dihydropyrimidine-2,4(1H, 3H)-dione (6g, 25.1 mmol) and 7-bromo-3-iodoimidazole [1,2-a] pyridine were dissolved in 1,4-dioxane (150 mL), and Cs₂CO₃ (16.7 g, 51.2 mmol), CuI (980 mg, 5.22 mmol), and (1R,2R)-N1, N2-dimethylcyclohexane-1,2-diamine (730 mg, 5.23 mmol) were added at 25°C. The mixture was stirred for 16 hours under a nitrogen atmosphere at 80 °C. After the reaction was completed, the mixture was poured into water (300 mL) and extracted with EA (150 mL×2). The organic phases were combined and washed once with water (200mL), and then washed once with saturated brine (200 mL). The organic phase was dried over anhydrous sodium sulfate, filtered and concentrated. The crude product was separated by column chromatography using EA/PE (1/10 to 1/0) to obtain 1-(7-bromoimidazo [1,2-a] pyridin-3-yl) -3- (4-methoxybenzyl) dihydropyrimidine-2,4 (1H, 3H) - dione as a yellow solid (3.1 g, 29% yield). LCMS: [M+H]⁺=429.0.

### Step 4: preparation of 1- (7-bromoimidazo[1,2-a]pyridin-3-yl)dihydropyrimidine-2,4(1H, 3H)-dione

The raw material 1-(7-bromoimidazo[1,2-a]pyridin-3-yl)-3-(4-methoxybenzyl) dihydropyrimidine-2,4 (1H, 3H)-dione (3.1 g, 7.48 mmol) was dissolved in TfOH (2.3 mL), and the solution was reacted for 4 h at 65 °C. After the reaction was completed, the mixture was concentrated. The pH value of the mixture was adjusted from 6 to 7 with TEA at 0 °C. The mixture was further concentrated, and EA (30ml) was added and stirred for half an hour. 1-(7-bromoimidazo [1,2-a] pyridin-3-yl) dihydropyrimidine-2,4 (1H, 3H) - dione (2.12 g, 76% yield) as a white solid was obtained after filtration and drying. LCMS: [M+H]⁺=309.0.

### Preparation of E3L-4: 1-(8-bromoimidazo[1,2-a] pyridin-3-yl)dihydropyrimidine-2,4(1H, 3H)-dione:

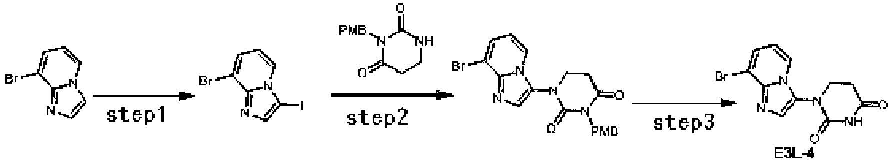

### Step 1: preparation of 8-bromo-3-iodoimidazole[1,2-a] pyridine:

The raw material 8-bromo-imidazole[1,2-a]pyridine (10 g, 50.1 mmol) was dissolved in DMF (150 mL), and NIS (13.6 g, 60.2 mmol) was added at 25 °C. The mixture was stirred at 100 °C for 1h. After the reaction was completed, the solution was poured into water (400 mL), then extracted with EA (200 mlx2). The organic phases were combined, washed once with water (200 ml), and then washed once with saturated brine (200 mL). The organic phase was dried over anhydrous sodium sulfate, filtered and concentrated. The crude product was separated by column chromatography using EA/PE (5%) to obtain 8-bromo-3-iodoimidazole [1,2-a] pyridine as a black brown solid (12.2 g, 77%yield).

LCMS: [M+H]⁺=322.9.

### Step 2: preparation of 1-(8-bromoimidazo[1,2-a]pyridin-3-yl)-3-(4-methoxybenzyl) dihydropyrimidine-2,4 (1H, 3H) - dione:

The raw material 3- (4-methoxybenzyl) dihydropyrimidine-2,4 (1H, 3H) - dione (4.4 g, 18.65 mmol) and 8-bromo-3-iodoimidazole [1,2-a] pyridine were dissolved in 1,4-dioxane (100 mL), and then Cs₂CO₃ (11.1 g, 34.1 mmol), CuI (705 mg, 3.71 mmol) and (1R, 2R)-N1, N2-dimethylcyclohexane-1,2- diamine (522 mg, 3.72 mmol) were added at 25 °C. Under the protection of nitrogen, the reaction was conducted at 80 °C for 16 h. After the reaction was completed, the solution was poured into water (200 mL), then extracted with EA (100 mL×2). The organic phases were combined, washed once with water (200 mL), and washed once with saturated brine (200 mL). The organic phase was dried over anhydrous sodium sulfate, filtered and concentrated. The crude product was separated by column chromatography using EA/PE (1/10 to 1/0) to obtain 1- (8-bromoimidazo [1,2-a]pyridin-3-yl)-3-(4-methoxybenzyl) dihydropyrimidine-2,4 (1H, 3H) - dione as a yellow solid (2.3 g, 29% yield). LCMS: [M+H]⁺=429.

### Step 3: preparation of 1-(8-bromoimidazo[1,2-a]pyridin-3-yl)dihydropyrimidine-2,4(1H, 3H)- dione

The raw material 1-(8-bromoimidazo[1,2-a]pyridin-3-yl)-3-(4-methoxybenzyl) dihydropyrimidine-2,4 (1H, 3H) - dione (2.3 g, 5.36 mmol) was dissolved in TfOH (1.5 mL), and the mixture was stirred at 65 °C for 4 h. After the reaction was completed, the mixture was concentrated. The pH value of the mixture was adjusted to 6-7 with TEA at 0 °C. The mixture was further concentrated, and EA (30 mL) was added and stirred for half an hour. 1-(8-bromoimidazo[1,2-a] pyridin-3-yl) dihydropyrimidine-2,4 (LCMS: [M+H]⁺=309.0) - dione as a white solid was obtained after filtration and drying. LCMS: [M+H]⁺=309.0.

### Preparation of E3L-5: 3-(4-bromo-3-methyl-2-oxo-2,3-dihydro-1h-benzo[d]imidazol-1-yl) piperidine-2,6-dione

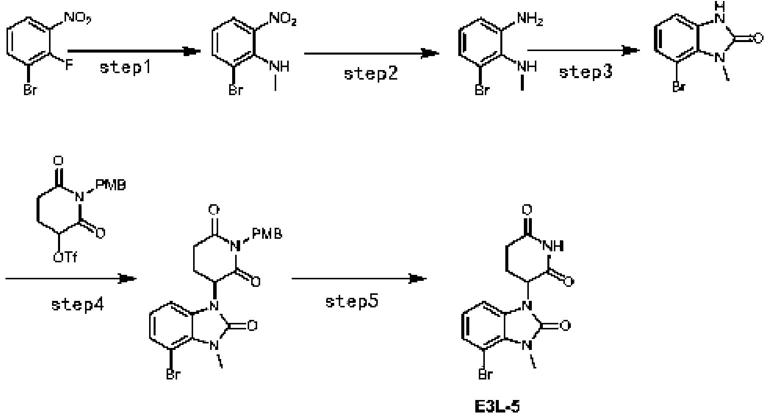

### Step 1: preparation of 2-bromo -N-methyl-6-nitroaniline

The raw material 1-bromo-2-fluoro-3-nitrobenzene (20 g, 91 mmol) was dissolved in THF (20 mL), and then methylamine (2 M, 200 mL) was added. The reaction was conducted at 60 °C for 12 h. After the reaction was completed, the mixture was poured into a solution of NaHCO₃ (20 mL) in water. The mixture was extracted with EA (100 mL×3). The organic phases were combined, and washed with saturated brine (2×100 mL). The organic phase was dried over anhydrous sodium sulfate, then filtered and concentrated. 2-bromo-n-methyl-6-nitroaniline as a red oil was obtained (20 g, 94% yield). LCMS(ESI), m/z: [M+H]⁺=230.9

### Step 2: preparation of 6-bromo-N1-toluene-1,2-diamine

The raw material 2-bromo-N-methyl-6-nitroaniline (11 g, 48 mmol) was dissolved in EA (150 mL), water (5 mL) and AcOH (50 mL). The mixture was heated to 50 °C, and then iron powder (12.2 g, 201 mmol) was added at this temperature. The mixture was then heated to 80 °C and continuously stirred for 4 hours. After the reaction was completed, the mixture was filtered. 100 mL of water was added into the mixture. The resulting mixture was extracted with EA (100 mL x 3). The organic phases were combined, washed once with water (200 mL), and washed once with saturated brine (200 mL). The organic phase was dried with anhydrous sodium sulfate, filtered and concentrated. 6-bromo-N1-methylbenzene-1,2-diamine as a crude red oil (10 g, yield:98%) was obtained. LCMS: [M+H]⁺=201.1.

### Step 3: preparation of 7-bromo-1-methyl-1,3-dihydro-2H-benzo [d] imidazole-2-one:

The raw material 6-bromo-N1-methylbenzene-1,2-diamine (10 g, 98 mmol) was dissolved in MeCN (150 mL), and then CDI (16 g, 98 mmol) was added. The reaction was conducted under the protection of nitrogen at 85 °C for 12 h. After the reaction was completed, the solvent was removed by rotary evaporation. The mixture was poured into water (100 mL). The solid precipitate was collected and washed with water to obtain 7-bromo-1-methyl-1,3-dihydro-2H-benzo[d]imidazole-2-one as a white solid (10.5 g, 89% yield). LCMS: [M+H]⁺=227.0.

### Step 4: preparation of 3-(4-bromo-3-methyl-2-oxo-2,3-dihydro-1H-benzo[d]imidazol-1-yl)-1-(4-methoxybenzyl) piperidine-2,6-dione:

The raw material 7-bromo-1-methyl-1,3-dihydro-2H-benzo[d]imidazole-2-one (12 g, 27 mmol) was dissolved in THF (300 mL), and then t-BuOK (3.6 g, 32 mmol) was added. The mixture was reacted at 0 °C for 0.5 h. A solution of 1- (4-methoxybenzyl) - 2,6-dioxypiperidine-3-yltrifluoromethanesulfonate (10.1 g, 27 mmol) in THF (50 mL) was added slowly into the mixture. The mixture was reacted for half an hour at 20 °C. After the reaction was completed, the reaction was quenched by adding saturated ammonium chloride aqueous solution (50 mL). The mixture was extracted with EA (100 mL). The organic phases were combined, washed with saturated brine, dried, filtered and concentrated. The crude product was purified by reverse HPLC (0.1%TFA), and 3-(4-bromo-3-methyl-2-oxo-2,3-dihydro-1H- benzo[d]imidazol-1-yl)-1-(4-methoxybenzyl) piperidine-2,6-dione was obtained as a yellow solid (6.8 g, yield 56%).

### Step 5: preparation of 4-(4-bromo-3-methyl-2-oxo-2,3-dihydro-1H-benzo[d]imidazol-1-yl) piperidine-2,6-dione:

The raw material 3-(4-bromo-3-methyl-2-oxo-2,3-dihydro-1H-benzo[d]imidazol-1-yl)-1-(4-methoxybenzyl)piperidine-2,6-dione (6.8 g, 13.6 mmol) was dissolved in TfOH (4.8 mL), and reacted at 65 °C for 4 h. After the reaction was completed, the solution was concentrated. The pH value of the mixture was adjusted to 6-7 with TEA at 0 °C. The resulting mixture was concentrated, and EA (100 mL) was added and stirred for half an hour. 4-(4-bromo-3-methyl-2-oxo-2,3-dihydro-1H-benzo [d] imidazol-1-yl) piperidine-2,6-dione as a white solid was obtained by filtration and drying.( 4.55 g, yield:81%). LCMS: [M+H]+=309.0.

### Preparation of E3L6: 3- (1-oxo-3,5,6,7-tetrahydropyrrole[3,4-f]isoindole-2(1H)-yl) piperidine-2,6-dione

### Preparation of 1- (4-methoxybenzyl) - 2,6-dioxypiperidine-3-yltrifluoromethanesulfonate:

To a solution of 3-hydroxy-1 - (4-methoxybenzyl) piperidine-2,6-dione (43.0 g, 173 mmol) in DCM (500 mL), pyridine (27.3 g, 345 mmol) was added. The mixture was cooled to 0 °C before trifluoromethanesulfonic anhydride (73.0 g, 258.74 mmol) was added. The mixture was stirred at 0 °C for 2 hours under the protection of nitrogen. After the reaction was completed, the solvent was removed by concentration. The crude product was separated by column chromatography to obtain 1- (4-methoxybenzyl) - 2,6-dioxypiperidine-3- yltrifluoromethanesulfonate as a light yellow solid (45.0 g, 68% yield). LCMS(ESI):m/z 381.32 [M+H]+.

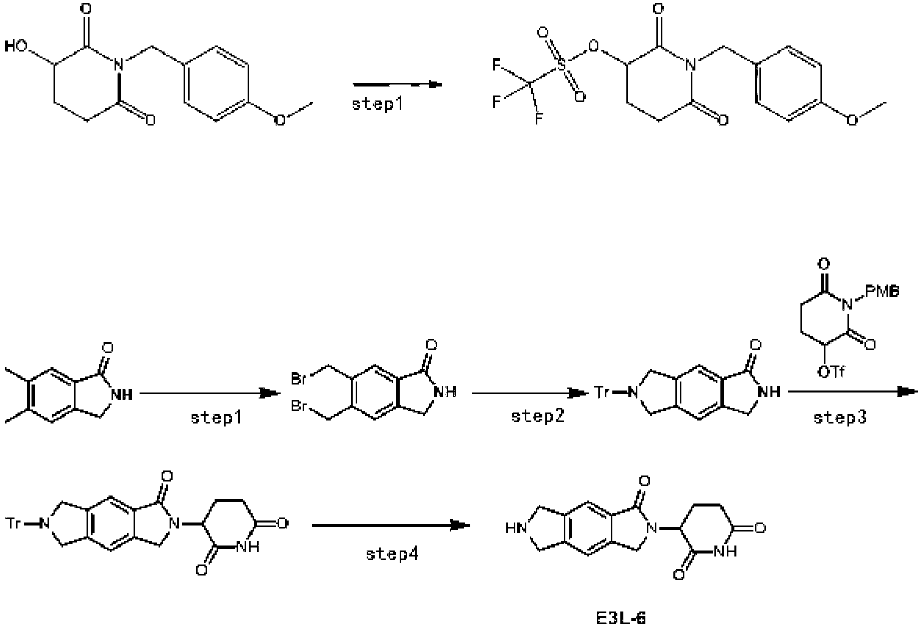

### Step 1: preparation of 5,6-bis (bromomethyl) isoindolin-1-one

5,6-dimethylisoindolin-1-one (9.7 g, 60 mmol) and NBS (32.2 g, 180 mmol) were dissolved in chloroform (120 ml). The mixture was heated to 80 °C, before dibenzoyl peroxide (336 mg, 1.2 mmol) was added. The reaction was carried out at 80 °C for 8 h. TLC (PE:EA = 5:1, Rf = 0.4) was used to monitor reaction. The mixture was cooled to room temperature after the reaction was completed. After filtration, the filtrate was washed with saturated NaHCO₃ aqueous solution (50 ml) and brine (50 ml), dried over MgSO₄ and concentrated. The organic phase was concentrated by rotary evaporation to obtain a crude of 5,6-bis (bromomethyl) isoindolin-1-one (11.44 g, 60%). LCMS: [M+H]⁺=317.91.

### Step 2: preparation of 6-triphenylmethyl-3,5,6,7-tetrahydropyrrole [3,4-f] isoindole-1 (2H) -one

DIEA (16 mL, 92 mmol) was added into a solution of 5,6-bis (bromomethyl) isoindolin-1-one (11.44 g, 36 mmol) in DMF (100 mL) at room temperature, and then triphenylamine (9.4 g, 36 mmol) was added. The mixture was stirred at 60 °C for 4 h, and then H₂O (400 mL) was poured into the mixture. The mixture was stirred vigorously for 15 min. The precipitate was collected by filtration and rinsed with H₂O (200 mL×2). The combined filtrate was dissolved in EA and extracted with EA (3×200 mL). The combined organic phase was washed with water (3×50 mL) and brine (2×50 mL), dried over MgSO₄ and concentrated. 6-triphenylmethyl-3,5,6,7-tetrahydropyrrole [3,4-f] isoindole-1 (2H)-one as a white solid (8.1 g, 54 %yield) was obtained after being purified by flash column chromatography (silica gel, DCM EtOAc 50:1 to 20:1). LCMS: [M+H]⁺=417.19.

### Step 3: preparation of 3- (1-oxo-6-triphenylmethyl-3, 5, 6,7-tetrahydropyrrole[3,4-f] isoindole-2 (1H) - yl) piperidine-2,6-dione:

The raw material 6-triphenylmethyl-3,5,6,7-tetrahydropyrrole[3,4-f]isoindole-1(2H)-one (8 g, 19.2 mmol) was dissolved in THF (60 mL), and then t-BuOK (2.6 g, 23 mmol) was added. The reaction was carried out at 0 °C for 0.5 h. Then a solution of 1-(4-methoxybenzyl)-2,6-dioxypiperidine-3-yltrifluoromethanesulfonate (8.6 g, 23 mmol) in THF (30 mL) was added . The mixture was stirred at 20 °C for half an hour. After the reaction was completed, the reaction was quenched by adding saturated ammonium chloride aqueous solution (80 mL). The mixture was extracted with EA (200 mL). The combined organic phase was washed with saturated brine, dried, then filtered and concentrated. The crude product was purified by reverse HPLC (0.1%EA) to give 3- (1-oxo-6-triphenylmethyl-3,5,6,7-tetrahydropyrrole [3, 4-f] isoindole-2 (1H) -yl) piperidine-2,6-dione as a yellow solid (5.66 g, 56% yield). LCMS: [M+H]⁺=528.22.

### Step 4: preparation of 3-(1-oxo-3,5,6,7-tetrahydropyrrole[3,4-f]isoindole-2(1H)-yl) piperidine-2,6-dione

3-(1-oxo-6-triphenylmethyl-3,5,6,7-tetrahydropyrrole[3,4-f]isoindole-2(1H)-yl)piperidine-2,6-dione (5.66 g, 10.8 mmol) was dissolved in TfOH (8 mL), and the mixture was reacted at 65 °C for 4 h. After the reaction was completed, the solution was concentrated. The pH value of the mixture was adjusted to 6 to 7 with TEA at 0 °C. The mixture was further concentrated, and then EA (200 mL) was added and stirred for half an hour at room temperature.

3-(1-oxo-3,5,6,7-tetrahydropyrrole[3,4-f]isoindole-2 (1H)-yl)piperidine-2,6-dione as a white solid (2.2 g, 72% yield) was obtained by filtration and drying. LCMS: [M+H]+=286.11.

### Preparation of E3L-7: 2-(2,6-dioxypiperidin-3-yl)- 6,7-dihydropyrrole [3,4-f] isoindole-1,3 (2H, 5H) - dione

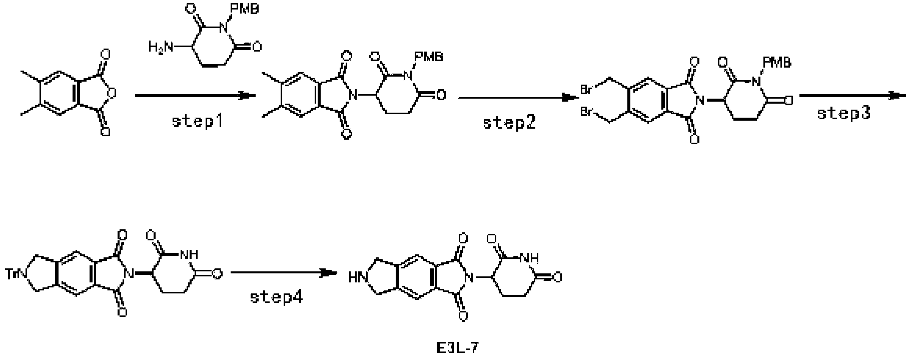

### Step 1: preparation of 2-(1-(4-methoxybenzyl)-2,6-dioxypiperidin-3-yl)-5,6-dimethyl isoindoline-1,3-dione:

To a solution of 5,6-dimethyl isobenzofuran-1,3-dione (3.52 g, 20 mmol) in acetic acid solution(40 mL), sodium acetate (3.28 g, 40 mmol) and 3-amino-1- (4-methoxybenzyl) piperidine-2,6-dione (4.96 g, 20 mmol) were added at room temperature. The mixture was heated to 100 °C and stirred for 12 h. The reaction mixture was concentrated under reduced pressure to remove most of the acetic acid. The residue was poured into water (200 mL) and stirred for 15 min. After filtration, the filter cake was washed with water (50 mL×2) and dried to obtain 2-(1-(4-methoxybenzyl)-2,6-dioxypiperidin-3-yl)- 5,6-dimethylisoindolin-1,3-dione as a grey white solid (6.51 g, 80% yield). LCMS: [M+H]⁺= 407.

### Step 2: 5,6-bis(bromomethyl)-2-(1-(4-methoxybenzyl)-2,6-dioxypiperidin-3-yl) isoindolin-1,3-dione

2-(1-(4-methoxybenzyl)-2,6-dioxypiperidin-3-yl)-5,6-dimethylisoindolin-1,3-dione (6.51 g, 16 mmol) and NBS (8.58 g, 48 mmol) were dissolved in chloroform (50 mL), and the mixture was heated to 80 °C, before dibenzoyl peroxide (112 mg, 0.4 mmol) was added. The mixture was stirred at this temperature for 8 h. After the reaction was completed, the mixture was cooled to room temperature. After filtration, the combined filtrate was washed with saturated NaHCO₃ aqueous solution (50 mL) and brine (50 mL), dried with MgSO₄, and concentrated by rotary evaporation to obtain a crude product of 5,6-bis(bromomethyl)-2-(1- (4-methoxybenzyl)-2,6-dioxypiperidin-3-yl)isoindolin-1,3-dione (4.95 g, 55%). LCMS: [M+H] ⁺=563.

### Step 3: preparation of 2-(2,6-dioxypiperidin-3-yl)-6-triphenylmethyl-6,7-dihydropyrrole[3,4-f] isoindole-1,3 (2H, 5H) - dione

DIEA (4.6 ml,26.4 mmol) was added into a solution of 5,6-bis(bromomethyl)-2- (1-(4-methoxybenzyl)-2,6-dioxypiperidin-3-yl)isoindolin-1,3-dione (4.95 g, 8.8 mmol) in DMF (50 mL), and then triphenylmethylamine (2.3 g, 8.8 mmol) was added. The reaction mixture was stirred at 60 °C for 4 h, and then poured into H₂O (200 mL) and stirred vigorously for 15 min. The precipitate was filtered out and rinsed with H₂O (100 mL×2). The combined filtrate was extracted with EA (3×100 mL). The resulting organic layer was washed with saturated brine (2×50 mL), dried with MgSO₄ and concentrated. The residue was purified by flash column chromatography (silica gel, DCM formula EA 50:1 to 20:1) to obtain 2- (2,6-dioxypiperidin-3-yl) - 6-triphenylmethyl-6,7-dihydropyrrole [3,4-f] isoindole-1,3 (2H, 5H) - dione (2.14 g, 45% yield). LCMS: [M+H]⁺=542.2.

### Step 4: preparation of 2- (2, 6-dioxypiperidin-3-yl) - 6,7-dihydropyrrole [3,4-f] isoindole-1,3 (2H, 5H) - dione:

The raw material of 2-(2,6-dioxypiperidin-3-yl)-6-triphenylmethyl-6,7-dihydropyrrole[3,4-f] isoindole-1,3 (2H, 5H)- dione (2.14 g, 3.96 mmol) was dissolved in TfOH (3.3 mL). The mixture was stirred at 65 °C for 4 h. After the reaction was completed, the solution was concentrated. The pH value of the residue was adjusted to 6 to 7 with TEA at 0 °C. The mixture was further concentrated. EA (50 mL) was added to the residue, and stirred for half an hour. 2-(2, 6-dioxypiperidin-3-yl)- 6,7-dihydropyrrole [3,4-f] isoindole-1,3 (2H, 5H) - dione as a white solid (416 mg, 35%) was obtained by filtration and drying. LCMS: [M+H]⁺=300.1.

### Preparation of E3L-8 piperidine hydrochloride: 1-(1-methyl-6-(piperidin-4-yl)-1H-indol-3-yl) dihydropyrimidine-2,4 (1H, 3H) - dione hydrochloride:

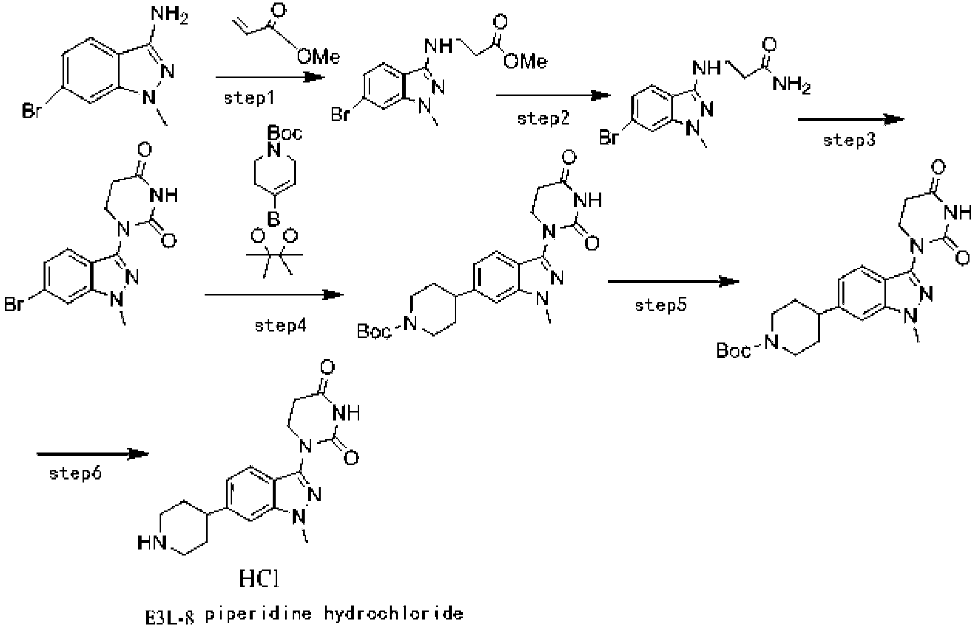

### Step 1: preparation of methyl 3-[(6-bromo-1-methyl-1H-indazole-3-yl) amino] propionate:

6 - Bromo - 1 - methyl - 1H - indazole - 3 - amine (3 g, 13.2 mmol) was prepared by mixing an equimolar mixture of DBU and lactic acid at room temperature with stirring for 16 hours. At 80 °C, methyl acrylate (9.9 g, 98 mmol) was added in 5 portions (2.0 g each at 24 - hour intervals) into a mixture containing the prepared 6 - bromo - 1 - methyl - 1H - indazole - 3 - amine(3 g, 13.2 mmol). After the reaction was completed, the mixture was quenched by adding sodium hypochlorite (30% aqueous solution, 5 mL). The mixture was extracted with ethyl acetate three times. The organic phases were combined, washed with water and brine, dried over anhydrous sodium sulfate, filtered and concentrated under reduced pressure. The residue was purified by silica gel chromatography (EA/PE=1:1) to give methyl 3-[(6-bromo-1-methyl-1H-indazole-3-yl) amino] propionate (2.0 g, yield 45%). LCMS: [M+H]⁺=327.12.

### Step 2: preparation of 3-[(6-bromo-1-methyl-1H-indazole-3-yl) amino] propionamide:

Methyl 3- ((6-bromo-1-methyl-1H-indazole-3-yl)amino) propionate (2.0 g, 6.1 mmol) was dissolved in a solution of ammonia in methanol (20 mL, 5 mol/L). The mixture was stirred for 24 h at room temperature. After the reaction was completed, the mixture was concentrated under reduced pressure to obtain 3-[(6-bromo-1-methyl-1H-indazole-3-yl)amino] propionamide (1.63 g, 90%) as a crude product, which was directly used in the next reaction. LCMS: [M+H]⁺=297.

### Step 3: preparation of 1-(6-bromo-1-methyl-1H-indazole-3-yl) dihydropyrimidine-2,4(IH, 3H) - dione:

Triphosgene (780 mg, 2.8 mmol) and DIEA (1.44 g, 11.2 mmol) were added to a solution of 3-((6-bromo-1-methyl-1H-indazole-3-yl) amino) propanamide (1.63 g, 5.59 mmol) in THF (30 mL). The mixture was stirred at 80 °C for 12h. After the reaction was completed, the mixture was cooled to room temperature, and quenched by adding saturated NH₄Cl aqueous solution (50 mL). The mixture was extracted with EA (50 mL×3), and the organic phases were combined. The combined organic phase was washed with water and brine, dried over anhydrous sodium sulfate, filtered and concentrated under reduced pressure. The residue was purified by silica gel chromatography (EA/PE=1:1) to give 1- (6-bromo-1-methyl-1H-indazole-3-yl) dihydropyrimidine-2,4 (1H, 3H) - dione (1.26 g, 70% yield). LCMS: [M+H]⁺=324.

### Step 4: preparation of tert-butyl 4-[3-(2,4-dioxotetrahydropyrimidin-1(2H)-yl)-1-methyl-1H-indazole-6-yl] - 3,6-dihydropyridine-1 (2H) - carboxylate

Cesium fluoride (1.20 g, 7.76 mmol) and Pd(dppf)Cl₂ (570 mg, 776 µmol) were added into a solution of 1-(6-bromo-1-methyl-1H-indazole-3-yl)dihydropyrimidine-2,4(1H, 3H)-dione (1.26 g, 3.88 mmol) and 4-(4,4,5,5-tetramethyl-1,3,2-dioxaborenyl-2-yl)-3,6-dihydropyridine-1(2H)-tert-butyl formate (2.43 g, 7.76 mmol) in 1,4-dioxane (20 mL) under a nitrogen atmosphere. The mixture was stirred at 85 °C for 3 h. The mixture was cooled to room temperature, diluted with ethyl acetate (50 mL) and filtered through diatomite/silica gel. After washing with ethyl acetate (20 mL), the filtrate was washed with water to separate the fractions. The combined organic layer was dried over Na₂SO₄, filtered and concentrated. The residue was purified by silica gel chromatography (EA:PE=10:1) to give tert-butyl 4-[3-(2,4-dioxotetrahydropyrimidin-1(2H)- yl) - 1-methyl-1H-indazole-6-yl] - 3,6-dihydro-pyridin-1(2H) - carboxylate (990 mg, 2.44 mmol, yield 60%). LCMS: [M+H]⁺=426.3.

### Step 5: preparation of tert-butyl 4-[3-(2,4-dioxotetrahydropyrimidin-1(2H)-yl)-1-methyl-1H-indazole-6-yl] piperidine-1-carboxylate:

Palladium (10%/carbon, 1.0g, 0.94mmol) was added into a solution of tert-butyl 4-[3-(2,4-dioxotetrahydropyrimidin-1 (2H)-yl) -1-methyl-1H-indazole-6-yl]-3,6-dihydro-pyridine-1 (2H) - carboxylate (990 mg, 2.32mmol) in methanol (20ml). The mixture was stirred under a hydrogen atmosphere for 24 hours at room temperature, and then filtered through diatomite. The filter cake was washed with dichloromethane (20 mL×3). The combined filtrate was concentrated under reduced pressure to obtain t-butyl 4-[3-(2,4-di oxytetrahydropyrimidin-1(2H)- yl)-1-methyl -1H-indazole-6-yl] piperidine-1-carboxylate (829 mg, 95% yield). LCMS: [M+H]⁺=427.

### Step 6: preparation of 1- (1-methyl-6-(piperidin-4-yl)-1H-indazole-3-yl) dihydropyrimidine-2,4 (1H, 3H)-dione hydrochloride:

Tert-butyl 4-[3-(2,4-dioxotetrahydropyrimidin-1(2H)-yl)-1-methyl-1H-indazole-6-yl] piperidine-1-carboxylate (829 mg, 2.2 mmol) and an aqueous solution of hydrochloric acid (4 M, 2.6 mL) were stirred in ethyl acetate (10 mL) overnight. The resulting mixture was concentrated under reduced pressure. The residue was washed and mashed in EA (5×20mL). 1- (1-methyl-6 -(piperidin-4-yl) - 1H-indazole-3-yl) dihydropyrimidine-2,4 (1H, 3H) - dione hydrochloride (720 mg, yield 90%) was obtained. LCMS: [M+H]⁺=328.2.

### E3L-9 difluoropyridine hydrochloride:

1-(6-(3,3-difluoropyridine-4-yl)-1-methyl-1H-indazole-3-yl)dihydropyrimidine-2,4(1H,3H)- dione hydrochloride was synthesized according to the embodiment of E3L-8 difluoropyridine hydrochloride. LCMS: [M+H]⁺=364.1.

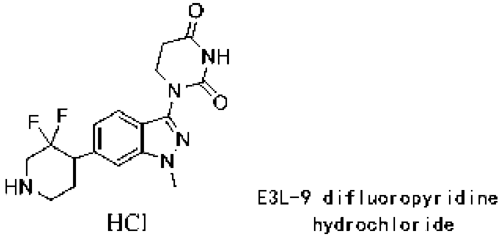

E3L-10 piperazine hydrochloride: 1-(6-(3,3-difluoropiridin-4-yl)-1-methyl-1H-indazole-3-yl) dihydropyrimidine-2,4(1H, 3H)-dione hydrochloride was synthesized according to the embodiment of E3L-8, LCMS: [M+H]⁺=347.

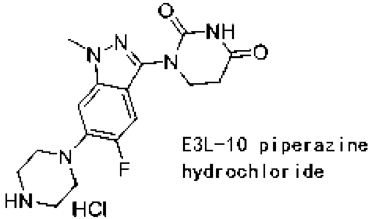

### Preparation of E3L-11 piperazine hydrochloride: 2-(2,6-dioxopiridin-3-yl)-2-fluoro-4 - (piperazine-1-yl) benzamide hydrochloride

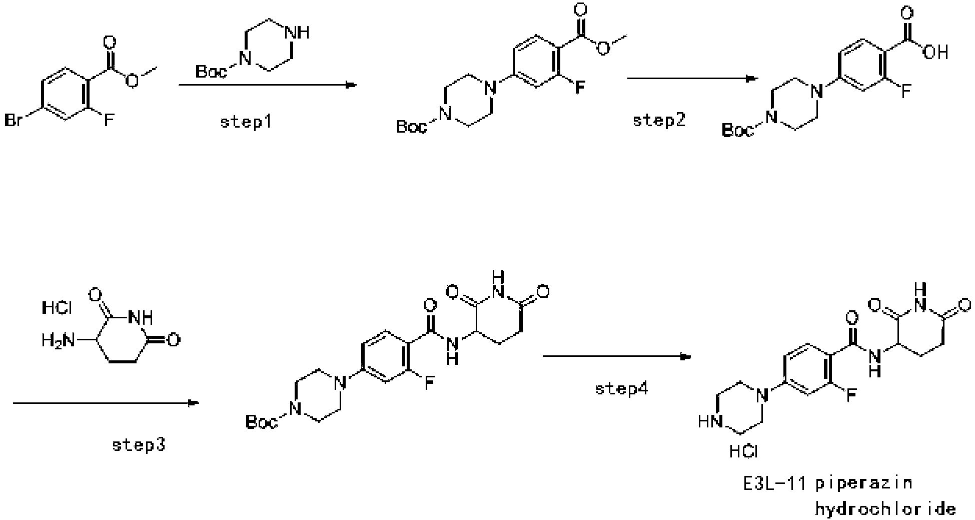

### Step1: preparation of 1-tert-butoxycarbonyl-4 - (3-fluoro-4-methoxyformylphenyl) piperazine

Methyl 2-fluoro-4-bromobenzoate (3 g, 12.9 mmol) was dissolved in toluene (30 mL), and 1-boc-piperazine (2.87 g, 15.5 mmol), palladium acetate (145 mg, 0.645 mmol), BINAP (803 mg, 1.29 mmol), and cesium carbonate (8.46 g, 25.8 mmol) were added into the solution. The mixture was stirred at 80 °C overnight under a nitrogen atmosphere. After the reaction was completed, the mixture was cooled to room temperature. The solvent was removed by rotary evaporation. 100 mL of ethyl acetate was added into the residue. The mixture was washed with water and brine, and dried with anhydrous sodium sulfate, filtered and concentrated under reduced pressure. The residue was purified by silica gel chromatography (EA/PA=1:5) to give 1-tert-butoxycarbonyl-4 - (3-fluoro-4-methoxyformylphenyl) piperazine (3.0 g, yield 69%). LCMS: [M+H]⁺=339.

### Step 2: preparation of tert-butyl 4- (3-fluoro-4-carboxyphenyl) piperazine-1-carboxylate:

1-tert-butoxycarbonyl-4- (3-fluoro-4-methoxyformylphenyl) piperazine (3.0 g, 8.88 mmol) was dissolved in THF (20 mL), and an aqueous solution of lithium hydroxide (852 mg, 35.5 mol) was added. The mixture was stirred at room temperature for 2 h. After the reaction was completed, the pH value of the mixture was adjusted to 6 to 7 by adding an aqueous solution of HCl (1M). The mixture was extracted with EA (50 mL×3). The organic phases were combined, washed with brine, dried over Na₂SO₄ and concentrated under reduced pressure. The residue was purified by silica gel chromatography (EA/PE=1:1) to give tert-butyl 4- (3-fluoro-4-carboxyphenyl) piperazine-1-carboxylate (2.5 g, 87%). LCMS: [M+H]⁺=352.

### Step 3: preparation of 2- (2,6-dioxopiridin-3-yl) -2-fluoro-4- (piperazine - (tert-butyl 4-carboxylate) - 1-yl) benzamide:

Tert-butyl 4- (3-fluoro-4-carboxyphenyl) piperazine-1-carboxylate (2.5 g, 7.72 mmol) was dissolved in DMF (30 mL), and 3-amino-2,6-piperidinedione hydrochloride (1.27 g, 7.72 mmol), HATU (4.4 g, 11.58 mmol), and DIEA(2.99 g, 23.16 mmol) were added. The mixture was stirred at 80 °C for 12 h. After the reaction was completed, saturated NH₄Cl aqueous solution (50 mL) was added to quench the reaction. The mixture was extracted with EA (50 mL×3). The organic phases were combined, washed with water and brine, and dried over anhydrous sodium sulfate, filtered and concentrated under reduced pressure. The residue was purified by silica gel chromatography (EA/PE=1:5) to obtain 2- (2,6-dioxopiridin-3-yl)- 2-fluoro-4-(piperazine-(tert-butyl 4-carboxylate)-1-yl)benzamide hydrochloride (1.7 g, 75% yield). LCMS: [M+H]⁺=435.

### Step 4: preparation of 2-(2,6-dioxopiridin-3-yl)-2-fluoro-4-(piperazine-1-yl)benzamide hydrochloride:

2- (2,6-dioxopiridin-3-yl)-2-fluoro-4-(piperazine - (tert-butyl 4-carboxylate)-1-yl) benzamide (1.7 g, 3.92 mmol) was dissolved in ethyl acetate (25 mL), and a solution of ethyl acetate in hydrochloric acid was added. The mixture was stirred at room temperature for 1 h. After the reaction was completed, the solvent was removed under reduced pressure. The residue was washed twice with ether and dried to obtain 2- (2,6-dioxopiridin-3-yl)-2-fluoro-4- (piperazin-1-yl) benzamide (1.1 g, yield 84.6%). LCMS: [M+H]⁺=335.

E3L-12 piperazine hydrochloride: N- (2,6-dioxopiridin-3-yl) -4- (piperazine-1-yl) benzamide hydrochloride was synthesized according to the embodiment of E3L-11. LCMS: [M+H]⁺=317.

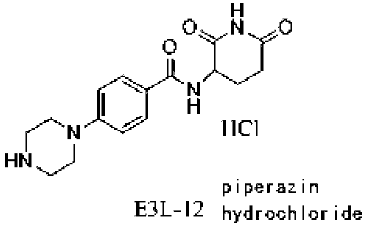

E3L-13 piperazine hydrochloride: N-(2,6-dioxo-piperazine-3-yl)-4-(piperazine-1-yl)-2-pyridinamide hydrochloride was synthesized according to the embodiment of E3L-12. LCMS: [M+H]+=317.

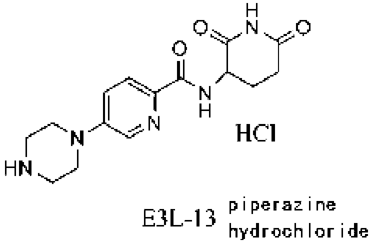

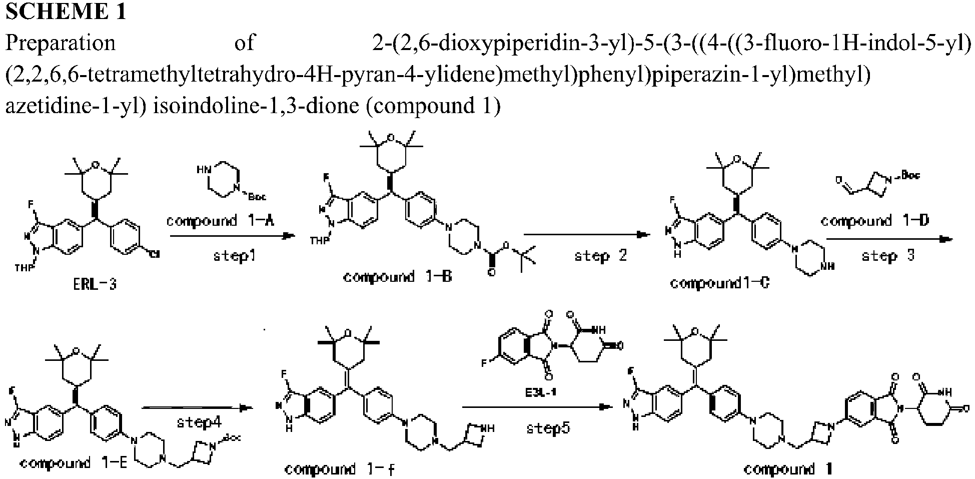

### Step 1: preparation of tert-butyl 4- (4-((3-fluoro-1-(tetrahydro-2H-pyran-4-yl)-1H-indazole-5-yl) (2,2,6,6-tetramethyltetrahydro-4H-pyran-4-ylidene)methyl)phenyl)piperazine-1-carboxylate:

Tert-butyl piperazine-1-carboxylate (173.5 mg, 0.93 mmol), Ruphos(58.0 mg, 0.12 mmol), Ruphos Palladacycle G3 (103.9 mg, 0.12 mmol), t-BuONa (179.1mg, 1.86mmol) and THF(5 mL) were added into a solution (50 mL) of 5- ((4-chlorophenyl) (2,2,6,6-tetramethyltetrahydro-4H-pyran-4-ylidene)methyl)-3-fluoro-1 - (tetrahydro-2H-pyran-4-yl)-1H indazole (300 mg, 0.62 mmol) in THF (50 mL). The resulting mixture was stirred at 85°C overnight. The reaction was quenched by adding water (2 mL) at room temperature. The resulting mixture was extracted with ethyl acetate (3×10 mL). The combined organic layer was dried over anhydrous sodium sulfate. After filtration, the filtrate was concentrated under reduced pressure. The residue was purified by silica gel column chromatography and then eluted with PE/EA (5:1) to obtain a white solid of tert-butyl 4-(4-((3-fluoro-1-(tetrahydro-2H-pyran-4-yl)-1H-indazole-5-yl) (2,2,6,6-tetramethyltetrahydro-4H-pyran-4-ylidene) methyl)phenyl)piperazine-1-carboxylate (145 mg, 36.89%). LCMS(ESI):m/z 633.22 [M+H]⁺.

### Step 2: preparation of 3-fluoro-5-((4-(piperazine-1-yl)phenyl) (2,2,6,6-tetramethyltetrahydro-4H-pyran-4-ylidene)methyl)-1H indazole

TFA (1.5 ml) was added into a solution of tert-butyl 4- (4-((3-fluoro-1-(tetrahydro- 2H-pyran-4-yl)-1H-indazole-5-yl)(2,2,6,6-tetramethyltetrahydro-4H-pyran-4-ylidene)methyl) phenyl) piperazine-1-carboxylate (145 mg, 0.23 mmol) in DCM (0.5 mL). The resulting mixture was stirred at room temperature for 12 h, and then the mixture was concentrated under reduced pressure. The residue was dissolved in ethyl acetate (2 mL). The pH value of the mixture was adjusted to 10 with saturated solution of K₂CO₃. The mixture was extracted with EtOAc (3×2 mL). The combined organic phase was dried over anhydrous sodium sulfate, and filtered. The filtrate was concentrated under reduced pressure. The residue was purified by preparative thin-layer chromatography (DCM/MeOH 5:1) to give 3-fluoro-5-((4-(piperazin-1-yl)phenyl) (2,2,6,6-tetramethyltetrahydro-4H-pyran-4-ylidene) methyl) -1H-indazole as a white solid (80 mg, 77.83%). LCMS(ESI):m/z 449.3 [M+H]⁺.

### Step 3: preparation of tert-butyl 3-((4-(4-((3-fluoro-1H-indazole-5-yl)

### (2,2,6,6-tetramethyltetrahydro -4H-pyran-4-ylidene)methyl)phenyl)piperazin-1-yl)methyl) azetidine-1-carboxylate:

### Tert-butyl 3-formyl monoheterocyclobutane-1-carboxylate (66.1 mg, 0.36 mmol) was added into a solution of 3-fluoro-5-((4-(piperazin-1-yl)phenyl)

(2,2,6,6-tetramethyltetrahydro-4H-pyran-4-ylidene) methyl)-1H indazole (80 mg, 0.18 mmol) in MeOH (0.5 mL), and stirred for 1 h at room temperature. The mixture was cooled to 0 °C before NaBH₃CN (22.4 mg, 0.36 mmol) was added. The resulting mixture was stirred overnight at room temperature. The reaction was quenched by adding water at room temperature. The resulting mixture was concentrated under vacuum. The resulting mixture was extracted with EtOAc (3×2 mL). The combined organic layer was dried over anhydrous sodium sulfate. After filtration, the filtrate was concentrated under reduced pressure. The residue was purified by preparative thin-layer chromatography (PE/EA 1:1) to give tert-butyl 3-((4-(4-((3-fluoro-1H-indazole-5-yl) (2,2,6,6-tetramethyltetrahydro-4H-pyran-4-ylidene)methyl)phenyl)piperazin-1-yl)methyl) azetidine-1-carboxylate as a white solid (80 mg, 72.61%). LCMS(ESI):m/z 618.4 [M+H]⁺.

### Step 4: preparation of 5-((4-(4-(azacyclobutan-3-ylmethyl)piperazin-1-yl)phenyl) (2,2,6,6-tetramethyltetrahydro-4H-pyran-4-ylidene)methyl)-3-fluoro-1H-indazole:

TFA (0.3 mL, 4.04 mmol) was added into a solution of tert-butyl-3-((4-(4- ((3-fluoro-1H-indazole-5-yl) (2,2.6,6-tetramethyltetrahydro-4H-pyran-4-ylidene)methyl)phenyl) piperazin-1-yl)methyl] azetidine-1-carboxylate (80 mg, 0.13 mmol) in DCM (0.5 mL). The resulting mixture was stirred at room temperature for 12 h. The resulting mixture was concentrated under reduced pressure. After filtration, the filtrate was concentrated under reduced pressure to obtain 5- ((4- (4- (azacyclobutan-3-ylmethyl) piperazin-1-yl)phenyl) (2,2,6,6-tetramethyltetrahydro-4H-pyran-4-ylidene)methyl)- 3-fluoro-1H-indazole as a yellow oily liquid (60 mg, 94.63%). The crude product was directly used in the next reaction without further purification. LCMS(ESI):m/z 518.32 [M+H]⁺.

### Step 5: preparation of 2-(2,6-dioxypiperidin-3-yl)-5-(3-(4-(4-(3-fluoro-1H-indol-5-yl) (2,2,6,6-tetramethyltetrahydro-4H-pyran-4-ylidene)phenyl)piperazin-1-yl)methyl) azetidine-1-yl) isoindoline-1,3-dione (compound 1):

To a solution of {4-[4-(azacyclobutan-3-methyl) piperazin-1-yl]phenyl}(2,2,6,6-tetramethoxy-4-ylidene)methyl)- 3-fluoro-1H-indazole (250 mg, 0.48 mmol) in DMSO (4 ml), 2- (2,6-dioxypiperidin-3-yl)-5-fluoroisoindole-1,3-dione (E3L-1) (293.5 mg, 1.06 mmol), and DIEA(312.1 mg, 2.42 mmol) were added. The resulting mixture was stirred at 100 °C for 12 h. The reaction was quenched by adding water (2 mL) at room temperature. The resulting mixture was extracted with ethyl acetate (3×5 mL). The combined organic layers were washed with water (3×3 mL) and dried over anhydrous sodium sulfate. After filtration, the filtrate was concentrated under reduced pressure. The residue was purified by reverse flash chromatography with the following conditions: column: Xselect CSH C18 OBD column 30×150 mm 5 µm, n; mobile phase A: water (0.05% TFA), mobile phase B: ACN; flow rate: 60ml/min; gradient: 24% B to 53% B in 10 min, 53% B; wavelength: 254nm; RT1 (min): 8.63. 2-(2,6-dioxypiperidin-3-yl)- 5-(3-(4-(4-(3-fluoro-1H-indol-5-yl) (2,2,6,6-tetramethyltetrahydro-4H-pyran-4-ylidene)phenyl) piperazin-1-yl)methyl) azetidine-1-yl)isoindole-1,3-dione as a yellow solid (compound 1) was obtained. LCMS (ES, m/z): 774.4[M+H]⁺; (27.8 mg, 7.44%) 1H NMR (DMSO, 400 MHz) δ (ppm): 12.54 (s, 1H), 11.10 (s, 1H), 9.86 (s, 1H), 7.70-7.68 (d, J =8.4 Hz, 1H),7.45-7.41 (m, 2H), 7.21-7.20 (d, J =1.6 Hz, 1H), 7.13-7.11 (d, J =8.4Hz, 2H), 6.97-6.95 (d, J =8.8Hz, 2H), 6.82 (s, 1H), 6.70-6.67 (d, J =10.4Hz, 1H), 5.06(m,1H), 4.28-4.23(m, 2H),3.88-3.82 (m,4H), 3.56-3.54 (s, 4H), 3.31 (m,1H), 3.16-3.14(m, 2H),3.00(m, 3H), 2.60(s, 1H), 2.50-2.49 (d, J =1.6 Hz, 4H), 2.21(m, 1H),1.17-1.12 (d, J =17.2 Hz,12H).

According to the synthetic route of compound 1, the following compounds were respectively synthesized by the following starting compounds or intermediates. (Synthetic steps were referred to above scheme 1, but the molar mass of each starting compound and intermediate was controlled unchanged):

| Compounds No. | Starting material | intermediate | Compounds structure |
|---|---|---|---|
| 4 | ERL-1 | 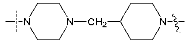 | 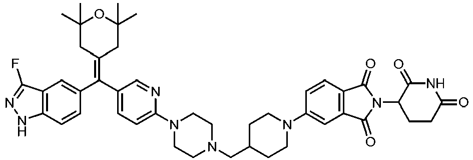 |
| 5 | ERL-4 | 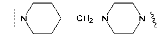 | 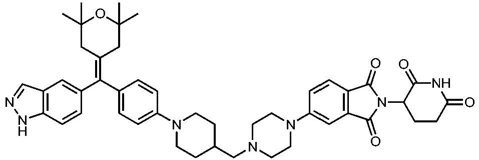 |
| 6 | ERL-3 | 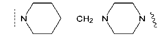 | 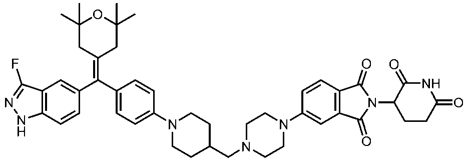 |
| 7 | ERL-1 | 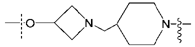 | 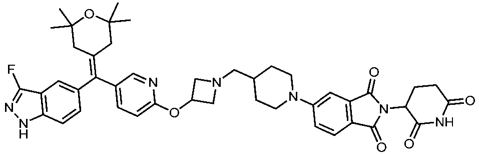 |
| 8 | ERL-3 | 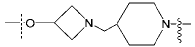 | 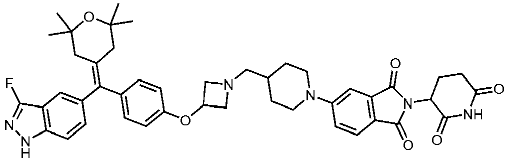 |
| 9 | ERL-5 | 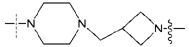 | 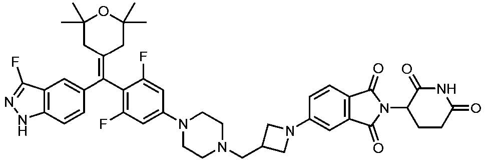 |
| 12 | ERL-5 | 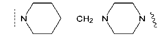 | 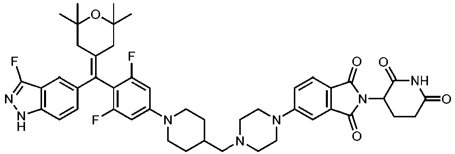 |
| 15 | ERL-5 | 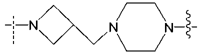 | 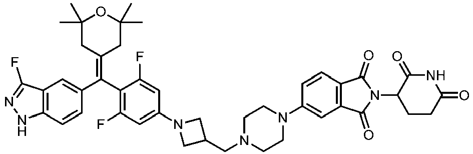 |
| 17 | ERL-3 | 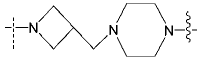 | 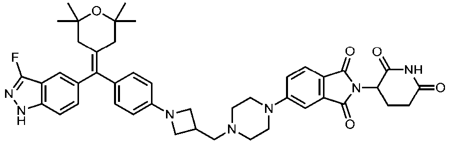 |
| 24 | ERL-5 | 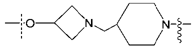 | 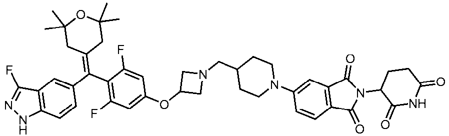 |
| 26 | ERL-5 | 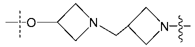 | 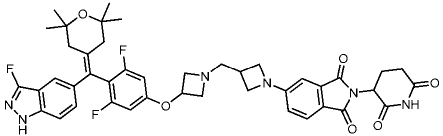 |
| 27 | ERL-1 | 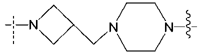 | 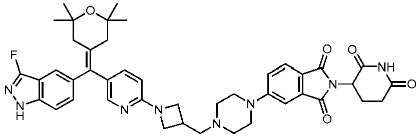 |
| 30 | ERL-1 | 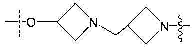 | 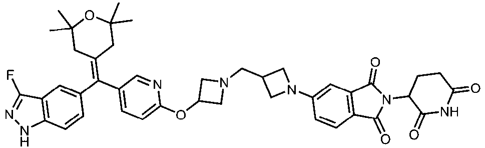 |
| 32 | ERL-3 | 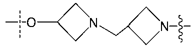 | 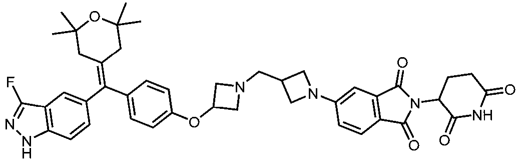 |
| 33 | ERL-3 | 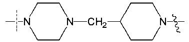 | 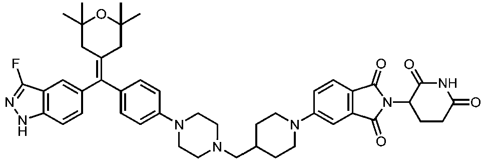 |
| 102 | ERL-5 | 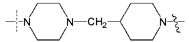 | 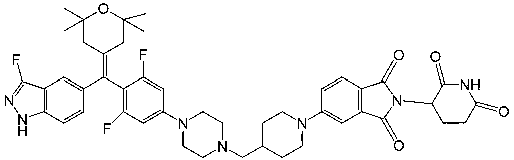 |
| 103 | ERL-1 | 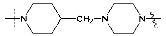 | 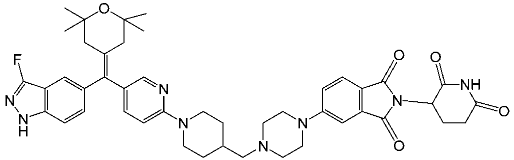 |
| 107 | ERL-3 | 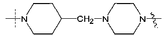 | 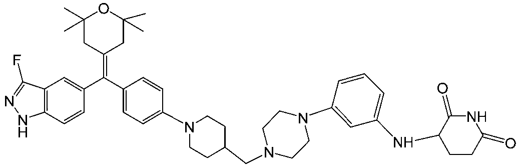 |
| 112 | ERL-1 | 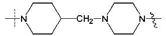 | 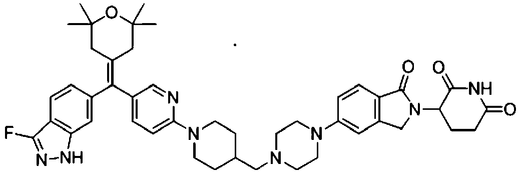 |
| 113 | | 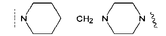 | 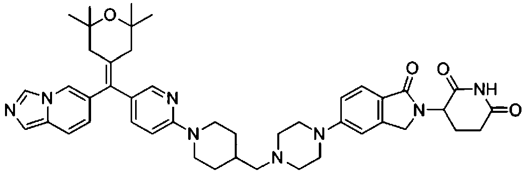 |
| 114 | 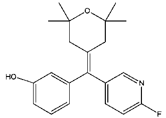 | 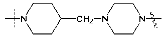 | 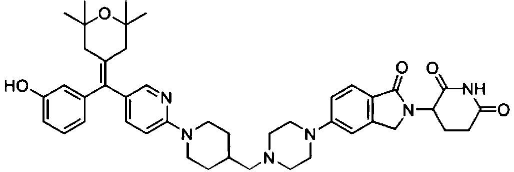 |
| 115 | ERL-3 | 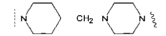 | 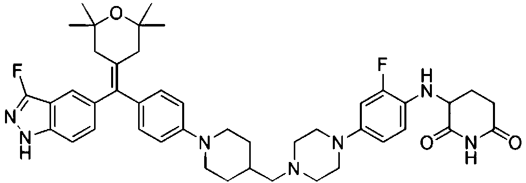 |
| 116 | 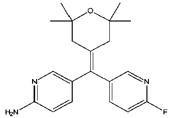 | 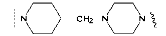 | 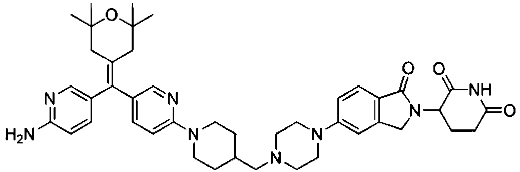 |
| 118 | ERL-1 | 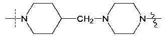 | 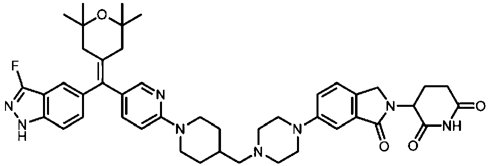 |
| 130 | ERL-4 | 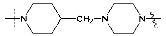 | 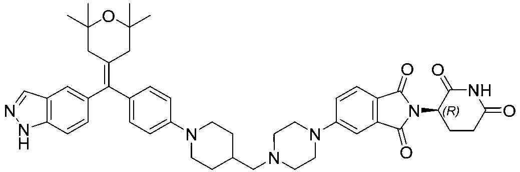 |
| 131 | ERL-4 | 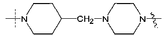 | 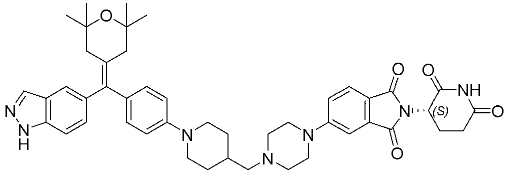 |

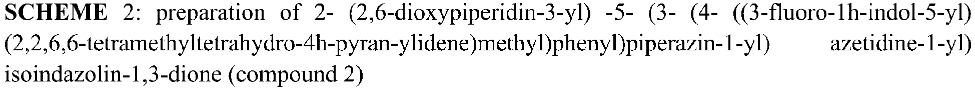

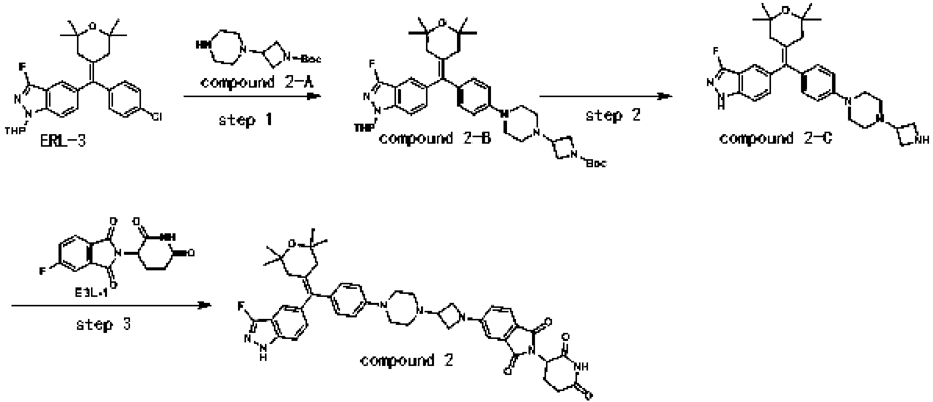

### Step 1: preparation of tert-butyl 3-(4-(4-((3-fluoro-1- (tetrahydro-2H-pyran-4-yl)-1H-indazole-5-yl) (2,2,6,6-tetramethyltetrahydro-4H-pyran-4-ylidene)methyl)phenyl)piperazin-1-yl) azetidine-1-carboxylate:

Tert-butyl 3-(piperazine-1-yl)azetidine-1-carboxylate (450 mg, 1.8 mmol), Ruphos Palladacycle G3 (104 mg, 0.12 mmol), Ruphos (58 mg, 0.12 mmol), and t-BuONa (179 mg, 1.8 mmol) were added into a solution of 5- ((4-chlorophenyl) (2,2,6,6-tetramethyltetrahydro-4H-pyran-4-ylidene) methyl)-3-fluoro-1- (tetrahydro-2H-pyran-4-yl) - 1H indazole (300 mg, 0.6 mmol) in THF (5 mL).

The mixture was stirred overnight at 85 °C under a nitrogen atmosphere. The reaction was quenched by adding water at room temperature. The resulting mixture was extracted with EA (3×20mL). The combined organic layers were dried over anhydrous sodium sulfate. After filtration, the filtrate was concentrated under reduced pressure. The residue was purified by preparative thin-layer chromatography (DCM/MeOH 10:1) to obtain tert-butyl 3-(4-4-((3-fluoro-1-(tetrahydro-2H-pyran-4-yl)-1H-indazole-5-yl] (2,2,6,6-tetramethyltetrahydro-4H-pyran-4-ylidene)methyl)phenyl)piperazin-1-yl] azetidine-1-carboxylate as a yellow oil (200 mg, 46.81%). LCMS (ES, m/z) : 688[M+H]⁺.

### Step 2: preparation of 5-((4-(4-(azacyclobutan-3-yl)piperazin-1-yl)phenyl) (2,2,6,6-tetramethyltetrahydro-4H-pyran-4-ylidene) methyl) - 3-fluoro-1H-indazole:

TFA (2 ml) was added to a solution of tert-butyl 3- (4-(4-((3-fluoro-1-(tetrahydro-2H- pyran-4-yl) - 1H-indazole-5-yl)(2,2,6,6-tetramethyltetrahydro-4H-pyran-4-ylidene)methyl)phenyl) piperazin-1-yl) azetidine-1-carboxylate ((180 mg, 0.3 mmol) in DCM (8 mL). The mixture was stirred overnight at room temperature under a nitrogen atmosphere. The resulting mixture was concentrated under reduced pressure. 5-((4-(4-(azacyclobutan-3-yl)piperazin-1-yl) phenyl) (2,2,6,6-tetramethyltetrahydro-4H-pyran-4-ylidene)methyl)-3-fluoro-1H-indazole as a yellow oil (110 mg, 83.46%) was obtained. The crude product was directly used in the next step without further purification. LCMS (ES, m/z) : 504[M+H]⁺.

### Step 3: preparation of 2-(2,6-dioxypiperidin-3-yl)-5-(3-(4-4-((3-fluoro-1H-indol-5-yl) (2,2,6,6-tetramethyltetrahydro-4H-pyran-4-ylidene)methyl)phenyl)piperazin-1-yl) azetidine-1-yl) isoindoline-1,3-dione:

2- (2,6-dioxypiperidin-3-yl)-5-fluoroisoindole-1,3-dione (121 mg, 0.4 mmol) and DIEA (128 mg, 1.0 mmol) were added into a solution of 5- ((4- (4- (azacyclobutan-3-yl) piperazin-1-yl) phenyl) (2,2,6,6-tetramethyltetrahydro-4H-pyran-4-ylidene) methyl)-3-fluoro-1-(tetrahydro-2H-pyran-4-yl)-1H indazole (100 mg, 0.2 mmol) in DMSO (1 mL). The resulting mixture was stirred at 100 °C for 4 h under a nitrogen atmosphere. The reaction was quenched by adding water at room temperature. The resulting mixture was extracted with EA (3×20 mL). The combined organic layers were washed with water (3×5 mL) and dried over anhydrous sodium sulfate. After filtration, the filtrate was concentrated under reduced pressure. The residue was purified by thin-layer chromatography (DCM/MeOH 10:1) to obtain a yellow solid. The crude product (100 mg) was purified by prep HPLC with the following conditions (column: Xselect CSH C18 OBD column 30× 150 mm 5 µm, n; mobile phase A: water (0.05% TFA), mobile phase B: ACN; flow rate: 60ml/min; gradient: 30% B to 59% B in 7 min, 59% B; wavelength: 254nm; RT1 (min): 5.25; operation times: 0) to obtain 2- (2,6-dioxypiperidin-3-yl)-5- (3-(4-4-((3-fluoro-1H-indol-5-yl) (2,2,6,6-tetramethyltetrahydro-4H-pyran-4-ylidene)methyl) phenyl)piperazin-1-yl) azetidine-1-yl) isoindazolin-1,3-dione as a yellow solid (90.5 mg, 59.51%). LCMS (ES, m/z): 760 [M+H]⁺;1H NMR (400 MHz, DMSO-d6) δ (ppm): 12.53 (s, 1H), 11.10 (s, 1H), 7.75-7.73 (d, J = 8.0 Hz, 1H), 7.45-7.41 (m, 2H), 7.29-7.27 (m, 1H), 7.21-7.18 (m, 1H), 7.12-7.08 (m, 2H), 6.98-6.91 (m, 3H), 6.77-6.75 (m, 1H), 5.10-5.06 (m, 1H), 4.36-4.30 (m, 4H), 3.91-2.84 (m, 9H), 2.61-2.55 (m, 2H),2.50-2.14 (d, J = 27.2 Hz, 4H), 2.89-2.86 (m, 1H), 2.05-2.0 (m, 1H), 1.17-1.10 (m, 12H).

According to the synthesis route of compound 2, the following compounds were synthesized by the following starting compounds or intermediates (see above scheme 2 for the synthesis steps, but the molar mass of each starting compound and intermediate was controlled unchanged):

| Compound No. | Starting material | intermediate | Compound structure |
|---|---|---|---|
| 3 | ERL-5 | 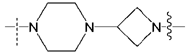 | 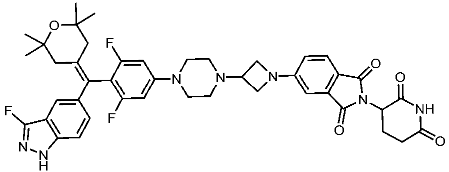 |
| 10 | ERL-5 | 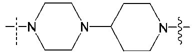 | 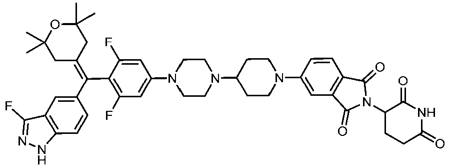 |
| 11 | ERL-5 | 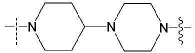 | 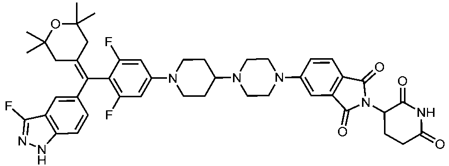 |
| 13 | ERL-3 | 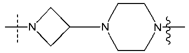 | 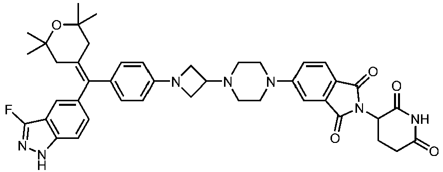 |
| 14 | ERL-5 | 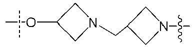 | 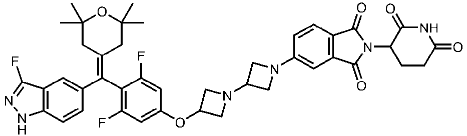 |
| 16 | ERL-3 | 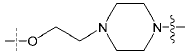 | 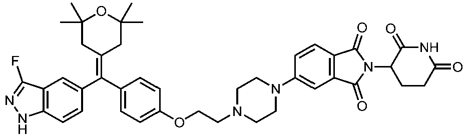 |
| 18 | ERL-1 | 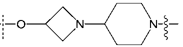 | 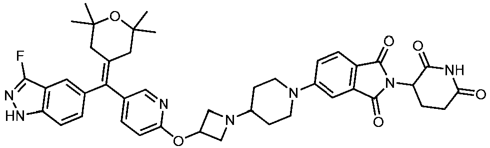 |
| 19 | ERL-1 | 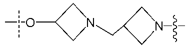 | 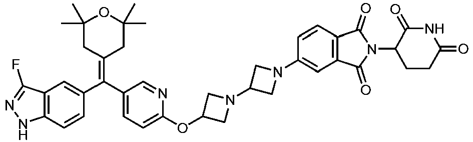 |
| 20 | ERL-3 | 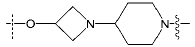 | 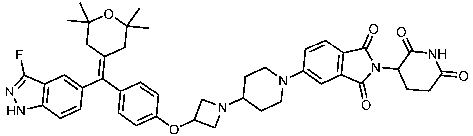 |
| 21 | ERL-3 | 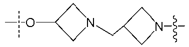 | 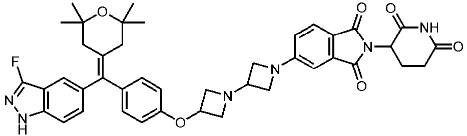 |
| 22 | ERL-3 | 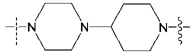 | 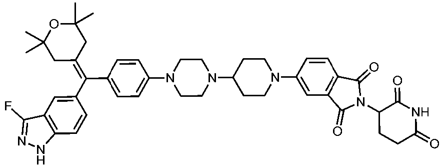 |
| 23 | ERL-5 | 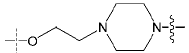 | 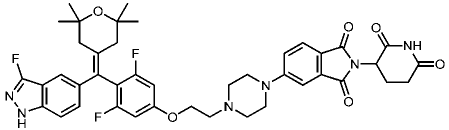 |
| 25 | ERL-5 | 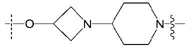 | 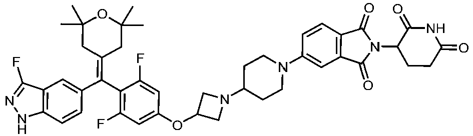 |
| 28 | ERL-1 | 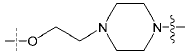 | 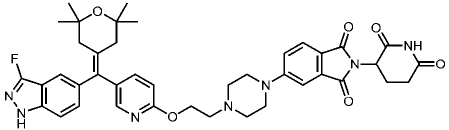 |
| 29 | ERL-5 | 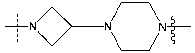 | 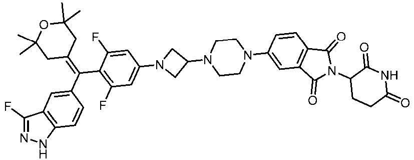 |
| 31 | ERL-3 | 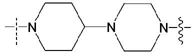 | 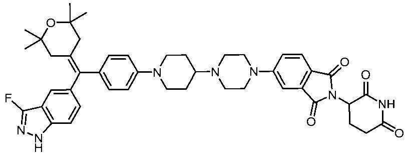 |
| 104 | ERL-1 | 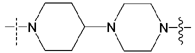 | 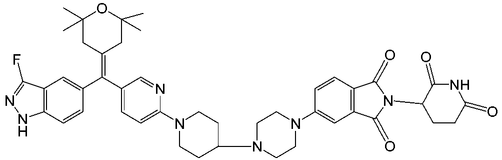 |
| 105 | ERL-1 | 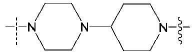 | 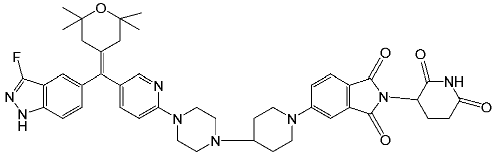 |
| 106 | ERL-2 | 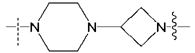 | 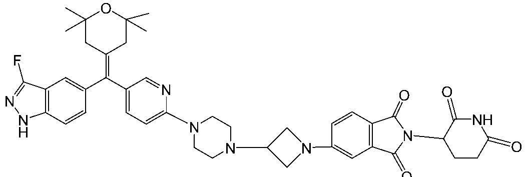 |
| 108 | ERL-4 | 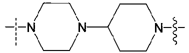 | 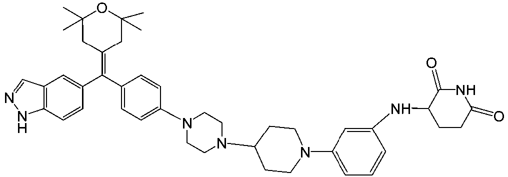 |
| 109 | ERL-1 | 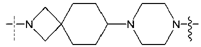 | 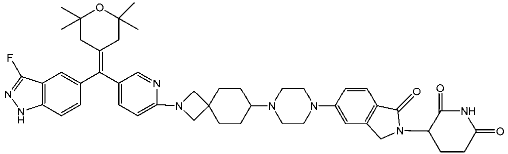 |

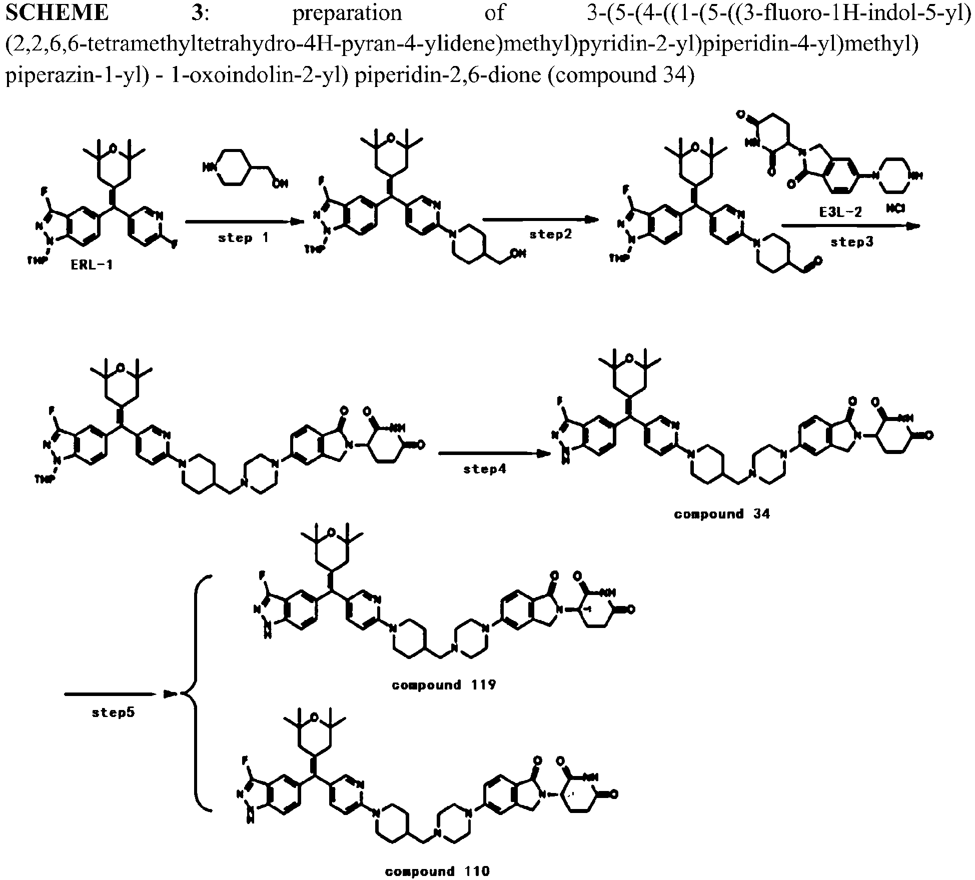

### Step 1: preparation of (1-(5-((3-fluoro-1-(tetrahydro-2H-pyran-4-yl)-1H-indazole-5-yl)

(2,2,6,6-tetramethyltetrahydro-4H-pyran-4-ylidene)methyl}pyridin-2-yl)piperidin-4-yl) methanol: K₂CO₃ (591 mg,4.5 mmol) was added into a solution of 3-fluoro-5-((6-fluoropyridin- 3-yl) (2,2,6,6-tetramethyltetrahydro-4H-pyran-4-ylidene)methyl-1-(tetrahydro-2H-pyran-4-yl)- 1H indazole (400 mg, 0.9 mmol) and piperidine-4-methanol (296 mg, 2.6 mmol) in DMSO (5 mL) at room temperature. The resulting mixture was stirred a under nitrogen atmosphere overnight at 130 °C. The resulting mixture was extracted with ethyl acetate (3×20 mL). The combined organic layers were washed with water (3×5 mL) and dried over anhydrous sodium sulfate. After filtration, the filtrate was concentrated under reduced pressure. The residue was purified by silica gel column chromatography, and then eluted with PE/EA (3:1) to obtain (1-(5-((3-fluoro-1-(tetrahydro-2H-pyran-4-yl)-1H-indazole-5-yl) (2,2,6,6-tetramethyltetrahydro -4H-pyran-4-ylidene)methyl)pyridin-2-yl)piperidin-4-yl) methanol as a yellow oil (400 mg, 83.09%). LCMS (ES, m/z) : 563[M+H]⁺.

### Step 2: preparation of 1-(5-((3-fluoro-1-(tetrahydro-2H-pyran-4-yl)-1H-indazole-5-yl) (2,2,6,6-tetramethyltetrahydro-4H-pyran-4-ylidene)methyl)pyridine-2-yl)piperidine-4-formaldehyd e:

Dess-Martin (174 mg, 0.4 mmol) was added to a solution of (1-(5-((3-fluoro-1-(tetrahydro-2H-pyran-4-yl)-1H-indazole-5-yl) (2,2,6,6-tetramethyltetrahydro-4H-pyran-4-ylidene)methyl}pyridin-2-yl)piperidin-4-yl) methanol (350 mg, 0.6 mmol) in DCM (3 mL) under a nitrogen atmosphere at 0 °C. The mixture was stirred at room temperature until the reaction was completed. The resulting mixture was filtered and the filter cake was washed with DCM (3×20 mL). The filtrate was concentrated under reduced pressure. The residue was purified by thin-layer chromatography (DCM/MeOH 9:1) to obtain 1-(5-((3-fluoro-1-(tetrahydro-2H-pyran-4-yl)-1H-indazole-5-yl) (2,2,6,6-tetramethyltetrahydro -4H-pyran-4-ylidene)methyl)pyridin-2-yl) piperidine-4-formaldehyde as a yellow oil (200 mg, 57.35%). LCMS (ES, m/z) : 561[M+H]⁺.

### Step 3: preparation of 3- (5- (4- ((1- (5- ((3-fluoro-1-(tetrahydro-2H-pyran-4-yl)-1H-indole-5-yl)] (2,2,6,6-tetramethyltetrahydro-4H-pyran-4-ylidene)methyl)pyridin-2-yl) piperidin-4-yl) methyl) piperazin-1-yl)-1-oxoisoindolin-2-yl)piperidin-2,6-dione:

To a solution of 3-[1-oxo-5-(piperazine-1-yl)-3H-indol-2-yl] piperidine-2,6-dione (70 mg, 0.2 mmol) and 1-(5-((3-fluoro-1-(tetrahydro-2H-pyran-4-yl) -1H-indazole-5-yl) (2,2,6,6-tetramethyltetrahydro-4H-pyran-4-ylidene)methyl)pyridine-2-yl) piperidine-4-formaldehyde(100 mg, 0.2 mmol) in DCE (2 mL), STAB (76 mg, 0.4 mmol) was added at room temperature. The resulting mixture was stirred overnight under a nitrogen atmosphere at room temperature. The reaction was quenched by adding water. The resulting mixture was extracted with DCM (3×10 mL). The combined organic layers were dried over anhydrous sodium sulfate. After filtration, the filtrate was concentrated under reduced pressure. 3-(5-(4-((1-(5-((3-fluoro-1-(tetrahydro-2H-pyran-4-yl) -1H-indol-5-yl) (2,2,6,6-tetramethyltetrahydro-4H-pyran-4-ylidene)methyl)pyridin-2-yl) piperidin-4-yl) methyl) piperazin-1-yl)-1-oxoisoindolin-2-yl) piperidin-2,6-dione as a yellow oil (100 mg, 64.22%) was obtained after purified by pre thin layer chromatography (PE/EA 1:1). LCMS (ES, m/z) : 873[M+H]⁺.

### Step 4: preparation of 3-(5-(4-((1-(5-((3-fluoro-1H-indol-5-yl) (2,2,6,6-tetramethyltetrahydro-4H-pyran-4-ylidene)methyl)pyridin-2-yl) piperidin-4-yl) methyl) piperazin-1-yl)-1-oxoisoindol-2-yl) piperidin-2,6-dione (compound 34):

3-(5-(4-((1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl) piperidin-4-yl) methyl) piperazin-1-yl)-1-oxoisoindolin-2-yl)piperidin-2,6-dione (120 mg, 0.14 mmol), DCM (8 mL), and TFA (2 mL) were added in to a 50mL round bottom flask. The resulting mixture was stirred overnight under a nitrogen atmosphere at room temperature. The resulting mixture was concentrated under reduced pressure. The crude product was purified by prep-HPLC with the following conditions (column: Xselect CSH C18 OBD column 30×150 mm 5 µm, n; mobile phase A: water (0.05% TFA), mobile phase B: ACN; flow rate: 60 mL/min; gradient: 16% B to 36% B in 7 min, 36% B; wavelength: 254 nm; RT1 (min): 5.68; operation times: 0) to obtain 3-(5-(4-((1-(5-((3-fluoro-1H-indol-5-yl) (2,2,6,6-tetramethyltetrahydro-4H-pyran -4-ylidene)methyl)pyridin-2-yl) piperidin-4-yl) methyl) piperazin-1-yl) -1-oxoindolin-2-yl) piperidin-2,6-dione as a white solid (74.1mg, 67.79%). LCMS (ES, m/z): 789 [M+H]⁺;1H NMR (400 MHz, DMSO-d6) δ (ppm): 12.58 (s, 1H), 10.99 (s, 1H), 9.49 (s, 1H), 7.97-7.96 (d, J = 2.4 Hz, 1H), 7.61-7.60 (d, J = 8.4 Hz, 1H), 7.53-7.44 (m, 3H),7.26-7.16 (m, 3H), 7.00-6.99 (d, J = 6.8 Hz, 1H), 5.10-5.05 (m, 1H), 4.39-4.34 (d, J = 17.2 Hz, 2H), 4.28-4.22 (m, 3H), 4.03-4.00 (d, J = 10.0 Hz, 3H), 3.65-3.63 (d, J = 8.4 Hz, 2H), 3.24-3.12 (m, 5H), 2.95-2.88 (m, 3H), 2.62-2.58 (d, J = 16.4 Hz, 1H), 2.41-2.34 (m, 1H), 2.24 (s, 2H), 2.16 (s, 2H), 1.97-1.93 (m, 1H), 1.86-1.84 (d, J = 12.3 Hz, 2H), 1.23-1.13 (m, 12H).

### Step 5: preparation of (R) 3-(5-(4-((1-(5-((3-fluoro-1H-indol-5-yl)

### (2,2,6,6-tetramethyltetrahydro-4H-pyran-4-ylidene)methyl)pyridin-2-yl)piperidin-4-yl)methyl) piperazin-1-yl)-1-oxoisoindolin-2-yl)piperidin-2,6-dione (compound 119) and (S) 3-(5-(4-((1-(5-((3-fluoro-1H-indol-5-yl)(2,2,6,6-tetramethyltetrahydro-4H-pyran-4-ylidene)methyl) pyridin-2-yl)piperidin-4-yl)methyl)piperazin-1-yl)-1-oxoisoindolin-2-yl)piperidin-2,6-dione (compound 110):

3-(5-(4-((1-(5-((3-fluoro-1H-indol-5-yl) (2,2,6,6-tetramethyltetrahydro-4H -pyran-4-ylidene)methyl)pyridin-2-yl) piperidin-4-yl) methyl) piperazin-1-yl)-1-oxoisoindolin-2-yl) piperidin-2,6-dione (compound 34)(50 mg) was purified by Chiral high performance liquid chromatography column CHIRAL-HPLC: column: JW-CHIRALART cellulose -SB, 4.6×100 mm; 3 µm; Flow phase A: Hex (0.1A): (IPA: DCM = 1:2) = 50:50; flow rate: 1 mL/min; gradient: 0% B to 0% B; injection volume: 5uL mL, for preparation of (S) 3-(5-(4-((1-(5-((3-fluoro-1H-indol-5-yl)(2,2,6,6-tetramethyltetrahydro-4H-pyran-4-ylidene)methyl) pyridin-2-yl) piperidin-4-yl) methyl) piperazin-1-yl)-1-oxoisoindolin-2-yl)piperidin-2,6-dione as a white solid (11 mg). LCMS (ES, m/z) : 815[M+H]⁺;CHIRAL-HPLC: Rt = 1.225; and (R)3-(5-(4-((1-(5-((3-fluoro-1H-indol-5-yl)(2,2,6,6-tetramethyltetrahydro-4H-pyran-4-ylidene)meth yl)pyridin-2-yl)piperidin-4-yl)methyl) piperazin-1-yl)-1-oxoisoindolin-2-yl)piperidin-2,6-dione as a white solid (compound 119) (14.2 mg) LCMS (ES, m/z) : 815[M+H]⁺;CHIRAL-HPLC: Rt = 1.769. According to the synthesis route of compound 34, the following compounds were synthesized by the following starting compounds or intermediates (see above scheme 3 for the synthesis steps, but the molar mass of each starting compound and intermediate was controlled unchanged):

| Compound No. | Starting material | intermediate | Compound structure |
|---|---|---|---|
| 39 | ERL-2 | 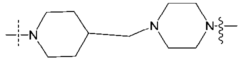 | 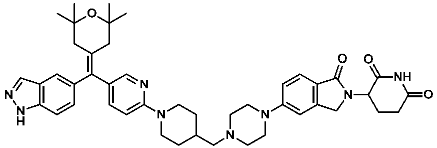 |
| 40 | ERL-1 | 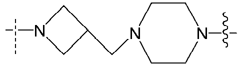 | 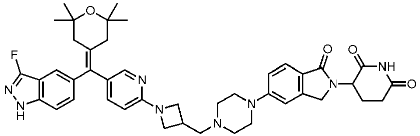 |
| 41 | ERL-3 | 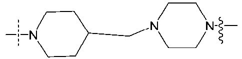 | 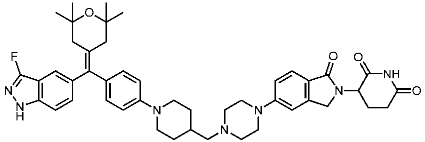 |
| 44 | ERL-1 | 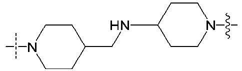 | 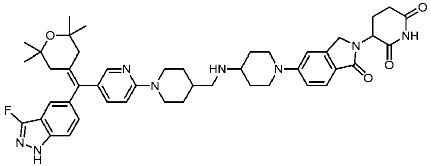 |
| 51 | ERL-1 | 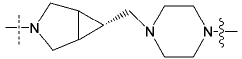 | 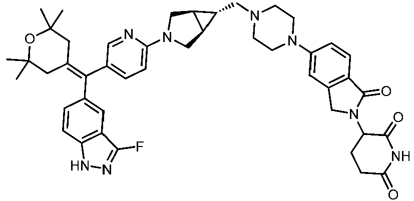 |
| 52 | ERL-1 | 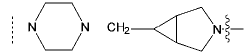 | 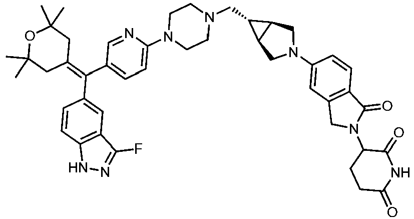 |
| 54 | ERL-1 | 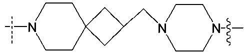 | 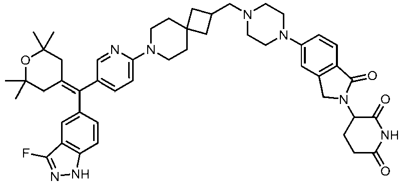 |
| 55 | ERL-5 | 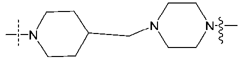 | 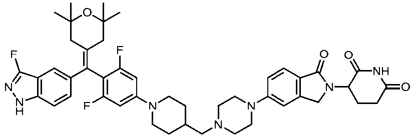 |
| 56 | ERL-4 | 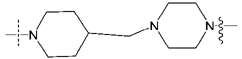 | 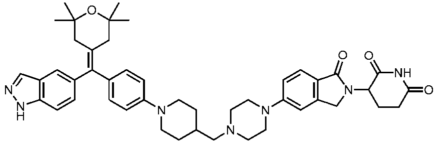 |
| 57 | ERL-1 | 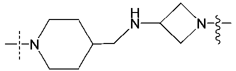 | 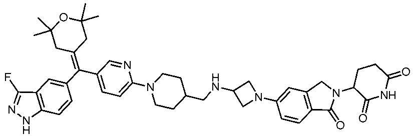 |
| 65 | ERL-1 | 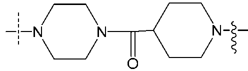 | 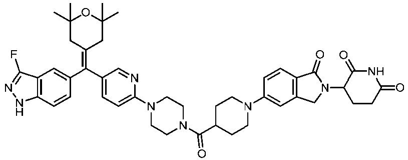 |
| 136 | ERL-2 | 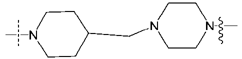 | 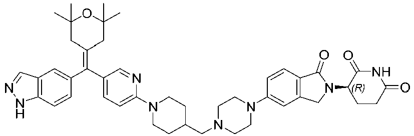 |
| 137 | ERL-2 | 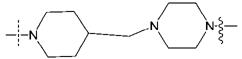 | 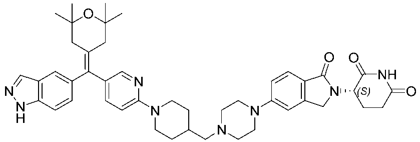 |

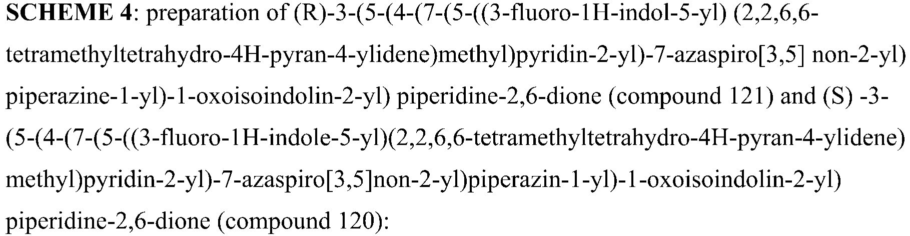

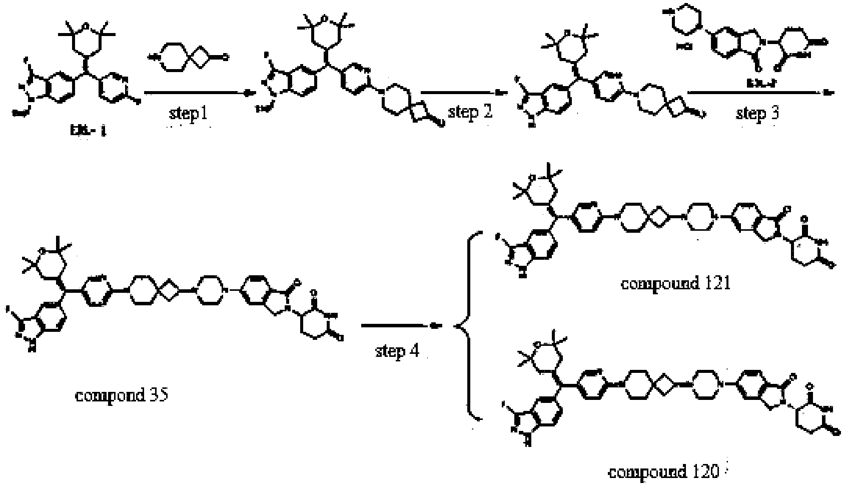

### Step 1: preparation of 7-(5-(3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl)-7-azaspiro[3,5] non-2-one:

7-azaspiro[3,5]non-2-one (267.94 mg, 1.926 mmol, 3equiv.) and K₂CO₃ (266.03 mg, 1.926 mmol, 3 equiv.) were added into a solution of 3-fluoro-5-((6-fluoropyridin-3-yl)(2,2,6,6-tetramethyltetrahydro-4H-pyran-4-ylidene)methyl-1-(tetrahydro-2H-pyran-4-yl)-1H indole (300 mg, 0.642 mmol, 1 equiv.) in DMSO (5 mL) at room temperature. The reaction mixture was irradiated with microwave at 130 °C under a nitrogen atmosphere. The reaction was quenched by adding water at room temperature. The resulting mixture was extracted with ethyl acetate and dried over anhydrous sodium sulfate. After filtration, the filtrate was concentrated under reduced pressure. The residue was purified by preparative thin-layer chromatography (PE/EA) to obtain 7-(5-(3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl)-7-azaspiro[3,5] non-2-one (160 mg, 42.50%). LCMS (ES, m/z) : 587[M+H]⁺.

### Step 2: preparation of a mixture of 7-(5-(3-fluoro-1H-indazole-5-yl)

### (2,2,6,6-tetramethyltetrahydro-4H-pyran-4-ylidene)methyl) pyridin-2-yl)-7-azaspiro[3,5] non-2-one:

TFA (1 mL, 13.463 mmol, 49.37 Eq.) was added into a solution of 7-(5-(3-fluoro-1-(tetrahydro-2H-pyran-4-yl) - 1H-indazole-5-yl)(2,2,6,6-tetramethyltetrahydro-4H-pyran-4-ylidene) methyl)pyridin-2-yl)- 7-azaspiro [3,5] non-2-one (160 mg, 0.273 mmol) in DCM (2 mL). The mixture was stirred overnight at room temperature. The resulting mixture was concentrated under vacuum. 7-(5-((3-fluoro-1H-indazole-5-yl) (2,2,6,6-tetramethyltetrahydro-4H-pyran-4-ylidene) methyl)pyridin-2-yl)-7-azaspiro[3,5] nonane-2-one as a brown oil (160 mg, 93.39%) was obtained. LCMS (ES, m/z) : 503[M+H]⁺.

### Step 3: preparation of 3-(5-(4-(7-(5-((3-fluoro-1H-indol-5-yl)

### (2,2,6,6-tetramethyltetrahydro-4H-pyran-4-ylidene)methyl)pyridin-2-yl)-7-azaspiro[3,5] nonan-2-yl) piperazin-1-yl)-1-oxoisoindolin-2-yl)piperidine-2,6-dione (compound 35):

7-(5-((3-fluoro-1H-indazole-5-yl)(2,2,6,6-tetramethyltetrahydro-4H-pyran-4-ylidene)methyl) pyridin-2-yl)-7-azaspiro [3,5]nonane-2-one (150 mg, 0.298 mmol, 1 equiv.) and 3-(1-oxo-5-(piperazin-1-yl)isoindolin-2-yl) piperidine-2,6-dione (146.99 mg, 0.447 mmol, 1.5 equiv.) were dissolved in a mixed solution of DCM and DMF, and NaOAc (122.41 mg, 1.490 mmol, 5 equiv.) was added. The mixture was stirred for 2 h at room temperature before STAB (126.50 mg, 0.596 mmol, 2 equiv.) was added. The resulting mixture was stirred overnight at room temperature. The reaction was quenched by adding water. The resulting mixture was extracted with ethyl acetate and dried over anhydrous sodium sulfate. After filtration, the filtrate was concentrated under reduced pressure. The crude product (100 mg) was purified by prep-HPLC with the following conditions (column: Sunfire (waters), 30×150 mm, 5 µm; mobile phase A: water (0.05% TFA), mobile phase B: ACN; flow rate: 25 mL/min; gradient: 21%B to 35%B in 8 min, 35% B; wavelength: 254 nm; RT1(min): 7.73; operation time: 0) to obtain 3-(5-(4-(7-(5-((3-fluoro-1H-indol-5-yl) (2,2,6,6-tetramethyltetrahydro-4H-pyran-4-ylidene) methyl)pyridin-2-yl)-7-azaspiro[3,5] nonan-2-yl)piperazin-1-yl)-1-oxoisoindolin-2-yl) piperidine-2,6-dione as a white solid (15.6 mg, 6.40%). LCMS (ES, m/z) : 815[M+H]⁺;1H NMR (400 MHz, DMSO-d6) δ (ppm):12.54 (s, 1H), 10.98 (s, 1H),7.91 (s, 1H), 7.60 (d,1H),7.56-7.40 (m, 3H), 7.26-7.12 (m, 3H), 6.98 (s, 1H),4.41-4.33 (d, 1H), 4.30-4.19 (d, 1H), 4.11-4.01 (d, 2H), 3.91-3.83 (d,1H), 3.58-3.40 (m, 6H), 3.17-3.08(m, 1H),3.08-2.89(m, 3H), 2.62-2.54 (m, 1H),2.43-2.35 (m, 1H), 2.26-2.21 (m, 4H), 2.19 (s, 2H), 2.12-2.08(m, 2H), 2.04-1.98(m, 1H), 1.18-1.13(d, 12H).

### Step 4: preparation of (R) 3-(5-(4-(7-(5-((3-fluoro-1H-indol-5-yl)

### (2,2,6,6-tetramethyltetrahydro-4H-pyran-4- ylidene) methyl) pyridin-2-yl)-7-azaspiro[3,5] nonan-2-yl) piperazin-1-yl) - 1-oxoisoindolin-2-yl) piperidine-2,6-dione (compound 121) and (S) -3-(5-(4-(7-(5-(3-fluoro-1H-indol-5-yl)(2,2,6,6-tetramethyltetrahydro-4H-pyran-4-methylene) methyl)pyridin-2-yl)-7-azaspiro[3,5]non-2-yl)piperazin-1-yl)-1-oxoisoindol-2-yl) piperidine-2,6-dione (compound 120):

3-(5-(4-(7-(5-((3-fluoro-1H-indol-5-yl)(2,2,6,6-tetramethyltetrahydro-4H-pyran-4-ylidene) methyl)pyridin-2-yl)-7-azaspiro[3,5]nonane-2-yl)piperazin-1-yl)-1-oxoisoindolin-2-yl) piperidine-2,6-dione (compound 35) (50 mg) was purified by CHIRAL-HPLC: column: JW-CHIRAL ART cellulose -SB, 4.6×100 mm; 3 µ m; flow phase a: Hex (0.1 A): (IPA: DCM = 1:2) = 50:50; flow rate: 1ml/min; gradient: 0% B to 0% B; injection volume: 5uL mL, for the preparation of (S) 3-(5-(4-(7-(5- ((3-fluoro-1H-indol-5-yl)(2,2,6,6-tetramethyltetrahydro-4H-pyran -4-ylidene)methyl) pyridin-2-yl)-7-azaspiro[3,5]nonan-2-yl) piperazin-1-yl)-1-oxoisoindolin-2-yl) piperidin-2,6-dione (compound 120) as a white solid (5.0 mg, 9.61%). LCMS (ES, m/z) : 815[M+H]⁺; Chiral HPLC: Rt = 3.269; and (R) 3-(5-(4-(7-(5-((3-fluoro-1H-indol-5-yl) (2,2,6,6-tetramethyltetrahydro-4H-pyran-4-ylidene)methyl)pyridin-2-yl)-7-azaspiro[3,5] nonan-2-yl) piperazin-1-yl) -1-oxoisoindolin-2-yl) piperidine-2,6-dione (compound 121) as a white solid (4.2 mg, 7.98%). LCMS (ES, m/z) : 815[M+H]⁺; Chiral HPLC: Rt = 4.556.

According to the synthesis route of compound 120 and 121, the following compounds were synthesized by the following starting compounds or intermediates (see above scheme 4 for the synthesis steps, but the molar mass of each starting compound and intermediate was controlled unchanged):

| Compound No. | Starting material | intermediate | Compound structure |
|---|---|---|---|
| 37 | ERL-3 | 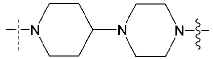 | 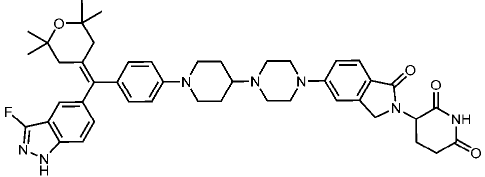 |
| 38 | ERL-5 | 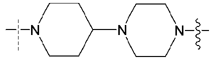 | 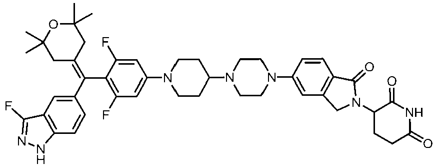 |
| 42 | ERL-3 | 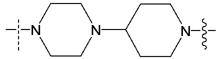 | 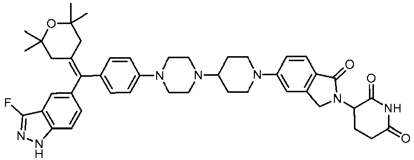 |
| 43 | ERL-1 | 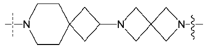 | 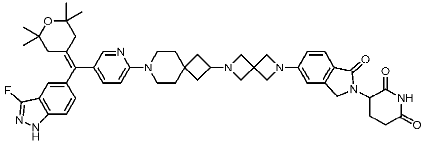 |
| 46 | ERL-1 | 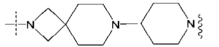 | 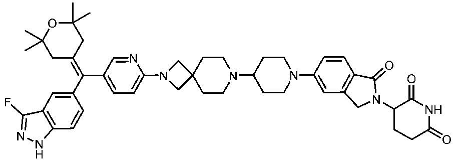 |
| 47 | ERL-1 | 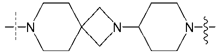 | 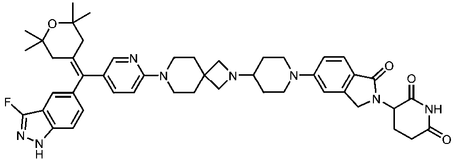 |
| 49 | ERL-1 | 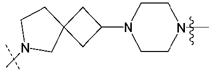 | 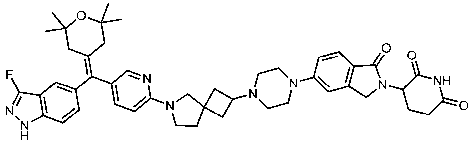 |
| *50* | ERL-1 | 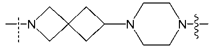 | 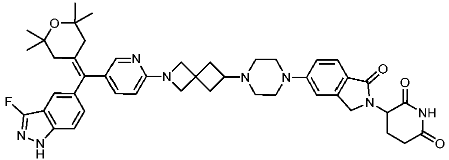 |
| 53 | ERL-1 | 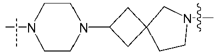 | 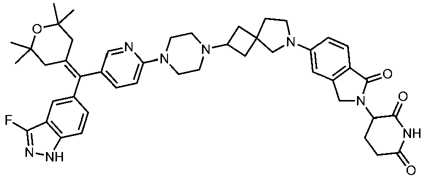 |
| 58 | ERL-4 | 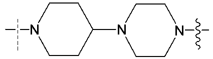 | 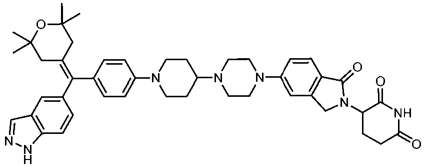 |
| 60 | ERL-1 | 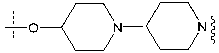 | 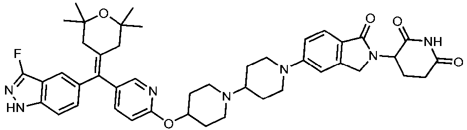 |
| 62 | ERL-3 | 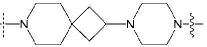 | 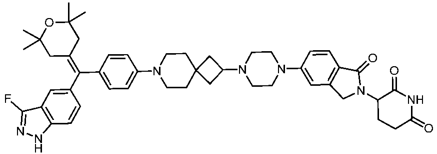 |
| 63 | ERL-1 | 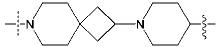 | 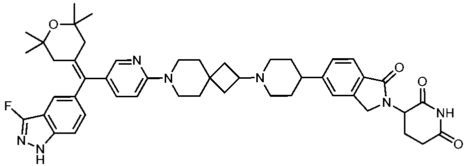 |
| 64 | ERL-1 | 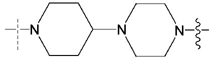 | 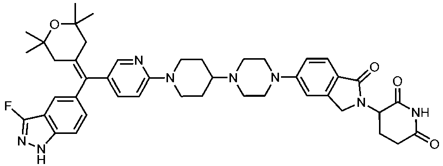 |
| 66 | ERL-4 | 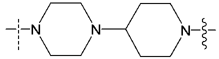 | 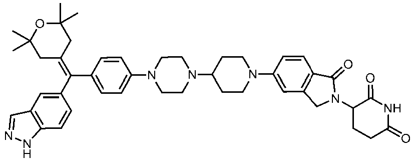 |
| 67 | ERL-1 | 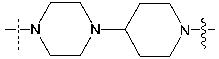 | 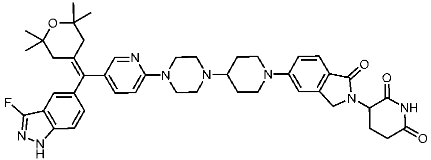 |
| 68 | ERL-1 | 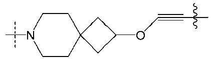 | 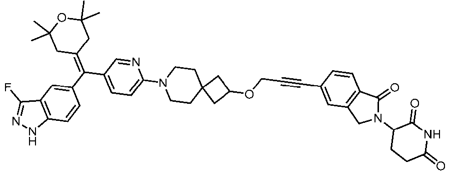 |
| 134 | ERL-3 | 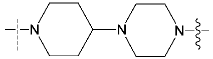 | 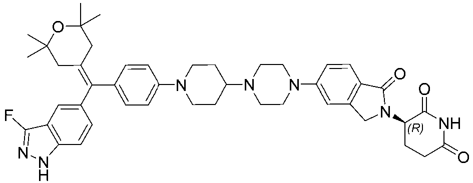 |
| 135 | ERL-3 | 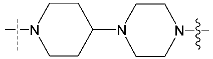 | 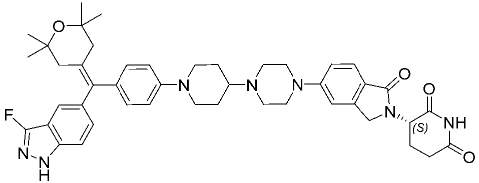 |
| 138 | ERL-1 | 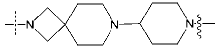 | 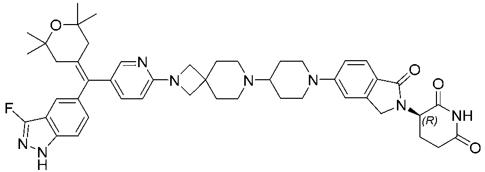 |
| 139 | ERL-1 | 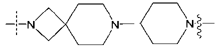 | 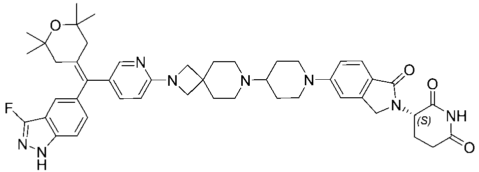 |
| 140 | ERL-1 | 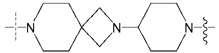 | 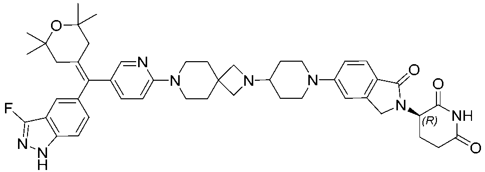 |
| 141 | ERL-1 | 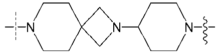 | 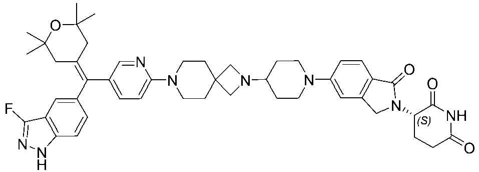 |

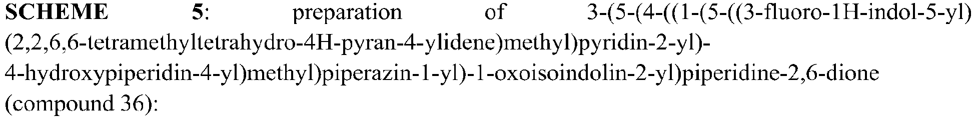

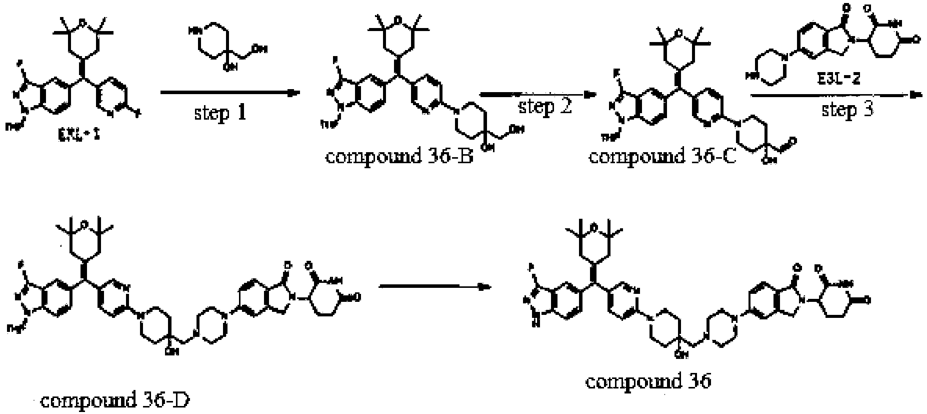

### Step 1: preparation of 1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl)-4-(hydroxymethyl) piperidin-4-ol

4-(hydroxymethyl)piperidin-4-ol (449 mg, 3.4 mmol) and DIEA (1106 mg, 8.6 mmol) were added to a solution of 3-fluoro-5-((6-fluoropyridin-3-yl) (2,2,6,6-tetramethyltetrahydro-4H-pyran-4-ylidene)methyl- 1-(tetrahydro-2H-pyran-4-yl)-1H indazole (800 mg, 1.7 mmol) in DMSO (5 mL). The resulting mixture was stirred at 110 °C under a nitrogen atmosphere overnight. The mixture was quenched by adding water at room temperature. The resulting mixture was extracted with ethyl acetate (3×20 mL). The combined organic layers were dried over anhydrous sodium sulfate. After filtration, the filtrate was concentrated under reduced pressure. The residue was purified by silica gel column chromatography using PE/EA (3:1) to obtain 1- (5- ((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl)-4-(hydroxymethyl) piperidin-4-ol as a yellow oil (600 mg, 60.59%). LCMS (ES, m/z) : 579[M+H]⁺.

### Step 2: preparation of 1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl) - 4-hydroxypiperidine-4-formaldehyde:

To a solution of 1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl)-4-(hydroxymethyl) piperidin-4-ol (100 mg, 0.17 mmol) in dimethyl sulfoxide solution (2 mL), IBX (194 mg, 0.7 mmol) was added at room temperature. The resulting mixture was stirred at room temperature for 4 h. The reaction was quenched by adding water at room temperature. The resulting mixture was extracted with EtOAc (3×20mL). The combined organic layers were washed with water (3×5 mL) and dried over anhydrous sodium sulfate. After filtration, the filtrate was concentrated under reduced pressure to obtain 1-(5-((3-fluoro-1- (tetrahydro-2H-pyran-4-yl)- 1H-indazole-5-yl) (2,2,6,6tetramethyltetrahydro-4H-pyran-4-ylidene)methyl)pyridin-2-yl)-4-hydroxypiperidine-4-for maldehyde as a gray-white solid (50 mg, 50.17%). LCMS (ES, m/z) : 577[M+H]⁺.

### Step 3: preparation of 3-(5-(4-((1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl) - 4-hydroxypiperidin-4-yl) methyl) piperazin-1-yl)- 1-oxoisoindolin-2-yl) piperidin-2,6-dione:

To a stirred solution of 1-(5-((3-fluoro-1-(tetrahydro-2H-pyran-4-yl)-1H-indazole-5-yl) (2,2,6,6-tetramethyltetrahydro-4H- pyran-4-ylidene)methyl)pyridin-2-yl)-4-hydroxypiperidine-4-formaldehyde (180 mg, 0.31 mmol) in DCM/DMF, 3-(1-oxo-5-(piperazin-1-yl) isoindol-2-yl)piperidine-2,6-dione (5 mg, 0.62 mmol) and STAB (132.30 mg, 0.62 mmol) were added. The resulting mixture was stirred overnight at room temperature. The reaction was quenched by adding water at room temperature. The resulting mixture was extracted with DCM (3×10 mL). The combined organic layers were washed with water *(3×5* mL) and dried over anhydrous sodium sulfate. After filtration, the filtrate was concentrated under reduced pressure to give 3-(5-(4-((1-(5-((3-fluoro-1- (tetrahydro-2H-pyran-4-yl) - 1H-indol-5-yl) (2,2,6,6-tetramethyltetrahydro-4H-pyran-4-ylidene)methyl)pyridin-2-yl)-4-hydroxypiperidin-4-yl)methyl)piperazin-1-yl)-1-oxoisoindolin-2-yl)piperidin-2,6-dione as a yellow oil (150 mg, 54.05%). LCMS (ES, m/z) : 889[M+H]⁺.

### Step 4: preparation of 3-(5-(4-((1-(5-((3-fluoro-1H-indol-5-yl) (2,2,6,6-tetramethyltetrahydro-4H-pyran-4-ylidene)methyl)pyridin-2-yl) - 4-hydroxypiperidin-4-yl)methyl)piperazin-1-yl)-1-oxoisoindolin-2-yl)piperidine-2,6-dione (compound 36):

TFA (1.5 mL) was added to a solution of 3-(5-(4-((1-(5-((3-fluoro-1 - (tetrahydro-2H-pyran-4-yl)-1H-indol-5-yl) (2,2,6,6-tetramethyltetrahydro-4H-pyran-4-ylidene) methyl) pyridin-2-yl)-1-oxoisoindolin-2-yl)piperidine-2,6-dione (150 mg, 0.17 mmol) in DCM (6 mL). The mixture was stirred overnight at room temperature under a nitrogen atmosphere. The resulting mixture was concentrated under reduced pressure. The crude product (100 mg) was purified by Prep-HPLC (column: Xselect CSH C18 OBD (waters) column 30×150 mm 5 µm, n; mobile phase A: water (0.1% TFA), mobile phase B: ACN; flow rate: 60ml/min; gradient: 11% B to 41% B in 7 min; wavelength: 254nm; RT1 (min): 5.89) to give 3-(5-(4-((1-(5-((3-fluoro-1H-indol-5-yl) (2,2,6,6-tetramethyltetrahydro-4H-pyran-4-ylidene)methyl)pyridin-2-yl) - 4-hydroxypiperidin-4-yl)methyl)piperazin-1-yl)-1-oxoisoindolin-2-yl)piperidine-2,6-dione (compound 36) as a white solid (23.4 mg, 17.06%). LCMS (ES, m/z) : 805[M+H]⁺;1H NMR (400 MHz, DMSO-d6) δ (ppm):12.56 (s, 1H), 10.97 (s, 1H),9.37(s, 1H), 7.98 (s,1H),7.61-7.58(d, J = 8.4 Hz, 1H), 7.51-7.41(m, 2H), 7.25-7.22 (m, 1H), 7.18-7.11 (m, 2H), 6.99-6.96 (d, J = 13.2 Hz, 1H), 5.45(s, 1H), 5.11-5.01 (m,1H), 4.41-4.18 (m, 2H), 3.98-3.81 (m, 4H), 3.71-3.63(s, 2H), 3.45-3.17 (m, 7H), 2.95-2.88 (m, 1H), 2.69-2.53 (m, 1H), 2.44-2.36 (m, 1H), 2.22(s, 2H), 2.15(s, 1H), 2.01-1.94(m, 1H), 1.81-1.73(m, 2H), 1.73-1.58(m, 2H), 1.19-1.01(m, 12H).

According to the synthesis route of compound 36, the following compounds were synthesized by the following starting compounds or intermediates (see above scheme 5 for the synthesis steps, but the molar mass of each starting compound and intermediate was controlled unchanged):

| Compound NO. | intermediate | Compound structure |
|---|---|---|
| 48 | 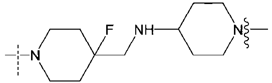 | 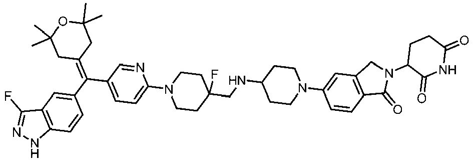 |
| 59 | 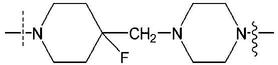 | 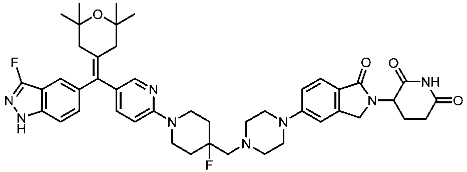 |
| 61 | 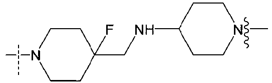 | 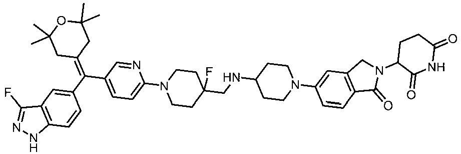 |
| 117 | 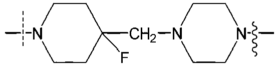 | 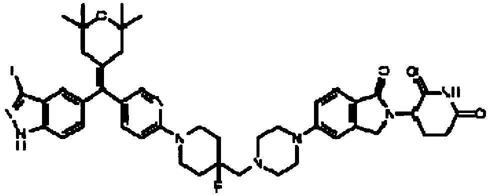 |
| 142 | 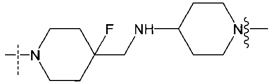 | 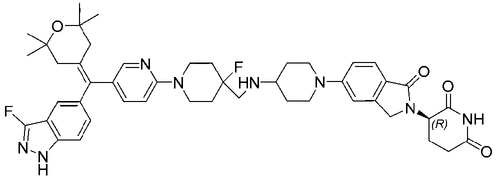 |
| 143 | 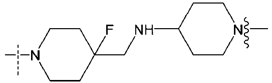 | 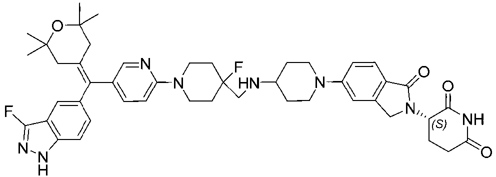 |

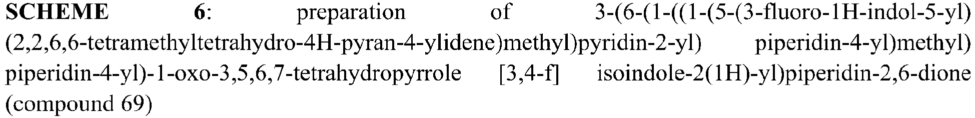

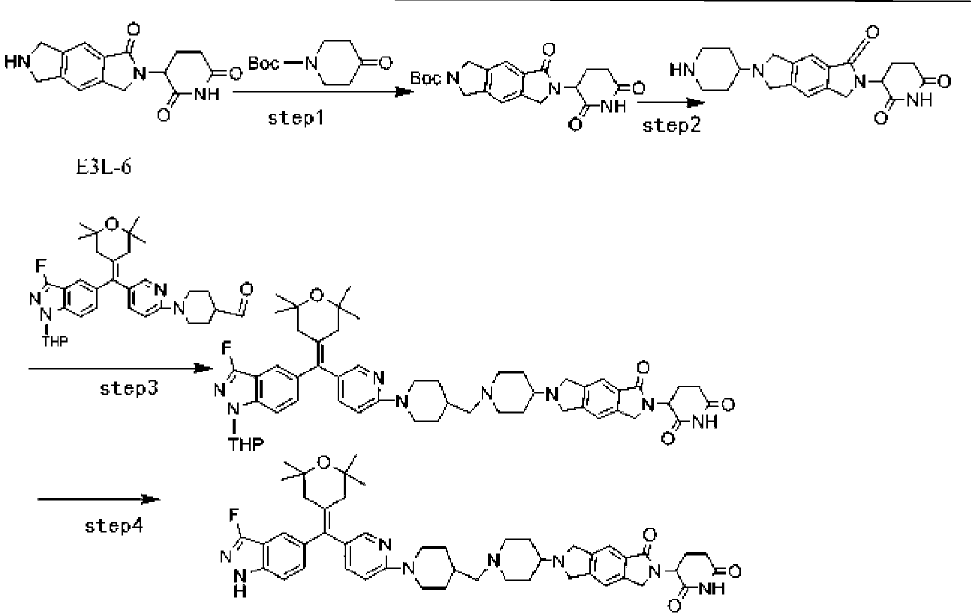

### Step 1: preparation of tert-butyl 4-(6-(2,6-dioxypiperidin-3-yl)-5-oxo-3,5,6,7-tetrahydropyrrole [3,4-f] isoindole-2(1H)-yl)piperidine-1-carboxylate:

3-(1-oxo-3,5,6,7-tetrahydropyrrole[3,4-f] isoindole-2(1H)-yl)piperidine-2,6-dione (143 mg, 0.5 mmol) and STAB(213.30 mg, 1 mmol) were added to a stirred solution of tert-butyl 4-oxo-piperidine-1-carboxylate (100 mg, 0.5 mmol) in DMA (1 mL). The resulting mixture was stirred overnight at room temperature. The reaction was quenched by adding water at room temperature. The resulting mixture was extracted with DCM (3×10 mL). The combined organic layers were washed with water (3×5 mL) and dried over anhydrous sodium sulfate. After filtration, the filtrate was concentrated under reduced pressure and tert-butyl 4-(6-(2,6-dioxypiperidin-3-yl)-5-oxo-3,5,6,7- tetrahydropyrrole [3,4-f] isoindole-2(1H)-yl)piperidine-1-carboxylate as a yellow oil was obtained. LCMS (ES, m/z) : 469.55[M+H]⁺.

### Step 2: preparation of 3-(1-oxo-6-(piperidin-4-yl)-3,5,6,7-tetrahydropyrrole [3,4-f]isoindole-2(1H) - yl) piperidine-2,6-dione:

TFA (2 mL) was added to a solution of tert-butyl 4-(6-(2,6-dioxypiperidin-3-yl)-5-oxo-3,5,6,7-tetrahydropyrrole[3,4-f] isoindole-2 (1H)- yl) piperidine-1-carboxylate (281 mg, 0.3 mmol) in DCM (8 mL). The mixture was stirred at room temperature overnight under a nitrogen atmosphere. The resulting mixture was concentrated under reduced pressure, dissolved in DCM (20 mL), and washed with an aqueous solution of NaHCO₃ (10 mL). After separation the aqueous phase was extracted with DCM (3×10 mL). The combined organic layers were washed with water (3×5 mL) and dried over anhydrous sodium sulfate. After filtration, the filtrate was concentrated under reduced pressure to give 3-(1-oxo-6- (piperidin-4-yl)-3,5,6,7- tetrahydropyrrole [3,4-f] isoindole-2(1H)-yl) piperidin-2,6-dione as a yellow oily liquid (88 mg, 80%). The crude product was directly used in the next step without further purification. LCMS (ES, m/z) : 504[M+H]⁺.

### Step 3: preparation of 3-(6-(1-((1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl)piperidin-4-yl)methyl) piperidin-4-yl)-1-oxo-3,5,6,7-tetrahydropyrrole[3,4-f] isoindole-2(1H)- yl) piperidin-2,6-dione:

To a stirred solution of 1-(5-((3-fluoro-1-(tetrahydro-2H-pyran-4-yl)-1H-indazole-5-yl) (2,2,6,6-tetramethyltetrahydro- 4H-pyran-4-ylidene)methyl) pyridin-2-yl) piperidine-4-formaldehyde(134.5 mg, 0.24 mmol) in DMA (1 mL), were added 3-(1-oxo-6-(piperidin-4-yl)- 3,5,6,7-tetrahydropyrrole [3,4-f] isoindole-2(1H)-yl)piperidine-2,6-dione (88 mg, 0.24 mmol) and STAB (106 mg, 0.5 mmol). The resulting mixture was stirred overnight at room temperature. The reaction was quenched by adding water at room temperature. The resulting mixture was extracted with DCM (3×10 mL). The combined organic layers were washed with water (3×5 mL) and dried over anhydrous sodium sulfate. After filtration, the filtrate was concentrated under reduced pressure. 3-(6-(1-(1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl) piperidin-4-yl) methyl) piperidin-4-yl)-1-oxo-3,5,6,7-tetrahydropyrrole [3,4-f] isoindole-2(1H)-yl)piperidin-2,6-dione (131mg, 60%) as a yellow oily liquid was obtained. LCMS (ES, m/z) : 914.15[M+H]⁺.

### Step 4: preparation of 3-(6-(1-((1-(5-((3-fluoro-1H-indol-5-yl)

### (2,2,6,6-tetramethyltetrahydro-4H-pyran-4-ylidene)methyl)pyridin-2-yl) piperidin-4-yl) methyl) piperidin-4-yl)-1-oxo-3,5,6,7-tetrahydropyrrole [3,4-f] isoindole-2(1H)-yl) piperidin-2,6-dione (compound 69):

TFA (0.5 mL) was added into a solution of 3-(6-(1-(1-(5-((3-fluoro-1- (tetrahydro-2H-pyran-4-yl) -1H-indol-5-yl) (2,2,6,6-tetramethyltetrahydro-4H-pyran-4-ylidene) methyl)pyridin-2-yl) piperidin-4-yl) methyl) piperidin-4-yl)-1oxo-3,5,6,7-tetrahydropyrrole [3,4-f] isoindole-2(1H) -yl) piperidin-2,6-dione (130 mg, 0.14 mmol) in DCM (1 mL). The mixture was stirred overnight at room temperature under a nitrogen atmosphere. The resulting mixture was concentrated under reduced pressure. The crude product (90 mg) was purified by prep-HPLC with the following conditions (column: Xselect CSH C18 OBD column (Waters) 30×150 mm 5 µm, n; mobile phase A: water (0.1% TFA), mobile phase B: ACN; flow rate: 60 mL/min; gradient: 11% B to 41\% B in 7 min; wavelength: 254 nm; RT1 (min): 5.89) to give 3-(6-(1-((1-(5-((3-fluoro-1H-indol-5-yl) (2,2,6,6-tetramethyltetrahydro-4H-pyran-4-ylidene)methyl)pyridin-2-yl) piperidin-4-yl) methyl) piperidin-4-yl)-1-oxo-3,5,6,7-tetrahydropyrrole [3,4-f] isoindole-2(1H)-yl)piperidin-2,6-dione (compound 69) as a white solid (35.4 mg, 30%). LCMS (ES, m/z) : 829.45[M+H]⁺ ;1H NMR (400 MHz, DMSO-d6) δ (ppm):12.55 (s, 1H), 10.96 (s, 1H),9.37(s, 1H), 7.64-7.58(d, J = 8.4 Hz, 1H), 7.52-7.42(m, 2H), 7.26-7.22 (m, 1H), 7.18-7.11 (m, 2H), 6.99-6.96 (d, J = 13.2 Hz, 1H), 5.45(s, 1H), 5.11-5.01 (m,1H), 4.41-4.18 (m, 2H), 3.98-3.81 (m, 4H), 3.72-3.58 (s, 6H), 3.45-3.17 (m, 3H), 2.95-2.88 (m, 1H), 2.69-2.52 (m, 2H), 2.44-2.36 (m, 1H), 2.22(s, 2H), 2.15(s, 1H), 2.01-1.94(m, 1H), 1.84-1.73(m, 4H), 1.75-1.56(m, 4H), 1.22-1.04(m, 12H).

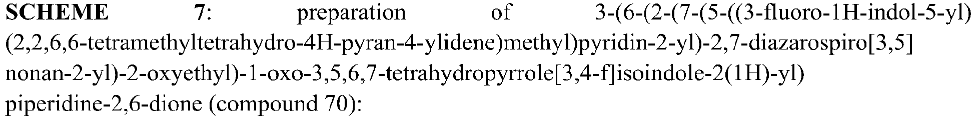

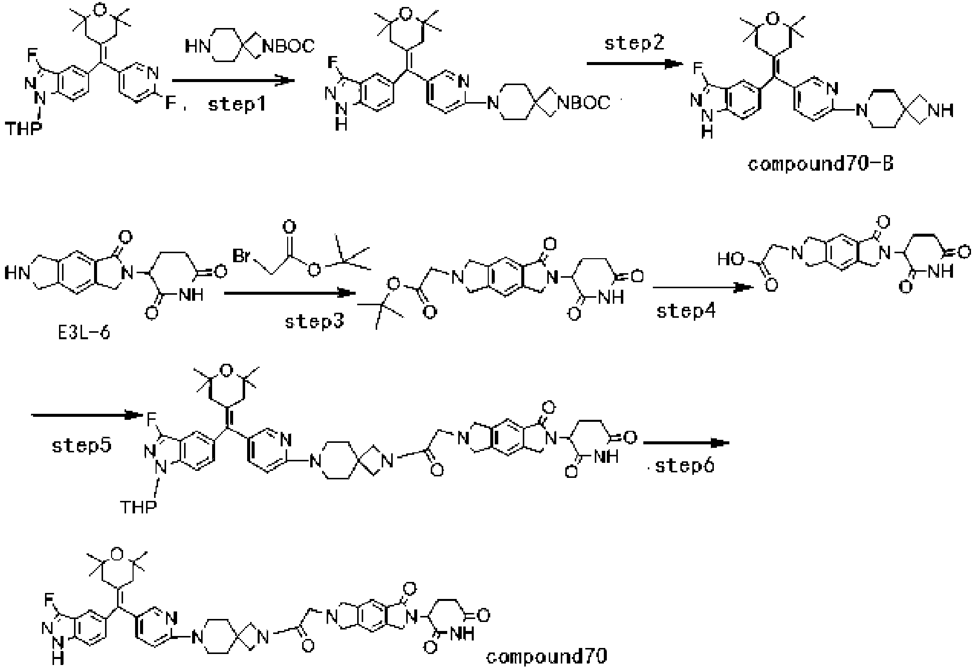

### Step 1: preparation of tert-butyl 7-(5-((3-fluoro-1H-indazole-5-yl)

### (2,2,6,6-tetramethyltetrahydro-4H-pyran-4-ylidene)methyl)pyridin-2-yl)-2,7-diazarospiro [3,5] nonane-2-carboxylate:

Tert-butyl 2,7-diazarospiro[3,5] nonane-2-carboxylate (136 mg, 0.6 mmol, 3 Eq.) and K₂CO₃ (208 mg, 1.5 mmol) were added into a solution of 3-fluoro-5-((6-fluoropyridin-3-yl)(2,2,6,6-tetramethyltetrahydro-4H-pyran-4-ylidene)methyl)-1-(tetrahydro-2H-pyran-4-yl)-1H indazole (234 mg, 0.5 mmol) in DMSO (5 mL) at room temperature. The final mixture was irradiated with microwave at 130 °C overnight. The reaction was quenched by adding water at room temperature. The resulting mixture was extracted with ethyl acetate and dried over anhydrous sodium sulfate. After filtration, the filtrate was concentrated under reduced pressure. The residue was purified by preparative thin-layer chromatography (PE/EA) to obtain tert-butyl 7-(5- ((3-fluoro-1H-indazole-5-yl)(2,2,6,6-tetramethyltetrahydro-4H-pyran-4-ylidene)methyl) pyridin-2-yl)-2,7-diazarospiro [3,5] nonane-2-carboxylate(162 mg, 55%).LCMS (ES, m/z) : 590.34[M+H]⁺.

### Step 2: preparation of 7-(5-((3-fluoro-1H-indazole-5-yl)

### (2,2,6,6-tetramethyltetrahydro-4H-pyran-4-ylidene) methyl) pyridin-2-yl)-2,7-diazarospiro [3,5] nonane:

TFA (2ml) was added to a solution of tert-butyl 7-(5-((3-fluoro-1H-indazole-5-yl) (2,2,6,6-tetramethyltetrahydro-4H-pyran-4-ylidene)methyl)pyridin-2-yl) - 2,7-diazarospiro [3,5] nonane-2-carboxylate (162 mg, 0.28 mmol) in DCM (4 mL), and the mixture was stirred over at room temperature under a nitrogen atmosphere. The resulting mixture was concentrated under reduced pressure. The residue was dissolved in DCM (20 mL), and washed with an aqueous solution of NaHCO₃ (10 mL). After separation, the aqueous phase was extracted with DCM (3×10 mL). The combined organic layers were dried over anhydrous sodium sulfate. The filtrate was concentrated under reduced pressure to give 7-(5-((3-fluoro-1H-indazole-5-yl) (2,2,6,6-tetramethyltetrahydro-4H-pyran-4-ylidene)methyl) pyridin-2-yl)-2,7-diazarospiro [3,5]nonane (compound 70-B) as a yellow oily liquid (114 mg, 85%). The crude product was directly used in the next step without further purification. LCMS (ES, m/z) : 490.28[M+H]⁺.

### Step 3: Preparation of tert-butyl 2-(6-(2,6-dioxopiperidin-3-yl)-5-oxo-3,5,6,7-tetrahydropyrrole [3,4-f] indole-2(1H)-yl) acetate:

Tert-butyl bromoacetate (1.07 g, 5.5 mmol) and DIEA (3.22 g, 25 mmol) were added into a stirred solution of 3-(1-oxo-3,5,6,7-tetrahydropyrrole[3,4-f]indole-2-(1H)-yl) piperidine-2,6-dione (1.43 g, 5 mmol) in MeCN (30 mL). The resulting mixture was stirred overnight at room temperature. The reaction was quenched by adding water at room temperature. The mixture was extracted with DCM (3×50 mL). The combined organic layers were dried over anhydrous sodium sulfate. After filtration, the filtrate was concentrated under reduced pressure. The crude product was purified by silica gel column chromatography and then eluted with DCM/MEOH (20:1) to give tert-butyl 2-(6-(2,6-dioxopiperidin-3-yl)-5-oxo-3,5,6,7-tetrahydropyrrole[3,4-flisoindole-2(1H)-yl) acetate as a yellow oil (1.6 g, 80%), which was used for the next reaction step. LCMS (ES, m/z) : 400.55[M+H]⁺.

### Step 4: preparation of 2-(6-(2,6-dioxypiperidin-3-yl)-5-oxo-3,5,6,7-tetrahydropyrrole[3,4-f] isoindole-2(1H)- yl) acetic acid:

TFA (10 mL) was added to a solution of tert-butyl 2-(6-(2,6-dioxypiperidin-3-yl)-5-oxo-3,5,6,7-tetrahydropyrrole [3,4-f] isoindole-2 (1H) - yl) carboxylate (1.6 g, 4 mmol) in DCM (10 mL). The mixture was stirred overnight at room temperature under a nitrogen atmosphere. The resulting mixture was concentrated under reduced pressure. The residue was dissolved in DCM (50 mL) and washed with H₂O (50 mL). After separation, the aqueous phase was extracted with DCM (3×10 mL). The combined organic layers were dried over anhydrous sodium sulfate and filtered. The filtrate was concentrated under reduced pressure to give 2-(6-(2,6-dioxypiperidin-3-yl)-5-oxo-3,5,6,7-tetrahydropyrrole[3,4-f] isoindole-2 (1H) - yl) acetic acid as a yellow oily liquid (1.16 g, 85%). The crude product was directly used in the next step without further purification. LCMS (ES, m/z) : 342.28[M+H]⁺.

### Step 5: preparation of 3-(6-(2-(7-(5-(3-fluoro-1-(tetrahydro-2H-pyran-4-yl)-1H-indazole-5-yl) (2,2,6,6-tetramethyltetrahydro-4H-pyran-4-ylidene)pyridin-2-yl)-2,7-diazarospiro[3,5] nonan-2-yl)-2-oxyethyl)-1-oxo-3,5,6,7-tetrahydropyrrole[3,4-f]isoindole-2(1H)-yl) piperidine-2,6-dione:

Compound 70B (114 mg, 0.24 mmol), HATU (137 mg, 0.36 mmol) and DIEA (62 mg, 0.48 mmol) were added into a solution of 2-(6-(2,6-dioxypiperidin-3-yl)- 5-oxo-3,5,6,7-tetrahydropyrrole [3,4-f] isoindole-2 (1H)- yl) acetic acid (82 mg, 0.24 mmol) in DMA (2 mL). The resulting mixture was stirred overnight at room temperature under a nitrogen atmosphere. The reaction was quenched by adding water (20 mL). After separation, the aqueous phase was extracted with EA (4×10 mL). The combined organic layers were dried over anhydrous sodium sulfate and filtered. The filtrate was concentrated under reduced pressure to give a crude product. The crude product was purified by silica gel column chromatography, and then eluted with DCM/MeoH (20:1) to give 3-(6-(2-(7-(5-(3-fluoro-1-(tetrahydro-2H-pyran-4-yl)-1H-indazole-5-yl)(2,2,6,6-tetramethyltetrahydro-4H-pyran-4-ylidene)pyridin-2-yl)-2,7-diaza-spiro[3,5] nonan-2-yl)-2-oxyethyl)-1-oxo-3,5,6,7-tetrahydropyrrole[3,4-f]isoindole-2(1H)-yl) piperidine-2,6-dione (118 mg, 55%). LCMS (ES, m/z): 899.55[M+H]⁺.

### Step 6: preparation of 3-(6-(2-(7-(5-((3-fluoro-1H-indol-5-yl) (2,2,6,6-tetramethyltetrahydro-4H-pyran-4-ylidene)methyl)pyridin-2-yl) - 2,7-diazarospiro [3,5] nonan-2-yl)-2-oxyethyl)-1-oxo-3,5,6,7-tetrahydropyrrole[3,4-f]isoindole-2(1H)-yl) piperidine-2,6-dione (compound 70):

TFA (0.5 ml) was added into a solution of 3-(6-(2-(7-(5-(3-fluoro-1-(tetrahydro-2H-pyran-4-yl)-1H-indazole-5-yl) (2,2,6,6-tetramethyltetrahydro-4H-pyran-4-ylidene)pyridin-2-yl)-2,7-diazaspiro[3,5] nonan-2-yl)-2-oxyethyl)-1-oxo-3,5,6,7-tetrahydropyrrole[3,4-f]isoindole-2(1H)-yl) piperidine-2,6-dione (118 mg, 0.13 mmol) in DCM (1 mL). The mixture was stirred overnight at room temperature under a nitrogen atmosphere. The resulting mixture was concentrated under reduced pressure. The crude product (90 mg) was purified by prep HPLC with the following conditions (column: Xselect CSH C18 OBD column 30×150 mm 5 µm, n; mobile phase a: water (0.1% TFA), mobile phase B: ACN; flow rate: 60ml/min; gradient: 11% B to 41% B in 7 min; wavelength: 254 nm; RT1 (min): 5.89) to give 3-(6-(2-(7-(5-((3-fluoro-1H-indol-5-yl) (2,2,6,6-tetramethyltetrahydro-4H-pyran-4-ylidene)methyl)pyridin-2-yl) - 2,7-diazarospiro [3,5] nonan-2-yl)-2-oxyethyl)-1-oxo-3,5,6,7-tetrahydropyrrole[3,4-f]isoindole-2(1H)-yl) piperidine-2,6-dione as a white solid (27 mg, 25%). LCMS (ES, m/z) : 815.4 [M+H]⁺;1H NMR (400 MHz, DMSO-d6) δ (ppm):12. 45 (s, 1H), 10.98 (s, 1H), 8.32-8.22(m, 2H), 8.02-7.62(m, 4H), 7.55-7.48 (m, 1H), 7.36-7.27 (m, 1H), 6.99-6.94 (m, 1H), 4.48-4.42 (m,1H), 4.40-4.20 (m, 2H), 3.99-3.80 (m, 4H), 3.72-3.58 (s, 6H), 3.46-3.18 (m, 2H), 3.28 (s, 2H), 2.45-2.36 (m, 1H), 2.05-1.94(m, 1H), 1.86-1.73(m, 4H), 1.76-1.56(m, 4H), 1.25-1.05(m, 12H).

According to the synthesis route of compound 70, the following compounds were synthesized by the following starting compounds or intermediates (see above scheme 7 for the synthesis steps, but the molar mass of each starting compound and intermediate was controlled unchanged):

| Comp ound No. | intermediate | Compound structure |
|---|---|---|
| 71 | 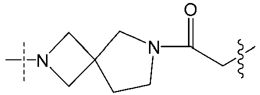 | 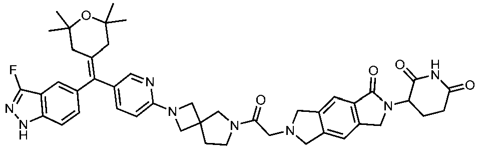 |
| 72 | 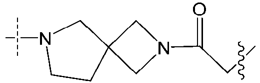 | 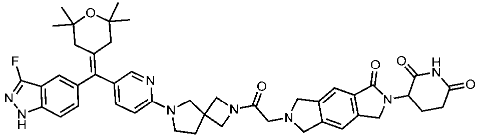 |
| 73 | 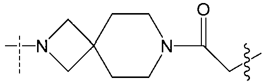 | 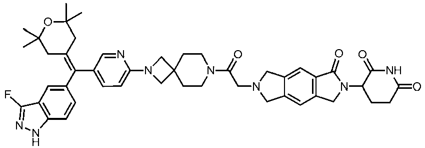 |
| 74 | 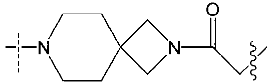 | 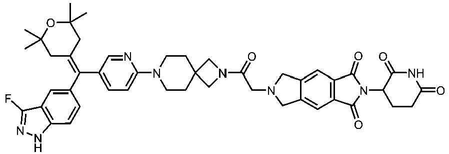 |

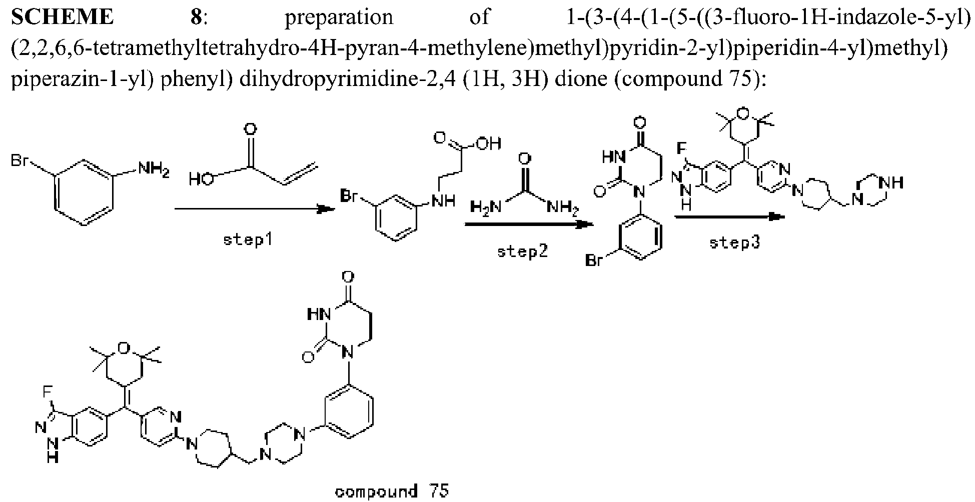

### Step 1: preparation of 3- (3-bromophenyl) aminopropionic acid:

An aqueous solution of m-bromoaniline (3 g, 17.439 mmol) was treated with acrylic acid (3.77 g, 52.317 mmol) at 70 °C for 3 h under a nitrogen atmosphere. Ethanol (6 mL) was added into the mixture after the mixture was cooled to room temperature. The resulting mixture was stirred at room temperature for 12 h. The resulting mixture was concentrated under vacuum. The residue was dissolved in dichloromethane. The residue was purified by silica gel column chromatography and eluted with PE/EA to give 3- (3-bromophenyl) aminopropionic acid as an oily liquid (4 g, 94%). LCMS (ES, m/z) : 244[M+H]⁺.

### Step 2: preparation of 1-(3-bromophenyl)dihydropyrimidine-2,4(1H, 3H) - dione:

3-((3-bromophenyl)amino)propionic acid (4 g, 16.388 mmol, 1 Eq.) and urea (1.97 g, 32.776 mmol, 2 Eq.) was boiled in 20 mL of acetic acid overnight. The pH value of above mixture was adjusted to 1 by adding an aqueous solution of HCl (10%). The resulting mixture was boiled for 30 min, and then cooled to room temperature. The reaction was quenched with water. The precipitated solid was collected by filtration and washed with water to give 1-(3-bromophenyl) dihydropyrimidine-2,4-dione as a white solid (2.55 g, 58%), LCMS (es, m/z): 269[M+H]⁺.

### Step 3: preparation of 1-(3-(4-(1-(5-(3-fluoro-1H-indazole-5-yl) (2,2,6,6-tetramethyltetrahydro-4H-pyran-4-methylene)methyl) pyridin-2-yl) piperidin-4-yl) methyl) piperazin-1-yl)phenyl) dihydropyrimidine-2,4 (1H, 3H) dione (compound 75):

The third generation pre catalyst t-BuXPhos (14.55 mg, 0.018 mmol), t-BuXPhos (15.53 mg, 0.037 mmol) and Cs₂CO₃ (178.78 mg, 0.549 mmol) were added into a solution of 3-fluoro-5-((6-(4-(piperazin-1-methyl)piperidin-1-yl)pyridin-3-yl) (2,2,6,6-tetramethyltetrahydro-4H-pyran-4-ylidene)methyl)-1H indazole (100 mg, 0.183 mmol) and 1-(3-bromophenyl)dihydropyrimidine-2,4-(1H,3H) in 1,4-dioxane (2 mL). The mixture was stirred overnight at 90 °C under a nitrogen atmosphere. The mixture was cooled to room temperature. The reaction was quenched by adding water. The mixture was extracted with ethyl acetate. The combined organic layers were dried over anhydrous sodium sulfate. After filtration, the filtrate was concentrated under reduced pressure. The crude product (100 mg) was purified by prep HPLC with the following conditions (column: Sunfire prep C18 OBD column, 19 x 150 mm, 5 µm; mobile phase A: water (0.05% TFA), mobile phase B: ACN; flow rate: 60 mL/min; gradient: 30% B to 50% B in 7.2 min; wavelength: 254/210 nm; RT1 (min): 8) to give 1-(3-(4-(1-(5-((3-fluoro-1H-indazole-5-yl)(2,2,6,6-tetramethyltetrahydro-4H-pyran-4-methylene) methyl) pyridin-2-yl) piperidin-4-yl) methyl) piperazin-1-yl) phenyl) dihydropyrimidine-2,4 (1H, 3H) dione (compound 75) as a white solid (4.0 mg, 2.88%). LCMS (ES, m/z) : 735[M+H]⁺; 1H NMR (400 MHz, DMSO-d6) δ (ppm):10.50 (s, 1H), 8.37 (s, 1H), 8.01 (s,1H), 7.86-7.84 (d,J=8.0 Hz, 1H), 7.73 (s, 1H), 7.64 (s, 1H),7.59-7.57 (d, J=8.0 Hz, 2H), 7.38-7.31 (m, 3H), 7.76-7.74 (d, J=8.0 Hz, 1H), 2.25-4.22 (d, J=12.0 Hz, 2H), 3.91-3.88 (t, 2H), 2.79-2.79(m, 8H),2.34-2.26(m, 6H), 2.18-2.11 (m, 4H),1.76-1.73 (m, 3H), 1.24-1.19(d, J=20.0 Hz, 12H), 1.13-1.08(m, 2H).

According to the synthesis route of compound 75, the following compounds were synthesized by the following starting compounds or intermediates (see above scheme 8 for the synthesis steps, but the molar mass of each starting compound and intermediate was controlled unchanged):

| Comp ound No. | Starting material | intermediate | Compound |
|---|---|---|---|
| 76 | 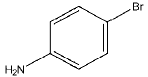 | 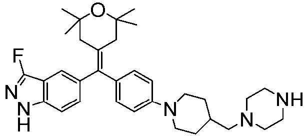 | 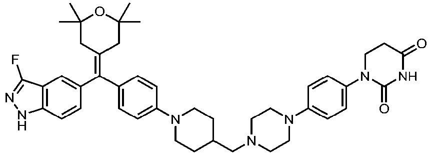 |
| 77 | 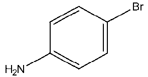 | N/A | 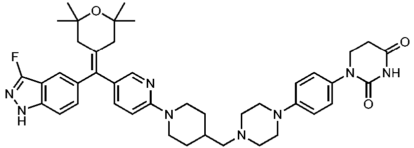 |
| 78 | 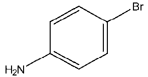 | 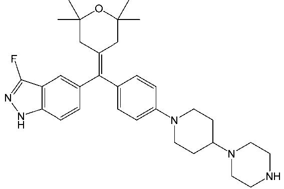 | 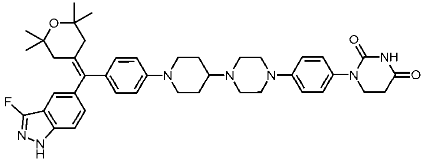 |
| 79 | 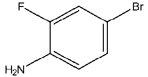 | 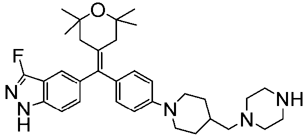 | 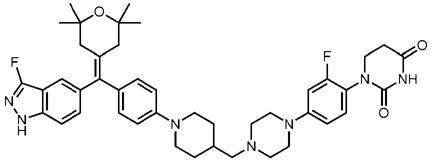 |
| 80 | 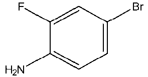 | N/A | 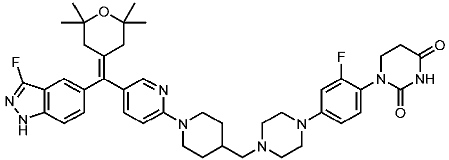 |
| 81 | 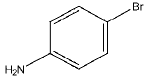 | 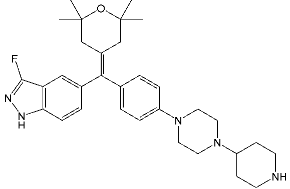 | 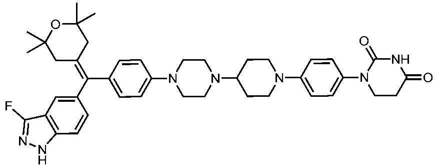 |
| 82 | 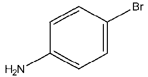 | 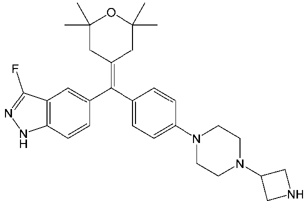 | 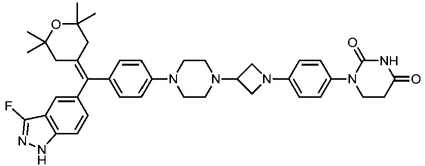 |

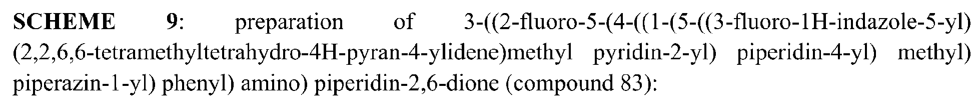

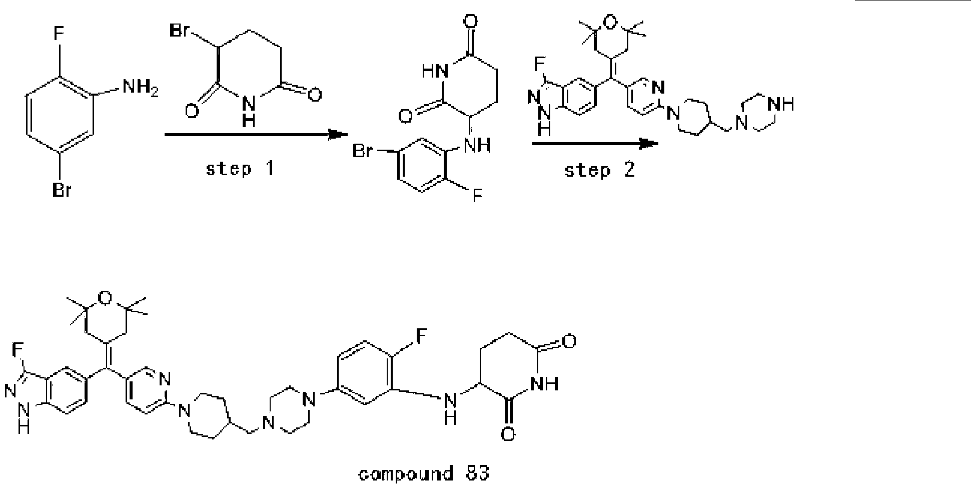

### Step 1: preparation of 3- ((5-bromo-2-fluorophenyl)amino)piperidine-2,6-dione:

5-bromo-2-fluoroaniline (1g, 5.3mmol), 3-bromo-2,6-dione(3.03 g, 15.8 mmol), silver trifluoromethanesulfonate (0.27 g, 1.05 mmol) and an aqueous solution of sodium hydroxide (1.33 g, 15.8 mmol) were stirred at 80 °C for 7 days under a nitrogen atmosphere. The reaction was quenched by adding water at room temperature. The resulting mixture was extracted with EtOAc (3×50mL). The combined organic layers were dried over anhydrous sodium sulfate. After filtration, the filtrate was concentrated under reduced pressure. The residue was mashed with Et₂O (3×10 mL). 3- ((4-bromo-2-fluorophenyl) amino)piperidine-2.6-dione (200mg, 12.62%) as a white solid was obtained. LCMS (ES, m/z) : 301 [M+H]⁺.

### Step 2: preparation of 3-((2-fluoro-5-(4-((1-(5-((3-fluoro-1H-indol-5-yl) (2,2,6,6-tetramethyltetrahydro-4H-pyran-4-ylidene)methyl)pyridin-2-yl) piperidin-4-yl) methyl) piperazin-1-yl)phenyl)amino) piperidin-2,6-dione (compound 83):

3-((4-bromo-2-fluorophenyl)amino)piperidin-2,6-dione (110 mg, 0.37 mmol), t-BuXPhos(16 mg, 0.04 mmol), and di-tert-butyl [2',4',6 '-tris(propane-2-yl)-[1,1'-biphenyl][1,1'-biphenyl]- 2-yl] phosphine were added to a solution of 3-fluoro-5-((6-(4- (piperazin-1-ylmethyl) piperidin-1-yl) pyridin) -3yl) (2,2,6,6-tetramethyltetrahydro-4H-pyran-4-ylidene) methyl)-1H- indazole (100 mg, 0.18 mmol) in 1,4-dioxane, and chlorine(2-dicyclohexylphosphino-2', 6'-di-isopropoxy-1,1'-biphenyl)(2-amino-1,1'-biphenyl-2-yl)palladium (II) (29 mg, 0.04 mmol) and Cs₂CO₃ (179 mg, 0.55 mmol) were added. The mixture was stirred overnight at 90 °C under a nitrogen atmosphere. The reaction was quenched by adding water at room temperature. The resulting mixture was extracted with ethyl acetate (3×20 mL). The combined organic layers were dried over anhydrous sodium sulfate. After filtration, the filtrate was concentrated under reduced pressure to obtain the crude product, which was purified by prep HPLC with the following conditions (column: Welch Utimate HS-C18,21.2* 250 mm, 7 µm; mobile phase A: water (0.05% TFA), mobile phase B: ACN; flow rate: 25 mL/min; gradient: 27% B to 50% B at 7 min, 50% B at 0.5 min; wavelength: 254/210 nm; RT1 (min): 6.45; numbers of operation: 0) to obtain 3-((2-fluoro-5-(4-((1-(5-((3-fluoro-1H-indazole -5-yl) (2,2,6,6-tetramethyltetrahydro-4H-pyran-4-ylidene)methyl)pyridin-2-yl) piperidin-4-yl) methyl) piperazin-1-yl) phenyl) amino) piperidin-2,6-dione (compound 83) as a white solid (9.5 mg, 6.21%). LCMS (ES, m/z) : 767.4[M+H]⁺;1H NMR (400 MHz, DMSO-d6) δ 10.86 (s, 1H), 8.79 (s, 1H), 7.61 (d, J =1.2 Hz, 1H), 7.56 (s, 1H), 7.42-7.39 (m, 1H), 7.29-7.27 (m, 2H), 7.08-7.06 (d, J = 8.0 Hz, 2H), 6.99-6.98 (m, 1H), 6.97-6.91 (m, 2H), 5.90 (s, 1H), 4.52 (s, 1H), 3.69-3.66 (d, J = 12.0 Hz, 5H), 3.23 (s, 4H), 2.79-2.70 (m, 1H), 2.66 - 2.56 (m, 4H), 2.35 (s, 1H), 2.23 (s, 2H), 2.17 (s, 2H), 2.11 (m, 2H), 1.82-1.79 (d, J = 11.6 Hz, 3H), 1.28 - 1.20 (m, 2H), 1.17-1.13 (d, J = 15.2 Hz, 12H).

According to the synthesis route of compound 83, the following compounds were synthesized by the following starting compounds or intermediates (see above scheme 9 for the synthesis steps, but the molar mass of each starting compound and intermediate was controlled unchanged):

| Comp ound No. | Starting material | intermediate | Compound structure |
|---|---|---|---|
| 84 | 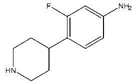 | 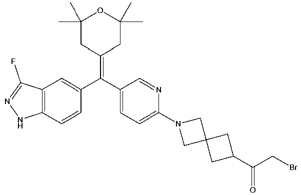 | 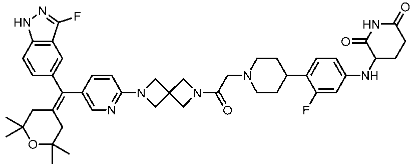 |
| 85 | 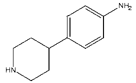 | 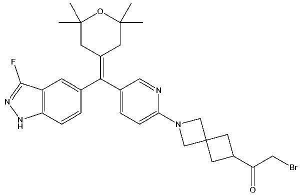 | 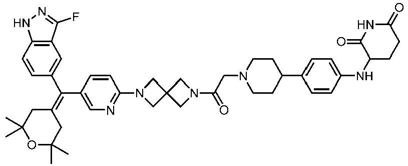 |
| 86 | 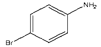 | 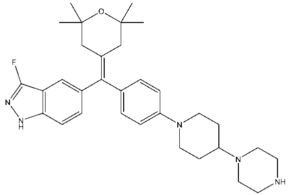 | 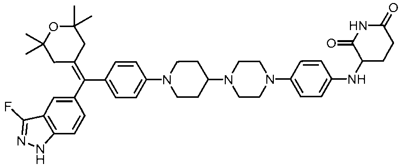 |
| 87 | 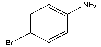 | 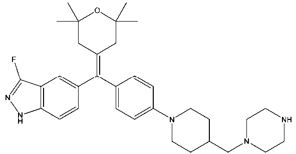 | 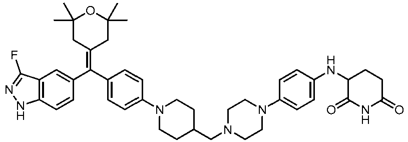 |
| 88 | 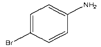 | 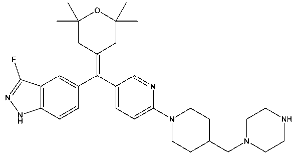 | 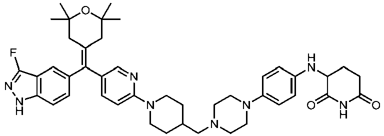 |
| 89 | 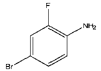 | 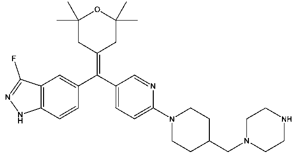 | 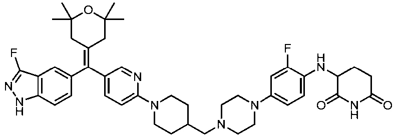 |
| 90 | 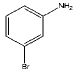 | 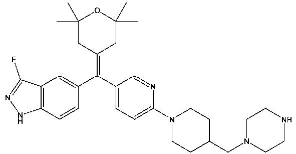 | 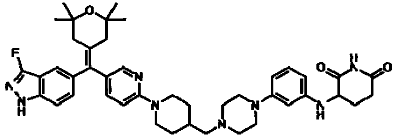 |
| 91 | 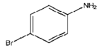 | 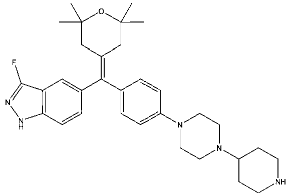 | 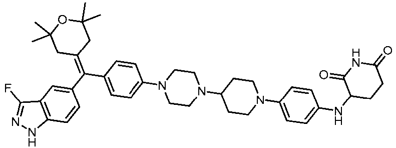 |

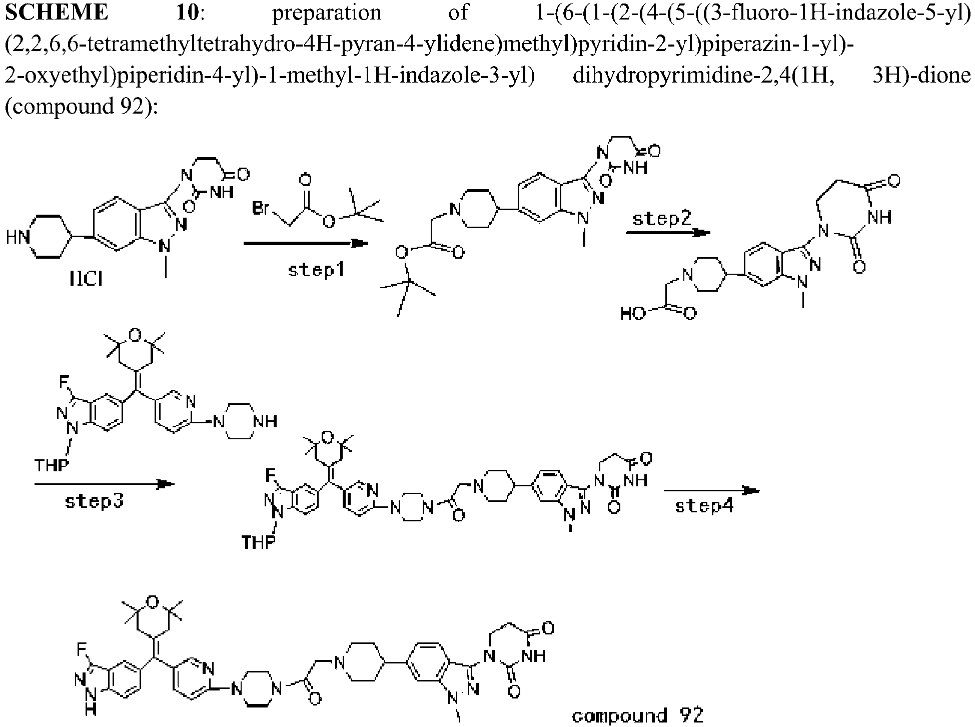

### Step 1: preparation of tert-butyl 2-(4-(3-(2,4-dioxotetrahydropyrimidin-1(2H)-yl)-1-methyl-1H-indazol-6-yl) piperidin-1-yl) acetate:

Tert-butyl bromoacetate (1.08 g, 5.5 mmol) and DIEA (3.23 g, 25 mmol) were added into a stirred solution of 1-(1-methyl-6-(piperidin-4-yl)-1H-indol-3-yl) dihydropyrimidine-2,4 (1H, 3H) - dione hydrochloride (1.80 g, *5* mmol) in MeCN (30 mL). The resulting mixture was stirred overnight at room temperature. The reaction was quenched by adding water at room temperature. The resulting mixture was extracted with DCM (3×50 mL). The combined organic layers were dried over anhydrous sodium sulfate. After filtration, the filtrate was concentrated under reduced pressure. The crude product was purified by silica gel column chromatography, and then eluted with DCM/MeoH (20:1) to give tert-butyl 2-(4-(3-(2,4-dioxotetrahydropyrimidin-1(2H)-yl)-1-methyl-1H-indazole-6-yl) piperidin-1-yl) acetate as a yellow oil (1.65 g, 75%), which was directly used in the next reaction without further purificaton. LCMS (ES, m/z) : 442.2[M+H]⁺.

### Step 2: preparation of 2-(4-(3-(2,4-dioxotetrahydropyrimidin-1 (2H)- yl) -1-methyl-1H-indazol-6-yl) piperidin-1-yl)acetic acid:

TFA (10 mL) was added to a solution of tert-butyl 2-(4-(3-(2,4-dioxotetrahydropyrimidin-1(2H) -yl) - 1-methyl-1H-indazole-6-yl) piperidin-1-yl) acetate (1.65 g, 3.74 mmol) in DCM (10 mL). The mixture was stirred overnight at room temperature under a nitrogen atmosphere. The resulting mixture was concentrated under reduced pressure. The residue was dissolved in DCM (50 mL) and washed with H₂O (50 mL). After separation, the aqueous phase was extracted with DCM (3×10 mL). The combined organic layers were dried over anhydrous sodium sulfate. After filtration, the filtrate was concentrated under reduced pressure to give 2-(4-(3- (2,4-dioxotetrahydropyrimidin-1 (2H)yl)-1-methyl-1H-indol-6-yl) piperidin-1-yl) acetic acid as a yellow oily liquid (1.15 g, 80%). The crude product was directly used in the next step without further purification. LCMS (ES, m/z) : 384[M+H]⁺.

### Step 3: preparation of 1-(6-(1-(2-(4-(5-((3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl)piperazin-1-yl)- 2-oxyethyl) piperidin-4-yl)-1-methyl-1H-indazole-3-yl)dihydropyrimidine-2,4 (1H, 3H) dione:

3-fluoro-5-((6-(piperazin-1-yl) pyridin-3-yl) (2,2,6,6-tetramethyltetrahydro-4H-pyran-4-ylidene) methyl)-1-(tetrahydro-2H-pyran-4-yl)-1H indazole(124 mg, 0.26 mmol), HATU (148 mg, 0.39 mmol) and DIEA (67 mg, 0.52 mmol) were added to a solution of 2-(4-(3-(2,4-dioxotetrahydropyrimidin-1(2H)yl)-1-methyl-1H-indol-6-yl) piperidin-1-yl) acetic acid (100 mg, 0.26 mmol) in DMA (2 mL). The resulting mixture was stirred overnight at room temperature under nitrogen atmosphere. The reaction was quenched by adding water (20 mL) and extracted with EA (4×10 mL). The combined organic layers were dried over anhydrous sodium sulfate and filtered. The filtrate was concentrated under reduced pressure to give a crude product, which was purified by silica gel column chromatography, and then eluted with DCM/MeOH (20:1) to obtain 1-(6-(1- (2-(4-(5-(3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl)piperazin-1-yl)- 2-oxyethyl) piperazin-4-yl)-1-methyl-1H-indazole-3-yl)dihydropyrimidine-2,4 (1H, 3H) dione (108 mg, 50%) LCMS (ES, m/z): 901.4[M+H]⁺.

### Step 4: preparation of 1-(6-(1-(2-(4-(5-(3-fluoro-1H-indazole-5-yl) (2,2,6,6-tetramethyltetrahydro-4H-pyran-4-ylidene)methyl)pyridin-2-yl)piperazin-1-yl)- 2-oxoethyl) piperidin-4-yl)-1-methyl-1H-indazole-3-yl)dihydropyrimidine-2,4 (1H, 3H)dione (compound 92):

TFA (0.5 mL) was added to a solution of 1-(6-(1-(2-(4-(5-(3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl)piperazin-1-yl)-2-oxyethyl)piperidin-4-yl)-1-methyl-1H-indazole-3-yl) dihydropyrimidine-2,4 (1H, 3H) dione (108 mg, 0.12 mmol) in DCM (1 mL).The mixture was stirred overnight at room temperature under a nitrogen atmosphere. The resulting mixture was concentrated under reduced pressure. The crude product was purified by prep-HPLC with the following conditions (column: Xselect CSH C18 OBD column 30x150 mm 5 µm, n; mobile phase A: water (0.1% TFA), mobile phase B: ACN; flow rate: 60 mL/min; gradient: 11% B to 41% B in 7 min; wavelength: 254 nm; RT1 (min): 5.89) to give 1-(6-(1-(2-(4- (5-((3-fluoro-1H-indazole-5-yl)(2,2,6,6-tetramethyltetrahydro-4H-pyran-4-ylidene)methyl) pyridin-2-yl)piperazin-1-yl)-2-oxyethyl)piperidin-4-yl)-1-methyl-1H-indazole -3-yl) dihydropyrimidine-2,4 (1H, 3H) -dione (compound 92) as a white solid (18 mg, 22%). LCMS (ES, m/z) : 817.4 [M+H]⁺; 1H NMR (400 MHz, DMSO-d6) δ (ppm): 12.48(s, 1H), 10.68(s, 1H), 8.32-8.15(m, 2H), 8.02-7.52(m, 5H), 7.36-7.27 (m, 1H), 6.59-6.54(m, 1H), 3.96 (s, 3H),3.85-3.52(m,10H), 3.28 (s, 2H), 2.75-2.68 (m, 3H), 2.55-2.50 (m, 2H),2.45-2.36 (m, 2H), 2.05-1.94(m, 4H), 1.76-1.58(m, 4H), 1.23-1.08(m, 12H)

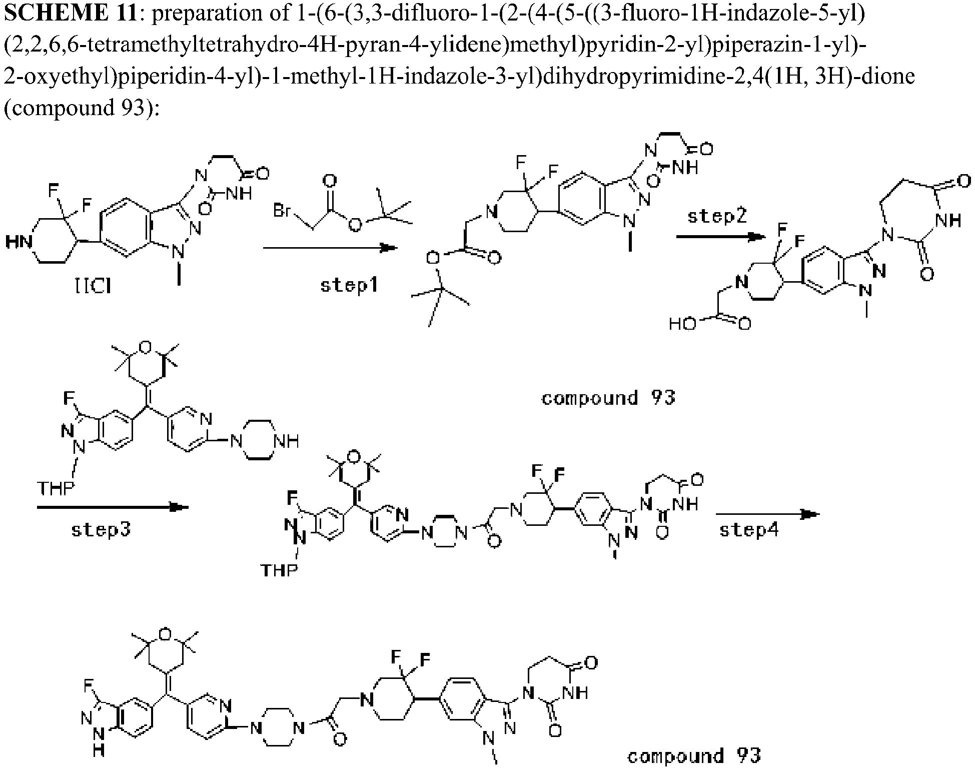

### Step 1: preparation of tert-butyl 2-(4-(3-(2,4-dioxotetrahydropyrimidin-1(2H)-yl)-1-methyl-1H-indol-6-yl)-3,3-difluoropiridine-1-yl)acetate:

Tert-butyl bromoacetate (1.08g, 5.5mmol) and DIEA (3.23 g, 25 mmol) were added into a stirred solution of 1-(6-(3,3-difluoropiridin-4-yl)-1-methyl-1H-indol-3-yl) dihydropyrimidine-2,4 (1H, 3H) - dione hydrochloride (1.80 g, 5 mmol) in MeCN (30 mL). The resulting mixture was stirred overnight at room temperature. The reaction was quenched by adding water at room temperature. The resulting mixture was extracted with DCM (3×50 mL). The combined organic layers were dried over anhydrous sodium sulfate. After filtration, the filtrate was concentrated under reduced pressure.

The crude product was purified by silica gel column chromatography, and then eluted with DCM/MeOH (20:1) to give tert-butyl 2- (4-(3-(2,4-dioxotetrahydropyrimidin-1 (2H)-yl)-1-methyl-1H-indol-6-yl)-3,3- difluoropyridine-1-yl) acetate as a yellow oil (1.65 g, 75%), which was directly used in the next reaction. LCMS (ES, m/z) : 478.2[M+H]⁺.

### Step 2: preparation of 2-(4-(3- (2,4-dioxotetrahydropyrimidin-1(2H)- yl) -1-methyl-1H-indol-6-yl) - 3,3-difluoropiridine-1-yl)acetic acid:

TFA (10ml) was added into a solution of tert-butyl 2-(4-(3-(2,4-dioxotetrahydropyrimidin-1(2H)-yl) -1-methyl-1H-indol-6-yl)-3,3 difluoropyridin-1-yl)acetate (1.65 g, 3.74 mmol) in DCM (10 mL). The mixture was stirred overnight at room temperature under a nitrogen atmosphere. The resulting mixture was concentrated under reduced pressure. The residue was dissolved in DCM (50 mL), washed with H₂O (50 mL) and separated. The aqueous phase was extracted with DCM (3 x10 mL). The combined organic layers were dried over anhydrous sodium sulfate and filtered. The filtrate was concentrated under reduced pressure to give 2-(4-(3-(2,4-dioxotetrahydropyrimidin-1(2H)yl) -1methyl-1H-indol-6-yl)-3,3- difluoropyridine-1-yl)acetic acid as a yellow oily liquid (1.15 g, 80%). The crude product was directly used in the next step without further purification. LCMS (ES, m/z) : 422[M+H]⁺.

### Step 3: preparation of 1-(6-(3,3-difluoro-1-(2-(4-(5-((3-fluoro-1-(tetrahydro-2H-pyran-4-yl)-1H-indazole-5-yl)(2,2,6,6-tetramethyltetrahydro-4H-pyran-4-ylidene)methyl)pyridin-2-yl) piperazin-1-yl)-2-oxyethyl) piperidin-4-yl)-1-methyl-1H-indazole-3-yl)dihydropyrimidine-2,4(1H, 3H)-dione:

3-fluoro-5-((6-(piperazine-1-yl)pyridin-3-yl) (2,2,6,6-tetramethyltetrahydro-4H-pyran-4-ylidene) methyl)-1-(tetrahydro-2H-pyran-4-yl)-1H indazole (124 mg, 0.26 mmol), HATU (148 mg, 0.39 mmol) and DIEA (67 mg, 0.52 mmol) were added into a solution of 2-(4-(3-(2,4-dioxotetrahydropyrimidin-1(2H)yl)-1-methyl-1H-indol-6-yl) -3,3-difluoropyridine-1-yl) acetic acid (100 mg, 0.26 mmol) in DMA (2 mL). The resulting mixture was stirred overnight at room temperature under a nitrogen atmosphere. The reaction was quenched by adding water (20 mL) and extracted with EA (4×10 mL). The combined organic layers were dried over anhydrous sodium sulfate and filtered. The filtrate was concentrated under reduced pressure to give a crude product. The crude product was purified by silica gel column chromatography, and then eluted with DCM/MeoH (20:1) to obtain 1-(6-(3,3-difluoro-1- (2- (4- (5- ((3-fluoro-1-(tetrahydro-2H-pyran-4-yl)-1H-indazole-5-yl) (2,2,6,6-tetramethyltetrahydro-4H-pyran-4-ylidene)methyl) pyridin-2-yl) piperazin-1-yl)-2-oxyethyl) piperazin-4-yl)-1-methyl-1H-indazole-3-yl)dihydropyrimidine-2,4(1H, 3H)-dione (108 mg, 50%) LCMS (ES, m/z): 937.4[M+H]⁺.

### Step 4: preparation of 1-(6-(3,3-difluoro-1-(2-(4-(5-(3-fluoro-1H-indazole-5-yl) (2,2,6,6-tetramethyltetrahydro-4H-pyran-4-ylidene)methyl)pyridin-2-yl)piperazin-1-yl)- 2-oxyethyl) piperidin-4-yl)-1-methyl-1H-indazole-3-yl) dihydropyrimidine-2,4 (1H, 3H) - dione (compound 93):

TFA (0.5 ml) was added into a solution of 1- (6-(3,3-difluoro-1-(2-(4-(5-((3-fluoro-1-(tetrahydro-2H-pyran-4-yl)-1H-indazole-5-yl) (2,2,6,6-tetramethyltetrahydro-4H-pyran-4-ylidene)methyl) pyridin-2-yl)piperazin-1-yl)- 2-oxyethyl) piperidin-4-yl)-1-methyl-1H-indazole-3-yl) dihydropyrimidine-2,4(1H, 3H) -dione (108 mg, 0.12 mmol) in DCM (1 mL). The mixture was stirred overnight at room temperature under a nitrogen atmosphere. The resulting mixture was concentrated under reduced pressure. The crude product was purified by prep-HPLC with the following conditions (column: Xselect CSH C18 OBD column 30×150mm 5 µm, n; mobile phase A: water (0.1% TFA), mobile phase B: ACN; flow rate: 60ml/min; gradient: 11% B to 41% B in 7 min; Wavelength: 254 nm; RT1 (min): 5.89) to give 1-(6-(3,3-difluoro-1-(2-(4-(5-((3-fluoro-1H-indazole-5-yl)(2,2,6,6-tetramethyltetrahydro -4H-pyran-4-ylidene)methyl) pyridin-2-yl)piperazin-1-yl)-2-oxyethyl)piperidin-4-yl)-1-methyl- 1H-indazole-3-yl) dihydropyrimidine-2,4 (1H, 3H) - dione (compound 93) (18 mg, 22%) as a white solid. LCMS (ES, m/z) : 853.4 [M+H]⁺; 1H NMR (400 MHz, DMSO-d6) δ (ppm):12.44(s, 1H), 10.66(s, 1H), 8.35-8.17(m, 2H), 8.05-7.52(m, 5H), 7.38-7.28(m, 1H), 6.59-6.55(m, 1H),4.22-4.16 (m, 1H),3.96 (s, 3H),3.85-3.52 (m,10H), 3.28 (s, 2H), 2.75-2.68 (m, 2H), 2.56-2.50 (m, 2H),2.46-2.38(m, 2H), 2.05-1.95(m, 4H), 1.80-1.58(m, 2H), 1.24-1.08(m, 12H).

According to the synthesis route of compound 93, the following compounds were synthesized by the following starting compounds or intermediates (see above scheme 11 for the synthesis steps, but the molar mass of each starting compound and intermediate was controlled unchanged):

| Compo und No. | Starting material | intermediate | Compound structure |
|---|---|---|---|
| 94 | N/A/ | 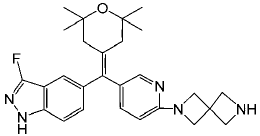 | 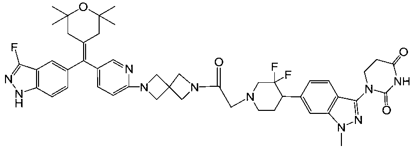 |
| 95 | 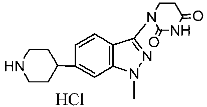 | 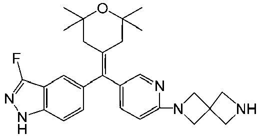 | 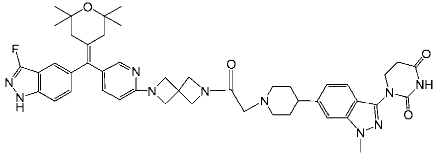 |
| 124 | 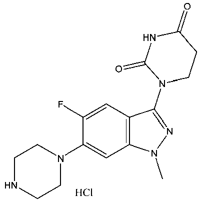 | 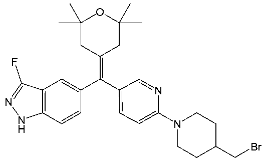 | 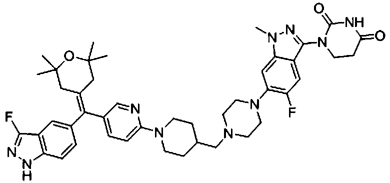 |
| 125 | 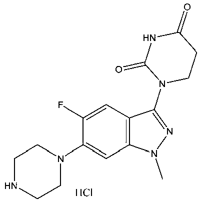 | 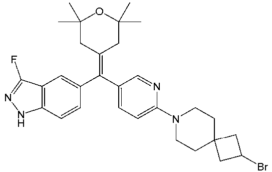 | 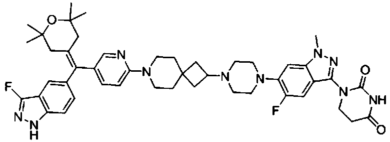 |

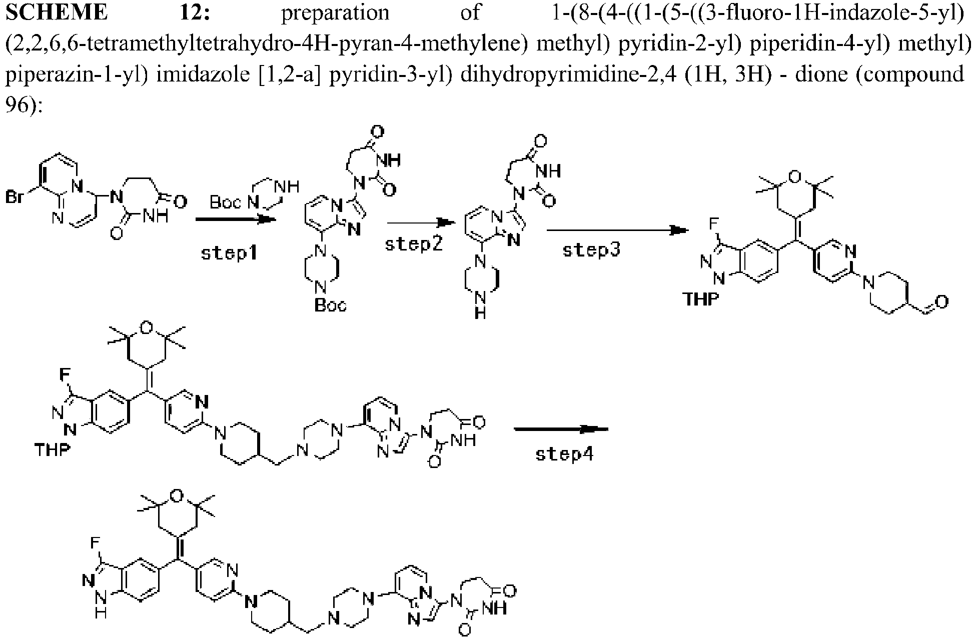

### Step 1: preparation of tert-butyl 4- (3-(2,4-dioxotetrahydropyrimidin-1(2H)-yl)imidazo[1,2-a] pyridin-8-yl)piperazine-1-carboxylate:

Tert-butyl piperazine-1-carboxylate(450 mg, 1.8 mmol), RuPhos Palladacycle G3(104 mg, 0.12 mmol), RuPhos (58 mg, 0.12 mmol) and t-BuONa (179 mg, 1.8 mmol) were added into a solution of 1- (8-bromoimidazo [1,2-a] pyridin-3-yl) dihydropyrimidine-2,4 (1H, 3H) - dione (300 mg, 0.6 mmol) in THF (5 mL). The resulting mixture was stirred overnight at 85 °C under a nitrogen atmosphere. The reaction was quenched by adding water at room temperature. The resulting mixture was extracted with EA (3×20 mL). The combined organic layers were dried over anhydrous sodium sulfate. After filtration, the filtrate was concentrated under reduced pressure. The residue was purified by preparative thin-layer chromatography (DCM/MeoH 10:1) to give tert-butyl 4-(3-(2,4-dioxotetrahydropyrimidin-1(2H)-yl)imidazo[1,2-a]pyridin-8-yl)piperazine-1-carboxylate as a yellow oil (200 mg, 46.81%). LCMS(ESI): m/z 415[M+H]⁺.

### Step 2: preparation of 1-(8-(piperazine-1-yl)imidazo[1,2-a]pyridin-3-yl)dihydropyrimidine-2,4 (1H, 3H) - dione:

TFA (2 mL) was added into a solution of tert-butyl 4-(3-(2,4-dioxotetrahydropyrimidin-1 (2H)-yl)imidazo[1,2-a]pyridin-8-yl)piperazine-1-carboxylate (180 mg, 0.3 mmol) in DCM (8 mL). The mixture was stirred overnight at room temperature under a nitrogen atmosphere. The resulting mixture was concentrated under reduced pressure to give 1-(8 (piperazine-1-yl)imidazo [1,2-a] pyridin-3-yl) dihydropyrimidine-2,4 (1H, 3H) - dione as a yellow oil (110 mg, 83.46%). The crude product was directly used in the next step without further purification. LCMS(ESI):m/z 315[M+H]⁺.

### Step 3: preparation of 1-(8-(4-((1-(5-((3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl) piperidin-4-yl)methyl) piperazin-1-yl)imidazole[1,2-a]pyridin-3-yl)dihydropyrimidine-2,4(1H, 3H) - dione:

STAB (106 mg, 0.5 mmol) was added into a stirred solution of (8- (piperazin-1-yl) imidazo [1,2-a] pyridin-3-yl) dihydropyrimidine-2,4 (1H, 3H) - Dione (88 mmol, 0.24 mmol) and 1-(5-((3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl) piperidine-4-formaldehyde (134.5 mmol, 0.24 mmol) in DMA (1 mL) at room temperature. The resulting mixture was stirred overnight at room temperature. The reaction was quenched by adding water at room temperature. The resulting mixture was extracted with DCM (3×10 mL). The combined organic layers were washed with water (3×5 mL) and dried over anhydrous sodium sulfate. After filtration, the filtrate was concentrated under reduced pressure to give 1-(8-(4-((1-(5-((3-fluoro-1- (tetrahydro-2H-pyran-4-yl)-1H-indazole-5-yl) (2,2,6,6-tetramethyltetrahydro-4H-pyran-4-ylidene)methyl)pyridin-2-yl) piperidin-4-yl) methyl) piperazin-1-yl) imidazole [1,2-a] pyridin-3-yl) dihydropyrimidine-2,4(1H, 3H)-dione as a yellow oily liquid (131 mg, 60%). LCMS(ESI):m/z 859.4 [M+H]⁺.

### Step 4: preparation of 1-(8-(4-((1-(5-((3-fluoro-1H-indazole-5-yl)(2,2,6,6-tetramethyltetrahydro-4H-pyran-4-ylidene)methyl)pyridin-2-yl) piperidin-4-yl) methyl) piperazin-1-yl) imidazole [1,2-a] pyridin-3-yl)dihydropyrimidine-2,4 (1H, 3H) - dione (compound 96):

TFA (0.5 mL) was added into a solution of 1-(8-(4-((1-(5-((3-fluoro-1-(tetrahydro-2H-pyran-4-yl)-1H-indazole-5-yl) (2,2,6,6-tetramethyltetrahydro-4H-pyran-4-ylidene) methyl)pyridin-2-yl) piperidin-4-yl) methyl) piperazin-1-yl) imidazole [1,2-a] pyridin-3-yl) dihydropyrimidine-2,4 (1H, 3H)-dione (130 mg, 0.14 mmol) in DCM (1 mL). The mixture was stirred overnight at room temperature under a nitrogen atmosphere. The resulting mixture was concentrated under reduced pressure. The crude product (90 mg) was purified by Prep-HPLC with the following conditions (column: Xselect CSH C18 OBD column 30×150 mm 5 µm, n; mobile phase A: water (0.1% TFA), mobile phase B: ACN; flow rate: 60 mL/min; gradient: 11% B to 41% B in 7 min; wavelength: 254nm; RT1 (min): 5.89) to give 1-(8-(4-((1-(5-((3-fluoro-1H-indazole-5-yl)(2,2,6,6-tetramethyltetrahydro-4H-pyran-4-methylene)methyl) pyridin-2-yl)piperidin-4-yl)methyl)piperazin-1-yl)imidazole[1,2-a]pyridin-3-yl) dihydropyrimidine-2,4(1H,3H)-dione (compound 96) as a white solid (35.4 mg, 30%). LCMS(ESI):m/z 775.4 [M+H]⁺. 1H NMR (400 MHz, DMSO-d6) δ (ppm):12.55 (s, 1H), 10.96 (s, 1H),9.37(s, 1H), 7.64-7.58(d, J = 8.4 Hz, 1H), 7.52-7.42(m, 2H), 7.26-7.22 (m, 1H), 7.18-7.11 (m, 2H), 6.99-6.96 (d, J = 13.2 Hz, 1H), 5.45(s, 1H), 5.11-5.01 (m,1H), 4.41-4.18 (m, 2H), 3.98-3.81 (m, 4H), 3.72-3.58 (s, 6H), 3.45-3.17 (m, 3H), 2.95-2.88 (m, 1H), 2.69-2.52 (m, 2H), 2.44-2.36 (m, 1H), 2.22(s, 2H), 2.15(s, 1H), 2.01-1.94(m, 1H), 1.84-1.73(m, 4H), 1.75-1.56(m, 4H), 1.22-1.04(m, 12H).

According to the synthesis route of compound 96, the following compounds were synthesized by the following starting compounds or intermediates (see above scheme 12 for the synthesis steps, but the molar mass of each starting compound and intermediate was controlled unchanged):

| Com poun d No. | Starting material | intermediate | Compound structure |
|---|---|---|---|
| 97 | N/A | 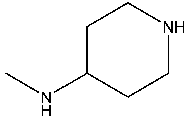 | 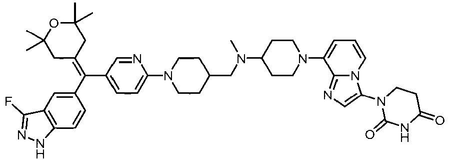 |
| 98 | 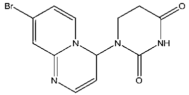 | 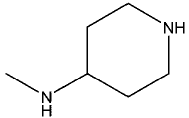 | 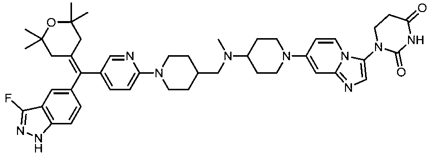 |

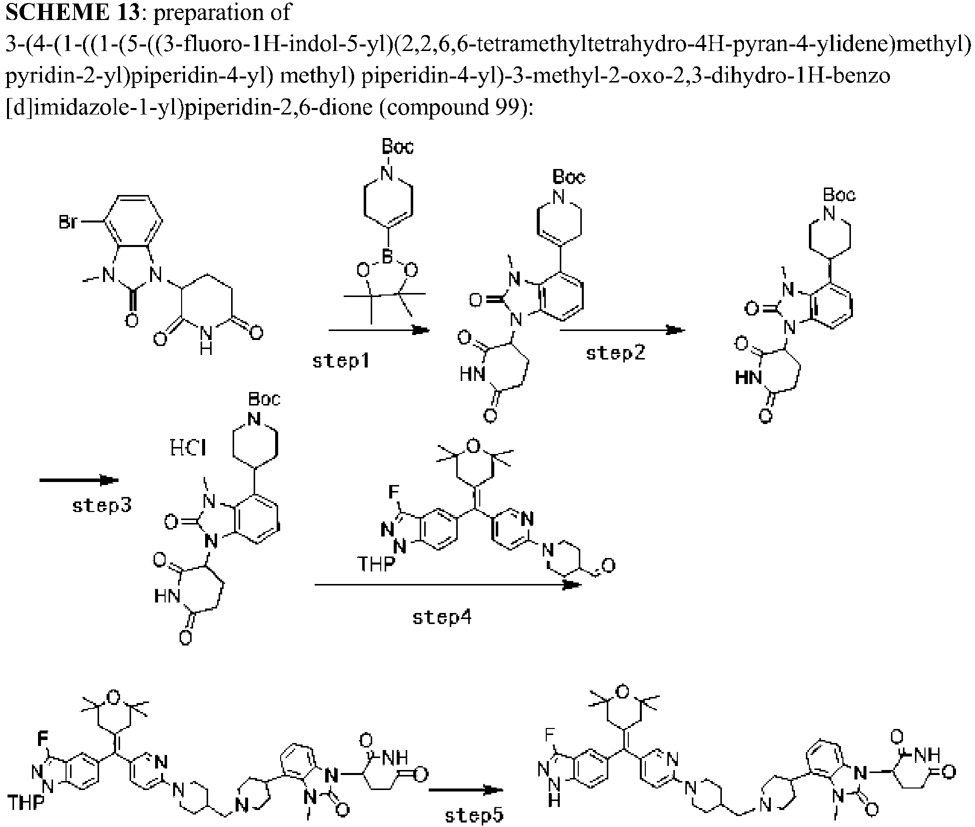

### Step 1: preparation of tert-butyl 4-(1-(2,6-dioxypiperidin-3-yl)-3-methyl-2-oxo-2,3-dihydro-1H-benzo[d]imidazol-4-yl)-3,6-dihydropyridine-1(2H)-carboxylate:

Cesium fluoride (1.14g, 7.40mmol) and Pd(dppf)Cl₂ (540mg, 740 µmol) were added into a solution of 3- (4-bromo-3-methyl-2-oxo-2,3-dihydro-1H-benzo[d]imidazol-1-yl) piperidine-2,6-dione (1.25g,3.70mmol) and tert-butyl 4-(4,4,5,5-tetramethyl-1,3,2-dioxaborolan-2-yl) -3,6-dihydropyridine-1(2H)-carboxylate(2.31 g, 7.40 mmol) in 1,4-dioxane (20 mL) under a nitrogen atmosphere. The mixture was stirred at 85 °C for 3 h. The mixture was cooled to room temperature, diluted with ethyl acetate (50 mL) and filtered through diatomite / silica gel. The filtrate was washed with water, and dried over Na₂SO₄, and then filtered. After washing with ethyl acetate (20 mL), the filtrate was concentrated. The residue was purified by silica gel chromatography (EA:PE=10:1) to obtain tert-butyl4-(1-(2,6-dioxypiperidin-3-yl)-3-methyl-2-oxo -2,3-dihydro-1H-benzo[d] imidazol-4-yl) -3,6-dihydropyridine-1(2H)-carboxylate (980 mg, yield 60%). LCMS (ESI) m/z: 441.2[M+H]⁺.

### Step 2: preparation of tert-butyl 4-(1-(2,6-dioxypiperidin-3-yl)-3-methyl-2-oxo-2,3-dihydro-1H-benzo[d] imidazol-4-yl)piperidine-1-carboxylate:

Palladium (10%/carbon, 0.95g, 0.89mmol) was added into a solution of 4-(1-(2,6-dioxypiperidin-3-yl)-3-methyl-2-oxo-2,3-dihydro-1H-benzo[d]imidazol-4-yl)-3,6-dihydropyridine-1(2H)-carboxylate (980 mg, 2.22 mmol) in methanol (20 mL). The mixture was stirred for 24 hours at room temperature under a hydrogen atmosphere, and then the reaction mixture was filtered through diatomite. The filter cake was washed with dichloromethane (20 mL×3). The combined organic layers were concentrated under reduced pressure to give tert-butyl 4-(1-(2,6-dioxypiperidin-3-yl)- 3-methyl-2-oxo-2,3-dihydro-1H-benzo[d]imidazol-4-yl) piperidine-1-carboxylate (930 mg, 94% yield). LCMS (ESI) m/z: 443.2 [M+H]⁺.

### Step 3: preparation of 3-(3-methyl-2-oxo-4-(piperidin-4-yl)-2,3-dihydro-1H-benzo[d] imidazol-1-yl) piperidine-2,6-dione hydrochloride:

Tert-butyl 4- (1-(2,6-dioxypiperidin-3-yl)-3-methyl-2-oxo-2,3-dihydro-1H-benzo [d] imidazol-4-yl) piperidine-1-carboxylate (930 mg, 2.09 mmol) was dissolved in a solution of hydrochloric acid in ethyl acetate (10 mL), and the mixture was stirred overnight. The resulting mixture was concentrated under reduced pressure. The residue was washed and mashed with ethyl acetate. 3-(3-methyl-2-oxo-4-(piperidin-4-yl)-2,3-dihydro-1H-benzo[d]imidazol-1-yl) piperidin-2,6-dione hydrochloride was obtained (610 mg, 85% yield). LCMS (ESI) m/z: 343.2 [M+H]⁺.

### Step 4: preparation of 3-(4-(1-((1-(5-((3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl) piperidin-4-yl)methyl) piperidin-4-yl)-3-methyl-2-oxo-2,3-dihydro-1H-benzo[d] imidazol-1-yl) piperidin-2,6-dione:

3-(3-methyl-2-oxo-4-(piperidin-4-yl)- 2,3-dihydro-1H-benzo[d] imidazol-1-yl) piperidine-2,6-dione hydrochloride(69mg, 0.2 mmol) was added into a stirred solution of 1-(5-((3-fluoro-1-(tetrahydro-2H-pyran-4-yl)-1H-indazole-5-yl) (2,2,6,6-tetramethyltetrahydro-4H-pyran-4-ylidene) methyl)pyridine-2-yl) piperidine-4-formaldehyde (100 mg, 0.2 mmol) in DCM (2 mL), and treated with NaOAc (82.4 mg, 1mmol) for 2 hours at room temperature before STAB (76 mg, 0.4 mmol) was added. The resulting mixture was stirred overnight under a nitrogen atmosphere at room temperature. The reaction was quenched by adding water. The resulting mixture was extracted with DCM (3×10 mL). The combined organic layers were dried over anhydrous sodium sulfate. After filtration, the filtrate was concentrated under reduced pressure.

3-(4-(1-(1-(5-((3-fluoro-1-(tetrahydro-2H-pyran-4-yl)-1H-indol-5-yl) (2,2,6,6-tetramethyltetrahydro-4H-pyran-4-ylidene)methyl)pyridin-2-yl) piperidin-4-yl) methyl) piperidin-4-yl)-3-methyl-2-oxo-2,3-dihydro-1H-benzo[d]imidazol-1-yl) piperidin-2,6-dione as a yellow oily liquid (108 mg, 61%) was obtained by purification using pre thin layer chromatography (PE/EA 1:1). LCMS (ESI) m/z: 887.5[M+H]⁺.

### Step 5: preparation of 3-(4-((1-(5-((3-fluoro-1H-indol-5-yl)

### (2,2,6,6-tetramethyltetrahydro-4H-pyran-4-ylidene)methyl)pyridin-2-yl) piperidin-4-yl) methyl) piperidin-4-yl)-3-methyl-2-oxo-2,3-dihydro-1H-benzo[d]imidazol-1-yl)piperidin-2,6-dione (compound 99):

In a 50 mL round bottom flask, DCM (8 mL), TFA (2 mL) and 3-(4-(1-(1-(5-((3-fluoro-1-(tetrahydro-2H-pyran-4-yl)- 1H-indazole-5-yl) (2,2,6,6-tetramethyltetrahydro-4H-pyran-4-ylidene) methyl)pyridin-2-yl) piperidin-4-yl) methyl) piperidin-4-yl)-3-methyl-2-oxo-2,3-dihydro-1H-benzo [d]imidazol-1-yl) piperidin-2,6-dione (108 mg, 0.12 mmol) were added. The resulting mixture was stirred overnight under a nitrogen atmosphere at room temperature. The resulting mixture was concentrated under reduced pressure to give a crude product, which was purified by prep-HPLC with the following conditions (column: Xselect CSH C18 OBD column 30×150 mm 5 µm, n; mobile phase A: water (0.05% TFA), mobile phase B: ACN; flow rate: 60 mL/min; gradient: 16% B to 36% B in 7 min, 36% B; wavelength: 254nm; RT1 (min): 5.68; operation times: 0) to give 3-(4-(1-((1-(5-((3-fluoro-1H-indol-5-yl) (2,2,6,6-tetramethyltetrahydro-4H-pyran-4-ylidene)methyl)pyridin-2-yl) piperidin-4-yl)methyl) piperidin-4-yl)-3-methyl-2-oxo-2,3-dihydro-1H-benzo[d]imidazol-1-yl)piperidin-2,6-dione (compound 99) as a white solid (44 mg, 27%). LCMS (ESI) m/z: 803.3 [M+H]⁺;1H NMR (400 MHz, DMSO-d6) δ (ppm): 12.52(s, 1H), 10.96(s, 1H), 9.46(s, 1H), 7.98-7.58(m, 3H), 7.50-7.44 (m, 2H),7.26-7.16 (m, 3H), 7.03-6.99 (d, J = 6.8 Hz, 1H), 5.10-5.05 (m, 1H), 4.39-4.34 (d, J = 17.2 Hz, 2H), 4.28-4.24(m, 1H), 4.034-4.00 (d, J = 10.0 Hz, 3H), 3.86 (s, 3H),3.65-3.63 (d, J = 8.4 Hz, 2H), 3.24-3.12 (m, 5H), 2.95-2.88 (m, 3H), 2.68-2.62(m, 2H), 2.41-2.34 (m, 1H), 2.25(s, 2H), 2.16 (s, 2H), 1.96-1.94(m, 1H), 1.86-1.83(d, J = 12.3 Hz, 2H), 1.22-1.10(m, 12H).

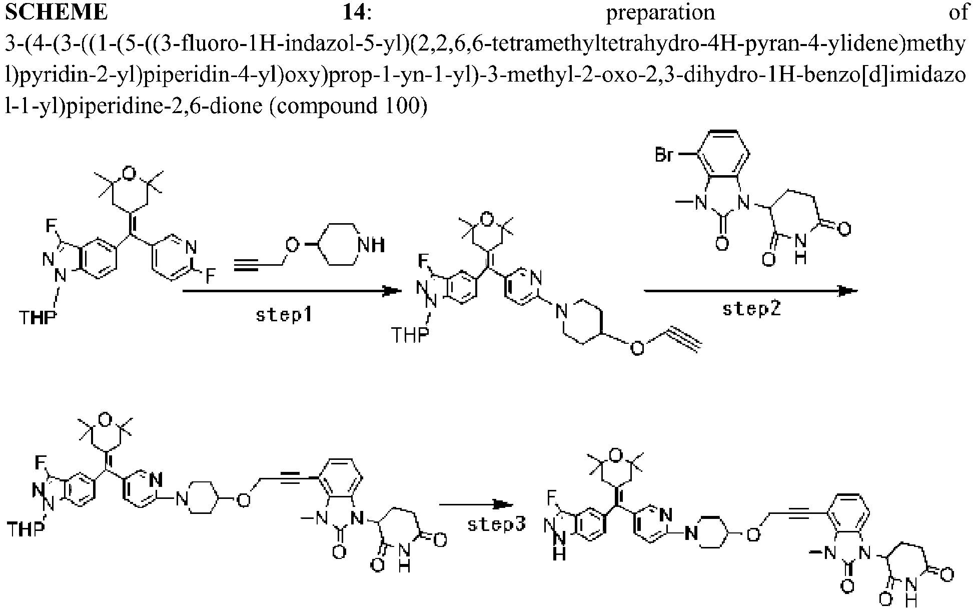

### Step 1: preparation of 3-fluoro-5-((6-(4-(propyl-2-yn-1-yloxy)piperidin-1-yl)pyridin-3-yl) (2,2,6,6-tetramethyltetrahydro-4H-pyran-4-ylidene)methyl)-1-(tetrahydro-2H-pyran-4-yl)-1H indazole:

DIEA (1.65 g, 12.74 mmol) was added dropwise into a stirred solution of 4-(prop-2-yn-1-yloxy)piperidine (780 mg, 5.60 mmol) and 3-fluoro-5-((6-fluoropyridin-3-yl) 2,2,6,6-tetramethyltetrahydro-4H-pyran-4-ylidene)methyl)-1-(tetrahydro-2H-pyran-4-yl)-1H indazole (1.19 g, 2.55 mmol) in DMSO (10 mL) at room temperature under a nitrogen atmosphere. The resulting mixture was stirred overnight at 120 °C under a nitrogen atmosphere. The reaction was quenched by adding water at room temperature. The resulting mixture was extracted with EtOAc (3×50 mL). The combined organic layers were washed with water (4×5 mL) and dried over anhydrous sodium sulfate. After filtration, the filtrate was concentrated under reduced pressure. The residue was purified by silica gel column chromatography and eluted with PE/ electro EA (1:1) to give 3-fluoro-5-((6-(4-(propyl-2-yn-1-yloxy)piperidin-1-yl)pyridin-3-yl} (2,2,6,6-tetramethyltetrahydro-4H-pyran-4-ylmethylene)methyl)-1-(tetrahydro-2H-pyran-4-yl)-1H indazole as a yellow oily liquid (400mg, 26.76%). LCMS (ESI) m/z: 587.3[M+H]⁺.

### Step 2: Preparation of 3-(4-(3-(1-(5-(3-fluoro-1-(tetrahydro-2H-pyran-4-yl)-1H-indazole-5-yl) (2,2,6,6-tetramethyltetrahydro-4H-pyran-4-ylidene)methyl)pyridin-2-yl)piperidin-4-yl)oxy) prop-1-yn-1-yl)-3-methyl-2-oxo-2,3-dihydro-1H-benzo[d]imidazol-1-yl) piperidine-2,6-dione 3- (4-bromo-3-methyl-2-oxo-2,3-dihydro-1H-benzo [d] imidazol-1-yl) piperidin-2,6-dione (150mg, 0.44mmol), 3-fluoro-5-((6-(4-(propyl-2-yn-1-yloxy)piperidin-1-yl)pyridin-3-yl)

(2,2,6,6-tetramethyltetrahydro-4H-pyran-4-ylidene)methyl)-1-(tetrahydro-2H-pyran-4-yl)-1H indazole (390 mg, 0.67 mmol), Pd (PPh₃)₄ (103 mg, 0.09 mmol), and 2-methylpropane-2-amine (97 mg, 1.33 mmol) were added into 1,4-dioxane (4 mL). The mixture was stirred overnight at 80 °C under a nitrogen atmosphere. The reaction was quenched by adding water at room temperature. The resulting mixture was extracted with ethyl acetate (3×20 mL). The combined organic layers were dried over anhydrous sodium sulfate. After filtration, the filtrate was concentrated under reduced pressure. The residue was purified by pre thin layer chromatography (DCM/MeOH 10:1) to give 3-(4-(3-((1-(5- ((3-fluoro-1-(tetrahydro-2H-pyran-4-yl)-1H-indazole-5-yl) (2,2,6,6-tetramethyltetrahydro-4H-ylidene)methyl)pyridin-2-yl)piperidin-4-yl)oxy)prop-1-yn-1-yl) - 3-methyl-2-oxo-2,3-dihydro-1H-benzo[d]imidazol-1-yl)piperidin-2,6-dione (150 mg, 40.07%) as a yellow oily liquid. LCMS (ESI) m/z: 844.4 [M+H]⁺.

### Step 3: preparation of 3-(4-(3-(1-(5-((3-fluoro-1H-indol-5-yl)

### (2,2,6,6-tetramethyltetrahydro-4H-pyran-4-ylidene)methyl)pyridin-2-yl)piperidin-4-yl)oxy) propyl-1-yn-1-yl)-3-methyl-2-oxo-2,3-dihydro-1H-benzo[d] imidazol-1-yl)piperidine-2,6-dione (compound 100):

TFA (1.5 mL) was added into a solution of 3-(4-(3-(1-(5-(3-fluoro-1-(tetrahydro-2H-pyran-4-yl)-1H-indazole-5-yl) (2,2,6,6-tetramethyltetrahydro-4H-pyran-4-ylidene) methyl)pyridin-2-yl)piperidin-4-yl)oxy)prop-1-yn-1-yl)-3-methyl-2-oxo-2,3-dihydro-1H-benzo[d] imidazol-1-yl)piperidin-2,6-dione (150 mg, 0.18 mmol) in DCM (0.6 mL). The mixture was stirred overnight at room temperature under a nitrogen atmosphere. The resulting mixture was concentrated under reduced pressure. The crude product was purified by Prep-HPLC with the following conditions (column: XselectCSH Prep Fluoro-Phenyl OBD C18 column, 30×150 mm, 5 µm; mobile phase A: water (0.05% TFA), mobile phase B: ACN; fow rate: 60 mL/min; gradient: 28% B to 43% B in 8 min; wavelength: 254 nm/220nm; RT1 (min): 6.68) to give 3-(4-(3-((1-(5-((3-fluoro-1H-indol-5-yl) (2,2,6,6-tetramethyltetrahydro-4H-pyran-4-ylidene)methyl)pyridin-2-yl)piperidin-4-yl)oxy) propyl-1-yn-1-yl)-3-methyl-2-oxy-2,3-dihydro-1H-benzo [D] imidazol-1-yl) piperidin-2,6-dione (compound 100) as a white solid (52.7 mg, 37.97%). LCMS (ESI) m/z: 760.3[M+H]⁺;1H NMR (400 MHz, DMSO-d6) δ 12.57 (s, 1H), 11.14 (s, 1H), 7.90 (d, J = 2.0 Hz, 1H), 7.60 - 7.55 (m, 2H), 7.46 - 7.43 (m, 1H), 7.26 - 7.24 (m, 1H), 7.19 - 7.11 (m, 3H), 7.05 - 7.01 (m, 1H), 5.43-5.38 (m, 1H), 4.54 (s, 2H), 3.90-3.84 (m, 3H), 3.65 (s, 3H), 3.37 - 3.35 (m, 2H), 2.95 - 2.82 (m, 1H), 2.73 - 2.60 (m, 2H), 2.23 - 2.15 (d, J = 32.4 Hz, 4H), 2.04 - 2.01 (m, 3H), 1.59 - 1.57 (s, 2H), 1.18 - 1.13 (d, J = 22.4 Hz, 12H).

According to the synthesis route of compound 100, the following compounds were synthesized by the following starting compounds or intermediates (see above scheme 14 for the synthesis steps, but the molar mass of each starting compound and intermediate was controlled unchanged):

| Compound No. | intermediate | Compound structure |
|---|---|---|
| 101 | 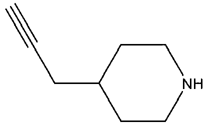 | 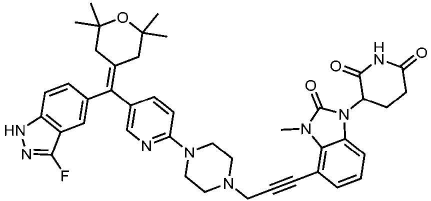 |

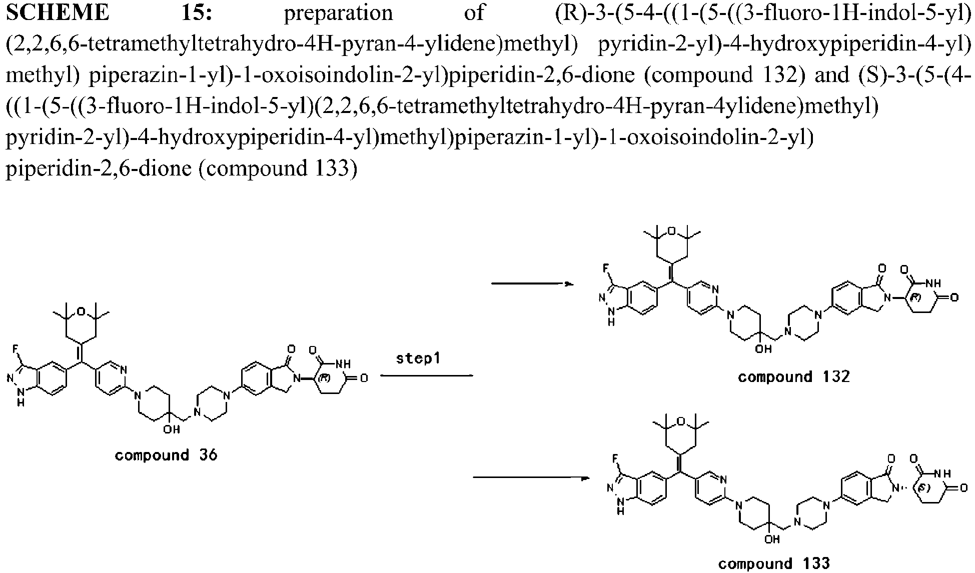

### Step 1:

### 3-(5-(4-((1-(5-((3-fluoro-1H-indol-5-yl)(2,2,6,6-tetramethyltetrahydro-4H-pyran-4-ylidene)methyl) pyridin-2-yl)-4-hydroxypiperidin-4-yl)methyl)piperazin-1-yl)-1-oxoisoindolin-2-yl)

piperidine-2,6-dione (compound 36) (50 mg) was purified by the following conditions (CHIRAL-HPLC: column: JW-CHIRAL ART cellulose -SB, 4.6*100 mm; 3 µ m; flow phase A: Hex (0.1 A): (IPA: DCM = 1:2)=50:50; flow rate: 1 mL/min; gradient: 0% B to 0% B; injection volume: 5 µL mL) for the preparation of (S)-3-(5-4-((1-(5-((3-fluoro-1H-indol-5-yl) (2,2,6,6-tetramethyltetrahydro-4H-pyran-4-ylidene) methyl) pyridin-2-yl)- 4-hydroxypiperidin-4-yl) methyl)piperazin-1-yl)-1-oxoisoindolin-2-yl) piperidin-2,6-dione (compound 133) as a white solid (6.0 mg); LCMS (ES, m/z) : 774.9[M+H]⁺; Chiral HPLC: Rt = 3.423; and (R) -3-(5-4-((1-(5-((3-fluoro-1H-indol-5-yl) (2,2,6,6-tetramethyltetrahydro-4H-pyran-4-ylidene)methyl) pyridin-2-yl)-4-hydroxypiperidin-4-yl)methyl)piperazin-1-yl)-1-oxoisoindolin-2-yl) piperidin-2,6-dione (compound 132) as a white solid (5.6 mg); LCMS (ES, m/z) : 774.9[M+H]⁺ Chiral HPLC: Rt = 4.619.

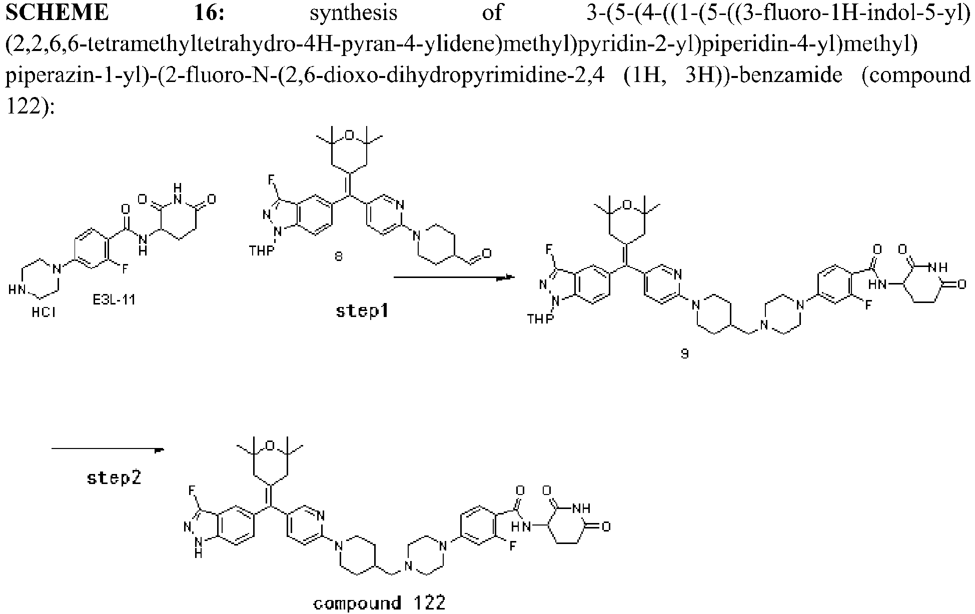

### Step 1: preparation of 3-(5-(4-((1-(5-((3-fluoro-1-(tetrahydro-2H-pyran-4-yl)-1H-indole-5-yl)] (2,2,6,6-tetramethyltetrahydro-4H-pyran-4-ylidene)methyl)pyridin-2-yl)piperidin-4-yl)methyl) piperazin-1-yl)-(2-fluoro-N-(2,6-dioxo-dihydropyrimidine-2,4(1H, 3H)) - benzamide:

2- (2,6-dioxo-piperidine-3-yl) - 2-fluoro-4 - (piperazine-1-yl) benzamide hydrochloride solution (70 mg, 0.2 mmol) was added into a stirred solution of 1-(5-((3-fluoro-1-(tetrahydro-2H-pyran-4-yl)-1H-indazole-5-yl)(2,2,6,6-tetramethyltetrahydro-4H-pyran-4-ylidene)methyl)pyridin-2-yl) piperidine-4-formaldehyde (100 mg, 0.2 mmol) in DCM (2 mL), and STAB(0.76 mg, 0.4 mmol) at room temperature. The resulting mixture was stirred overnight under a nitrogen atmosphere at room temperature. The reaction was quenched by adding water. The resulting mixture was extracted with DCM (3×10 mL). The combined organic layers were dried over anhydrous sodium sulfate. After filtration, the filtrate was concentrated under reduced pressure. The residue was purified by pre-thin layer chromatography (PE/EA 1:1), and 3-(5-(4-((1-(5-((3-fluoro-1-(tetrahydro-2H-pyran-4-yl)-1H-indol-5-yl)] (2,2,6,6-tetramethyltetrahydro-4H-pyran-4-ylidene)methyl)pyridin-2-yl)piperidin-4-yl)methyl) piperazin-1-yl)-(2-fluoro-N-(2,6-dioxo-dihydropyrimidine-2,4(1H, 3H))-benzamide as a yellow oily liquid (100 mg, 64.22%) was obtained. LCMS (ES, m/z) : 879[M+H]⁺.

### Step 2: preparation of 3-(5-(4-((1-(5-((3-fluoro-1H-indol-5-yl)

### (2,2,6,6-tetramethyltetrahydro-4H-pyran-4-ylidene)methyl) pyridin-2-yl)piperidin-4-yl)methyl) piperazin-1-yl)-(2-fluoro-N-(2,6-dioxo-dihydropyrimidine-2,4(1H, 3H)) - benzoylamine (compound 122):

In a 50ml round bottom flask, 3-(5-(4-((1-(5-((3-fluoro-1-(tetrahydro-2H-pyran-4-yl)- 1H-indol-5-yl] (2,2,6,6-tetramethyltetrahydro-4H-pyran-4-ylidene)methyl)pyridin-2-yl)piperidin-4-yl)methyl) piperazin-1-yl) - (2-fluoro-N-(2,6-dioxo-dihydropyrimidine-2,4(1H, 3H)) - benzamide (120 mg, 0.14 mmol), DCM (8 mL) and TFA (2 mL) were added. The resulting mixture was stirred overnight under a nitrogen atmosphere at room temperature. The resulting mixture was concentrated under reduced pressure. The crude product was purified by prep-HPLC with the following conditions (column: Xselect CSH C18 OBD column 30×150 mm 5 µm, n; mobile phase A: water (0.05% TFA), mobile phase B: ACN; flow rate: 60 mL/min; gradient: 16% B to 36% B in 7 min, 36% B; wavelength: 254nm; RT1 (min): 5.68; operation times: 0) to give 3- (5- (4- ((1-(5-((3-fluoro-1 - (tetrahydro-2H-pyran-4-yl) - 1H-indol-5-yl] (2,2,6,6-tetramethyltetrahydro-4H-pyran-4-ylidene) methyl)pyridin-2-yl)piperidin-4-yl)methyl)piperazin-1-yl)-(2-fluoro-N- - (2,6-dioxo-dihydropyrimidine-2,4 (1H, 3H)) - benzamide as a white solid (74.1mg, 67.79%). LCMS (ES, m/z): 789 [M+H]⁺; 1H NMR (400 MHz, DMSO-d6) δ (ppm): 12.58 (s, 1H), 10.99 (s, 1H), 9.49 (s, 1H), 7.97-7.96 (d, J = 2.4 Hz, 1H), 7.61-7.60 (d, J = 8.4 Hz, 1H), 7.53-7.44 (m, 3H),7.26-7.16 (m, 3H), 7.00-6.99 (d, J = 6.8 Hz, 1H), 5.10-5.05 (m, 1H), 4.39-4.34 (d, J = 17.2 Hz, 2H), 4.28-4.22 (m, 3H), 4.03-4.00 (d, J = 10.0 Hz, 3H), 3.65-3.63 (d, J = 8.4 Hz, 2H), 3.24-3.12 (m, 5H), 2.95-2.88 (m, 3H), 2.62-2.58 (d, J = 16.4 Hz, 1H), 2.41-2.34 (m, 1H), 2.24 (s, 2H), 2.16 (s, 2H), 1.97-1.93 (m, 1H), 1.86-1.84 (d, J = 12.3 Hz, 2H), 1.23-1.13 (m, 12H).

According to the synthesis route of compound 122, the following compounds were synthesized by the following starting compounds or intermediates (see above scheme 16 for the synthesis steps, but the molar mass of each starting compound and intermediate was unchanged):

| Compoun d No. | Starting material | Intermediate | Compound structure |
|---|---|---|---|
| 123 | N/A | 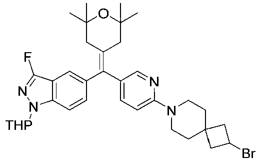 | 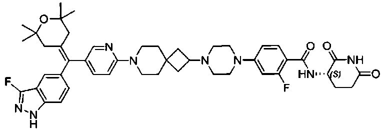 |
| 126 | 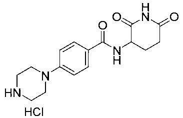 | N/A | 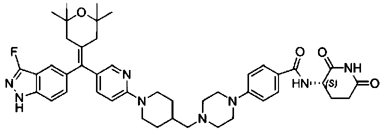 |
| 127 | 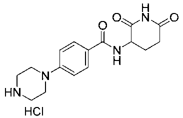 | 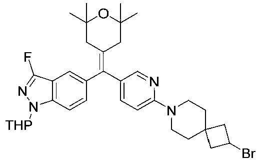 | 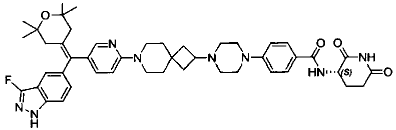 |
| 128 | 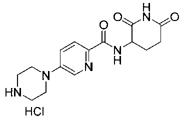 | N/A | 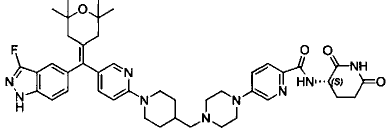 |
| 129 | 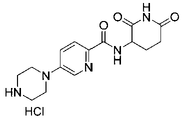 | 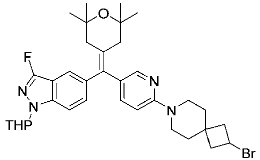 | 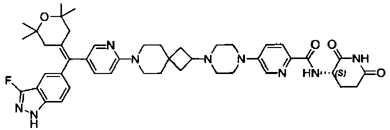 |

The compounds and related NMR data of this application are shown in the following table:

| com pou nd NO | Compound structure | MS [M+H]+ | NMR |
|---|---|---|---|
| 1 | 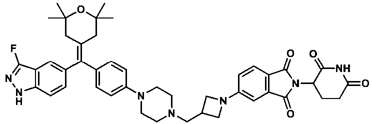 | 773.9 | ¹H NMR (400 MHz, chloroform-*d*) δ 9.80 (s, 1H), 8.06 - 8.01 (m, 1H), 7.65 - 7.58 (m, 2H), 7.39 - 7.33 (m, 3H), 7.28 - 7.22 (m, 1H), 7.13 - 7.07 (m, 1H), 6.77 - 6.71 (m, 2H), 5.41 - 5.36 (m, 1H), 3.74 - 3.67 (m, 2H), 3.53 - 3.46 (m, 2H), 3.25 - 3.18 (m, 4H), 2.75 - 2.68 (m, 1H), 2.66 - 2.51 (m, 6H), 2.49 - 2.42 (m, 1H), 2.39 - 2.30 (m, 3H), 2.22 - 2.13 (m, 3H), 1.82 - 1.73 (m, 1H), 1.28 - 1.24 (m, 12H). |
| 2 | 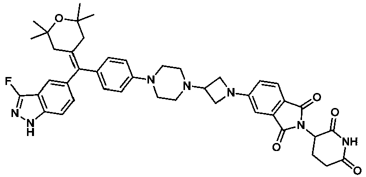 | 759.9 | 1H NMR (400 MHz, chloroform-*d*) δ 9.80 (s, 1H), 8.06 - 8.01 (m, 1H), 7.65 - 7.58 (m, 2H), 7.39 - 7.33 (m, 3H), 7.28 - 7.22 (m, 1H), 7.13 - 7.07 (m, 1H), 6.77 - 6.71 (m, 2H), 5.41 - 5.36 (m, 1H), 3.71 - 3.64 (m, 2H), 3.64 - 3.57 (m, 2H), 3.43 - 3.36 (m, 1H), 3.33 - 3.20 (m, 4H), 2.81 - 2.75 (m, 2H), 2.67 - 2.61 (m, 2H), 2.61 - 2.57 (m, 1H), 2.57 - 2.51 (m, 1H), 2.32 (s, 2H), 2.22 - 2.13 (m, 3H), 1.82 - 1.73 (m, 1H), 1.28 - 1.24 (m, 12H). |
| 3 | 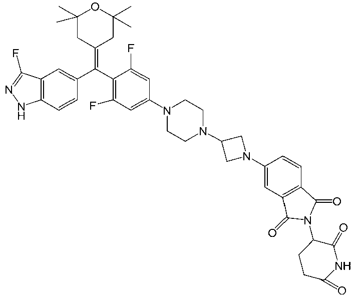 | 796 | 1H NMR (400 MHz, DMSO-d6) δ (ppm): 12.52 (s, 1H), 11.10 (s, 1H), 7.75-7.73 (d, J = 8.0 Hz, 1H), 7.29-7.26 (m, 1H), 7.22-7.16 (m, 1H), 7.12-7.08 (m, 2H), 6.98-6.90 (m, 3H), 6.77-6.75 (m, 1H), 5.10-5.06 (m, 1H), 4.36-4.31 (m, 4H), 3.92-2.83(m, 9H), 2.90-2.85(m, 1H), 2.61-2.55 (m, 2H),2.50-2.14 (d, J = 27.2 Hz, 4H), 2.09-2.0 (m, 1H), 1.17-1.10 (m, 12H). |
| 4 | 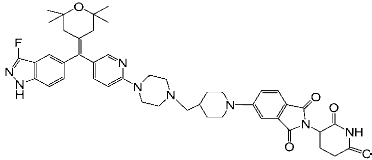 | 803 | 1H NMR (DMSO, 400 MHz) δ (ppm):12.44 (s, 1H), 11.08 (s, 1H), 8.27(s, 1H), 7.96-8.18(m, 2H), 7.70-7.68 (d, J =8.4 Hz, 1H),7.45-7.41 (m, 1H), 7.35-7.24 (d, J =1.6 Hz, 2H), 6.97-6.95 (d, J =8.8Hz, 2H), 6.82 (s, 1H), 6.72-6.56 (d, J =10.4Hz, 1H), 5.06(m,1H), 4.28-4.23(m, 2H),3.88-3.82 (m,4H), 3.56-3.54 (s, 4H), 3.31 (m, 1H), 3.16-3.14(m, 2H),3.00(m,3H), 2.60(s, 1H), 2.50-2.49 (d, J =1.6 Hz, 4H), 2.21(m, 1H), 1.35-1.30(m, 4H) , 1.25 - 1.14 (d, J = 17.2 Hz, 12H). |
| 5 | 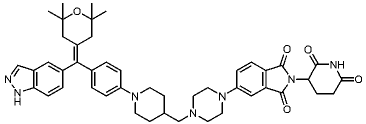 | 785 | 1H NMR (400 MHz, DMSO-d6) δ (ppm):13.05 (s, 1H), 10.97 (s, 1H), 9.41 (s, 1H), 8.21 (s, 1H),8.04 (d, J = 0.8 Hz, 1H), 7.96-7.95 (d, J = 2.0 Hz, 1H), 7.61-7.57 (m, 2H),7.50-7.48 (d, J = 8.8 Hz, 1H), 7.41 (d, J = 8.8 Hz, 1H), 7.19-7.13 (m, 3H), 6.98-6.89 (m, 1H), 5.10-5.05 (m, 1H),4.37-4.22 (m, 2H), 4.02-3.99 (m, 4H), 3.64 (s, 2H), 3.21-3.11 (m, 5H), 2.90-2.87 (m, 3H), 2.70-2.60 (m, 1H), 2.40-2.35 (m, 1H), 2.25-2.17 (m, 4H), 1.98-1.95 (m, 1H), 1.85-1.78(m, 2H), 1.17-1.12 (m, 13H). |
| 6 | 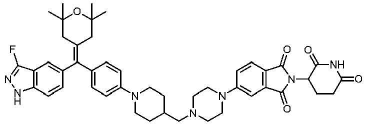 | 802 | 1H NMR (400 MHz, DMSO-d6) δ (ppm):12.55 (s, 1H), 11.11 (s, 1H), 7.78-7.76 (d, J = 8.4 Hz, 1H), 7.52-7.35 (m, 4H), 7.20-7.18 (m, 1H),7.11-7.09 (d, J = 8.4 Hz, 2H), 6.99-6.97 (d, J = 8.0 Hz, 2H), 5.13-5.09 (m, 1H),4.23-4.20 (m, 2H), 3.36-3.31 (m, 2H), 3.17-3.14 (m, 4H), 2.95-2.72 (m, 3H), 2.61-2.52 (m, 1H), 2.22-2.14 (d, J = 30.8 Hz, 4H), 2.08-2.02 (m, 2H), 1.89-1.85(m, 1H), 1.36-1.33(m, 2H), 1.17-1.12 (m, 12H). |
| 7 | 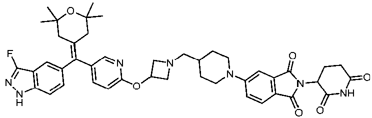 | 789 | 1H NMR (DMSO, 400 MHz) δ (ppm):12.42 (s, 1H), 11.07 (s, 1H), 8.27(s, 1H), 8.16-7.98(m, 2H), 7.70-7.66 (d, J =8.4 Hz, 1H),7.46-7.41 (m, 1H), 7.35-7.24 (d, J =1.6 Hz, 2H), 6.98-6.95 (d, J =8.8Hz, 2H), 6.82 (s, 1H), 6.72-6.56 (d, J =10.4Hz, 1H), 5.06(m,1H), 4.44-4.38(m, 1H),4.28-4.23(m, 2H),3.88-3.82 (m,4H), 3.56-3.54 (s, 4H), 3.31 (m,1H), 3.00(m,3H), 2.60(s, 1H), 2.50-2.49 (d, J =1.6 Hz, 4H), 2.21(m, 1H), 1.35-1.30(m, 4H),1.26-1.12 (m,12H). |
| 8 | 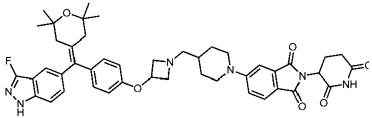 | 789 | 1H NMR (DMSO, 400 MHz) δ (ppm): 12.43 (s, 1H), 11.08 (s, 1H), 8.28(s, 1H), 8.16-7.98(m, 2H), 7.70-7.66 (d, J =8.4 Hz, 1H),7.46-7.41 (m, 1H), 7.35-7.24 (d, J =1.6 Hz, 2H), 6.98-6.95 (d, J =8.8Hz, 2H), 6.89-6.81 (m, 2H), 6.74-6.58 (d, J =10.4Hz, 1H), 5.06(m,1H), 4.44-4.38(m, 1H),4.28-4.23(m, 2H),3.88-3.82 (m,4H), 3.56-3.54 (s, 4H), 3.32 (m,1H), 3.00(m,3H), 2.60(s, 1H), 2.50-2.49 (d, J =1.6 Hz, 4H), 2.21(m, 1H), 1.35-1.30(m, 4H),1.24-1.13 (m,12H). |
| 9 | 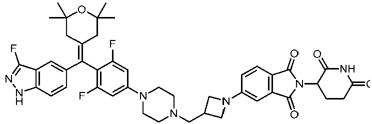 | 810 | 1H NMR (DMSO, 400 MHz) δ (ppm): 12.44 (s, 1H), 11.12 (s, 1H), 9.85 (s, 1H), 7.72-7.68 (d, J =8.4 Hz, 1H),7.45-7.41 (m, 2H), 7.21-7.20 (d, J =1.6 Hz, 1H), 7.13-7.11 (d, J =8.4Hz, 2H), 6.60-6.52 (d, J =10.4Hz, 2H), 5.06(m,1H), 4.28-4.23(m, 2H),3.88-3.82 (m,4H), 3.56-3.54 (s, 4H), 3.31 (m,1H), 3.16-3.14(m, 2H),3.00(m, 3H), 2.60(s, 1H), 2.53-2.49 (d, J =1.6 Hz, 4H), 2.21(m, 1H),1.24-1.12 (m,12H). |
| 10 | 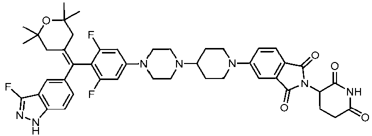 | 824 | 1H NMR (400 MHz, DMSO-d6) δ 12.65 (s, 1H), 11.08(s, 1H), 7.72 (d, J = 8.4 Hz, 1H), 7.56 - 7.43 (m, 2H), 7.42 - 7.33 (m, 2H), 7.31 - 7.20 (m, 1H), 6.72 (d, J = 11.1 Hz, 2H), 5.10 (dd, J = 13.0, 5.3 Hz, 1H), 4.22 (d, J = 8.5 Hz, 2H), 3.96 (d, J = 12.6 Hz, 3H), 3.64 (d, J = 8.6 Hz, 2H), 3.47 (s, 1H), 3.20 (d, J = 9.5 Hz, 3H), 2.86 (ddd, J = 17.3, 14.1, 5.5 Hz, 1H), 2.76 (t, J = 12.4 Hz, 2H), 2.55 (s, 2H), 2.21 (s, 2H), 2.09 (q, J = 11.0, 10.3 Hz,5H), 1.72 - 1.60 (m, 2H), 1.14-1.20(m, 12H). |
| 11 | 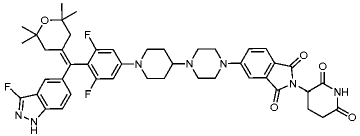 | 824 | 1H NMR (400 MHz, DMSO-d6) δ 12.59 (s, 1H), 11.12 (s, 1H), 7.77 (d, J = 8.4 Hz, 1H), 7.52 - 7.43 (m, 2H), 7.42 - 7.33 (m, 2H), 7.31 - 7.20 (m, 1H), 6.73 (d, J = 11.1 Hz, 2H), 5.10 (dd, J = 13.0, 5.3 Hz, 1H), 4.25 (d, J = 8.5 Hz, 2H), 3.97 (d, J = 12.6 Hz, 3H), 3.64 (d, J = 8.6 Hz, 2H), 3.47 (s, 1H), 3.21 (d, J = 9.5 Hz, 3H), 2.89 (ddd, J = 17.3, 14.1, 5.5 Hz, 1H), 2.76 (t, J = 12.4 Hz, 2H), 2.55 (s, 2H), 2.21 (s, 2H), 2.09 (q, J = 11.0, 10.3 Hz,5H), 1.68 - 1.60 (m, 2H), 1.22-1.12 (m, 12H). |
| 12 | 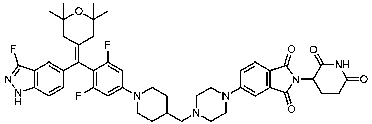 | 838 | 1H NMR (400 MHz, DMSO-d6) δ 10.98 (s, 1H), 9.34 (s, 1H), 7.73 (dd, J = 8.8, 2.1 Hz, 1H), 7.61 (d, J = 8.4 Hz, 1H), 7.41 (s, 1H), 7.32 (dd, J = 8.7, 1.5 Hz, 1H), 7.21 - 7.13 (m, 2H), 6.68 (d, J = 11.3 Hz, 2H), 5.77 (dt, J = 9.7, 2.6 Hz, 1H), 5.08 (dd, J = 13.3, 5.1 Hz, 1H), 4.36 (d, J = 17.1 Hz, 1H), 4.24 (d, J = 17.1 Hz, 1H), 4.01 (d, J = 10.9 Hz, 2H), 3.83 (dd, J = 13.4, 7.3 Hz, 3H), 3.72 - 3.67 (m, 1H), 3.15 - 3.08 (m, 2H), 2.92 (ddd, J = 17.3, 13.6, 5.4 Hz, 1H), 2.76 (t, J = 11.8 Hz, 2H), 2.64 - 2.55 (m, 1H), 2.29 - 2.18 (m, 2H), 2.09 (s, 2H), 2.05 (s, 1H), 2.04 - 1.88 (m, 2H), 1.81 (d, J = 12.2 Hz, 2H), 1.71 (t, J = 4.7 Hz, 1H), 1.60 - 1.45 (m, 2H), 1.24-1.12 (m, 12H). |
| 13 | 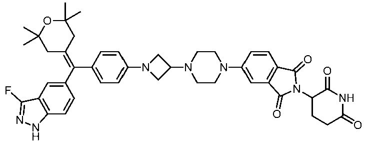 | 760 | 1H NMR (400 MHz, DMSO-d6) δ 12.54 (s, 1H), 11.11 (s, 1H), 7.77 (d, J = 8.5 Hz, 1H), 7.51 (d, J = 2.3 Hz, 1H), 7.49 - 7.34 (m, 4H), 7.24 - 7.11 (m, 2H), 7.07 (d, J = 8.4 Hz, 2H), 6.47 (d, J = 8.4 Hz, 2H), 5.11 (dd, J = 12.9, 5.4 Hz, 1H), 4.30 - 3.90 (m, 8H), 3.25 (s, 1H), 2.97 - 2.83 (m, 2H), 2.65 - 2.53 (m, 2H), 2.18 (d, J = 22.0 Hz, 4H), 2.07 - 1.99 (m, 1H), 1.24-1.13(m, 12H). |
| 14 | 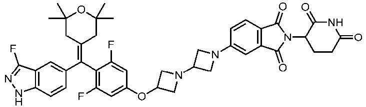 | 783 | 1H NMR (400 MHz, DMSO-d6) δ 12.61 (s, 1H), 11.10 (s, 1H), 7.71 (d, J = 8.2 Hz, 1H), 7.48 (dd, J = 8.7, 2.3 Hz, 1H), 7.41 (s, 1H), 7.25 (dd, J = 8.7, 1.6 Hz, 1H), 6.91 (d, J = 2.1 Hz, 1H), 6.84 - 6.72 (m, 3H), 5.09 (dt, J = 13.0, 7.5 Hz, 2H), 4.42 (s, 1H), 4.28 (d, J = 9.5 Hz, 2H), 4.09 - 4.02 (m, 2H), 2.91 - 2.82 (m, 1H), 2.64 - 2.51 (m, 7H), 2.23 (s, 2H), 2.05 (s, 2H), 1.22-1.12 (m, 12H). |
| 15 | 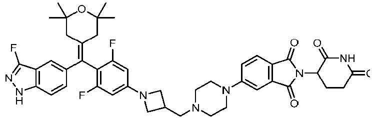 | 810 | 1H NMR (DMSO, 400 MHz) δ (ppm):12.54 (s, 1H), 11.10 (s, 1H), 9.82 (s, 1H), 7.72-7.68 (d, J =8.4 Hz, 1H),7.45-7.41 (m, 2H), 7.21-7.20 (d, J =1.6 Hz, 1H), 7.13-7.11 (d, J =8.4Hz, 2H), 6.60-6.52 (d, J =10.4Hz, 2H), 5.06(m,1H), 4.28-4.20(m, 2H),3.88-3.82 (m,4H), 3.56-3.54 (s, 4H), 3.30 (m, 1H), 3.16-3.14(m, 2H),3.02(m, 3H), 2.63(s, 1H), 2.53-2.49 (d, J =1.6 Hz, 4H), 2.21(m, 1H),1.26-1.10 (m, 12H). |
| 16 | 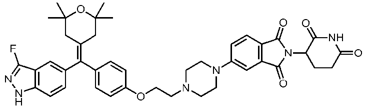 | 750 | 1H NMR (400 MHz, DMSO-d6) δ 12.58 (s, 1H), 11.11 (s, 1H), 9.96 (s, 3H), 8.12 (d, J = 2.4 Hz, 1H), 7.77 (d, J = 8.4 Hz, 1H), 7.62 (dd, J = 8.5, 2.4 Hz, 1H), 7.54 (s, 1H), 7.49 (d, J = 2.3 Hz, 1H), 7.45 (dd, J = 8.7, 2.3 Hz, 1H), 7.18-7.36 (m, 2H), 6.64-6.86 (m, 2H), 5.10 (dd, J = 12.9, 5.4 Hz, 1H), 4.63 (t, J = 4.8 Hz, 2H), 4.23 (s, 1H), 3.68 (s, 1H), 3.62 (s, 2H), 2.90 (ddd, J = 17.3, 14.0, 5.5 Hz, 2H), 2.64 - 2.54 (m, 2H), 2.18 (d, J = 8.1 Hz, 4H), 2.10 - 1.96 (m, 2H), 1.22-1.12 (m, 12H). |
| 17 | 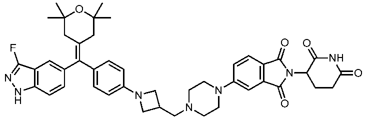 | 774 | 1H NMR (400 MHz, DMSO-d6) δ 12.60(s, 1H), 11.08 (s, 1H), 9.93 (s, 1H), 7.76 (d, J = 8.5 Hz, 1H), 7.57 - 7.49 (m, 2H), 7.46 (dd, J = 8.7, 2.3 Hz, 1H), 7.37 (dd, J = 8.7, 2.3 Hz, 1H), 7.15-7.25 (m, 2H), 6.58-6.78 (m, 2H), 5.12 - 5.07 (m, 1H), 4.23 (d, J = 10.9 Hz, 4H), 3.87 (s, 2H), 3.27 (s, 2H), 3.18 (s, 1H), 2.97 - 2.83 (m, 2H), 2.73 - 2.58 (m, 3H), 2.46 (s, 2H), 2.21 (s, 2H), 2.16 (s, 2H), 2.10 - 1.99 (m, 2H), 1.22-1.12 (m, 12H). |
| 18 | 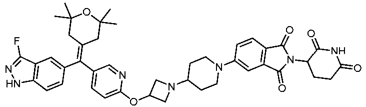 | 776 | 1H NMR (400 MHz, Methanol-d4) δ 7.93 (d, J = 2.1 Hz, 1H), 7.86 (dd, J = 9.5, 2.1 Hz, 1H), 7.72 (d, J = 8.5 Hz, 1H), 7.59 (s, 1H), 7.53 - 7.44 (m, 2H), 7.43 (d, J = 2.3 Hz, 1H), 7.32 (ddd, J = 12.8, 8.7, 2.0 Hz, 2H), 5.10 (dd, J = 12.6, 5.5 Hz, 1H), 4.54 (t, J = 2.7 Hz, 1H), 4.44 - 4.31 (m, 3H), 4.27 - 4.18 (m, 2H), 3.58 (dd, J = 13.4, 2.9 Hz, 1H), 3.50 (d, J = 14.2 Hz, 1H), 3.29 - 3.11 (m, 3H), 2.91 - 2.83 (m, 1H), 2.81 - 2.70 (m, 2H), 2.41 (s, 2H), 2.32 (s, 2H), 2.17 - 1.98 (m, 4H), 1.95 (t, J = 4.8 Hz, 2H), 1.30 -1.24 (m, 12H). |
| 19 | 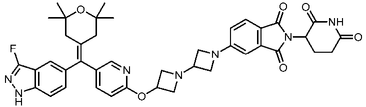 | 748 | 1H NMR (400 MHz, DMSO-d6) δ 12.64 (s, 1H), 11.10 (s, 1H), 8.17 (d, J = 2.0 Hz, 1H), 7.97 (dd, J = 9.3, 2.0 Hz, 1H), 7.71 (d, J = 8.2 Hz, 1H), 7.62 (s, 1H), 7.50 (dd, J = 8.6, 2.3 Hz, 1H), 7.38 - 7.28 (m, 2H), 6.95 (d, J = 2.1 Hz, 1H), 6.79 (dd, J = 8.4, 2.1 Hz, 1H), 5.31 - 5.20 (m, 1H), 5.08 (dd, J = 12.9, 5.3 Hz, 1H), 4.53 (t, J = 3.0 Hz, 1H), 4.45 (q, J = 8.5 Hz, 2H), 4.39 - 4.25 (m, 3H), 4.19 (dd, J = 9.4, 5.2 Hz, 1H), 3.82 (dd, J = 12.8, 3.1 Hz, 1H), 3.63 (d, J = 13.1 Hz, 2H), 2.89 (ddd, J = 17.4, 14.2, 5.5 Hz, 1H), 2.65 - 2.54 (m, 2H), 2.19 (d, J = 18.2 Hz, 3H), 2.07 - 1.97 (m, 1H), 1.26 - 1.10 (m, 12H). |
| 20 | 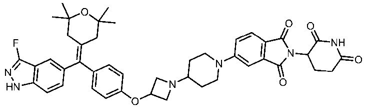 | 775 | 1H NMR (300 MHz, DMSO-d6) δ 12.58 (s, 1H), 11.11 (s, 1H), 10.60 (s, 1H), 7.70 (dd, J = 8.5, 2.1 Hz, 1H), 7.51 - 7.39 (m, 3H), 7.35 - 7.28 (m, 1H), 7.20 (tt, J = 8.4, 3.8 Hz, 3H), 6.84 (t, J = 8.7 Hz, 2H), 5.08 (dt, J = 11.2, 5.6 Hz, 2H), 4.65 (s, 2H), 4.35 (s, 1H), 4.20 (d, J = 13.2 Hz, 3H), 2.99 - 2.94 (m, 1H), 2.94 - 2.81 (m, 2H), 2.68 - 2.54 (m, 2H), 2.23 - 1.94 (m, 7H), 1.39-1.15 (m, 12H). |
| 21 | 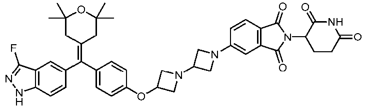 | 747 | 1H NMR (400 MHz, DMSO-d6) δ 12.55 (s, 1H), 11.11 (s, 1H), 7.71 (d, J = 8.2 Hz, 1H), 7.47 (s, 1H), 7.43 (dd, J = 8.6, 2.4 Hz, 1H), 7.21 (td, J = 8.1, 7.5, 5.7 Hz, 3H), 6.90 (s, 1H), 6.84 (d, J = 8.4 Hz, 2H), 6.75 (dd, J = 8.3, 2.2 Hz, 1H), 5.08 (dd, J = 12.8, 5.5 Hz, 2H), 4.27 (s, 2H), 4.06 (s, 2H), 2.89 (ddd, J = 17.8, 13.8, 5.4 Hz, 2H), 2.61 (d, J = 3.3 Hz, 1H), 2.56 (d, J = 8.0 Hz, 2H), 2.16 (d, J = 11.9 Hz, 4H), 2.08 - 1.94 (m, 2H), 1.22-1.12 (m, 12H). |
| 22 | 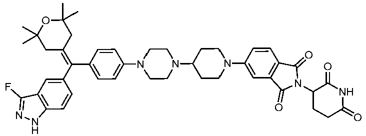 | 788 | 1H NMR (400 MHz, DMSO-d6) δ 12.54 (s, 1H), 11.10 (s, 1H), 9.87 (s, 1H), 8.02 (d, J = 2.4 Hz, 1H), 7.77 (d, J = 8.4 Hz, 1H), 7.53 7.48 (m, 2H), 7.45 (dt, J = 7.5, 3.3 Hz, 2H), 7.36-7.24 (m, 2H), 6.96-6.78 (m, 2H), 5.11 (dd, J = 12.9, 5.4 Hz, 1H), 4.43 (d, J = 12.9 Hz, 2H), 4.24 (d, J = 9.4 Hz, 2H), 3.64 (d, J = 8.8 Hz, 2H), 3.20 (d, J = 9.7 Hz, 3H), 2.96 - 2.87 (m, 2H), 2.84 (t, J = 8.8 Hz, 2H), 2.64 - 2.53 (m, 2H), 2.23 (s, 2H), 2.17 (d, J = 11.3 Hz, 4H), 2.09 - 1.99 (m, 1H), 1.69 - 1.56 (m, 2H), 1.20-1.12 (m, 12H). |
| 23 | 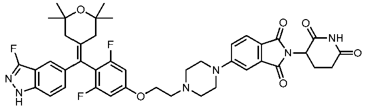 | 785 | 1H NMR (400 MHz, DMSO-d6) δ 12.56 (s, 1H), 11.10 (s, 1H), 9.94 (s, 1H), 8.12 (d, J = 2.4 Hz, 1H), 7.76 (d, J = 8.4 Hz, 1H), 7.62 (dd, J = 8.5, 2.4 Hz, 1H), 7.54 (s, 1H), 7.49 (d, J = 2.3 Hz, 1H), 7.45 (dd, J = 8.7, 2.3 Hz, 1H), 7.35 (dd, J = 8.6, 2.4 Hz, 1H), 7.25 (dd, J = 8.7, 1.6 Hz, 1H), 6.88 (s, 2H), 5.10 (dd, J = 12.9, 5.4 Hz, 1H), 4.63 (t, J = 4.8 Hz, 2H), 4.23 (s, 1H), 3.68 (s, 1H), 3.62 (s, 2H), 2.90 (ddd, J = 17.3, 14.0, 5.5 Hz, 2H), 2.64 - 2.54 (m, 2H), 2.18 (d, J = 8.1 Hz, 4H), 2.12 - 1.96 (m, 2H), 1.18-1.12 (m, 12H). |
| 24 | 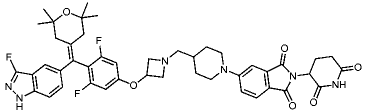 | 825 | 1H NMR (400 MHz, DMSO-d6) δ 12.62 (s, 1H), 11.10 (s, 1H), 7.67 (d, J = 8.4 Hz, 1H), 7.49 (dd, J = 8.7, 2.3 Hz, 1H), 7.42 (s, 1H), 7.34 (d, J = 2.3 Hz, 1H), 7.25 (ddd, J = 8.7, 3.5, 1.8 Hz, 2H), 6.76 (dd, J = 8.5, 5.7 Hz, 2H), 5.07 (dd, J = 12.8, 5.4 Hz, 2H), 4.77 (s, 1H), 4.55 - 4.47 (m, 1H), 4.34 (s, 1H), 4.23 - 4.17 (m, 1H), 4.08 (d, J = 13.1 Hz, 2H), 3.19 (d, J = 7.7 Hz, 2H), 3.00 - 2.81 (m, 4H), 2.62 - 2.53 (m, 3H), 2.05 (s, 3H), 1.91 (s, 1H), 1.74 (t, J = 12.1 Hz, 2H), 1.24 - 1.15 (m, 14H). |
| 25 | 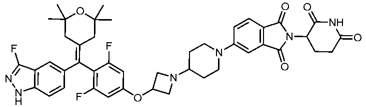 | 811 | 1H NMR (300 MHz, DMSO-d6) δ 12.58 (s, 1H), 11.11 (s, 1H), 10.60 (s, 1H), 7.70 (dd, J = 8.5, 2.1 Hz, 1H), 7.50 - 7.40 (m, 2H), 7.34-7.24 (m, 2H), 6.86 (m, 2H), 5.08 (dt, J = 11.2, 5.6 Hz, 2H), 4.65 (s, 2H), 4.35 (s, 1H), 4.20 (d, J = 13.2 Hz, 3H), 2.99 - 2.94 (m, 1H), 2.94 - 2.81 (m, 2H), 2.68 - 2.54 (m, 2H), 2.23 - 1.94 (m, 7H), 1.39 (d, J = 10.4 Hz, 2H), 1.18-1.12 (m, 12H). |
| 26 | 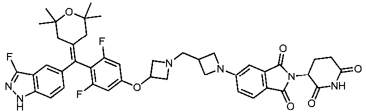 | 797 | 1H NMR (400 MHz, DMSO-d6) δ 12.62 (s, 1H), 11.09 (s, 1H), 10.39 (s,0.5H), 9.98 (s, 0.5H), 7.68 (d, J = 8.3 Hz, 1H), 7.49 (dd, J = 8.7, 2.3 Hz, 1H), 7.42 (s, 1H), 7.29 - 7.20 (m, 2H), 7.10 (s, 0.5H), 6.97 (s, 0.5H), 6.80 (dd, J = 12.5, 5.3 Hz, 3H), 6.67 (dd, J = 8.4, 2.1 Hz, 1H), 5.14 (s, 1H), 5.07 (dd, J = 12.9, 5.3 Hz, 2H), 4.73 (s, 1H), 4.52 (s, 1H), 4.27 (s, 1H), 4.14 (d, J = 6.5 Hz, 2H), 3.81 (dd, J = 8.7, 5.5 Hz, 2H), 3.01 (s, 1H), 2.89 (ddd, J = 17.4, 14.1, 5.5 Hz, 2H), 2.57 (dd, J = 15.9, 12.0 Hz, 1H), 2.24 (s, 2H), 2.05 (s, 1H), 2.05 - 1.97 (m, 1H), 1.20-1.13(m, 12H). |
| 27 | 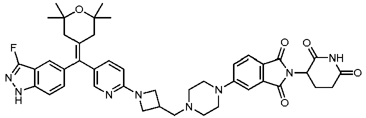 | 775 | 1H NMR (400 MHz, DMSO-d6) δ 12.59 (s, 1H), 11.12 (s, 1H), 9.96 (s, 1H), 7.97 (d, J = 2.2 Hz, 1H), 7.78 (d, J = 8.5 Hz, 1H), 7.57 - 7.49 (m, 3H), 7.46 (dd, J = 8.7, 2.3 Hz, 1H), 7.37 (dd, J = 8.7, 2.3 Hz, 1H), 7.25 (d, J = 8.7 Hz, 1H), 6.56 (s, 1H), 5.12 - 5.07 (m, 1H), 4.25 (d, J = 10.9 Hz, 4H), 3.87 (s, 2H), 3.27 (s, 2H), 3.18 (s, 1H), 2.97 - 2.83 (m, 2H), 2.73 - 2.58 (m, 3H), 2.46 (s, 2H), 2.21 (s, 2H), 2.16 (s, 2H), 2.10 - 1.99 (m, 2H), 1.18 - 1.14 (m, 12H). |
| 28 | 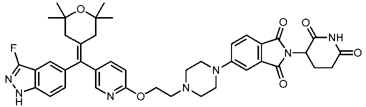 | 750 | 1H NMR (400 MHz, DMSO-d6) δ 12.58 (s, 1H), 11.11 (s, 1H), 9.96 (s, 3H), 8.12 (d, J = 2.4 Hz, 1H), 7.77 (d, J = 8.4 Hz, 1H), 7.62 (dd, J = 8.5, 2.4 Hz, 1H), 7.54 (s, 1H), 7.49 (d, J = 2.3 Hz, 1H), 7.45 (dd, J = 8.7, 2.3 Hz, 1H), 7.36 (dd, J = 8.6, 2.4 Hz, 1H), 7.25 (dd, J = 8.7, 1.6 Hz, 1H), 6.86 (d, J = 8.4 Hz, 1H), 5.10 (dd, J = 12.9, 5.4 Hz, 1H), 4.63 (t, J = 4.8 Hz, 2H), 4.23 (s, 1H), 3.68 (s, 1H), 3.62 (s, 2H), 2.90 (ddd, J = 17.3, 14.0, 5.5 Hz, 2H), 2.64 - 2.54 (m, 2H), 2.18 (d, J = 8.1 Hz, 4H), 2.10 - 1.96 (m, 2H), 1.18-1.10 (m, 12H). |
| 29 | 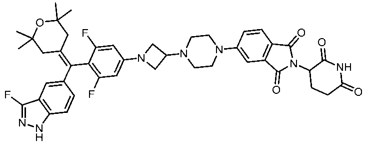 | 796 | 1H NMR (400 MHz, DMSO-d6) δ 12.58 (s, 1H), 11.10 (s, 1H), 7.77 (d, J = 8.4 Hz, 1H), 7.52 - 7.43 (m, 2H), 7.42 - 7.33 (m, 2H), 7.23 (dd, J = 8.7, 1.5 Hz, 2H), 6.24 (d, J = 9.0 Hz, 2H), 5.10 (dd, J = 13.0, 5.4 Hz, 2H), 4.14 (s, 2H), 4.05 (s, 2H), 2.90 (ddd, J = 17.1, 14.0, 5.5 Hz, 2H), 2.64 - 2.53 (m, 2H), 2.23 (s, 2H), 2.09 - 1.98 (m, 4H), 1.18-1.10 (m, 12H). |
| 30 | 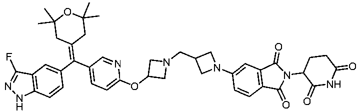 | 762 | 1H NMR (400 MHz, DMSO-d6) δ 12.64 (s, 1H), 11.09 (s, 1H), 8.05 (d, J = 2.1 Hz, 1H), 7.84 (dd, J = 9.4, 2.0 Hz, 1H), 7.68 - 7.60 (m, 2H), 7.52 - 7.43 (m, 2H), 7.33 - 7.28 (m, 1H), 6.81 (d, J = 2.2 Hz, 1H), 6.67 (dt, J = 8.4, 2.6 Hz, 1H), 5.07 (dt, J = 12.9, 4.3 Hz, 1H), 4.39 (d, J = 13.2 Hz, 2H), 4.28 - 4.20 (m, 2H), 4.13 (dd, J = 8.3, 4.1 Hz, 2H), 4.02 (dd, J = 15.0, 7.2 Hz, 2H), 3.86 (dd, J = 8.6, 5.6 Hz, 2H), 3.76 (d, J = 12.7 Hz, 1H), 3.50 (d, J = 13.3 Hz, 1H), 3.35 (td, J = 6.4, 2.0 Hz, 1H), 3.17 (dt, J = 14.5, 7.2 Hz, 1H), 2.91 - 2.82 (m, 1H), 2.59 (d, J = 13.9 Hz, 1H), 2.22 (s, 1H), 2.15 (s, 2H), 2.04 - 1.97 (m, 1H), 1.23 - 1.12 (m, 12H). |
| 31 | 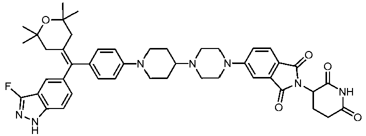 | 788 | 1H NMR (400 MHz, DMSO-d6) δ 12.60 (s, 1H), 11.16 (s, 1H), 9.87 (s, 1H), 8.01 (d, J = 2.4 Hz, 1H), 7.77 (d, J = 8.4 Hz, 1H), 7.53 - 7.48 (m, 2H), 7.45 (dt, J = 7.5, 3.3 Hz, 2H), 7.34-7.25 (m, 2H), 6.96-6.82 (m, 2H), 5.11 (dd, J = 12.9, 5.4 Hz, 1H), 4.43 (d, J = 12.9 Hz, 2H), 4.26 (d, J = 9.4 Hz, 2H), 3.64 (d, J = 8.8 Hz, 2H), 3.22 (d, J = 9.7 Hz, 3H), 2.96 - 2.87 (m, 2H), 2.84 (t, J = 8.8 Hz, 2H), 2.64 - 2.53 (m, 2H), 2.23 (s, 2H), 2.17 (d, J = 11.3 Hz, 4H), 2.09 - 1.99 (m, 1H), 1.69 - 1.56 (m, 2H), 1.20-1.12 (m, 12H). |
| 32 | 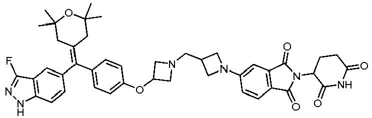 | 761 | 1H NMR (400 MHz, DMSO-d6) δ 12.56 (s, 1H), 11.10 (s, 1H), 10.19 (m, 1H), 7.68 (d, J = 8.3 Hz, 1H), 7.48 (s, 1H), 7.43 (dd, J = 8.8, 2.3 Hz, 1H), 7.20 (t, J = 7.6 Hz, 3H), 6.88 - 6.78 (m, 3H), 6.67 (dd, J = 8.3, 2.1 Hz, 1H), 5.13 - 4.98 (m, 3H), 4.70 (s, 1H), 4.50 (s, 1H), 4.23 (s, 1H), 4.17 - 4.09 (m, 2H), 3.81 (dd, J = 8.7, 5.4 Hz, 2H), 3.02 (s, 1H), 2.88 (ddd, J = 17.3, 14.1, 5.4 Hz, 2H), 2.60 (d, J = 3.5 Hz, 1H), 2.16 (d, J = 11.9 Hz, 4H), 2.05 - 1.95 (m, 2H), 1.22-1.12 (m, 12H). |
| 33 | 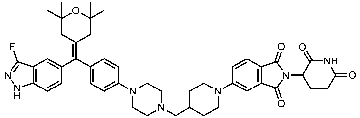 | 702 | 1H NMR (400 MHz, DMSO-d6) δ 12.54 (s, 1H), 11.10 (s, 1H), 9.35 (s, 1H), 7.69 (d, J = 8.5 Hz, 1H), 7.47 - 7.35 (m, 3H), 7.28 (dd, J = 8.8, 2.3 Hz, 1H), 7.20 (dd, J = 8.7, 1.5 Hz, 1H), 7.15 - 7.08 (m, 2H), 6.96 (d, J = 8.6 Hz, 2H), 5.08 (dd, J = 12.9, 5.4 Hz, 1H), 4.11 (d, J = 13.1 Hz, 3H), 3.80 (d, J = 12.4 Hz, 3H), 3.61 (d, J = 11.3 Hz, 2H), 3.16 - 2.96 (m, 8H), 2.91 - 2.82 (m, 1H), 2.63 - 2.54 (m, 2H), 2.21 (s, 2H), 2.14 (s, 2H), 2.02 (dp, J = 11.1, 3.7, 3.2 Hz, 1H), 1.84 (d, J = 12.5 Hz, 2H), 1.31 (d, J = 12.4 Hz, 1H), 1.24-1.13 (m, 12H). |
| 34 | 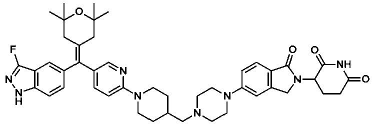 | 789 | 1H NMR (400 MHz, chloroform-d) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.59 - 4.53 (m, 1H), 4.53 - 4.51 (m, 1H), 4.46 - 4.42 (m, 1H), 3.66 - 3.52 (m, 4H), 3.28 - 3.21 (m, 4H), 2.66 - 2.46 (m, 8H), 2.31 (s, 2H), 2.22 (s, 2H), 2.11 - 2.01 (m, 1H), 1.93 - 1.83 (m, 5H), 1.73 - 1.63 (m, 1H), 1.26 (s, 12H). |
| 35 | 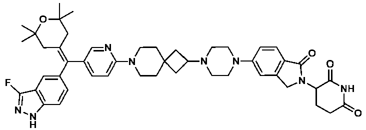 | 815 | 1H NMR (400 MHz, chloroform-d) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.59 - 4.53 (m, 1H), 4.53 - 4.51 (m, 1H), 4.46 - 4.42 (m, 1H), 3.74 - 3.68 (m, 2H), 3.52 - 3.47 (m, 2H), 3.33 - 3.20 (m, 4H), 2.81 - 2.75 (m, 2H), 2.74 - 2.66 (m, 1H), 2.66 - 2.60 (m, 2H), 2.60 - 2.54 (m, 1H), 2.54 - 2.46 (m, 1H), 2.31 (s, 2H), 2.11 - 2.01 (m, 1H), 1.82 - 1.63 (m, 5H), 1.62 - 1.52 (m, 4H), 1.28 - 1.24 (m, 12H). |
| 36 | 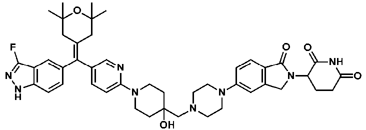 | 774.9 | 1H NMR (400 MHz, chloroform-d) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.59 - 4.53 (m, 1H), 4.53 - 4.51 (m, 1H), 4.46 - 4.42 (m, 1H), 3.62 - 3.53 (m, 3H), 3.51 - 3.43 (m, 2H), 3.29 - 3.23 (m, 4H), 2.76 (s, 2H), 2.70 - 2.65 (m, 2H), 2.65 - 2.60 (m, 2H), 2.60 - 2.54 (m, 1H), 2.54 - 2.46 (m, 1H), 2.31 (s, 2H), 2.11 - 2.01 (m, 1H), 1.94 - 1.81 (m, 4H), 1.73 - 1.63 (m, 1H), 1.28 - 1.24 (m, 12H). |
| 37 | 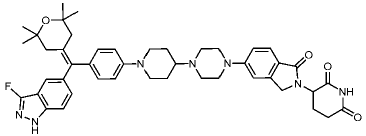 | 774.29 | 1H NMR (400 MHz, DMSO-d6) δ (ppm):12.50 (s, 1H), 10.98 (s, 1H), 7.60 (d,1H), 7.46-7.41 (m, 2H), 7.23-7.12 (m, 3H), 7.10 (d, 2H),6.95-6.88 (d, 2H), 5.08-5.00 (m, 1H), 4.41-4.33 (d, 1H), 4.28-4.19 (d,1H), 4.12-4.01 (d, 2H), 3.91-3.83(d, 2H),3.72-3.63(d, 2H), 3.30-3.13 (m, 4H),3.12-3.05 (m, 2H), 2.93-2.86 (m,1H), 2.73-2.66 (m, 1H), 2.59-2.51(m, 2H), 2.33-2.32(m, 2H), 2.13-2.02(m, 2H), 2.01-1.98(m.2H), 1.85-1.74(m,2H), 1.23(s, 1H), 1.16-1.11(d,12H). |
| 38 | 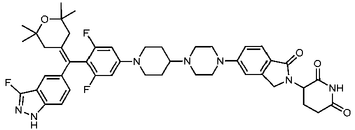 | 805.08 | 1H NMR (400 MHz, DMSO-d6) δ (ppm):12.54 (s, 1H), 10.96 (s, 1H), 7.60 (d,1H), 7.23-7.12 (m, 3H), 7.12 (d, 2H),6.98-6.89 (d, 2H), 5.06 (m, 1H), 4.41-4.33 (d, 1H), 4.28-4.19 (d,1H), 4.12-4.01 (d, 2H), 3.91-3.83(d, 2H),3.72-3.63(d, 2H), 3.30-3.13 (m, 4H),3.12-3.05 (m, 2H), 2.93-2.86 (m,lH), 2.73-2.66 (m, 1H), 2.59-2.51(m, 2H), 2.33-2.32(m, 2H), 2.13-2.02(m, 2H), 2.01-1.98(m.2H),1.85-1.74(m,2H), 1.23(s, 1H), 1.18-1.11(d,12H). |
| 39 | 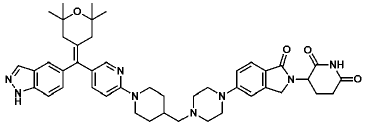 | 771.06 | 1H NMR (400 MHz, DMSO-d6) δ (ppm):13.05 (s, 1H), 10.97 (s, 1H), 8.04 (d, J = 0.8 Hz, 1H), 7.96-7.95 (d, J = 2.0 Hz, 1H), 7.61-7.57 (m, 2H),7.50-7.48 (d, J = 8.8 Hz, 1H), 7.41 (d, J = 8.8 Hz, 1H), 7.19-7.13 (m, 3H), 6.98-6.89 (m, 1H), 5.10-5.05 (m, 1H),4.37-4.22 (m, 4H), 4.02-3.99 (m, 4H), 3.64 (s, 2H), 3.21-3.11 (m, 5H), 2.90-2.87 (m, 3H), 2.70-2.60 (m, 1H), 2.40-2.35 (m, 1H), 2.25-2.17 (m, 4H), 1.98-1.95 (m, 1H), 1.85-1.78(m, 2H), 1.17-1.12 (m, 13H). |
| 40 | 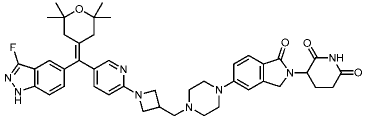 | 761.39 | 1H NMR (400 MHz, DMSO-d6) δ (ppm): 12.56 (s, 1H), 11.02 (s, 1H), 7.98-7.96 (d, J = 2.4 Hz, 1H), 7.64-7.60 (d, J = 8.4 Hz, 1H), 7.55-7.44 (m, 3H),7.26-7.16 (m, 3H), 7.00-6.99 (d, J = 6.8 Hz, 1H), 5.10-5.05 (m, 1H), 4.39-4.34 (d, J = 17.2 Hz, 2H), 4.29-4.22 (m, 3H), 4.03-4.00 (d, J = 10.0 Hz, 3H), 3.65-3.63 (d, J = 8.4 Hz, 2H), 3.25-3.12 (m, 5H), 2.95-2.88 (m, 3H), 2.62-2.58 (d, J = 16.4 Hz, 1H), 2.41-2.34 (m, 1H), 2.24 (s, 2H), 2.16 (s, 2H), 1.98-1.93 (m, 1H), 1.25-1.12 (m, 12H). |
| 41 | 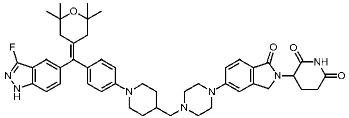 | 789 | 1H NMR (400 MHz, DMSO-d6) δ (ppm): 12.56 (s, 1H), 10.98 (s, 1H), 7.98-7.95 (d, J = 2.4 Hz, 1H), 7.64-7.60 (d, J = 8.4 Hz, 1H), 7.53-7.45 (m, 3H),7.28-7.16 (m, 3H), 7.00-6.79 (d, J = 6.8 Hz, 2H), 5.10-5.05 (m, 1H), 4.39-4.34 (d, J = 17.2 Hz, 2H), 4.28-4.22 (m, 3H), 4.03-4.00 (d, J = 10.0 Hz, 3H), 3.65-3.63 (d, J = 8.4 Hz, 2H), 3.24-3.12 (m, 5H), 2.95-2.88 (m, 3H), 2.62-2.58(d, J = 16.4 Hz, 1H), 2.41-2.34 (m, 1H), 2.24 (s, 2H), 2.16 (s, 2H), 1.97-1.93 (m, 1H), 1.86-1.84 (d, J = 12.3 Hz, 2H), 1.23-1.13 (m, 12H). |
| 42 | 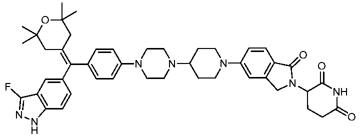 | 788.3 | 1H NMR (400 MHz, DMSO-d6) δ (ppm): 12.58(s, 1H), 10.96 (s, 1H), 7.98-7.95 (d, J = 2.4 Hz, 1H), 7.65-7.60 (d, J = 8.4 Hz, 1H), 7.53-7.45 (m, 3H),7.28-7.16 (m, 3H), 7.02-6.79 (d, J = 6.8 Hz, 2H), 5.10-5.05 (m, 1H), 4.38-4.32 (d, J = 17.2 Hz, 2H), 4.28-4.21 (m, 3H), 4.05-4.00 (d, J = 10.0 Hz, 3H), 3.68-3.63 (d, J = 8.4 Hz, 2H), 3.24-3.12 (m, 5H), 2.92-2.86 (m, 2H), 2.62-2.58 (d, J = 16.4 Hz, 1H), 2.41-2.34 (m, 1H), 2.24 (s, 2H), 2.16 (s, 2H), 1.88-1.84 (d, J = 12.3 Hz, 2H), 1.25-1.13 (m, 12H). |
| 43 | 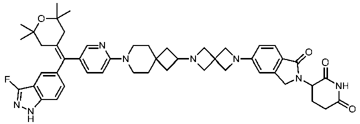 | 827.4 | 1H NMR (400 MHz, DMSO-d6) δ (ppm): 12.54 (s, 1H), 11.02 (s, 1H), 7.97-7.96 (d, J = 2.4 Hz, 1H), 7.61-7.60 (d, J = 8.4 Hz, 1H), 7.53-7.44 (m, 3H),7.26-7.16 (m, 3H), 7.00-6.99 (d, J = 6.8 Hz, 1H), 5.10-5.05 (m, 1H), 4.39-4.34 (d, J = 17.2 Hz, 2H), 4.28-4.22 (m, 3H), 4.03-4.00 (d, J = 10.0 Hz, 3H), 3.68-3.64 (d, J = 8.4 Hz, 2H), 3.26-3.15 (m, 5H), 2.96-2.88 (m, 3H), 2.62-2.58 (d, J = 16.4 Hz, 1H), 2.41-2.34 (m, 1H), 2.25 (s, 2H), 2.18 (s, 2H), 1.97-1.93 (m, 1H), 1.88-1.84 (d, J = 12.3 Hz, 2H), 1.26-1.13 (m, 12H). |
| 44 | 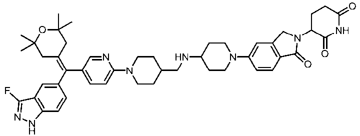 | 803.43 | 1H NMR (400 MHz, DMSO-d6) δ (ppm):12.58 (s, 1H), 10.99 (s, 1H), 7.98 (s,1H),7.61-7.58(d, J = 8.4 Hz, 1H), 7.55-7.42(m, 2H), 7.28-7.22 (m, 1H), 7.18-7.11 (m, 2H), 6.99-6.96 (d, J = 13.2 Hz, 1H), 5.45(s, 1H), 5.11-5.01 (m,1H), 4.65(s, 1H),4.41-4.18 (m, 2H), 3.98-3.81 (m, 3H), 3.71-3.63(s, 2H), 3.45-.317 (m, 3H), 2.95-2.88 (m, 1H), 2.69-2.53 (m, 1H), 2.44-2.36 (m, 2H), 2.22(m, 2H), 2.15(s, 1H), 2.01-1.94(m, 2H), 1.90-1.74(m, 4H), 1.73-1.58(m, 2H), 1.22-1.05(m, 12H). |
| 45 | 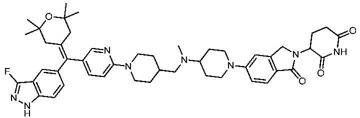 | 817.45 | 1H NMR (400 MHz, DMSO-d6) δ (ppm):12.52 (s, 1H), 10.93 (s, 1H), 7.96 (s,1H),7.62-7.58(d, J = 8.4 Hz, 1H), 7.56-7.42(m, 2H), 7.28-7.22 (m, 1H), 7.20-7.12 (m, 2H), 6.99-6.96 (d, J = 13.2 Hz, 1H), 5.13-5.01 (m,1H), 4.65(s, 1H),4.41-4.18 (m, 2H), 3.98-3.81 (m, 3H), 3.71-3.63(m, 2H), 3.45-3.19 (m, 3H), 2.95-2.88 (m, 1H), 2.69-2.53 (m, 1H), 2.44-2.36 (m, 2H), 2.26(s, 3H), 2.23-2.16(m, 2H), 2.15(s, 1H), 2.01-1.94(m, 2H), 1.90-1.76(m, 4H), 1.72-1.54(m, 2H), 1.25-1.12(m, 12H). |
| 46 | 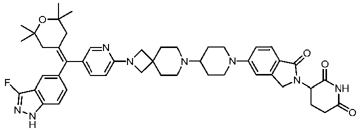 | 815.4 | 1H NMR (400 MHz, DMSO-d6) δ (ppm):12.58 (s, 1H), 10.99 (s, 1H), 7.95(s,1H),7.63-7.58(d, J = 8.4 Hz, 1H), 7.58-7.45(m, 2H), 7.29-7.22 (m, 1H), 7.20-7.12 (m, 2H), 6.99-6.96 (d, J = 13.2 Hz, 1H), 5.15-5.01 (m,1H), 4.65(s, 1H),4.41-4.18 (m, 2H), 3.98-3.81 (m, 3H), 3.71-3.63(m, 2H), 3.46-3.15 (m, 3H), 2.98-2.88 (m, 1H), 2.70-2.54 (m, 1H), 2.44-2.38 (m, 2H), 2.27(s, 3H), 2.22-2.16(m, 2H), 2.16(s, 1H), 2.01-1.94(m, 2H), 1.92-1.75(m, 4H), 1.74-1.54(m, 2H), 1.22-1.08(m, 12H). |
| 47 | 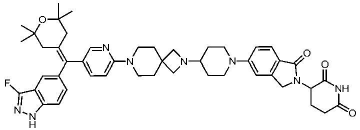 | 815.4 | 1H NMR (400 MHz, DMSO-d6) δ (ppm):12.56 (s, 1H), 10.95 (s, 1H), 7.98 (s,1H),7.64-7.58(d, J = 8.4 Hz, 1H), 7.56-7.42(m, 2H), 7.28-7.22 (m, 1H), 7.20-7.12 (m, 2H), 7.00-6.96 (d, J = 13.2 Hz, 1H), 5.13-5.01 (m,1H), 4.65(s, 1H),4.41-4.18 (m, 2H), 3.98-3.81 (m, 3H), 3.71-3.63(m, 2H), 3.45-3.19 (m, 3H), 2.95-2.88 (m, 1H), 2.69-2.53 (m, 1H), 2.46-2.34 (m, 4H), 2.22-2.10(m, 2H), 2.06-1.95(m, 2H), 1.92-1.76(m, 4H), 1.75-1.56(m, 2H), 1.28-1.10(m, 12H). |
| 48 | 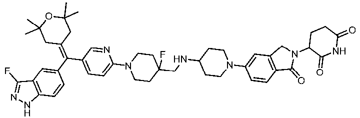 | 821.4 | 1H NMR (400 MHz, DMSO-d6) δ (ppm):12.55 (s, 1H), 10.98 (s, 1H),7.97 (s,1H),7.63-7.58(d, J = 8.4 Hz, 1H), 7.54-7.43(m, 2H), 7.26-7.22 (m, 1H), 7.18-7.12 (m, 2H), 6.99-6.96 (d, J = 13.2 Hz, 1H), 5.46(s, 1H), 5.11-5.01 (m,1H), 4.44-4.18 (m, 2H), 3.98-3.84 (m, 4H), 3.74-3.63(s, 2H), 3.45-3.17 (m, 7H), 2.97-2.88 (m, 1H), 2.69-2.53 (m, 1H), 2.44-2.36 (m, 1H), 2.22(s, 2H), 2.15(s, 1H), 1.81-1.73(m, 2H), 1.73-1.58(m, 2H), 1.19-1.01(m, 12H). |
| 49 | 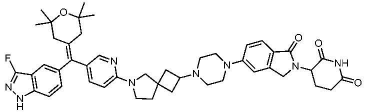 | 801.4 | 1H NMR (400 MHz, DMSO-d6) δ (ppm):12.55(s, 1H), 10.99(s, 1H), 7.84-7.78(m,1H), 7.47-7.40 (m, 2H), 7.24-7.10 (m, 2H), 7.12-7.06 (md, 2H),6.94-6.88 (d, 2H), 5.08-5.02 (m, 1H), 4.45-4.33 (d, 1H), 4.28-4.19 (d,1H), 4.12-4.01 (d, 2H), 3.91-3.83(d, 2H),3.74-3.65(d, 2H), 3.32-3.13 (m, 4H),3.12-3.05 (m, 2H), 2.96-2.86 (m,1H), 2.73-2.66 (m, 1H), 2.59-2.51(m, 2H), 2.35-2.32(m, 2H), 2.13-2.02(m, 2H), 2.05-1.94(m, 3H), 1.92-1.74(m, 3H), 1.28-1.22(m, 1H), 1.20-1.12(d,12H). |
| 50 | 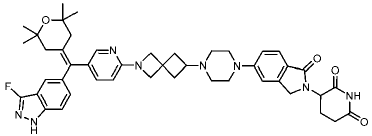 | 787.4 | 1H NMR (400 MHz, DMSO-d6) δ (ppm):12.52 (s, 1H), 10.96 (s, 1H), 7.82-7.78(m,1H), 7.46-7.41 (m, 2H), 7.25-7.12 (m, 2H), 7.10-7.06 (md, 2H),6.95-6.88 (d, 2H), 5.08-5.00 (m, 1H), 4.42-4.33 (d, 1H), 4.28-4.19 (d,1H), 4.12-4.01 (d, 2H), 3.91-3.83(d, 2H),3.74-3.65(d, 2H), 3.32-3.13 (m, 4H),3.12-3.05 (m, 2H), 2.96-2.86 (m,1H), 2.73-2.66 (m, 1H), 2.59-2.51(m, 2H), 2.33-2.32(m, 2H), 2.13-2.02(m, 2H), 2.01-1.98(m.2H), 1.85-1.74(m,2H), 1.28-1.22(m, 1H), 1.18-1.10(d,12H). |
| 51 | 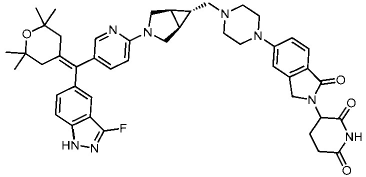 | 787.38 | 1H NMR (400 MHz, DMSO-d6) δ (ppm): 12.56 (s, 1H), 10.95 (s, 1H), 7.96-7.92 (d, J = 2.4 Hz, 1H), 7.62-7.60 (d, J = 8.4 Hz, 1H), 7.53-7.44 (m, 3H),7.26-7.16 (m, 3H), 7.00-6.99 (d, J = 6.8 Hz, 1H), 5.10-5.05 (m, 1H), 4.40-4.34 (d, J = 17.2 Hz, 2H), 4.28-4.22 (m, 3H), 4.07-4.02 (d, J = 10.0 Hz, 3H), 3.68-3.63 (d, J = 8.4 Hz, 2H), 3.27-3.12 (m, 5H), 2.95-2.88 (m, 3H), 2.66-2.58 (d, J = 16.4 Hz, 1H), 2.41-2.34 (m, 1H), 2.25 (s, 2H), 2.14 (s, 2H), 1.97-1.93 (m, 1H), 1.25-1.12 (m, 12H). |
| 52 | 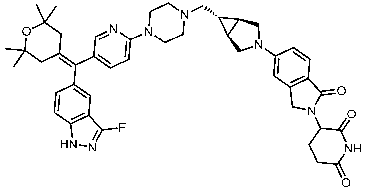 | 787.4 | 1H NMR (400 MHz, DMSO-d6) δ (ppm): 12.58 (s, 1H), 10.99 (s, 1H), 7.98-7.96 (d, J = 2.4 Hz, 1H), 7.62-7.60 (d, J = 8.4 Hz, 1H), 7.53-7.44 (m, 3H),7.26-7.16 (m, 3H), 7.02-6.99 (d, J = 6.8 Hz, 1H), 5.10-5.05 (m, 1H), 4.39-4.34 (d, J = 17.2 Hz, 2H), 4.28-4.22 (m, 3H), 4.03-4.00 (d, J = 10.0 Hz, 3H), 3.65-3.63 (d, J = 8.4 Hz, 2H), 3.24-3.12 (m, 5H), 2.95-2.88 (m, 3H), 2.62-2.58 (d, J = 16.4 Hz, 1H), 2.41-2.34 (m, 1H), 2.24 (s, 2H), 2.18 (s, 2H), 1.97-1.93 (m, 1H), 1.25-1.11 (m, 12H). |
| 53 | 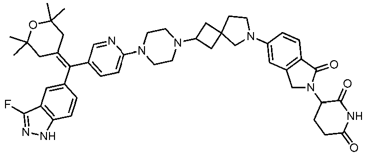 | 801.4 | 1H NMR (400 MHz, DMSO-d6) δ (ppm):12.56(s, 1H), 10.95(s, 1H), 7.85-7.78(m,1H), 7.48-7.41 (m, 2H), 7.24-7.11(m, 2H), 7.13-7.06 (md, 2H),6.94-6.88 (d, 2H), 5.08-5.02 (m, 1H), 4.45-4.33 (d, 1H), 4.28-4.19 (d,1H), 4.12-4.01 (d, 2H), 3.91-3.83(d, 2H),3.74-3.65(d, 2H), 3.32-3.13 (m, 4H),3.12-3.05 (m, 2H), 2.96-2.86 (m,1H), 2.73-2.66 (m, 1H), 2.59-2.51(m, 2H), 2.35-2.32(m, 2H), 2.13-2.02(m, 2H), 2.06-1.94(m, 3H), 1.94-1.74(m, 3H), 1.28-1.23(m, 1H), 1.21-1.13(d,12H). |
| 54 | 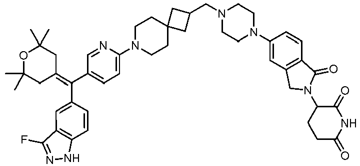 | 829.4 | 1H NMR (400 MHz, DMSO-d6) δ (ppm): 12.54(s, 1H), 10.98(s, 1H), 7.96-7.94 (d, J = 2.4 Hz, 1H), 7.62-7.60 (d, J = 8.4 Hz, 1H), 7.53-7.44 (m, 3H),7.26-7.16 (m, 3H), 7.00-6.99 (d, J = 6.8 Hz, 1H), 5.10-5.05 (m, 1H), 4.39-4.34 (d, J = 17.2 Hz, 2H), 4.28-4.22 (m, 3H), 4.04-4.00 (d, J = 10.0 Hz, 3H), 3.65-3.63 (d, J = 8.4 Hz, 2H), 3.25-3.12 (m, 5H), 2.95-2.88 (m, 3H), 2.63-2.58 (d, J = 16.4 Hz, 1H), 2.45-2.34 (m, 2H), 2.24 (s, 2H), 2.16 (s, 2H), 1.97-1.90 (m, 2H), 1.86-1.84 (d, J = 12.3 Hz, 2H), 1.23-1.12(m, 12H). |
| 55 | 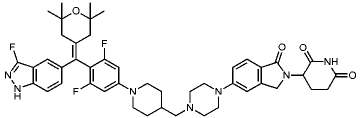 | 824.2 | 1H NMR (400 MHz, DMSO-d6) δ 10.98 (s, 1H), 9.34 (s, 1H), 7.73 (dd, J = 8.8, 2.1 Hz, 1H), 7.61 (d, J = 8.4 Hz, 1H), 7.41 (s, 1H), 7.32 (dd, J = 8.7, 1.5 Hz, 1H), 7.21 - 7.13 (m, 2H), 6.68 (d, J = 11.3 Hz, 2H), 5.77 (dt, J = 9.7, 2.6 Hz, 1H), 5.08 (dd, J = 13.3, 5.1 Hz, 1H), 4.36 (d, J = 17.1 Hz, 1H), 4.24 (d, J = 17.1 Hz, 1H), 4.01 (d, J = 10.9 Hz, 2H), 3.83 (dd, J = 13.4, 7.3 Hz, 3H), 3.72 - 3.67 (m, 1H), 3.19 (d, J = 11.2 Hz, 2H), 3.15 - 3.08 (m, 2H), 2.92 (ddd, J = 17.3, 13.6, 5.4 Hz, 1H), 2.76 (t, J = 11.8 Hz, 2H), 2.64 - 2.55 (m, 1H), 2.29 - 2.18 (m, 2H), 2.09 (s, 2H), 2.05 (s, 1H), 2.04 - 1.88 (m, 2H), 1.81 (d, J = 12.2 Hz, 2H), 1.71 (t, J = 4.7 Hz, 1H), 1.60 - 1.45 (m, 2H), 1.15 (d, J = 3.9 Hz, 12H). |
| 56 | 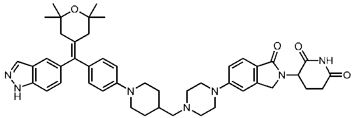 | 784 | 1H NMR (400 MHz, DMSO-d6) δ 11.11 (s, 1H), 8.03 (d, J = 1.0 Hz, 1H), 7.78 (d, J = 8.5 Hz, 1H), 7.56 - 7.48 (m, 2H), 7.47 (d, J = 8.5 Hz, 1H), 7.37 (dd, J = 8.7, 2.2 Hz, 1H), 7.28 (s, 1H), 7.17 - 7.06 (m, 4H), 7.05 - 6.91 (m, 3H), 5.11 (dd, J = 12.9, 5.4 Hz, 1H), 4.22 (d, J = 13.5 Hz, 2H), 3.68 (t, J = 13.5 Hz, 3H), 3.62 (s, 1H), 3.34 (t, J = 12.5 Hz, 2H), 2.96 - 2.87 (m, 1H), 2.79 (t, J = 12.0 Hz, 2H), 2.65 - 2.53 (m, 2H), 2.23 (s, 2H), 2.16 (s, 2H), 2.08 - 2.00 (m, 2H), 1.87 (d, J = 12.7 Hz, 2H), 1.38 (d, J = 12.3 Hz, 1H), 1.32 (d, J = 11.6 Hz, 1H), 1.22-1.12 (m, 12H). |
| 57 | 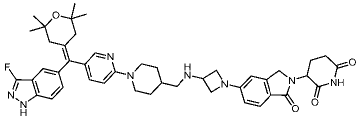 | 775.4 | 1H NMR (400 MHz, DMSO-d6) δ 12.56 (s, 1H), 10.93 (s, 1H), 8.40 (s, 1H), 7.99 - 7.94 (m, 1H), 7.58 - 7.48 (m, 3H), 7.45 (dd, J = 9.0, 2.3 Hz, 2H), 7.24 (d, J = 8.5 Hz, 1H), 7.15 - 7.06 (m, 2H), 6.97 (d, J = 10.1 Hz, 1H), 5.06 (dd, J = 13.3, 5.1 Hz, 1H), 4.33 (d, J = 16.9 Hz, 2H), 4.23 (t, J = 15.9 Hz, 4H), 4.02 (d, J = 13.3 Hz, 3H), 2.98 - 2.85 (m, 7H), 2.64 - 2.55 (m, 1H), 2.40 (td, J = 13.1, 4.5 Hz, 1H), 2.24 (s, 2H), 2.16 (s, 2H), 2.10 -1.92m, 4H), 1.22-1.12 (m, 12H). |
| 58 | 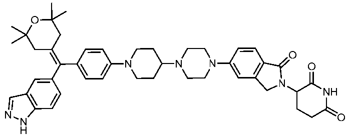 | 756 | 1H NMR (400 MHz, DMSO-d6) δ (ppm): 12.57 (s, 1H), 10.98(s, 1H), 7.96-7.94 (d, J = 2.4 Hz, 1H), 7.62-7.60 (d, J = 8.4 Hz, 1H), 7.53-7.44 (m, 3H),7.26-7.16 (m, 3H), 7.00-6.99 (d, J = 6.8 Hz, 1H), 5.10-5.05 (m, 1H), 4.39-4.34 (d, J = 17.2 Hz, 2H), 4.28-4.22 (m, 3H), 4.04-4.00 (d, J = 10.0 Hz, 3H), 3.65-3.63 (d, J = 8.4 Hz, 2H), 3.25-3.12 (m, 5H), 2.95-2.88 (m, 3H), 2.63-2.58 (d, J = 16.4 Hz, 1H), 2.45-2.34 (m, 2H), 2.24 (s, 2H), 2.16 (s, 2H), 1.97-1.90 (m, 2H), 1.86-1.84 (d, J = 12.3 Hz, 2H), 1.234-1.11 (m, 12H). |
| 59 | 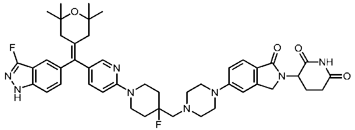 | 807 | 1H NMR (400 MHz, DMSO-d6) δ 12.57 (s, 1H), 10.97 (s, 1H), 8.01 (d, J = 2.4 Hz, 1H), 7.60 (d, J = 8.3 Hz, 1H), 7.51 (s, 1H), 7.50 - 7.41 (m, 2H), 7.24 (dd, J = 8.8, 1.4 Hz, 2H), 7.14 (d, J = 13.2 Hz, 3H), 6.98 (d, J = 10.0 Hz, 2H), 5.07 (dd, J = 13.3, 5.1 Hz, 2H), 4.36 (d, J = 17.0 Hz, 2H), 4.24 (d, J = 17.1 Hz, 2H), 4.12 (d, J = 13.0 Hz, 2H), 3.21 (t, J = 12.2 Hz, 3H), 2.98 - 2.85 (m, 2H), 2.64 - 2.55 (m, 2H), 2.40 (td, J = 13.1, 4.5 Hz, 2H), 2.24 (s, 2H), 2.16 (s, 2H), 2.08 - 1.93 (m, 4H), 1.85 (d, J = 10.7 Hz, 1H), 1.77 (d, J = 13.5 Hz, 1H), 1.22-1.13 (m, 12H). |
| 60 | 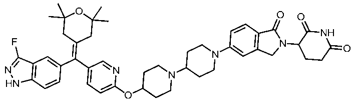 | 790 | 1H NMR (400 MHz, DMSO-d6) δ 12.56 (s, 1H), 10.95 (s, 1H), 8.07 (dd, J = 4.2, 2.4 Hz, 1H), 7.63 - 7.57 (m, 1H), 7.55 (dd, J = 5.5, 3.0 Hz, 1H), 7.53 (d, J = 3.5 Hz, 1H), 7.45 (dd, J = 8.7, 2.3 Hz, 1H), 7.24 (dd, J = 8.8, 1.5 Hz, 1H), 7.12 (dt, J = 10.8, 2.6 Hz, 2H), 6.80 (dd, J = 15.8, 8.5 Hz, 1H), 5.29 (d, J = 3.9 Hz, 1H), 5.18 (dt, J = 11.0, 6.3 Hz, 1H), 5.06 (dd, J = 13.2, 5.1 Hz, 1H), 4.34 (d, J = 17.4 Hz, 2H), 4.22 (d, J = 16.9 Hz, 2H), 4.12 - 4.03 (m, 3H), 3.61 - 3.39 (m, 4H), 3.25 - 3.15 (m, 2H), 2.98 - 2.86 (m, 3H), 2.65 - 2.56 (m, 1H), 2.46 - 2.28 (m, 3H), 2.07 (d, J = 19.9 Hz, 1H), 1.98 (d, J = 6.0 Hz, 1H), 1.76 (dd, J = 38.0, 12.5 Hz, 4H), 1.22-1.12 (m, 12H). |
| 61 | 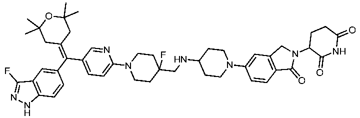 | 821 | 1H NMR (400 MHz, DMSO-d6) δ 12.57 (s, 1H), 10.96 (s, 1H), 8.69 (s, 2H), 8.00 (d, J = 2.3 Hz, 1H), 7.56 (s, 1H), 7.51 (t, J = 8.9 Hz, 2H), 7.45 (dd, J = 8.7, 2.4 Hz, 1H), 7.24 (dd, J = 8.7, 1.5 Hz, 1H), 7.15 - 7.07 (m, 2H), 7.00 (s, 1H), 5.06 (dd, J = 13.3, 5.1 Hz, 2H), 4.33 (d, J = 16.9 Hz, 1H), 4.21 (d, J = 16.9 Hz, 1H), 4.13 (d, J = 13.0 Hz, 2H), 4.03 (d, J = 12.9 Hz, 2H), 3.38 (s, 1H), 3.33 (s, 1H), 3.22 (d, J = 13.3 Hz, 2H), 2.90 (q, J = 12.0 Hz, 3H), 2.60 (d, J = 16.7 Hz, 1H), 2.38 (dd, J = 13.0, 4.5 Hz, 1H), 2.23 (s, 2H), 2.15 (s, 2H), 2.11 (d, J = 12.0 Hz, 2H), 1.97 (d, J = 4.7 Hz, 2H), 1.95 (s, 1H), 1.83 (s, 1H), 1.64 (d, J = 11.8 Hz, 2H), 1.22-1.12 (m, 12H). |
| 62 | 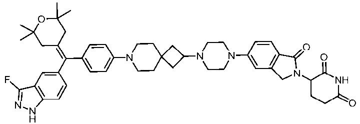 | 814 | 1H NMR (400 MHz, DMSO-d6) δ 12.44 (s, 1H), 11.04 (s, 1H), 7.46 - 7.38 (m, 2H), 7.22 - 7.13 (m, 3H), 7.08 (d, J = 8.2 Hz, 2H), 6.97 (d, J = 8.4 Hz, 2H), 5.07 (dd, J = 13.3, 5.1 Hz, 1H), 4.06 (d, J = 13.0 Hz, 3H), 3.83 (d, J = 8.6 Hz, 1H), 3.49 (d, J = 11.4 Hz, 2H), 3.18 (s, 2H), 3.10 (s, 3H), 3.02 - 2.85 (m, 3H), 2.61 (d, J = 3.5 Hz, 1H), 2.39 (dd, J = 13.2, 4.6 Hz, 1H), 2.24 (d, J = 8.6 Hz, 2H), 2.21 (s, 2H), 2.14 (s, 2H), 2.10 - 1.92 (m, 4H), 1.72-1.66(m, 4H),1.22-1.12 (m, 16H). |
| 63 | 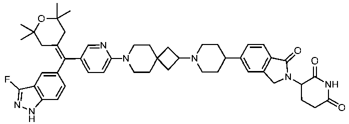 | 814 | 1H NMR (400 MHz, DMSO-d6) δ 12.57 (s, 1H), 11.00 (s, 1H), 7.93 (s, 1H), 7.72 (d, J = 8.0 Hz, 1H), 7.54 (d, J = 7.4 Hz, 2H), 7.51 - 7.38 (m, 3H), 7.24 (d, J = 8.3 Hz, 1H), 7.04 (s, 1H), 5.14 - 5.09 (m, 1H), 4.46 (d, J = 17.5 Hz, 2H), 4.32 (d, J = 17.4 Hz, 2H), 3.74 (d, J = 8.3 Hz, 2H), 2.94 (dd, J = 38.2, 12.9 Hz, 4H), 2.43 - 2.38 (m, 1H), 2.23 (s, 3H), 2.15 (s, 2H), 2.09 (d, J = 14.3 Hz, 3H), 2.02 (s, 2H), 1.88 (d, J = 13.1 Hz, 2H), 1.66 (s, 2H), 1.62 (s, 2H), 1.20-1.12 (m, 12H). |
| 64 | 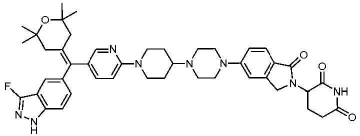 | 789 | 1H NMR (400 MHz, DMSO-d6) δ 12.58 (s, 1H), 11.12 (s, 1H), 9.87 (s, 1H), 8.01 (d, J = 2.4 Hz, 1H), 7.77 (d, J = 8.4 Hz, 1H), 7.53 - 7.48 (m, 2H), 7.45 (dt, J = 7.5, 3.3 Hz, 2H), 7.37 (dd, J = 8.6, 2.3 Hz, 1H), 7.23 (dd, J = 8.8, 1.5 Hz, 1H), 6.96 (d, J = 8.9 Hz, 1H), 5.11 (dd, J = 12.9, 5.4 Hz, 1H), 4.43 (d, J = 12.9 Hz, 2H), 4.26 (d, J = 9.4 Hz, 2H), 3.64 (d, J = 8.8 Hz, 2H), 3.22 (d, J = 9.7 Hz, 3H), 2.96 - 2.87 (m, 2H), 2.84 (t, J = 8.8 Hz, 2H), 2.64 - 2.53 (m, 2H), 2.23 (s, 2H), 2.17 (d, J = 11.3 Hz, 4H), 2.09 - 1.99 (m, 1H), 1.69 - 1.56 (m, 2H), 1.22-1.12 (s, 12H). |
| 65 | 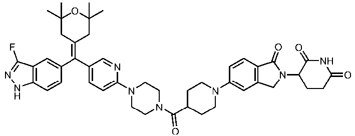 | 803 | 1H NMR (400 MHz, DMSO-d6) δ 12.57(s, 1H), 10.95(s, 1H), 7.97(s, 1H), 7.70-7.40(m, 4H), 7.30-7.20(m, 1H), 7.15-6.95(m, 3H), 5.10-5.00(m, 1H), 4.40-4.14(m, 2H), 3.97-3,92 (m, 1H), 3.75-3.55(m, 6H), 3.06-2.80(m, 4H), 2.78-2.52(m, 2H), 2.42-2.10(m, 6H), 2.00-1.90(m, 1H), 1.80-1.60(m, 4H), 1.50-1.30(m, 2H), 1.12-1.22 (m, 12H). |
| 66 | 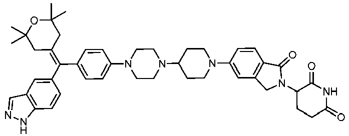 | 756 | 1H NMR (400 MHz, DMSO-d6) δ 10.96 (s, 1H), 8.03 (d, J = 1.0 Hz, 1H), 7.59 - 7.50 (m, 2H), 7.46 (d, J = 8.5 Hz, 1H), 7.17 - 7.07 (m, 5H), 6.95 (d, J = 8.8 Hz, 2H), 5.06 (dd, J = 13.3, 5.1 Hz, 1H), 4.34 (d, J = 16.9 Hz, 1H), 4.21 (d, J = 16.9 Hz, 1H), 4.09 (d, J = 12.8 Hz, 2H), 3.85 (d, J = 12.8 Hz, 2H), 3.64 (d, J = 11.6 Hz, 2H), 3.20 - 3.10 (m, 2H), 2.99 - 2.83 (m, 5H), 2.64 - 2.55 (m, 1H), 2.40 (td, J = 13.1, 4.6 Hz, 1H), 2.22 (s, 2H), 2.17 (d, J = 11.4 Hz, 4H), 2.02 - 1.93 (m, 1H), 1.70 (d, J = 12.1 Hz, 2H), 1.22-1.12 (s, 6H). |
| 67 | 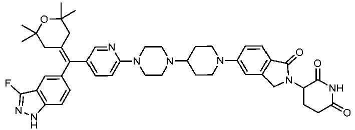 | 775 | 1H NMR (400 MHz, DMSO-d6) δ 12.52 (s, 1H), 10.96 (s, 1H), 9.40 (s, 1H), 7.74 (s, 1H), 7.48 - 7.41 (m, 2H), 7.12 (d, J = 8.2 Hz, 4H), 6.95-6.90 (m, 1H), 5.06 (dd, J = 13.3, 5.1 Hz, 2H), 4.34 (d, J = 16.9 Hz, 2H), 4.21 (d, J = 16.9 Hz, 2H), 4.09 (d, J = 12.8 Hz, 2H), 3.86 (d, J = 13.1 Hz, 2H), 3.64 (d, J = 11.7 Hz, 2H), 3.16 (d, J = 11.4 Hz, 2H), 2.92 (s, 2H), 2.87 (d, J = 11.7 Hz, 2H), 2.21 (s, 2H), 2.18 (s, 1H), 2.14 (s, 3H), 1.97 (d, J = 6.7 Hz, 1H), 1.22-1.12 (m, 12H). |
| 68 | 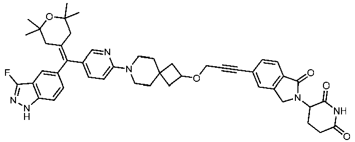 | 815 | 1H NMR (400 MHz, DMSO-d6) δ 12.56 (s, 1H), 11.00 (s, 1H), 7.89 (d, J = 2.3 Hz, 1H), 7.76 - 7.68 (m, 2H), 7.61 - 7.52 (m, 3H), 7.44 (dd, J = 8.5, 2.3 Hz, 1H), 7.25 (d, J = 8.8 Hz, 1H), 7.08 (s, 1H), 5.12 (dd, J = 13.3, 5.1 Hz, 1H), 4.44 (s, 1H), 4.35 (d, J = 8.1 Hz, 3H), 4.23 (p, J = 6.9 Hz, 2H), 3.52 (s, 2H), 2.91 (ddd, J = 17.9, 13.5, 5.4 Hz, 1H), 2.65 - 2.56 (m, 1H), 2.46 - 2.35 (m, 2H), 2.24 (d, J = 11.4 Hz, 3H), 2.15 (s, 2H), 2.05 - 1.97 (m, 1H), 1.76 (dd, J = 11.9, 7.1 Hz, 2H), 1.61 (s, 4H), 1.22-1.12 (m, 12H). |
| 69 | 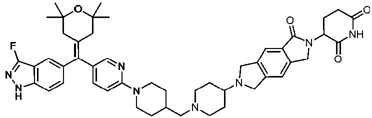 | 829 | 1H NMR (400 MHz, chloroform-d) δ 9.58 (s, 1H), 8.32 - 8.28 (m, 1H), 8.08 - 8.03 (m, 1H), 7.99 - 7.95 (m, 1H), 7.65 - 7.59 (m, 1H), 7.54 - 7.49 (m, 1H), 7.31 - 7.25 (m, 2H), 6.73 - 6.68 (m, 1H), 4.59 - 4.53 (m, 1H), 4.45 - 4.41 (m, 1H), 4.38 - 4.35 (m, 1H), 4.33 - 4.27 (m, 1H), 4.00 - 3.94 (m, 1H), 3.94 - 3.89 (m, 1H), 3.89 - 3.84 (m, 1H), 3.66 - 3.52 (m, 4H), 2.86 - 2.78 (m, 1H), 2.78 - 2.71 (m, 2H), 2.66 - 2.58 (m, 4H), 2.58 - 2.54 (m, 1H), 2.54 - 2.46 (m, 2H), 2.31 (s, 2H), 2.22 (s, 2H), 2.11 - 2.01 (m, 1H), 1.91 - 1.82 (m, 6H), 1.82 - 1.76 (m, 2H), 1.73 - 1.63 (m, 1H), 1.62 - 1.53 (m, 2H), 1.28 - 1.24 (m, 12H). |
| 70 | 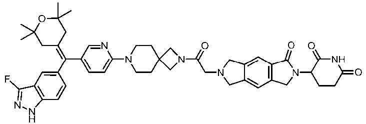 | 815 | 1H NMR (400 MHz, chloroform-d) δ 9.58 (s, 1H), 8.32 - 8.28 (m, 1H), 8.08 - 8.03 (m, 1H), 7.97 - 7.93 (m, 1H), 7.65 - 7.59 (m, 1H), 7.54 - 7.49 (m, 1H), 7.31 - 7.25 (m, 2H), 6.73 - 6.68 (m, 1H), 4.59 - 4.53 (m, 1H), 4.45 - 4.41 (m, 1H), 4.38 - 4.35 (m, 1H), 4.02 - 3.97 (m, 1H), 3.97 - 3.90 (m, 2H), 3.90 - 3.86 (m, 1H), 3.73 - 3.68 (m, 2H), 3.66 (s, 2H), 3.56 (s, 2H), 3.54 - 3.48 (m, 2H), 3.30 (s, 2H), 2.63 - 2.54 (m, 1H), 2.54 - 2.46 (m, 1H), 2.31 (s, 2H), 2.11 - 2.01 (m, 1H), 1.89 - 1.83 (m, 2H), 1.76 - 1.71 (m, 2H), 1.71 - 1.63 (m, 1H), 1.28 - 1.24 (m, 12H). |
| 71 | 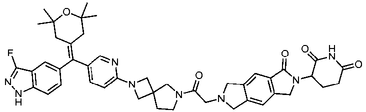 | 801.4 | 1H NMR (400 MHz, DMSO-d6) δ (ppm):12.45 (s, 1H), 10.96 (s, 1H), 7.62(m,1H), 7.46-7.41 (m, 2H), 7.23-7.12 (m, 3H), 7.12-7.08 (m, 2H),6.96-6.88 (m, 2H), 5.06-5.02 (m, 1H), 4.40-4.33 (m, 1H), 4.28-4.19 (m,1H), 4.12-4.00 (m, 2H), 3.92-3.83(m, 2H),3.75-3.60(m, 2H), 3.35-3.10 (m, 4H),3.29 (s, 2H), 2.59-2.51(m, 2H), 2.33-2.32(m, 2H), 2.15-2.05(m, 2H), 2.00-1.92(m.2H),1.88-1.82(m, 2H),1.16-1.13(m,12H). |
| 72 | 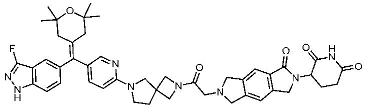 | 801.4 | 1H NMR (400 MHz, DMSO-d6) δ (ppm):12.47 (s, 1H), 11.02(s, 1H), 7.64(m,1H), 7.46-7.40 (m, 2H), 7.23-7.15 (m, 3H), 7.10-7.08 (m, 2H),6.98-6.90 (m, 2H), 5.08-5.02 (m, 1H), 4.40-4.33 (m, 1H), 4.28-4.19 (m,1H), 4.15-4.00 (m, 2H), 3.92-3.83(m, 2H),3.76-3.65(m, 2H), 3.37-3.16 (m, 4H),3.30 (s, 2H), 2.60-2.51(m, 2H), 2.35-2.32(m, 2H), 2.16-2.05(m, 2H), 2.02-1.92(m.2H), 1.88-1.84(m, 2H), 1.18-1.12(m,12H). |
| 73 | 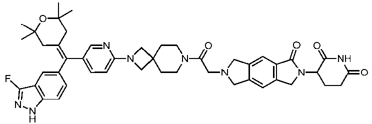 | 771.06 | 1H NMR (400 MHz, DMSO-d6) δ (ppm):12.56 (s, 1H), 10.96 (s, 1H), 8.38-8.25(m, 2H), 8.02-7.62(m, 4H), 7.55-7.48 (m, 1H), 7.36-7.27 (m, 1H), 6.99-6.94 (m, 1H), 4.48-4.42 (m,1H), 4.45-4.24 (m, 2H), 4.02-3.85 (m, 4H), 3.72-3.58 (s, 6H), 3.53-3.25 (m, 2H), 3.32 (s, 2H), 2.48-2.36 (m, 1H), 2.08-1.95(m, 1H), 1.88-1.73(m, 4H), 1.76-1.56(m, 4H), 1.26-1.05(m, 12H). |
| 74 | 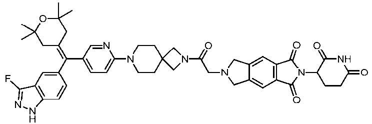 | 829.3 | 1H NMR (400 MHz, DMSO-d6) δ (ppm):12.40 (s, 1H), 11.02 (s, 1H), 8.35-8.22(m, 2H), 8.05-7.68(m, 4H), 7.56-7.48 (m, 1H), 7.38-7.28 (m, 1H), 7.00-6.92 (m, 1H), 4.48-4.42(m,1H), 4.42-4.25 (m, 2H), 3.99-3.80 (m, 4H), 3.75-3.56 (s, 6H), 3.28 (s, 2H), 2.45-2.36 (m, 1H), 2.05-1.94(m, 1H), 1.88-1.73(m, 4H), 1.78-1.56(m, 4H), 1.26-1.05(m, 12H). |
| 75 | 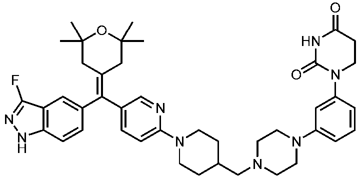 | 735 | 1H NMR (400 MHz, chloroform-d) δ 9.08 (s, 1H), 8.32 - 8.28 (m, 1H), 8.08 - 8.03 (m, 1H), 7.65 - 7.59 (m, 1H), 7.54 - 7.49 (m, 1H), 7.31 - 7.25 (m, 1H), 7.20 - 7.12 (m, 2H), 6.73 - 6.68 (m, 1H), 6.50 - 6.44 (m, 1H), 6.38 - 6.33 (m, 1H), 3.91 - 3.85 (m, 1H), 3.83 - 3.77 (m, 1H), 3.66 - 3.52 (m, 4H), 3.27 - 3.21 (m, 4H), 2.74 - 2.67 (m, 2H), 2.66 - 2.59 (m, 3H), 2.58 - 2.53 (m, 2H), 2.53 - 2.47 (m, 1H), 2.31 (s, 2H), 2.22 (s, 2H), 1.92 - 1.82 (m, 5H), 1.28 - 1.24 (m, 12H). |
| 76 | 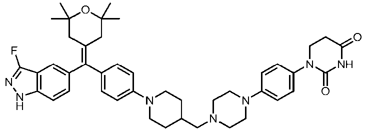 | 734 | 1H NMR (400 MHz, DMSO-d6) δ 10.46 (s, 1H), 9.07 (s, 1H), 7.82 (dd, J = 8.7, 2.2 Hz, 1H), 7.77 - 7.70 (m, 2H), 7.62 (s, 1H), 7.57 - 7.49 (m, 2H), 7.36 (dd, J = 8.8, 1.6 Hz, 1H), 7.26 - 7.17 (m, 1H), 7.14-7.10 (m, 2H), 7.00 (s, 2H), 3.87 (t, J = 6.6 Hz, 2H), 3.70 (d, J = 11.9 Hz, 2H), 3.35 (s, 5H), 3.22 (t, J = 6.8 Hz, 2H), 2.90 (s, 2H), 2.79 - 2.70 (m, 4H), 2.24 (s, 2H), 2.18 (s, 2H), 1.86-1.78 (m, 3H), 1.32-1.12 (m, 14H). |
| 77 | 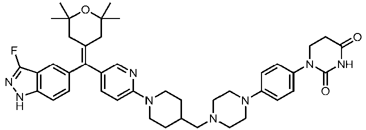 | 735 | 1H NMR (400 MHz, DMSO-d6) δ 10.48 (s, 1H), 9.08 (s, 1H), 7.82 (dd, J = 8.7, 2.2 Hz, 1H), 7.77 - 7.70 (m, 2H), 7.62 (s, 1H), 7.57 - 7.49 (m, 2H), 7.36-7.30 (m, 1H), 7.26 - 7.17 (m, 1H), 7.13 (d, J = 8.0 Hz, 2H), 6.55-6.60 (m, 1H), 3.87 (t, J = 6.6 Hz, 2H), 3.70 (d, J = 11.9 Hz, 2H), 3.35 (s, 5H), 3.22 (t, J = 6.8 Hz, 2H), 2.90 (s, 2H), 2.79 - 2.70 (m, 4H), 2.24 (s, 2H), 2.18 (s, 2H), 1.86-1.78 (m, 3H), 1.32-1.12 (m, 14H). |
| 78 | 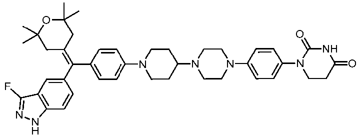 | 720 | 1H NMR (400 MHz, DMSO-d6) δ 10.48 (s, 1H), 10.21 (s, 1H), 8.83 (s, 1H), 8.58 (s, 1H), 7.78 - 7.70 (m, 2H), 7.58 - 7.48 (m, 2H), 7.14 (d, J = 8.3 Hz, 2H), 6.96 (d, J = 8.7 Hz, 2H), 3.86 (t, J = 6.6 Hz, 3H), 3.15 (s, 3H), 3.00 - 2.89 (m, 3H), 2.75 (t, J = 6.6 Hz, 2H), 2.29 (s, 1H), 2.24 (s, 2H), 2.18 (s, 2H), 1.82-1.74 (m, 2H), 1.22-1.14 (m, 12H). |
| 79 | 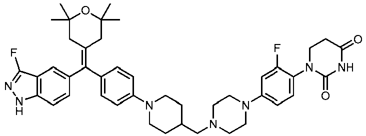 | 765 | 1H NMR (400 MHz, DMSO-d6) δ 10.84 (s, 1H), 8.79 (s, 3H), 7.63 (dd, J = 8.9, 2.2 Hz, 1H), 7.42 (dd, J = 12.4, 2.5 Hz, 1H), 7.29 (dd, J = 8.9, 2.6 Hz, 2H), 7.16-7.08 (m, 3H), 6.99 (t, J = 9.2 Hz, 2H), 6.94 (s, 1H), 6.94 - 6.86 (m, 2H), 4.53 (s, 1H), 3.68 (d, J = 12.0 Hz, 4H), 3.23 (s, 1H), 2.79 (dt, J = 17.9, 9.3 Hz, 3H), 2.70 (s, 1H), 2.66 - 2.56 (m, 3H), 2.24 (s, 2H), 2.17 (s, 2H), 2.11 (h, J = 4.3 Hz, 3H), 1.84-1.76 (m, 3H), 1.28-1.14 (m, 15H). |
| 80 | 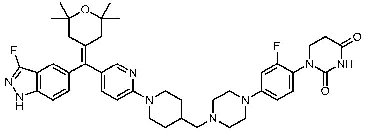 | 766 | 1H NMR (400 MHz, DMSO-d6) δ 10.86 (s, 1H), 8.79 (s, 3H), 7.63 (dd, J = 8.9, 2.2 Hz, 1H), 7.56 (s, 1H), 7.42 (dd, J = 12.4, 2.5 Hz, 1H), 7.29 (dd, J = 8.9, 2.6 Hz, 2H), 7.08 (d, J = 8.1 Hz, 2H), 6.99 (t, J = 9.2 Hz, 2H), 6.94 (s, 1H), 6.93 - 6.89 (m, 1H), 4.53 (s, 1H), 3.68 (d, J = 12.0 Hz, 4H), 3.23 (s, 1H), 2.79 (dt, J = 17.9, 9.3 Hz, 3H), 2.70 (s, 1H), 2.66 - 2.56 (m, 3H), 2.24 (s, 2H), 2.17 (s, 2H), 2.11 (h, J = 4.3 Hz, 3H), 1.84-1.76 (m, 3H), 1.28-1.14 (m, 15H). |
| 81 | 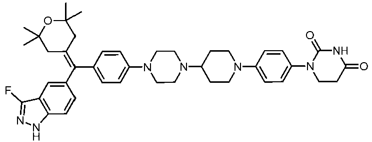 | 720 | 1H NMR (400 MHz, DMSO-d6) δ 10.46 (s, 1H), 10.21 (s, 1H), 8.83 (s, 1H), 8.58 (s, 1H), 7.78 - 7.70 (m, 2H), 7.58 - 7.49 (m, 2H), 7.15 (d, J = 8.3 Hz, 2H), 6.97 (d, J = 8.7 Hz, 2H), 3.86 (t, J = 6.6 Hz, 3H), 3.15 (s, 3H), 3.00 - 2.89 (m, 3H), 2.75 (t, J = 6.6 Hz, 2H), 2.29 (s, 1H), 2.24 (s, 2H), 2.18 (s, 2H), 1.82-1.76 (m, 2H), 1.22-1.12 (m, 12H). |
| 82 | 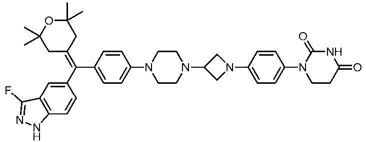 | 692 | 1H NMR (400 MHz, DMSO-d6) δ 10.46 (s, 1H), 8.73 (s, 1H), 7.80 (dd, J = 8.8, 2.2 Hz, 1H), 7.74 (d, J = 8.6 Hz, 2H), 7.61 (s, 1H), 7.53 (d, J = 8.7 Hz, 2H), 7.35 (dd, J = 8.9, 1.5 Hz, 1H), 7.14 - 7.06 (m, 2H), 7.00 - 6.89 (m, 2H), 3.86 (t, J = 6.6 Hz, 5H), 3.20 (s, 4H), 2.75 (t, J = 6.6 Hz, 2H), 2.61 (s, 3H), 2.25 (s, 2H), 2.18 (s, 2H), 1.24 -1.12 (m, 12H). |
| 83 | 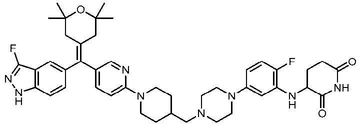 | 767 | 1H NMR (400 MHz, chloroform-d) δ 9.53 (s, 1H), 8.32 - 8.28 (m, 1H), 8.08 - 8.03 (m, 1H), 7.65 - 7.59 (m, 1H), 7.54 - 7.49 (m, 1H), 7.31 - 7.25 (m, 1H), 6.96 - 6.90 (m, 1H), 6.76 - 6.68 (m, 2H), 6.39 - 6.34 (m, 1H), 5.50 - 5.44 (m, 1H), 4.38 - 4.31 (m, 1H), 3.66 - 3.52 (m, 4H), 3.27 - 3.21 (m, 4H), 2.66 - 2.59 (m, 3H), 2.59 - 2.47 (m, 5H), 2.31 (s, 2H), 2.22 (s, 2H), 2.04 - 1.94 (m, 1H), 1.92 - 1.82 (m, 5H), 1.61 - 1.51 (m, 1H), 1.28 - 1.24 (m, 12H). |
| 84 | 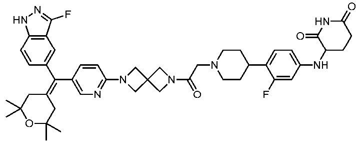 | 789.4 | 1H NMR (400 MHz, DMSO-d6) δ (ppm):12.45 (s, 1H), 10.96 (s, 1H), 7.62(m,1H), 7.46-7.41 (m, 2H), 7.23-7.12 (m, 3H), 7.12-7.08 (m, 2H),6.96-6.88 (m, 2H), 5.06-5.02 (m, 1H), 4.40-4.33 (m, 1H), 4.28-4.19 (m,lH), 4.12-4.00 (m, 2H), 3.92-3.83(m, 2H),3.75-3.60(m, 2H), 3.35-3.10 (m, 4H),3.29 (s, 2H), 2.59-2.51(m, 2H), 2.33-2.32(m, 2H), 2.15-2.05(m, 2H), 2.00-1.92(m.2H), 1.88-1.82(m, 2H), 1.16-1.13(m, 12H). |
| 85 | 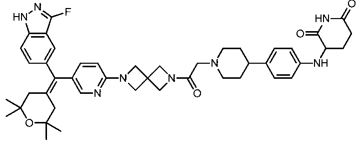 | 801.4 | 1H NMR (400 MHz, DMSO-d6) δ (ppm):12.47 (s, 1H), 11.02(s, 1H), 7.64(m,1H), 7.46-7.40 (m, 2H), 7.23-7.15 (m, 3H), 7.10-7.08 (m, 2H),6.98-6.90 (m, 2H), 5.08-5.02 (m, 1H), 4.40-4.33 (m, 1H), 4.28-4.19 (m,1H), 4.15-4.00 (m, 2H), 3.92-3.83(m, 2H),3.76-3.65(m, 2H), 3.37-3.16 (m, 4H),3.30 (s, 2H), 2.60-2.51(m, 2H), 2.35-2.32(m, 2H), 2.16-2.05(m, 2H), 2.02-1.92(m.2H), 1.88-1.84(m, 2H), 1.18-1.12(m,12H). |
| 86 | 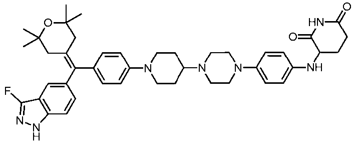 | 734 | 1H NMR (400 MHz, DMSO-d6) δ 10.82 (s, 1H), 10.20 (s, 1H), 8.82 (s, 1H), 8.59 (s, 1H), 7.55 (d, J = 10.5 Hz, 2H), 7.35 (d, J = 8.5 Hz, 2H), 7.27 (d, J = 8.8 Hz, 1H), 7.14 (d, J = 8.3 Hz, 2H), 6.97 (d, J = 8.3 Hz, 2H), 6.83 (d, J = 8.6 Hz, 2H), 4.42 (dd, J = 11.6, 4.8 Hz, 1H), 3.85 (s, 2H), 3.47 (d, J = 12.8 Hz, 4H), 3.15 (s, 1H), 2.96 (d, J = 14.1 Hz, 2H), 2.90 (d, J = 4.9 Hz, 1H), 2.79 - 2.70 (m, 1H), 2.62 (dt, J = 17.7, 4.3 Hz, 2H), 2.32 - 2.21 (m, 4H), 2.20 - 2.09 (m, 3H), 1.94 (qd, J = 12.5, 4.7 Hz, 1H), 1.84 1.75 (m, 2H), 1.24 -1.14 (m, 13H). |
| 87 | 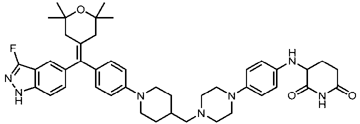 | 748 | 1H NMR (400 MHz, DMSO-d6) δ10.86 (s, 1H), 10.21 (s, 1H), 7.85 (s, 1H), 7.77 (dd, J = 8.8, 2.2 Hz, 1H), 7.70 (d, J = 1.3 Hz, 4H), 7.61 (s, 1H), 7.36 - 7.29 (m, 2H), 7.11 (d, J = 8.1 Hz, 2H), 4.75 (dd, J = 8.5, 3.3 Hz, 1H), 3.70 (d, J = 12.2 Hz, 6H), 3.34 (s, 4H), 2.94 (s, 1H), 2.72 (s, 1H), 2.60 (dt, J = 15.1, 8.4 Hz, 2H), 2.50 - 2.36 (m, 5H), 2.23 (s, 2H), 2.17 (s, 2H), 2.01 - 1.94 (m, 1H), 1.83 (d, J = 13.1 Hz, 2H), 1.29-1.14 (m, 14H). |
| 88 | 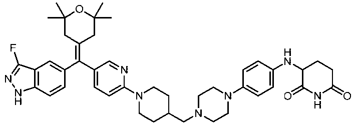 | 749 | 1H NMR (400 MHz, DMSO-d6) δ10.88 (s, 1H), 10.22 (s, 1H), 7.85 (s, 1H), 7.78-7.70 (m, 4H), 7.61 (s, 1H), 7.36 - 7.29 (m, 2H), 7.12-7.08 (m, 1H), 6.60-6.66 (m, 1H), 4.75 (dd, J = 8.5, 3.3 Hz, 1H), 3.70 (d, J = 12.2 Hz, 6H), 3.34 (s, 4H), 2.94 (s, 1H), 2.72 (s, 1H), 2.60 (dt, J = 15.1, 8.4 Hz, 2H), 2.50 - 2.36 (m, 5H), 2.23 (s, 2H), 2.17 (s, 2H), 2.01 - 1.94 (m, 1H), 1.83 (d, J = 13.1 Hz, 2H), 1.28-1.12 (m, 14H). |
| 89 | 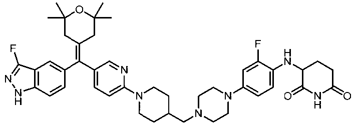 | 767 | 1H NMR (400 MHz, DMSO-d6) δ 10.86 (s, 1H), 8.79 (s, 3H), 7.63 (dd, J = 8.9, 2.2 Hz, 1H), 7.56 (s, 1H), 7.42 (dd, J = 12.4, 2.5 Hz, 1H), 7.30-7.25 (m, 2H), 7.10-7.08 (m, 2H), 6.99 (t, J = 9.2 Hz, 2H), 6.93 - 6.89 (m, 1H), 4.53 (s, 1H), 3.68 (d, J = 12.0 Hz, 4H), 3.23 (s, 1H), 2.79 (dt, J = 17.9, 9.3 Hz, 3H), 2.70 (s, 1H), 2.66 - 2.56 (m, 3H), 2.24 (s, 2H), 2.17 (s, 2H), 2.11 (h, J = 4.3 Hz, 3H), 1.81 (d, J = 12.4 Hz, 3H), 1.28 - 1.20 (m, 3H), 1.16 (d, J = 15.3 Hz, 12H). |
| 90 | 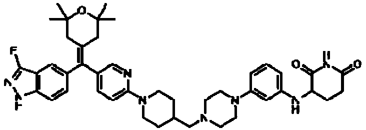 | 735 | 1H NMR (400 MHz, DMSO-d6) δ (ppm):10.50 (s, 1H), 8.37 (s, 1H), 8.01 (s,1H), 7.86-7.84 (d,J=8.0 Hz, 1H), 7.73 (s, 1H), 7.64 (s, 1H),7.59-7.57 (d, J=8.0 Hz, 2H), 7.38-7.31 (m, 3H), 7.76-7.74 (d, J=8.0 Hz, 1H), 2.25-4.22 (d, J=12.0 Hz, 2H), 3.91-3.88 (t, 2H), 2.79-2.79(m, 8H),2.34-2.26(m, 6H), 2.18-2.11 (m, 4H),1.76-1.73 (m, 3H), 1.24-1.19(d, J=20.0 Hz, 12H), 1.13-1.08(m, 2H). |
| 91 | 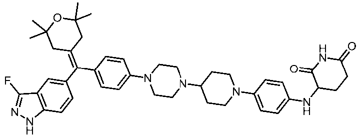 | 734 | 1H NMR (400 MHz, DMSO-d6) δ 10.84 (s, 1H), 10.22 (s, 1H), 8.84 (s, 1H), 8.60 (s, 1H), 7.56 (d, J = 10.5 Hz, 2H), 7.35 (d, J = 8.5 Hz, 2H), 7.27 (d, J = 8.8 Hz, 1H), 7.14 (d, J = 8.3 Hz, 2H), 6.97 (d, J = 8.3 Hz, 2H), 6.83 (d, J = 8.6 Hz, 2H), 4.42 (dd, J = 11.6, 4.8 Hz, 1H), 3.85 (s, 2H), 3.47 (d, J = 12.8 Hz, 4H), 3.15 (s, 1H), 2.96 (d, J = 14.1 Hz, 2H), 2.90 (d, J = 4.9 Hz, 1H), 2.79 - 2.70 (m, 1H), 2.62 (dt, J = 17.7, 4.3 Hz, 2H), 2.32 - 2.21 (m, 4H), 2.20 -1.92 (m, 4H), 1.84- 1.75 (m, 2H), 1.24 -1.12 (m, 13H). |
| 92 | 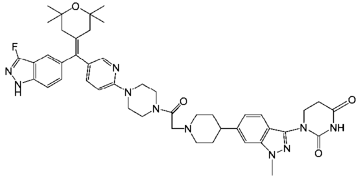 | 817 | 1H NMR (500 MHz, chloroform-d) δ 9.14 (s, 1H), 8.30 (d, J = 2.0 Hz, 1H), 8.08 - 8.03 (m, 1H), 7.87 (d, J = 8.5 Hz, 1H), 7.62 (d, J = 7.6 Hz, 1H), 7.54 - 7.48 (m, 1H), 7.31 - 7.25 (m, 2H), 7.04 - 6.98 (m, 1H), 6.71 (d, J = 7.9 Hz, 1H), 4.07 - 4.01 (m, 1H), 4.01 - 3.95 (m, 4H), 3.71 - 3.60 (m, 8H), 3.18 (s, 2H), 2.79 - 2.59 (m, 7H), 2.31 (s, 2H), 1.97 - 1.82 (m, 4H), 1.26 (s, 12H). |
| 93 | 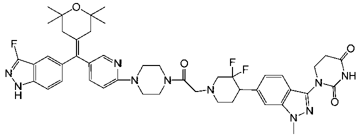 | 853 | 1H NMR (500 MHz, chloroform-d) δ 9.14 (s, 1H), 8.30 (d, J = 2.0 Hz, 1H), 8.08 - 8.03 (m, 1H), 7.77 (d, J = 7.8 Hz, 1H), 7.62 (d, J = 7.6 Hz, 1H), 7.57 - 7.48 (m, 2H), 7.31 - 7.25 (m, 1H), 7.20 - 7.14 (m, 1H), 6.71 (d, J = 7.9 Hz, 1H), 4.07 - 4.01 (m, 1H), 4.01 - 3.95 (m, 4H), 3.69 (d, J = 2.0 Hz, 1H), 3.68 - 3.65 (m, 4H), 3.65 - 3.56 (m, 3H), 3.47 - 3.33 (m, 1H), 3.32 - 3.26 (m, 2H), 3.22 - 3.10 (m, 1H), 2.80 - 2.67 (m, 4H), 2.31 (s, 2H), 2.03 - 1.85 (m, 2H), 1.29 (s, 6H), 1.24 (s, 6H). |
| 94 | 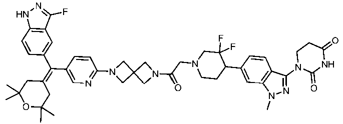 | 865.4 | 1H NMR (400 MHz, DMSO-d6) δ (ppm):12.45 (s, 1H), 10.76 (s, 1H), 8.38-8.17(m, 2H), 8.02-7.52(m, 5H), 7.38-7.28 (m, 1H), 6.59-6.55 (m, 1H), 4.25-4.16 (m, 1H), 3.96 (s, 3H),3.82-3.58 (m,10H), 3.28 (s, 2H), 2.76-2.68 (m, 2H), 2.56-2.50 (m, 2H),2.46-2.38 (m, 2H), 2.05-1.95(m, 4H), 1.82-1.58(m, 2H), 1.26-1.12(m, 12H). |
| 95 | 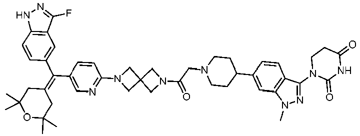 | 829.4 | 1H NMR (400 MHz, DMSO-d6) δ (ppm):12.48 (s, 1H), 10.69 (s, 1H), 8.35-8.15(m, 2H), 8.05-7.52(m, 5H), 7.38-7.27 (m, 1H), 6.60-6.54 (m, 1H), 3.97 (s, 3H),3.86-3.58 (m,10H), 3.28 (s, 2H), 2.75-2.68 (m, 3H), 2.55-2.50 (m, 2H),2.44-2.36 (m, 2H), 2.05-1.94(m, 4H), 1.79-1.58(m, 4H), 1.23-1.08(m, 12H). |
| 96 | 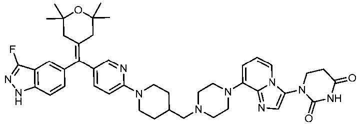 | 775 | 1H NMR (400 MHz, chloroform-d) δ 9.18 (s, 1H), 8.30 (d, J = 2.0 Hz, 1H), 8.08 - 8.03 (m, 1H), 8.03 - 7.99 (m, 1H), 7.62 (d, J = 7.6 Hz, 1H), 7.54 - 7.49 (m, 1H), 7.37 (s, 1H), 7.31 - 7.25 (m, 2H), 7.14 - 7.09 (m, 1H), 6.71 (d, J = 7.8 Hz, 1H), 4.02 - 3.96 (m, 1H), 3.95 - 3.90 (m, 1H), 3.66 - 3.52 (m, 4H), 3.35 - 3.23 (m, 4H), 2.74 - 2.68 (m, 1H), 2.68 - 2.60 (m, 6H), 2.54 - 2.47 (m, 1H), 2.31 (s, 2H), 2.22 (s, 2H), 1.92 - 1.83 (m, 5H), 1.28 - 1.24 (m, 12H). |
| 97 | 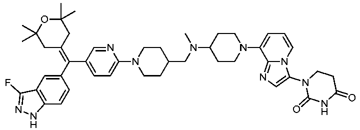 | 803.4 | 1H NMR (400 MHz, DMSO-d6) δ (ppm):12.45 (s, 1H), 10.76 (s, 1H), 8.38-8.17(m, 2H), 8.02-7.52(m, 5H), 7.38-7.28 (m, 1H), 6.59-6.55 (m, 1H), 4.25-4.16 (m, 1H), 3.96 (s, 3H),3.82-3.58 (m,10H), 3.28 (s, 2H), 2.76-2.68 (m, 2H), 2.56-2.50 (m, 2H),2.46-2.38 (m, 2H), 2.05-1.95(m, 4H), 1.82-1.58(m, 2H), 1.26-1.12(m, 12H). |
| 98 | 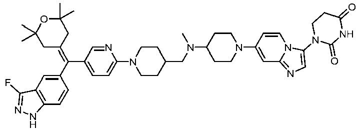 | 803.4 | 1H NMR (400 MHz, DMSO-d6) δ (ppm):12.48 (s, 1H), 10.69 (s, 1H), 8.35-8.15(m, 2H), 8.05-7.52(m, 5H), 7.38-7.27 (m, 1H), 6.60-6.54 (m, 1H), 3.97 (s, 3H),3.86-3.58 (m,10H), 3.28 (s, 2H), 2.75-2.68(m, 3H), 2.55-2.50 (m, 2H),2.44-2.36 (m, 2H), 2.05-1.94(m, 4H), 1.79-1.58(m, 4H), 1.23-1.08(m, 12H). |
| 99 | 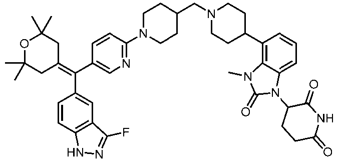 | 804 | 1H NMR (400 MHz, chloroform-d) δ 9.85 (s, 1H), 8.30 (d, J = 2.3 Hz, 1H), 8.08 - 8.03 (m, 1H), 7.62 (d, J = 7.6 Hz, 1H), 7.54 - 7.49 (m, 1H), 7.31 - 7.25 (m, 2H), 7.14 - 7.07 (m, 1H), 7.01 - 6.95 (m, 1H), 6.71 (d, J = 7.8 Hz, 1H), 4.80 - 4.74 (m, 1H), 3.66 - 3.52 (m, 4H), 3.46 (s, 3H), 2.93 - 2.85 (m, 1H), 2.66 - 2.48 (m, 8H), 2.31 (s, 2H), 2.22 (s, 2H), 2.11 - 2.02 (m, 1H), 2.02 - 1.81 (m, 9H), 1.73 - 1.63 (m, 1H), 1.28 - 1.24 (m, 12H). |
| 100 | 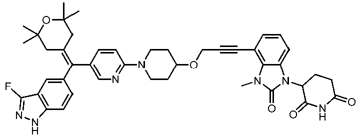 | 759.9 | ¹H NMR (400 MHz, chloroform-d) δ 9.85 (s, 1H), 8.30 (d, *J* = 2.0 Hz, 1H), 8.08 - 8.03 (m, 1H), 7.62 (d, *J* = 7.6 Hz, 1H), 7.54 - 7.49 (m, 1H), 7.42 - 7.37 (m, 1H), 7.31 - 7.23 (m, 3H), 6.71 (d, *J=* 7.8 Hz, 1H), 4.80 - 4.74 (m, 1H), 4.18 (s, 2H), 3.89 - 3.83 (m, 1H), 3.73 - 3.64 (m, 2H), 3.55 - 3.45 (m, 5H), 2.63 - 2.48 (m, 2H), 2.31 (s, 2H), 2.12 - 2.02 (m, 1H), 1.98 - 1.88 (m, 2H), 1.80 - 1.71 (m, 2H), 1.71 - 1.63 (m, 1H), 1.28 - 1.24 (m, 12H). |
| 101 | 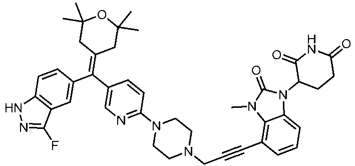 | 745.4 | 1H NMR (400 MHz, DMSO-d6) δ (ppm):12.45 (s, 1H), 10.76 (s, 1H), 8.38-8.17(m, 2H), 8.02-7.52(m, 5H), 7.38-7.28 (m, 1H), 6.59-6.55 (m, 1H), 4.25-4.16 (m, 1H), 3.96 (s, 3H),3.82-3.58 (m,10H), 3.28 (s, 2H), 2.76-2.68 (m, 2H), 2.56-2.50 (m, 2H),2.46-2.38 (m, 2H), 2.05-1.95(m, 4H), 1.82-1.58(m, 2H), 1.26-1.12(m, 12H). |
| 102 | 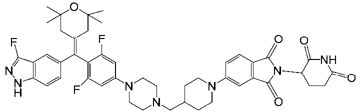 | 838 | 1H NMR (400 MHz, chloroform-*d*) δ 9.80 (s, 1H), 8.04 - 7.99 (m, 1H), 7.65 - 7.58 (m, 2H), 7.34 (d, *J* = 1.9 Hz, 1H), 7.31 - 7.26 (m, 1H), 7.11 - 7.05 (m, 1H), 6.40 - 6.34 (m, 2H), 5.41 - 5.36 (m, 1H), 3.51 - 3.42 (m, 2H), 3.29 - 3.24 (m, 2H), 3.24 - 3.20 (m, 2H), 3.11 - 3.02 (m, 2H), 2.66 - 2.47 (m, 8H), 2.32 (s, 2H), 2.25 (s, 2H), 2.22 - 2.13 (m, 1H), 1.89 - 1.81 (m, 1H), 1.81 - 1.68 (m, 5H), 1.28 - 1.24 (m, 12H). |
| 103 | 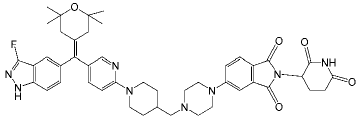 | 803 | 1H NMR (400 MHz, chloroform-d) δ 9.80 (s, 1H), 8.30 (d, *J* = 2.0 Hz, 1H), 8.08 - 8.03 (m, 1H), 7.65 - 7.59 (m, 2H), 7.54 - 7.49 (m, 1H), 7.34 (d, *J=* 1.9 Hz, 1H), 7.31 - 7.25 (m, 1H), 7.11 - 7.05 (m, 1H), 6.71 (d, *J* = 7.8 Hz, 1H), 5.41 - 5.36 (m, 1H), 3.66 - 3.52 (m, 4H), 3.29 - 3.22 (m, 4H), 2.66 - 2.47 (m, 8H), 2.431 (s, 2H), 2.22 - 2.13 (m, 3H), 1.93 - 1.82 (m, 5H), 1.82 - 1.73 (m, 1H), 1.28 - 1.24 (m, 12H). |
| 104 | 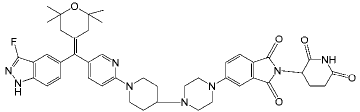 | 789 | ¹H NMR (400 MHz, chloroform-*d*) δ 9.80 (s, 1H), 8.30 (d, *J*= 2.0 Hz, 1H), 8.08 - 8.03 (m, 1H), 7.65 - 7.59 (m, 2H), 7.54 - 7.49 (m, 1H), 7.34 (d, *J=* 1.9 Hz, 1H), 7.31 - 7.25 (m, 1H), 7.11 - 7.05 (m, 1H), 6.71 (d, *J* = 7.8 Hz, 1H), 5.41 - 5.36 (m, 1H), 3.81 - 3.72 (m, 2H), 3.54 - 3.45 (m, 2H), 3.32 - 3.19 (m, 4H), 2.75 - 2.70 (m, 2H), 2.66 - 2.57 (m, 3H), 2.57 - 2.51 (m, 1H), 2.49 - 2.42 (m, 1H), 2.31 (s, 2H), 2.22 - 2.13 (m, 3H), 1.93 - 1.83 (m, 2H), 1.82 - 1.74 (m, 1H), 1.74 - 1.64 (m, 2H), 1.28 - 1.24 (m, 12H). |
| 105 | 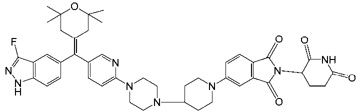 | 789 | 1H NMR (400 MHz, chloroform-*d*) δ 9.80 (s, 1H), 8.30 (d, *J*= 2.0 Hz, 1H), 8.08 - 8.03 (m, 1H), 7.65 - 7.59 (m, 2H), 7.54 - 7.48 (m, 1H), 7.34 (d, *J=* 1.9 Hz, 1H), 7.31 - 7.25 (m, 1H), 7.11 - 7.05 (m, 1H), 6.74 - 6.69 (m, 1H), 5.41 - 5.36 (m, 1H), 3.80 - 3.74 (m, 2H), 3.67 - 3.61 (m, 2H), 3.56 - 3.47 (m, 2H), 3.24 - 3.15 (m, 2H), 2.87 - 2.82 (m, 2H), 2.77 - 2.72 (m, 2H), 2.66 - 2.51 (m, 2H), 2.50 - 2.43 (m, 1H), 2.31 (s, 2H), 2.22 - 2.13 (m, 3H), 1.93 - 1.83 (m, 2H), 1.82 - 1.73 (m, 1H), 1.71 - 1.61 (m, 2H), 1.28 - 1.24 (m, 12H). |
| 106 | 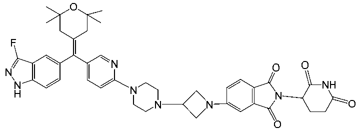 | 761 | ¹H NMR (400 MHz, chloroform-*d*) δ 9.80 (s, 1H), 8.30 (d, *J*= 2.0 Hz, 1H), 8.08 - 8.03 (m, 1H), 7.65 - 7.59 (m, 2H), 7.54 - 7.48 (m, 1H), 7.36 (d, *J=* 1.9 Hz, 1H), 7.31 - 7.25 (m, 1H), 7.13 - 7.07 (m, 1H), 6.71 (d, *J=* 7.9 Hz, 1H), 5.41 - 5.36 (m, 1H), 3.80 - 3.74 (m, 2H), 3.71 - 3.64 (m, 2H), 3.64 - 3.57 (m, 4H), 3.43 - 3.36 (m, 1H), 2.85 - 2.80 (m, 2H), 2.72 - 2.67 (m, 2H), 2.66 - 2.51 (m, 2H), 2.31 (s, 2H), 2.22 - 2.13 (m, 3H), 1.82 - 1.73 (m, 1H), 1.28 - 1.24 (m, 12H). |
| 107 | 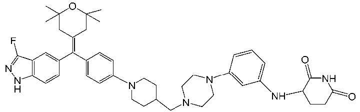 | 748 | ¹H NMR (400 MHz, chloroform-*d*) δ 9.53 (s, 1H), 8.06 - 8.01 (m, 1H), 7.61 (d, *J* = 7.7 Hz, 1H), 7.39 - 7.33 (m, 2H), 7.28 - 7.22 (m, 1H), 7.12 - 7.05 (m, 1H), 6.77 - 6.71 (m, 2H), 6.40 - 6.33 (m, 2H), 6.17 - 6.13 (m, 1H), 5.78 (d, *J* = 7.5 Hz, 1H), 4.39 - 4.32 (m, 1H), 3.33 - 3.27 (m, 2H), 3.27 - 3.22 (m, 7H), 2.75 - 2.69 (m, 2H), 2.65 - 2.58 (m, 5H), 2.58 - 2.54 (m, 3H), 2.54 - 2.49 (m, 2H), 2.43 - 2.20 (m, 8H), 2.06 - 1.96 (m, 1H), 1.76 - 1.66 (m, 2H), 1.66 - 1.62 (m, 1H), 1.62 - 1.48 (m, 6H), 1.31 - 1.20 (m, 16H). |
| 108 | 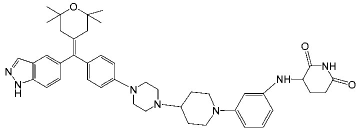 | 716 | H NMR (400 MHz, chloroform-*d*) δ 9.53 (s, 1H), 8.25 (d, *J*= 1.7 Hz, 1H), 7.57 (d, *J* = 7.7 Hz, 1H), 7.39 - 7.33 (m, 3H), 7.30 - 7.24 (m, 1H), 7.12 - 7.05 (m, 1H), 6.77 - 6.71 (m, 2H), 6.40 - 6.34 (m, 2H), 6.18 - 6.13 (m, 1H), 5.78 (d, *J* = 7.5 Hz, 1H), 4.39 - 4.32 (m, 1H), 3.57 - 3.48 (m, 2H), 3.33 - 3.25 (m, 3H), 3.25 - 3.14 (m, 3H), 2.75 - 2.70 (m, 2H), 2.66 - 2.61 (m, 2H), 2.58 - 2.50 (m, 2H), 2.50 - 2.43 (m, 1H), 2.32 (s, 2H), 2.06 - 1.96 (m, 1H), 1.93 - 1.83 (m, 2H), 1.71 - 1.61 (m, 2H), 1.61 - 1.51 (m, 1H), 1.28 - 1.24 (m, 12H). |
| 109 | 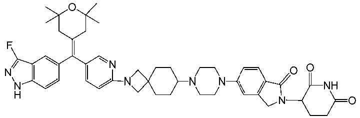 | 815 | ¹H NMR (400 MHz, chloroform-*d*) δ 9.58 (s, 1H), 8.32 - 8.28 (m, 1H), 8.08 - 8.03 (m, 1H), 7.65 - 7.57 (m, 2H), 7.54 - 7.48 (m, 1H), 7.31 - 7.25 (m, 1H), 6.92 - 6.86 (m, 1H), 6.79 - 6.75 (m, 1H), 6.71 - 6.65 (m, 1H), 4.59 - 4.53 (m, 1H), 4.53 - 4.51 (m, 1H), 4.46 - 4.42 (m, 1H), 3.84 (s, 2H), 3.72 (s, 2H), 3.33 - 3.20 (m, 4H), 2.75 - 2.69 (m, 2H), 2.65 - 2.61 (m, 2H), 2.61 - 2.54 (m, 1H), 2.54 - 2.46 (m, 1H), 2.46 - 2.40 (m, 1H), 2.31 (s, 2H), 2.11 - 2.01 (m, 1H), 1.77 - 1.71 (m, 2H), 1.71 - 1.63 (m, 2H), 1.61 - 1.51 (m, 4H), 1.45 - 1.34 (m, 2H), 1.28 - 1.24 (m, 12H). |
| 110 | 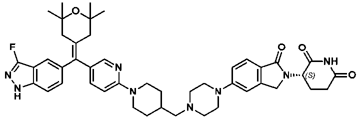 | 789 | 1H NMR (400 MHz, DMSO-d6) δ (ppm): 12.58 (s, 1H), 10.94 (s, 1H), 7.97-7,96 (d, J = 2.0 Hz, 1H),7.53-7.40 (m, 3H), 7.29-7 .26 (m, 1H), 7.20-7.18 (m, 1H), 7.06-7.04 (m, 2H), 6.74-6.72 ( m, 1H), 5.07-5.02 (m, 1H), 4.34-4.18 (m, 4H), 3.32-3.28 (m, 4 H), 2.90-2.77 (m, 1H), 2.75-2,72 (m, 2H), 2.60-2.59 (m, 1H), 2. 56-2.50 (m, 3H), 2.49-2.48 (m, 1H), 2.35-2.24 (m, 2H), 2.20-2. 18 (m, 2H), 2.14-1.95 (m, 3H), 1.79-1.76 (m, 3H), 1.17-1.03 ( m, 15H),LCMS (ES, m/z): 789 [M+H]+. |
| 112 | 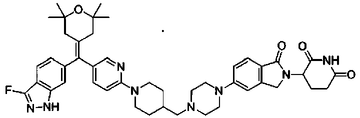 | 789 | ¹H NMR (400 MHz, chloroform-*d*) δ 9.58 (s, 1H), 8.33 - 8.29 (m, 1H), 8.15 - 8.08 (m, 1H), 7.76 - 7.72 (m, 1H), 7.62 - 7.57 (m, 1H), 7.56 - 7.50 (m, 1H), 7.32 - 7.26 (m, 1H), 6.92 - 6.86 (m, 1H), 6.79 - 6.75 (m, 1H), 6.73 - 6.68 (m, 1H), 4.59 - 4.53 (m, 1H), 4.53 - 4.51 (m, 1H), 4.46 - 4.42 (m, 1H), 3.66 - 3.52 (m, 4H), 3.28 - 3.21 (m, 4H), 2.66 - 2.46 (m, 8H), 2.29 (s, 2H), 2.22 (s, 2H), 2.11 - 2.01 (m, 1H), 1.92 - 1.82 (m, 5H), 1.73 - 1.63 (m, 1H), 1.28 - 1.24 (m, 12H). |
| 113 | 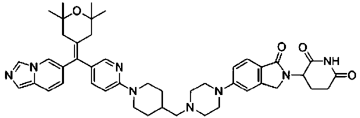 | 771 | ¹H NMR (400 MHz, chloroform-*d*) δ 9.58 (s, 1H), 8.38 - 8.34 (m, 1H), 8.31 - 8.27 (m, 1H), 7.80 - 7.75 (m, 1H), 7.62 - 7.57 (m, 1H), 7.55 - 7.49 (m, 1H), 6.92 - 6.86 (m, 1H), 6.79 - 6.75 (m, 1H), 6.75 - 6.67 (m, 2H), 6.36 - 6.29 (m, 1H), 4.77 - 4.70 (m, 1H), 4.59 - 4.53 (m, 1H), 4.53 - 4.51 (m, 1H), 4.46 - 4.42 (m, 1H), 3.98 - 3.87 (m, 2H), 3.66 - 3.52 (m, 4H), 3.28 - 3.21 (m, 4H), 2.66 - 2.49 (m, 8H), 2.49 - 2.46 (m, 1H), 2.28 - 2.24 (m, 4H), 2.11 - 2.01 (m, 1H), 1.92 - 1.82 (m, 5H), 1.73 - 1.63 (m, 1H), 1.29 (s, 6H), 1.24 (s, 6H). |
| 114 | 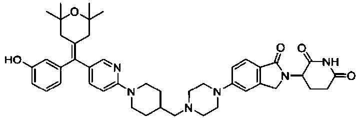 | 747 | ¹H NMR (400 MHz, chloroform-*d*) δ 9.58 (s, 1H), 8.75 (s, 1H), 8.28 - 8.24 (m, 1H), 7.62 - 7.57 (m, 1H), 7.51 - 7.46 (m, 1H), 7.27 - 7.20 (m, 1H), 7.20 - 7.16 (m, 1H), 6.92 - 6.85 (m, 2H), 6.79 - 6.72 (m, 2H), 6.72 - 6.68 (m, 1H), 4.59 - 4.54 (m, 1H), 4.54 - 4.51 (m, 1H), 4.46 - 4.42 (m, 1H), 3.66 - 3.52 (m, 4H), 3.28 - 3.21 (m, 4H), 2.66 - 2.46 (m, 8H), 2.29 (s, 2H), 2.22 (s, 2H), 2.11 - 2.01 (m, 1H), 1.92 - 1.82 (m, 5H), 1.73 - 1.63 (m, 1H), 1.28 - 1.24 (m, 12H). |
| 115 | 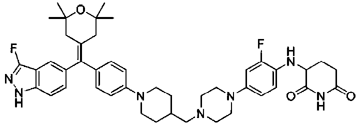 | 766 | ¹H NMR (400 MHz, chloroform-*d*) δ 9.53 (s, 1H), 8.06 - 8.01 (m, 1H), 7.63 - 7.58 (m, 1H), 7.39 - 7.33 (m, 2H), 7.28 - 7.22 (m, 1H), 6.95 - 6.89 (m, 1H), 6.77 - 6.71 (m, 2H), 6.65 - 6.59 (m, 1H), 6.45 - 6.40 (m, 1H), 5.23 - 5.18 (m, 1H), 4.40 - 4.33 (m, 1H), 3.50 - 3.41 (m, 2H), 3.28 - 3.21 (m, 4H), 3.15 - 3.06 (m, 2H), 2.66 - 2.59 (m, 3H), 2.58 - 2.54 (m, 3H), 2.54 - 2.50 (m, 2H), 2.32 (s, 2H), 2.04 - 1.94 (m, 1H), 1.90 - 1.81 (m, 1H), 1.81 - 1.68 (m, 4H), 1.61 - 1.51 (m, 1H), 1.28 - 1.24 (m, 12H). |
| | | | |
| 116 | 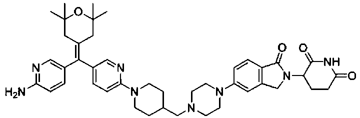 | 747 | ¹H NMR (400 MHz, DMSO-d6) δ 9.58 (s, 1H), 8.33 - 8.29 (m, 2H), 7.62 - 7.57 (m, 1H), 7.57 - 7.50 (m, 2H), 6.92 - 6.86 (m, 1H), 6.86 - 6.80 (m, 1H), 6.79 - 6.75 (m, 1H), 6.74 - 6.68 (m, 1H), 5.26 (s, 2H), 4.59 - 4.53 (m, 1H), 4.53 - 4.51 (m, 1H), 4.46 - 4.42 (m, 1H), 3.66 - 3.52 (m, 4H), 3.28 - 3.21 (m, 4H), 2.66 - 2.46 (m, 8H), 2.29 (s, 2H), 2.22 (s, 2H), 2.11 - 2.01 (m, 1H), 1.92 - 1.82 (m, 5H), 1.73 - 1.63 (m, 1H), 1.28 - 1.24 (m, 12H). |
| 117 | 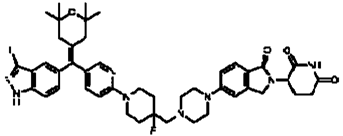 | 807 | ¹H NMR (400 MHz, DMSO-d6) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.59 - 4.53 (m, 1H), 4.53 - 4.51 (m, 1H), 4.46 - 4.42 (m, 1H), 3.64 - 3.48 (m, 4H), 3.29 - 3.23 (m, 4H), 2.77 - 2.64 (m, 6H), 2.63 - 2.54 (m, 1H), 2.54 - 2.46 (m, 1H), 2.31 (s, 2H), 2.26 - 2.12 (m, 4H), 2.11 - 1.93 (m, 3H), 1.73 - 1.63 (m, 1H), 1.28 - 1.24 (m, 12H). |
| 118 | 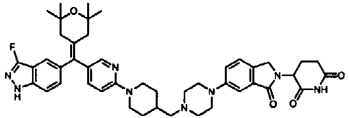 | 789 | ¹H NMR (400 MHz, DMSO-d6) δ 9.58 (s, 1H), 8.32 - 8.28 (m, 1H), 8.08 - 8.03 (m, 1H), 7.65 - 7.59 (m, 1H), 7.54 - 7.49 (m, 1H), 7.37 (s, 1H), 7.31 - 7.25 (m, 1H), 7.25 - 7.20 (m, 1H), 6.96 - 6.90 (m, 1H), 6.73 - 6.68 (m, 1H), 4.59 - 4.53 (m, 1H), 4.43 - 4.39 (m, 1H), 4.38 - 4.34 (m, 1H), 3.66 - 3.52 (m, 4H), 3.29 - 3.22 (m, 4H), 2.66 - 2.46 (m, 8H), 2.31 (s, 2H), 2.22 (s, 2H), 2.11 - 2.01 (m, 1H), 1.92 - 1.82 (m, 5H), 1.73 - 1.63 (m, 1H), 1.28 - 1.24 (m, 12H). |
| 119 | 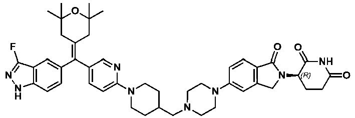 | 789 | ¹H NMR (400 MHz, chloroform-*d*) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.63 - 4.57 (m, 1H), 4.54 - 4.51 (m, 1H), 4.46 - 4.42 (m, 1H), 3.66 - 3.52 (m, 4H), 3.28 - 3.21 (m, 4H), 2.66 - 2.53 (m, 6H), 2.53 - 2.42 (m, 2H), 2.31 (s, 2H), 2.22 - 2.12 (m, 3H), 2.11 - 2.02 (m, 1H), 1.93 - 1.81 (m, 5H), 1.28 - 1.24 (m, 12H). |
| 120 | 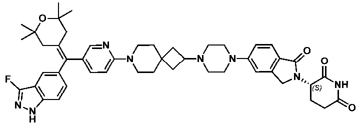 | 815 | ¹H NMR (400 MHz, chloroform-*d*) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.63 - 4.57 (m, 1H), 4.54 - 4.51 (m, 1H), 4.46 - 4.42 (m, 1H), 3.74 - 3.68 (m, 2H), 3.52 - 3.47 (m, 2H), 3.33 - 3.20 (m, 4H), 2.81 - 2.75 (m, 2H), 2.74 - 2.66 (m, 1H), 2.66 - 2.60 (m, 2H), 2.58 - 2.46 (m, 2H), 2.31 (s, 2H), 2.17 - 2.07 (m, 1H), 2.01 - 1.91 (m, 1H), 1.82 - 1.70 (m, 4H), 1.62 - 1.52 (m, 4H), 1.28 - 1.24 (m, 12H). |
| 121 | 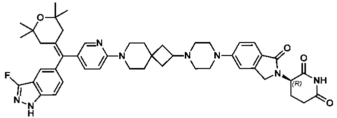 | 815 | ¹H NMR (400 MHz, chloroform-*d*) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.63 - 4.57 (m, 1H), 4.54 - 4.51 (m, 1H), 4.46 - 4.42 (m, 1H), 3.74 - 3.68 (m, 2H), 3.52 - 3.47 (m, 2H), 3.33 - 3.20 (m, 4H), 2.81 - 2.75 (m, 2H), 2.74 - 2.66 (m, 1H), 2.66 - 2.60 (m, 2H), 2.58 - 2.46 (m, 2H), 2.31 (s, 2H), 2.17 - 2.07 (m, 1H), 2.01 - 1.91 (m, 1H), 1.82 - 1.70 (m, 4H), 1.62 - 1.52 (m, 4H), 1.28 - 1.24 (m, 12H). |
| 122 | 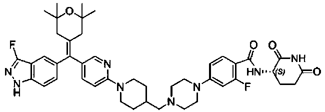 | 795 | ¹H NMR (400 MHz, chloroform-*d*) δ 9.60 (s, 1H), 8.32 - 8.28 (m, 1H), 8.08 - 8.03 (m, 1H), 7.85 - 7.80 (m, 1H), 7.68 - 7.59 (m, 2H), 7.54 - 7.49 (m, 1H), 7.31 - 7.25 (m, 1H), 6.75 - 6.68 (m, 3H), 4.33 - 4.26 (m, 1H), 3.66 - 3.52 (m, 4H), 3.28 - 3.21 (m, 4H), 2.66 - 2.46 (m, 8H), 2.31 (s, 2H), 2.22 (s, 2H), 1.98 - 1.82 (m, 7H), 1.28 - 1.24 (m, 12H). |
| 123 | 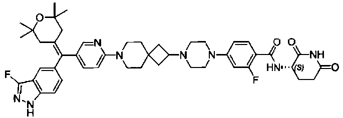 | 821 | ¹H NMR (400 MHz, chloroform-*d*) δ 9.60 (s, 1H), 8.32 - 8.28 (m, 1H), 8.08 - 8.03 (m, 1H), 7.85 - 7.80 (m, 1H), 7.68 - 7.59 (m, 2H), 7.54 - 7.49 (m, 1H), 7.31 - 7.25 (m, 1H), 6.75 - 6.68 (m, 3H), 4.36 - 4.28 (m, 1H), 3.74 - 3.68 (m, 2H), 3.52 - 3.47 (m, 2H), 3.32 - 3.21 (m, 4H), 2.81 - 2.75 (m, 2H), 2.74 - 2.66 (m, 1H), 2.66 - 2.60 (m, 2H), 2.58 - 2.46 (m, 2H), 2.31 (s, 2H), 1.98 - 1.87 (m, 2H), 1.82 - 1.70 (m, 4H), 1.62 - 1.52 (m, 4H), 1.28 - 1.24 (m, 12H). |
| 124 | 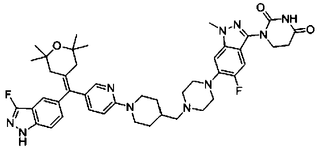 | 807 | ¹H NMR (400 MHz, chloroform-*d*) δ 9.14 (s, 1H), 8.32 - 8.28 (m, 1H), 8.08 - 8.03 (m, 1H), 7.65 - 7.59 (m, 1H), 7.54 - 7.48 (m, 2H), 7.31 - 7.25 (m, 1H), 7.07 - 7.03 (m, 1H), 6.73 - 6.68 (m, 1H), 4.07 - 4.01 (m, 1H), 4.01 - 3.95 (m, 4H), 3.66 - 3.52 (m, 4H), 3.25 - 3.19 (m, 4H), 2.79 - 2.69 (m, 2H), 2.69 - 2.60 (m, 5H), 2.54 - 2.47 (m, 1H), 2.31 (s, 2H), 1.92 - 1.81 (m, 5H), 1.26 (s, 12H). |
| 125 | 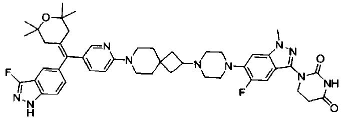 | 833 | 1H NMR (500 MHz, chloroform-*d*) δ 9.14 (s, 1H), 8.32 - 8.28 (m, 1H), 8.08 - 8.03 (m, 1H), 7.65 - 7.59 (m, 1H), 7.54 - 7.48 (m, 2H), 7.31 - 7.25 (m, 1H), 7.07 - 7.03 (m, 1H), 6.73 - 6.68 (m, 1H), 4.07 - 4.01 (m, 1H), 4.01 - 3.95 (m, 4H), 3.74 - 3.68 (m, 2H), 3.52 - 3.47 (m, 2H), 3.33 - 3.22 (m, 4H), 2.84 - 2.79 (m, 2H), 2.79 - 2.73 (m, 1H), 2.73 - 2.67 (m, 3H), 2.67 - 2.64 (m, 1H), 2.31 (s, 2H), 1.82 - 1.70 (m, 4H), 1.62 - 1.52 (m, 4H), 1.26 (s, 12H). |
| 126 | 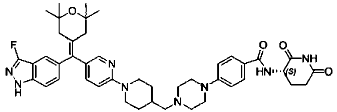 | 777 | 1H NMR (400 MHz, chloroform-*d*) δ 9.60 (s, 1H), 8.32 - 8.28 (m, 1H), 8.08 - 8.02 (m, 2H), 7.70 - 7.65 (m, 2H), 7.65 - 7.59 (m, 1H), 7.54 - 7.49 (m, 1H), 7.31 - 7.25 (m, 1H), 7.04 - 6.99 (m, 2H), 6.73 - 6.68 (m, 1H), 4.33 - 4.26 (m, 1H), 3.66 - 3.52 (m, 4H), 3.25 - 3.18 (m, 4H), 2.66 - 2.46 (m, 8H), 2.31 (s, 2H), 2.22 (s, 2H), 1.99 - 1.91 (m, 1H), 1.91 - 1.85 (m, 5H), 1.28 - 1.24 (m, 12H). |
| 127 | 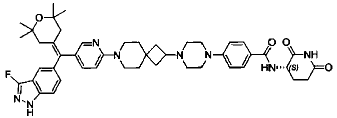 | 803 | 1H NMR (400 MHz, chloroform-*d*) δ 9.60 (s, 1H), 8.32 - 8.28 (m, 1H), 8.08 - 8.02 (m, 2H), 7.70 - 7.65 (m, 2H), 7.65 - 7.59 (m, 1H), 7.54 - 7.49 (m, 1H), 7.31 - 7.25 (m, 1H), 7.04 - 6.99 (m, 2H), 6.73 - 6.68 (m, 1H), 4.36 - 4.29 (m, 1H), 3.74 - 3.68 (m, 2H), 3.52 - 3.47 (m, 2H), 3.33 - 3.20 (m, 4H), 2.81 - 2.75 (m, 2H), 2.74 - 2.66 (m, 1H), 2.66 - 2.60 (m, 2H), 2.58 - 2.46 (m, 2H), 2.31 (s, 2H), 1.99 - 1.84 (m, 2H), 1.82 - 1.70 (m, 4H), 1.62 - 1.52 (m, 4H), 1.28 - 1.24 (m, 12H). |
| 128 | 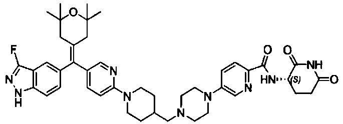 | 778 | ¹H NMR (400 MHz, chloroform-*d*) δ 9.60 (s, 1H), 8.32 - 8.28 (m, 1H), 8.27 - 8.21 (m, 1H), 8.20 - 8.16 (m, 1H), 8.08 - 8.03 (m, 1H), 7.84 - 7.79 (m, 1H), 7.65 - 7.59 (m, 1H), 7.54 - 7.49 (m, 1H), 7.31 - 7.25 (m, 1H), 7.20 - 7.15 (m, 1H), 6.73 - 6.68 (m, 1H), 4.37 - 4.29 (m, 1H), 3.66 - 3.52 (m, 4H), 3.30 - 3.20 (m, 4H), 2.66 - 2.59 (m, 3H), 2.58 - 2.46 (m, 5H), 2.31 (s, 2H), 2.22 (s, 2H), 1.99 - 1.90 (m, 1H), 1.90 - 1.81 (m, 6H), 1.26 (s, 12H). |
| 129 | 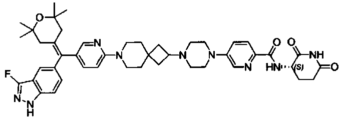 | 804 | ¹H NMR (400 MHz, chloroform-*d*) δ 9.60 (s, 1H), 8.32 - 8.28 (m, 1H), 8.27 - 8.21 (m, 1H), 8.20 - 8.16 (m, 1H), 8.08 - 8.03 (m, 1H), 7.84 - 7.79 (m, 1H), 7.65 - 7.59 (m, 1H), 7.54 - 7.49 (m, 1H), 7.31 - 7.25 (m, 1H), 7.20 - 7.15 (m, 1H), 6.73 - 6.68 (m, 1H), 4.37 - 4.30 (m, 1H), 3.74 - 3.68 (m, 2H), 3.52 - 3.47 (m, 2H), 3.31 - 3.20 (m, 4H), 2.81 - 2.75 (m, 2H), 2.74 - 2.66 (m, 1H), 2.65 - 2.60 (m, 2H), 2.58 - 2.46 (m, 2H), 2.31 (s, 2H), 1.99 - 1.84 (m, 2H), 1.82 - 1.70 (m, 4H), 1.62 - 1.52 (m, 4H), 1.28 - 1.24 (m, 12H). |
| 130 | 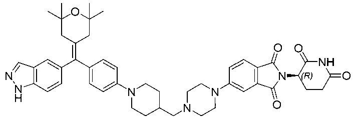 | 785 | 1H NMR (400 MHz, DMSO-d6) δ (ppm):13.05 (s, 1H), 10.97 (s, 1H), 9.41 (s, 1H), 8.21 (s, 1H),8.04 (d, J = 0.8 Hz, 1H), 7.96-7.95 (d, J = 2.0 Hz, 1H), 7.61-7.57 (m, 2H),7.50-7.48 (d, J = 8.8 Hz, 1H), 7.41 (d, J = 8.8 Hz, 1H), 7.19-7.13 (m, 3H), 6.98-6.89 (m, 1H), 5.10-5.05 (m, 1H),4.37-4.22 (m, 2H), 4.02-3.99 (m, 4H), 3.64 (s, 2H), 3.21-3.11 (m, 5H), 2.90-2.87 (m, 3H), 2.70-2.60 (m, 1H), 2.40-2.35 (m, 1H), 2.25-2.17 (m, 4H), 1.98-1.95 (m, 1H), 1.85-1.78(m, 2H), 1.17-1.12 (m, 13H). |
| 131 | 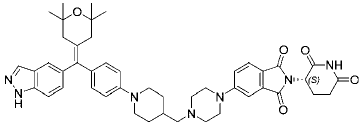 | 785 | 1H NMR (400 MHz, DMSO-d6) δ (ppm):13.05 (s, 1H), 10.97 (s, 1H), 9.41 (s, 1H), 8.21 (s, 1H),8.04 (d, J = 0.8 Hz, 1H), 7.96-7.95 (d, J = 2.0 Hz, 1H), 7.61-7.57 (m, 2H),7.50-7.48 (d, J = 8.8 Hz, 1H), 7.41 (d, J = 8.8 Hz, 1H), 7.19-7.13 (m, 3H), 6.98-6.89 (m, 1H), 5.10-5.05 (m, 1H),4.37-4.22 (m, 2H), 4.02-3.99 (m, 4H), 3.64 (s, 2H), 3.21-3.11 (m, 5H), 2.90-2.87 (m, 3H), 2.70-2.60 (m, 1H), 2.40-2.35 (m, 1H), 2.25-2.17 (m, 4H), 1.98-1.95 (m, 1H), 1.85-1.78(m, 2H), 1.17-1.12 (m, 13H). |
| 132 | 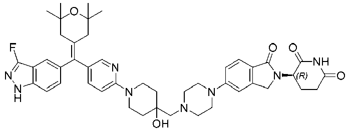 | 774.9 | ¹H NMR (400 MHz, chloroform-*d*) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.59 - 4.53 (m, 1H), 4.53 - 4.51 (m, 1H), 4.46 - 4.42 (m, 1H), 3.62 - 3.53 (m, 3H), 3.51 - 3.43 (m, 2H), 3.29 - 3.23 (m, 4H), 2.76 (s, 2H), 2.70 - 2.65 (m, 2H), 2.65 - 2.60 (m, 2H), 2.60 - 2.54 (m, 1H), 2.54 - 2.46 (m, 1H), 2.31 (s, 2H), 2.11 - 2.01 (m, 1H), 1.94 - 1.81 (m, 4H), 1.73 - 1.63 (m, 1H), 1.28 - 1.24 (m, 12H). |
| 133 | 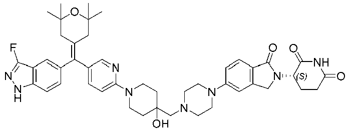 | 774.9 | ¹H NMR (400 MHz, chloroform-*d*) δ 9.58 (s, 1H), 8.32 - 8.28 (m, 1H), 8.08 - 8.03 (m, 1H), 7.65 - 7.57 (m, 2H), 7.54 - 7.49 (m, 1H), 7.31 - 7.25 (m, 1H), 6.92 - 6.86 (m, 1H), 6.79 - 6.75 (m, 1H), 6.73 - 6.68 (m, 1H), 4.59 - 4.53 (m, 1H), 4.53 - 4.51 (m, 1H), 4.46 - 4.42 (m, 1H), 3.62 - 3.53 (m, 3H), 3.51 - 3.43 (m, 2H), 3.29 - 3.23 (m, 4H), 2.76 (s, 2H), 2.70 - 2.65 (m, 2H), 2.65 - 2.60 (m, 2H), 2.60 - 2.54 (m, 1H), 2.54 - 2.46 (m, 1H), 2.31 (s, 2H), 2.11 - 2.01 (m, 1H), 1.94 - 1.81 (m, 4H), 1.73 - 1.63 (m, 1H), 1.28 - 1.24 (m, 12H). |
| 134 | 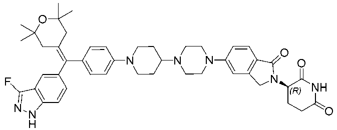 | 774.29 | 1H NMR (400 MHz, DMSO-d6) δ (ppm):12.50 (s, 1H), 10.98 (s, 1H), 7.60 (d,1H), 7.46-7.41 (m, 2H), 7.23-7.12 (m, 3H), 7.10 (d, 2H),6.95-6.88 (d, 2H), 5.08-5.00 (m, 1H), 4.41-4.33 (d, 1H), 4.28-4.19 (d,1H), 4.12-4.01 (d, 2H), 3.91-3.83(d, 2H),3.72-3.63(d, 2H), 3.30-3.13 (m, 4H),3.12-3.05 (m, 2H), 2.93-2.86 (m,1H), 2.73-2.66 (m, 1H), 2.59-2.51(m, 2H), 2.33-2.32(m, 2H), 2.13-2.02(m, 2H), 2.01-1.98(m.2H), 1.85-1.74(m,2H), 1.23(s, 1H), 1.16-1.11(d,12H). |
| 135 | 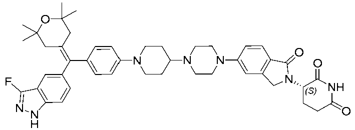 | 774.29 | 1H NMR (400 MHz, DMSO-d6) δ (ppm):12.50 (s, 1H), 10.98 (s, 1H), 7.60 (d,1H), 7.46-7.41 (m, 2H), 7.23-7.12 (m, 3H), 7.10 (d, 2H),6.95-6.88 (d, 2H), 5.08-5.00 (m, 1H), 4.41-4.33 (d, 1H), 4.28-4.19 (d,1H), 4.12-4.01 (d, 2H), 3.91-3.83(d, 2H),3.72-3.63(d, 2H), 3.30-3.13 (m, 4H),3.12-3.05 (m, 2H), 2.93-2.86 (m,1H), 2.73-2.66 (m, 1H), 2.59-2.51(m, 2H), 2.33-2.32(m, 2H), 2.13-2.02(m, 2H), 2.01-1.98(m.2H), 1.85-1.74(m,2H), 1.23(s, 1H), 1.16-1.11(d,12H). |
| 136 | 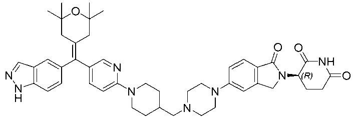 | 771.06 | 1H NMR (400 MHz, DMSO-d6) δ (ppm):13.05 (s, 1H), 10.97 (s, 1H), 8.04 (d, J = 0.8 Hz, 1H), 7.96-7.95 (d, J = 2.0 Hz, 1H), 7.61-7.57 (m, 2H),7.50-7.48 (d, J = 8.8 Hz, 1H), 7.41 (d, J = 8.8 Hz, 1H), 7.19-7.13 (m, 3H), 6.98-6.89 (m, 1H), 5.10-5.05 (m, lH),4.37-4.22 (m, 4H), 4.02-3.99 (m, 4H), 3.64 (s, 2H), 3.21-3.11 (m, 5H), 2.90-2.87 (m, 3H), 2.70-2.60 (m, 1H), 2.40-2.35 (m, 1H), 2.25-2.17 (m, 4H), 1.98-1.95 (m, 1H), 1.85-1.78(m, 2H), 1.17-1.12 (m, 13H). |
| 137 | 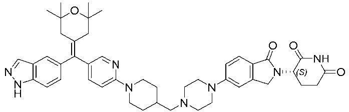 | 771.06 | 1H NMR (400 MHz, DMSO-d6) δ (ppm):13.05 (s, 1H), 10.97 (s, 1H), 8.04 (d, J = 0.8 Hz, 1H), 7.96-7.95 (d, J = 2.0 Hz, 1H), 7.61-7.57 (m, 2H),7.50-7.48 (d, J = 8.8 Hz, 1H), 7.41 (d, J = 8.8 Hz, 1H), 7.19-7.13 (m, 3H), 6.98-6.89 (m, 1H), 5.10-5.05 (m, lH),4.37-4.22 (m, 4H), 4.02-3.99 (m, 4H), 3.64 (s, 2H), 3.21-3.11 (m, 5H), 2.90-2.87 (m, 3H), 2.70-2.60 (m, 1H), 2.40-2.35 (m, 1H), 2.25-2.17 (m, 4H), 1.98-1.95 (m, 1H), 1.85-1.78(m, 2H), 1.17-1.12 (m, 13H). |
| 138 | 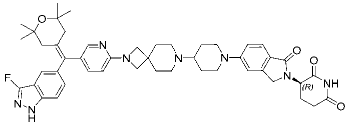 | 815.4 | 1H NMR (400 MHz, DMSO-d6) δ (ppm):12.58 (s, 1H), 10.99 (s, 1H), 7.95(s,1H),7.63-7.58(d, J = 8.4 Hz, 1H), 7.58-7.45(m, 2H), 7.29-7.22 (m, 1H), 7.20-7.12 (m, 2H), 6.99-6.96 (d, J = 13.2 Hz, 1H), 5.15-5.01 (m,1H), 4.65(s, 1H),4.41-4.18 (m, 2H), 3.98-3.81 (m, 3H), 3.71-3.63(m, 2H), 3.46-3.15 (m, 3H), 2.98-2.88 (m, 1H), 2.70-2.54 (m, 1H), 2.44-2.38 (m, 2H), 2.27(s, 3H), 2.22-2.16(m, 2H), 2.16(s, 1H), 2.01-1.94(m, 2H), 1.92-1.75(m, 4H), 1.74-1.54(m, 2H), 1.22-1.08(m, 12H). |
| 139 | 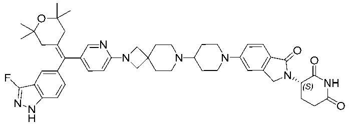 | 815.4 | 1H NMR (400 MHz, DMSO-d6) δ (ppm):12.58 (s, 1H), 10.99 (s, 1H), 7.95(s,1H),7.63-7.58(d, J = 8.4 Hz, 1H), 7.58-7.45(m, 2H), 7.29-7.22 (m, 1H), 7.20-7.12 (m, 2H), 6.99-6.96 (d, J = 13.2 Hz, 1H), 5.15-5.01 (m,1H), 4.65(s, 1H),4.41-4.18 (m, 2H), 3.98-3.81 (m, 3H), 3.71-3.63(m, 2H), 3.46-3.15 (m, 3H), 2.98-2.88 (m, 1H), 2.70-2.54 (m, 1H), 2.44-2.38 (m, 2H), 2.27(s, 3H), 2.22-2.16(m, 2H), 2.16(s, 1H), 2.01-1.94(m, 2H), 1.92-1.75(m, 4H), 1.74-1.54(m, 2H), 1.22-1.08(m, 12H). |
| 140 | 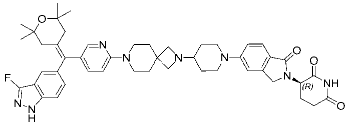 | 815.4 | 1H NMR (400 MHz, DMSO-d6) δ (ppm):12.56 (s, 1H), 10.95 (s, 1H), 7.98 (s,lH),7.64-7.58(d, J = 8.4 Hz, 1H), 7.56-7.42(m, 2H), 7.28-7.22 (m, 1H), 7.20-7.12 (m, 2H), 7.00-6.96 (d, J = 13.2 Hz, 1H), 5.13-5.01 (m,1H), 4.65(s, 1H),4.41-4.18 (m, 2H), 3.98-3.81 (m, 3H), 3.71-3.63(m, 2H), 3.45-3.19 (m, 3H), 2.95-2.88 (m, 1H), 2.69-2.53 (m, 1H), 2.46-2.34 (m, 4H), 2.22-2.10(m, 2H), 2.06-1.95(m, 2H), 1.92-1.76(m, 4H), 1.75-1.56(m, 2H), 1.28-1.10(m, 12H). |
| 141 | 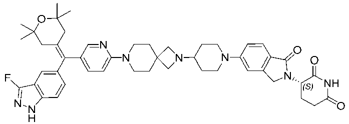 | 815.4 | 1H NMR (400 MHz, DMSO-d6) δ (ppm):12.56 (s, 1H), 10.95 (s, 1H), 7.98 (s,lH),7.64-7.58(d, J = 8.4 Hz, 1H), 7.56-7.42(m, 2H), 7.28-7.22 (m, 1H), 7.20-7.12 (m, 2H), 7.00-6.96 (d, J = 13.2 Hz, 1H), 5.13-5.01 (m,1H), 4.65(s, 1H),4.41-4.18 (m, 2H), 3.98-3.81 (m, 3H), 3.71-3.63(m, 2H), 3.45-3.19 (m, 3H), 2.95-2.88 (m, 1H), 2.69-2.53 (m, 1H), 2.46-2.34 (m, 4H), 2.22-2.10(m, 2H), 2.06-1.95(m, 2H), 1.92-1.76(m, 4H), 1.75-1.56(m, 2H), 1.28-1.10(m, 12H). |
| 142 | 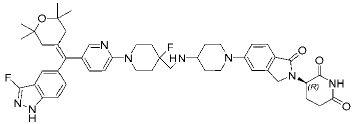 | 821.4 | 1H NMR (400 MHz, DMSO-d6) δ (ppm):12.55 (s, 1H), 10.98 (s, 1H),7.97 (s,lH),7.63-7.58(d, J = 8.4 Hz, 1H), 7.54-7.43(m, 2H), 7.26-7.22 (m, 1H), 7.18-7.12 (m, 2H), 6.99-6.96 (d, J = 13.2 Hz, 1H), 5.46(s, 1H), 5.11-5.01 (m,lH), 4.44-4.18 (m, 2H), 3.98-3.84 (m, 4H), 3.74-3.63(s, 2H), 3.45-3.17 (m, 7H), 2.97-2.88 (m, 1H), 2.69-2.53 (m, 1H), 2.44-2.36 (m, 1H), 2.22(s, 2H), 2.15(s, 1H), 1.81-1.73(m, 2H), 1.73-1.58(m, 2H), 1.19-1.01( m , 12 H ) . |
| 143 | 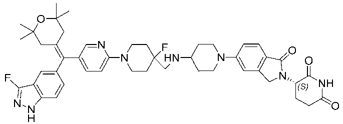 | 821.4 | 1H NMR (400 MHz, DMSO-d6) δ (ppm):12.55 (s, 1H), 10.98 (s, 1H),7.97 (s,lH),7.63-7.58(d, J = 8.4 Hz, 1H), 7.54-7.43(m, 2H), 7.26-7.22 (m, 1H), 7.18-7.12 (m, 2H), 6.99-6.96 (d, J = 13.2 Hz, 1H), 5.46(s, 1H), 5.11-5.01 (m,lH), 4.44-4.18 (m, 2H), 3.98-3.84 (m, 4H), 3.74-3.63(s, 2H), 3.45-3.17 (m, 7H), 2.97-2.88 (m, 1H), 2.69-2.53 (m, 1H), 2.44-2.36 (m, 1H), 2.22(s, 2H), 2.15(s, 1H), 1.81-1.73(m, 2H), 1.73-1.58(m, 2H), 1.19 - 1. 01 ( m , 1 2 H ) . |

### Biological Assays

The following assays were used to measure the effects of the compounds of the present specification. But these assays are not intended to limit the scope of the application.

### Test example 1. The ER degradation activity of the compounds disclosed herein in MCF-7 cells.

ERα-positive human breast cancer cells MCF-7 (ATCC, HTB-22) were cultured with EMEM medium (ATCC, catalog number 30-2003) containing 10% fetal bovine serum (Thermo Fisher, catalog number 10099141) and 10 µg/mL bovine insulin in 25 cm² or 75 cm² plastic tissue culture flasks in a 37 °C incubator with 5% CO₂. The cells were passaged 2 to 3 times per week. MCF-7 cells were seeded in 6-well plate at 1×10⁶ cells/well with 1.8 mL culture medium per well, and cultured in a 37 °C incubator with 95% air and 5% CO₂. After 24 hours, the test compounds were added: the compounds were serially diluted 10-fold in DMSO starting from 10 mM (dissolved in DMSO) to prepare gradient stock solutions. A complete culture medium containing 1% DMSO was prepared for further dilution, temporarily named as the "dilution buffer." Working solutions were prepared by diluting each stock solution with 99 times the volume of dilution buffer, that is, by adding 1 µL of the 10 mM stock solution to 99 µL of the dilution buffer and mixing thoroughly.

Finally, 10 µL of the working solution was added to the cell-seeded culture plate. The final concentration of DMSO in the cell culture medium was 0.2%, and the final concentration of the test compounds ranged from 1 nM to 100 nM. The cells were then incubated at 37 °C for 6 hours.

The culture medium was removed, and the cells were washed twice with ice cold PBS. 1 mL of PBS was added to each well, and the cells were collected into EP tubes using a cell scraper, and centrifuged at 4 °C, 4000 rpm for 5 minutes. PBS was aspirated. Total protein was extracted using the Minute^{™} Mammalian Tissue/Cell Total Protein Extraction Reagent. The protein concentration was measured using the BCA protein assay kit. 30 µg of protein was taken and mixed with 5× SDS-PAGE loading buffer, boiled for 10 minutes, and cooled. The samples were loaded into wells of a precast gel, with constant voltage at 80 V for 30 minutes, and then the voltage was changed to 120 V after the samples entered the resolving gel. The "sandwich" (sponge - filter paper - gel - membrane - filter paper - sponge) was assembled for Bio-Rad's Transblot. Transfer was performed for 1 hour at 0.3 amp at 4 °C. The sample was blocked with 5% fat-free milk for 1 hour, and incubated with Estrogen Receptor alpha Antibody (Thermo Fisher, catalog number MA5-14104) overnight at 4 °C. The membrane was washed with TBST to remove non-specifically bound antibodies, and then incubated with goat anti-mouse IgG-HRP secondary antibody for 1 hour at room temperature, and was visualized using ECL. The protein degradation level was calculated by measuring the grayscale intensity.

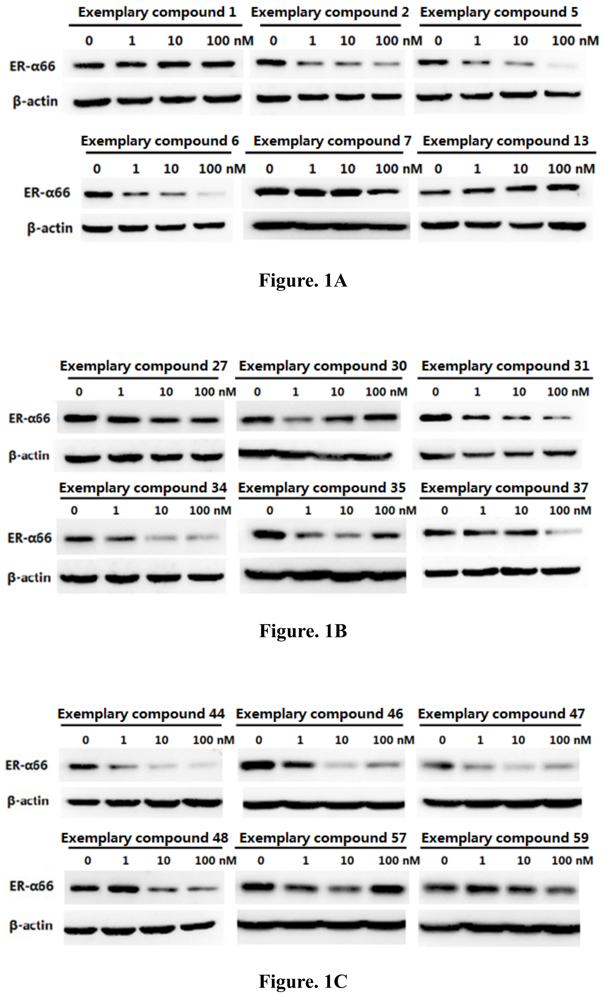

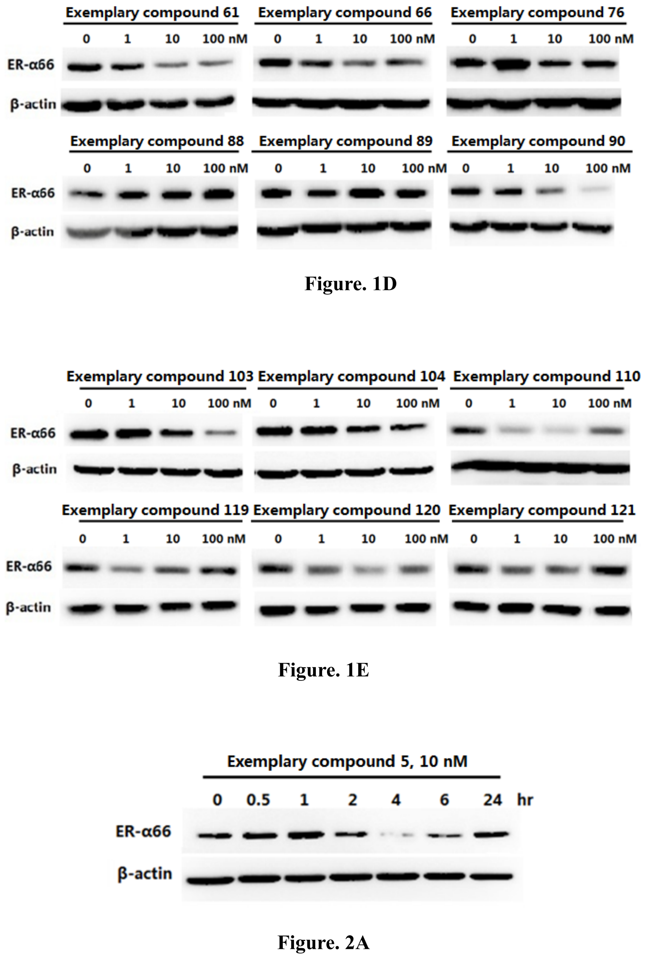

Figure 1A shows the ERα degradation in MCF-7 cells after 6 hours of treatment with exemplary compounds 1, 2, 5, 6, 7, and 13 disclosed herein. Figure 1B shows the ERα degradation in MCF-7 cells after 6 hours of treatment with exemplary compounds 27, 30, 31, 34, 35, and 37 disclosed herein. Figure 1C shows the ERα degradation in MCF-7 cells after 6 hours of treatment with exemplary compounds 44, 46, 47, 48, 57, and 59 disclosed herein. Figure 1D shows the ERα degradation in MCF-7 cells after 6 hours of treatment with exemplary compounds 61, 66, 76, 88, 89, and 90 disclosed herein. Figure 1E shows the ERα degradation in MCF-7 cells after 6 hours of treatment with exemplary compounds 103, 104, 110, 119, 120, and 121 disclosed herein.

The DC50 values (i.e., the concentration of the test compounds required to degrade 50% of the target protein) were calculated and shown in Table 1. A corresponds to a DC50 value below 10 nM, B corresponds to a DC50 value greater than 10 nM and less than 100 nM, and C corresponds to DC50 values greater than 100 nM.

**Table 1. The DC50 values of exemplary compounds in MCF-7 cells**

| **Exemplary compound** | **DC50** | **Exemplary compound** | **DC50** | **Exemplary compound** | **DC50** |
|---|---|---|---|---|---|
| 1 | C | 49 | C | 97 | B |
| 2 | A | 50 | C | 98 | C |
| 3 | C | 51 | C | 99 | B |
| 4 | C | 52 | C | 100 | C |
| 5 | A | 53 | C | 101 | C |
| 6 | A | 54 | C | 102 | C |
| 7 | B | 55 | C | 103 | B |
| 8 | C | 56 | A | 104 | C |
| 9 | C | 57 | A | 105 | A |
| 10 | C | 58 | C | 106 | C |
| 11 | A | 59 | B | 107 | C |
| 12 | B | 60 | C | 108 | C |
| 13 | C | 61 | A | 109 | A |
| 14 | C | 62 | C | 110 | A |
| 15 | B | 63 | C | 112 | B |
| 16 | C | 64 | C | 113 | C |
| 17 | C | 65 | C | 114 | B |
| 18 | C | 66 | B | 115 | C |
| 19 | C | 67 | C | 116 | C |
| 20 | C | 68 | C | 117 | C |
| 21 | C | 69 | A | 118 | C |
| 22 | A | 70 | B | 119 | A |
| 23 | C | 71 | C | 120 | A |
| 24 | B | 72 | C | 121 | A |
| 25 | C | 73 | C | 122 | A |
| 26 | A | 74 | C | 123 | B |
| 27 | B | 75 | C | 124 | B |
| 28 | C | 76 | B | 125 | A |
| 29 | C | 77 | C | 126 | A |
| 30 | C | 78 | C | 127 | A |
| 31 | A | 79 | B | 128 | B |
| 32 | C | 80 | C | 129 | B |
| 33 | C | 81 | C | 130 | A |
| 34 | A | 82 | C | 131 | A |
| 35 | A | 83 | A | 132 | A |
| 36 | A | 84 | C | 133 | B |
| 37 | B | 85 | C | 134 | B |
| 38 | B | 86 | C | 135 | B |
| 39 | A | 87 | C | 136 | A |
| 40 | C | 88 | C | 137 | A |
| 41 | B | 89 | C | 138 | A |
| 42 | B | 90 | A | 139 | B |
| 43 | C | 91 | C | 140 | A |
| 44 | A | 92 | C | 141 | A |
| 45 | B | 93 | C | 142 | A |
| 46 | A | 94 | C | 143 | B |
| 47 | A | 95 | C | | |
| 48 | A | 96 | A | | |

### Test example 2. The time-dependent ERα degradation activity of the compounds disclosed herein in MCF-7 cells.

ERα-positive human breast cancer cells MCF-7 (ATCC, HTB-22) were cultured with EMEM medium (ATCC, catalog number 30-2003) containing 10% fetal bovine serum (Thermo Fisher, catalog number 10099141) and 10 µg/mL bovine insulin in 25 cm² or 75 cm² plastic tissue culture flasks in a 37 °C incubator with 5% CO₂. The cells were passaged 2 to 3 times per week.

MCF-7 cells were seeded in 6-well plate at 1×10⁶ cells/well with 1.8 mL culture medium per well, and cultured in a 37 °C incubator with 95% air and 5% CO₂. After 24 hours, the test compounds were added: the compounds were serially diluted 10-fold in DMSO starting from 10 mM (dissolved in DMSO) to prepare gradient stock solutions. A complete culture medium containing 1% DMSO was prepared for further dilution, temporarily named as the "dilution buffer." Working solutions were prepared by diluting each stock solution with 99 times the volume of dilution buffer, that is, by adding 1 µL of the 10 mM stock solution to 99 µL of the dilution buffer and mixing thoroughly.

Finally, 10 µL of the working solution was added to the cell-seeded culture plate. The final concentration of DMSO in the cell culture medium was 0.2%, and the final concentration of the test compounds was 10 nM. The cells were then incubated at 37 °C for 0.5, 1, 2, 4, 6 and 24 hours respectively.

The culture medium was removed, and the cells were washed twice with ice cold PBS. 1 mL of PBS was added to each well, and the cells were collected into EP tubes using a cell scraper, and centrifuged at 4 °C, 4000 rpm for 5 minutes. PBS was aspirated. Total protein was extracted using the Minute^{™} Mammalian Tissue/Cell Total Protein Extraction Reagent. The protein concentration was measured using the BCA protein assay kit. 30 µg of protein was taken and mixed with 5× SDS-PAGE loading buffer, boiled for 10 minutes, and cooled. The samples were loaded into wells of a precast gel, with constant voltage at 80 V for 30 minutes, and then the voltage was changed to 120 V after the samples entered the resolving gel. The "sandwich" (sponge - filter paper - gel - membrane - filter paper - sponge) was assembled for Bio-Rad's Transblot. Transfer was performed for 1 hour at 0.3 amp at 4 °C. The sample was blocked with 5% fat-free milk for 1 hour, and incubated with Estrogen Receptor alpha Antibody (Thermo Fisher, catalog number MA5-14104) overnight at 4 °C. The membrane was washed with TBST to remove non-specifically bound antibodies, and then incubated with goat anti-mouse IgG-HRP secondary antibody for 1 hour at room temperature, and was visualized using ECL. The protein degradation level was calculated by measuring the grayscale intensity.

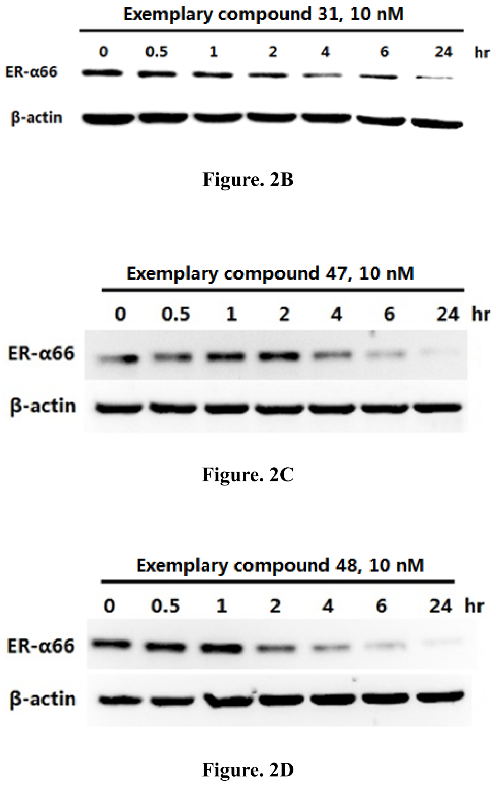

Figure 2A-2D shows the time-dependent ERα degradation activity in the MCF-7 cell line of 10 nM exemplary compounds 5, 31, 47, and 48 disclosed herein.

### Test example 3. The inhibitory effect of the compounds disclosed herein on the proliferation of MCF-7 cells.

ERα-positive human breast cancer cells MCF-7 (ATCC, HTB-22) were cultured with EMEM medium (ATCC, catalog number 30-2003) containing 10% fetal bovine serum (Thermo Fisher, catalog number 10099141) and 10 µg/mL bovine insulin in 25 cm² or 75 cm² plastic tissue culture flasks in a 37 °C incubator with 5% CO₂. The cells were passaged 2 to 3 times per week.

MCF-7 cells were seeded in 96-well plate at 3×10³ cells/well with 90 µL culture medium per well, and cultured in a 37 °C incubator with 95% air and 5% CO₂. After 24 hours, the test compounds were added: the compounds were serially diluted 10-fold in DMSO starting from 10 mM (dissolved in DMSO) to prepare gradient stock solutions. A complete culture medium containing 1% DMSO was prepared for further dilution, temporarily named as the "dilution buffer." Working solutions were prepared by diluting each stock solution with 99 times the volume of dilution buffer, that is, by adding 1 µL of the 10 mM stock solution to 99 µL of the dilution buffer and mixing thoroughly. Finally, 10 µL of the working solution was added to the cell-seeded culture plate. The final concentration of DMSO in the cell culture medium was 0.2%, and the final concentration of the test compounds ranged from 1 pM to 10 µM. The cells were then incubated at 37 °C for 3 days. The culture medium was removed and replaced with 90µL per well of fresh medium. According to the above compound dilution method, the test compounds were added again. On the 6th day, the CellTiter Glo luminescent cell viability assay kit (Promag, item number G7573) was added for cell viability measurement.

Cell viability was measured using the CellTiter-Glo assay kit, and the half-maximal growth inhibitory concentration (GI50) of the compounds was calculated using the Prism software.

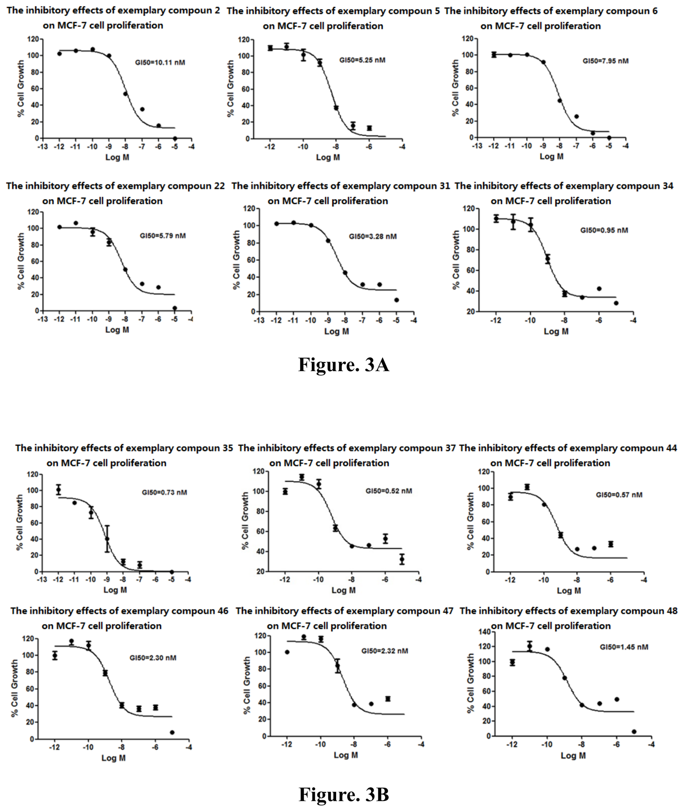

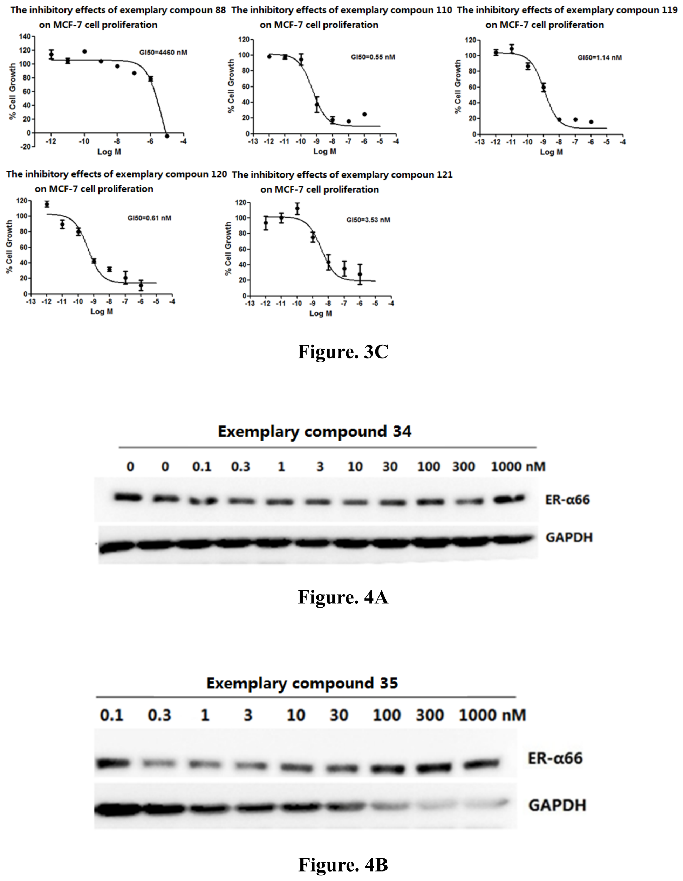

Figure 3A shows the inhibitory effects of exemplary compounds 2, 5, 6, 22, 31, and 34 disclosed herein on MCF-7 cell proliferation after 6 days treatment. Figure 3B shows the inhibitory effects of exemplary compounds 35, 37, 44, 46, 47and 48 disclosed herein on MCF-7 cell proliferation after 6 days treatment. Figure 3C shows the inhibitory effects of exemplary compounds 88, 110, 119, 120 and 121 disclosed herein on MCF-7 cell proliferation after 6 days treatment.

The GI50 values (i.e., the concentration of the test compounds required to inhibit 50% of the cell proliferation) were calculated and shown in Table 2. A corresponds to a GI50 value below 10 nM, B corresponds to a GI50 value greater than 10 nM and less than 100 nM, and C corresponds to GI50 values greater than 100 nM.

**Table 2. The GI50 values of exemplary compounds in MCF-7 cells**

| **Exemplary compound** | **GI50** |
|---|---|
| 2 | B |
| 5 | A |
| 6 | A |
| 22 | A |
| 31 | A |
| 34 | A |
| 35 | A |
| 37 | A |
| 44 | A |
| 46 | A |
| 47 | A |
| 48 | A |
| 77 | C |
| 88 | C |
| 89 | C |
| 90 | C |
| 110 | A |
| 119 | A |
| 120 | A |
| 121 | A |

### Test example 4. The ER degradation activity of the compounds disclosed herein in T-47D cells.

ERα-positive human breast cancer cells T-47D (ATCC, HTB-133) were cultured with RPMI-1640 medium (Thermo Fisher, catalog number 61870-036) containing 10% fetal bovine serum (Thermo Fisher, catalog number 10099141) and 10 µg/mL bovine insulin in 25 cm² or 75 cm² plastic tissue culture flasks in a 37°C incubator with 5% CO₂. The cells were passaged 2 to 3 times per week.

T-47D cells were seeded in 6-well plate at 1×10⁶ cells/well with 1.8 mL culture medium per well, and cultured in a 37 °C incubator with 95% air and 5% CO₂. After 24 hours, the test compounds were added: the compounds were serially diluted 10-fold in DMSO starting from 10 mM (dissolved in DMSO) to prepare gradient stock solutions. A complete culture medium containing 1% DMSO was prepared for further dilution, temporarily named as the "dilution buffer." Working solutions were prepared by diluting each stock solution with 99 times the volume of dilution buffer, that is, by adding 1 µL of the 10 mM stock solution to 99 µL of the dilution buffer and mixing thoroughly. Finally, 10 µL of the working solution was added to the cell-seeded culture plate. The final concentration of DMSO in the cell culture medium was 0.2%, and the final concentration of the test compounds ranged from 0.1 nM to 1000 nM. The cells were then incubated at 37°C for 4 hours. The culture medium was removed, and the cells were washed twice with ice cold PBS. 1 mL of PBS was added to each well, and the cells were collected into EP tubes using a cell scraper, and centrifuged at 4 °C, 4000 rpm for 5 minutes. PBS was aspirated. Total protein was extracted using the Minute^{™} Mammalian Tissue/Cell Total Protein Extraction Reagent. The protein concentration was measured using the BCA protein assay kit. 30 µg of protein was taken and mixed with 5× SDS-PAGE loading buffer, boiled for 10 minutes, and cooled. The samples were loaded into wells of a precast gel, with constant voltage at 80 V for 30 minutes, and then the voltage was changed to 120 V after the samples entered the resolving gel. The "sandwich" (sponge - filter paper - gel - membrane - filter paper - sponge) was assembled for Bio-Rad's Transblot. Transfer was performed for 1 hour at 0.3 amp at 4 °C. The sample was blocked with 5% fat-free milk for 1 hour, and incubated with Estrogen Receptor alpha Antibody (Thermo Fisher, catalog number MA5-14104) overnight at 4°C. The membrane was washed with TBST to remove non-specifically bound antibodies, and then incubated with goat anti-mouse IgG-HRP secondary antibody for 1 hour at room temperature, and was visualized using ECL. The protein degradation level was calculated by measuring the grayscale intensity.

Figure 4A shows the ERα degradation in T-47D cells after 4 hours of treatment with exemplary compound 34 disclosed herein. Figure 4B shows the ERα degradation in T-47D cells after 4 hours of treatment with exemplary compound 35 disclosed herein.

### Test example 5. The ER degradation activity of the compounds disclosed herein in MCF-7 cells expressing the ERα Y537S mutant.

MCF-7 cells expressing the ERα Y537S mutant was constructed via lentiviral infection.

MCF-7/ERα Y537S cells were seeded in 6-well plate at 1×10⁶ cells/well with 1.8 mL culture medium per well, and cultured in a 37 °C incubator with 95% air and 5% CO₂. After 24 hours, the test compounds were added: the compounds were serially diluted 10-fold in DMSO starting from 10 mM (dissolved in DMSO) to prepare gradient stock solutions. A complete culture medium containing 1% DMSO was prepared for further dilution, temporarily named as the "dilution buffer." Working solutions were prepared by diluting each stock solution with 99 times the volume of dilution buffer, that is, by adding 1 µL of the 10 mM stock solution to 99 µL of the dilution buffer and mixing thoroughly. Finally, 10 µL of the working solution was added to the cell-seeded culture plate. The final concentration of DMSO in the cell culture medium was 0.2%, and the final concentration of the test compounds ranged from 0.1 nM to 1000 nM. The cells were then incubated at 37 °C for 4 and 6 hours respectively.

The culture medium was removed, and the cells were washed twice with ice cold PBS. 1 mL of PBS was added to each well, and the cells were collected into EP tubes using a cell scraper, and centrifuged at 4 °C, 4000 rpm for 5 minutes. PBS was aspirated. Total protein was extracted using the Minute^{™} Mammalian Tissue/Cell Total Protein Extraction Reagent. The protein concentration was measured using the BCA protein assay kit. 30 µg of protein was taken and mixed with 5× SDS-PAGE loading buffer, boiled for 10 minutes, and cooled. The samples were loaded into wells of a precast gel, with constant voltage at 80 V for 30 minutes, and then the voltage was changed to 120 V after the samples entered the resolving gel. The "sandwich" (sponge - filter paper - gel - membrane - filter paper - sponge) was assembled for Bio-Rad's Transblot. Transfer was performed for 1 hour at 0.3 amp at 4 °C. The sample was blocked with 5% fat-free milk for 1 hour, and incubated with Estrogen Receptor alpha Antibody (Thermo Fisher, catalog number MA5-14104) overnight at 4°C. The membrane was washed with TBST to remove non-specifically bound antibodies, and then incubated with goat anti-mouse IgG-HRP secondary antibody for 1 hour at room temperature, and was visualized using ECL. The protein degradation level was calculated by measuring the grayscale intensity.

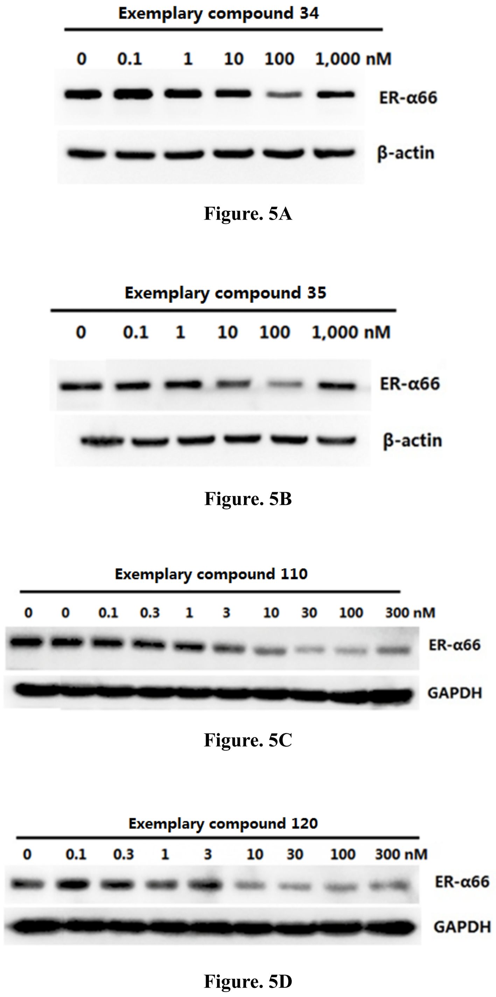

Figure 5A shows the ERα degradation in MCF-7/ERα Y537S cells after 4 hours of treatment with exemplary compound 34 disclosed herein. Figure 5B shows the ERα degradation in MCF-7/ERα Y537S cells after 4 hours of treatment with exemplary compound 35 disclosed herein. Figure 5C shows the ERα degradation in MCF-7/ERα Y537S cells after 6 hours of treatment with exemplary compound 110 disclosed herein. Figure 5D shows the ERα degradation in MCF-7/ERα Y537S cells after 6 hours of treatment with exemplary compound 120 disclosed herein.

### Test example 6. The ER degradation activity of the compounds disclosed herein in MCF-7 celles expressing the ERα D538G mutant.

MCF-7 cells expressing the ERα D538G mutant were constructed via lentiviral infection. MCF-7/ERα D538G cells were seeded in 6-well plate at 1×10⁶ cells/well with 1.8 mL culture medium per well, and cultured in a 37 °C incubator with 95% air and 5% CO₂. After 24 hours, the test compounds were added: the compounds were serially diluted 10-fold in DMSO starting from 10 mM (dissolved in DMSO) to prepare gradient stock solutions. A complete culture medium containing 1% DMSO was prepared for further dilution, temporarily named as the "dilution buffer." Working solutions were prepared by diluting each stock solution with 99 times the volume of dilution buffer, that is, by adding 1 µL of the 10 mM stock solution to 99 µL of the dilution buffer and mixing thoroughly. Finally, 10 µL of the working solution was added to the cell-seeded culture plate. The final concentration of DMSO in the cell culture medium was 0.2%, and the final concentration of the test compounds ranged from 0.1 nM to 1000 nM. The cells were then incubated at 37 °C for 6 hours.

The culture medium was removed, and the cells were washed twice with ice cold PBS. 1 mL of PBS was added to each well, and the cells were collected into EP tubes using a cell scraper, and centrifuged at 4 °C, 4000 rpm for 5 minutes. PBS was aspirated. Total protein was extracted using the Minute^{™} Mammalian Tissue/Cell Total Protein Extraction Reagent. The protein concentration was measured using the BCA protein assay kit. 30 µg of protein was taken and mixed with 5× SDS-PAGE loading buffer, boiled for 10 minutes, and cooled. The samples were loaded into wells of a precast gel, with constant voltage at 80 V for 30 minutes, and then the voltage was changed to 120 V after the samples entered the resolving gel. The "sandwich" (sponge - filter paper - gel - membrane - filter paper - sponge) was assembled for Bio-Rad's Transblot. Transfer was performed for 1 hour at 0.3 amp at 4 °C. The sample was blocked with 5% fat-free milk for 1 hour, and incubated with Estrogen Receptor alpha Antibody (Thermo Fisher, catalog number MA5-14104) overnight at 4°C. The membrane was washed with TBST to remove non-specifically bound antibodies, and then incubated with goat anti-mouse IgG-HRP secondary antibody for 1 hour at room temperature, and was visualized using ECL. The protein degradation level was calculated by measuring the grayscale intensity.

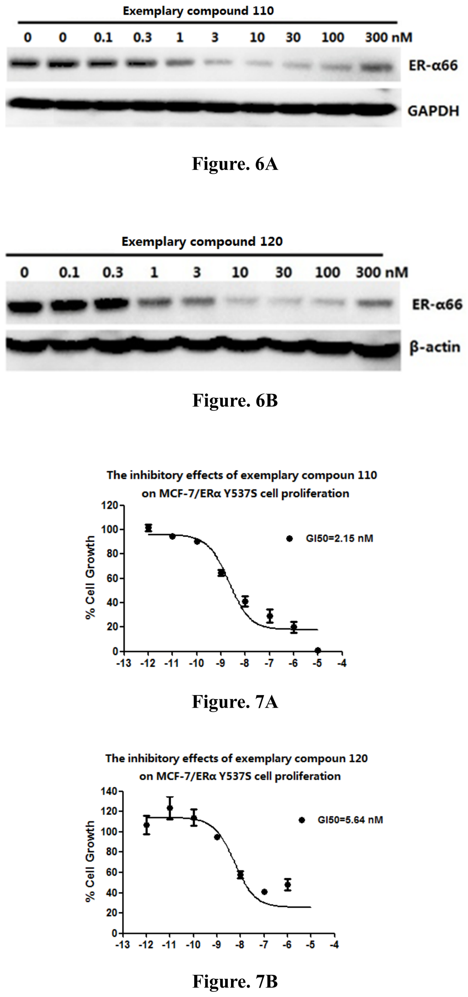

Figure 6A shows the ERα degradation in MCF-7/ERα D538G cells after 6 hours of treatment with exemplary compound 110 disclosed herein. Figure 6B shows the ERα degradation in MCF-7/ERα D538G cells after 6 hours of treatment with exemplary compound 120 disclosed herein.

### Test example 7. The inhibitory effect of the compound disclosed herein on the proliferation of MCF-7 cells expressing ER α Y537S mutant.

MCF-7/ ERα Y537S cells were seeded in 96-well plate at 3×10³ cells/well with 90 µL culture medium per well, and cultured in a 37 °C incubator with 95% air and 5% CO₂. After 24 hours, the test compounds were added: the compounds were serially diluted 10-fold in DMSO starting from 10 mM (dissolved in DMSO) to prepare gradient stock solutions. A complete culture medium containing 1% DMSO was prepared for further dilution, temporarily named as the "dilution buffer." Working solutions were prepared by diluting each stock solution with 99 times the volume of dilution buffer, that is, by adding 1 µL of the 10 mM stock solution to 99 µL of the dilution buffer and mixing thoroughly. Finally, 10 µL of the working solution was added to the cell-seeded culture plate. The final concentration of DMSO in the cell culture medium was 0.2%, and the final concentration of the test compounds ranged from 1 pM to 10 µM. The cells were then incubated at 37 °C for 3 days. The culture medium was removed and replaced with 90µL per well of fresh medium. According to the above compound dilution method, the test compounds were added again. On the 6th day, the CellTiter Glo luminescent cell viability assay kit (Promag, item number G7573) was added for cell viability measurement. Cell viability was assessed using the CellTiter-Glo assay kit, and the half-maximal growth inhibitory concentration (GI50) of the compounds was calculated using the Prism software.

Figure 7A shows the inhibitory effects of exemplary compound 110 disclosed herein on MCF-7/ ERα Y537S cell proliferation after 6 days treatment. Figure 7B shows the inhibitory effects of exemplary compound 120 disclosed herein on MCF-7/ ERα Y537S cell proliferation after 6 days treatment.

### Test example 8. The anti-tumor activity of the compounds disclosed herein in MCF-7 xenografts expressing ER α Y537S mutant.

MCF-7/ERα Y537S tumors that had been passaged in mice for more than three but less than ten generations were transplanted as approximately 3×3×3 mm tumor blocks into the subcutaneous right flank of female Balb/c nude mice. When the tumor volume in the tumor-bearing mice reached around 200 mm³, the mice were randomly divided into four groups according to tumor volume and body weight: vehicle group, 1 mpk group, 3 mpk group, and 10 mpk group, 7 mice in each group. The day of grouping was designated as D0, and daily oral gavage administration began on this day and last for a total of 17 days. The 17th day after administration was designated as Day 17.

The tumor volume was measured twice weekly using a caliper, and the body weight of the mice was measured using an analytical balance. The length and width of the tumor were measured, and the formula for tumor volume calculation is: Tumor Volume (TV) = 0.5 × length × width× width. The T/C value was calculated based on the tumor volume, where T is the mean relative tumor volume (RTV) of the treated groups, and C is the mean RTV of the vehicle group. The RTV is the ratio of the tumor volume after administration to the tumor volume before administration. The tumor growth inhibition rate (TGITV) (%) = (1 - T/C) × 100%.

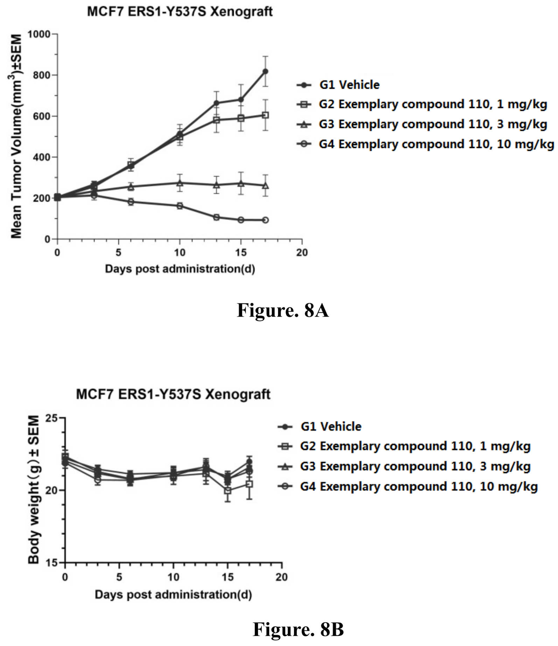

Figure 8A shows exemplary compound 110 disclosed herein exhibited dose-dependent tumor growth inhibition in MCF-7/ERα Y537S xenografts mice.

Figure 8B shows exemplary compound 110 disclosed herein had no effect on body weight in MCF-7/ERα Y537S xenograft mice.

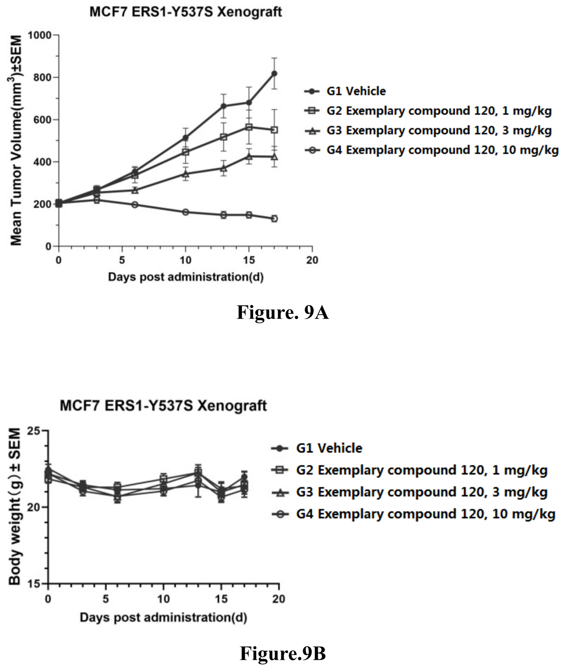

Figure 9A shows exemplary compound 120 disclosed herein exhibited dose-dependent tumor growth inhibition in MCF-7/ERα Y537S xenograft mice.

Figure 9B shows exemplary compound 120 disclosed herein had no effect on body weight in MCF-7/ERα Y537S xenograft mice.

## Claims

1. A compound targeting an estrogen receptor represented by formula (I) or a pharmaceutically acceptable salt, solvate, hydrate, or isomer thereof, 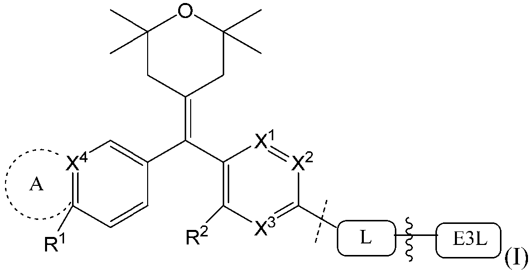
wherein 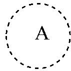
is absent, substituted or unsubstituted 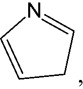
substituented or unsubstituted 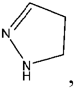 X⁴ is N or CRa, the substituent is selected from the group consisting of halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl;
when A is absent, R¹ is selected from the group consisting of halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl, preferably R₁ is selected from -NH₂ or -OH;
X¹ is N or CR³;
X² or X³ is independently selected from CH, CR³ and N;
R² and R³ are each independently selected from H, halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl;
Ra is selected from the group consisting of H, halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl;
L is any one selected from the group consisting of: 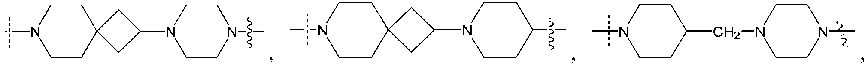 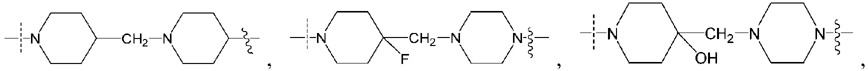 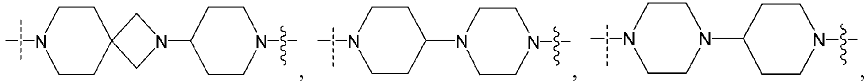 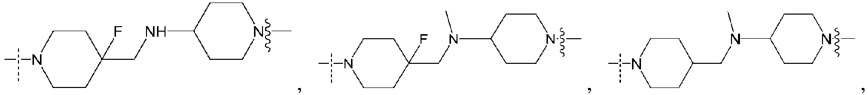 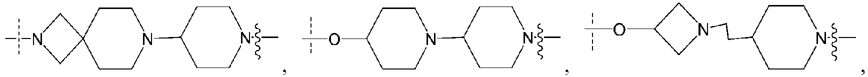 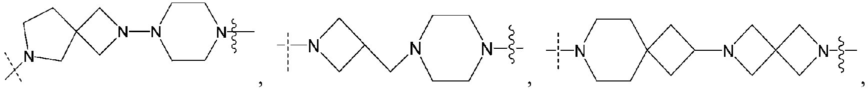 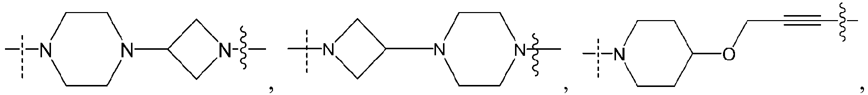 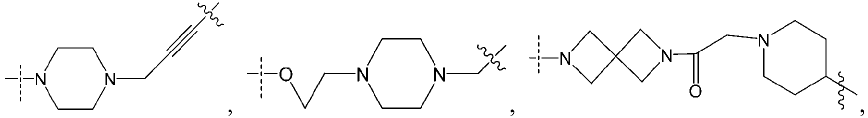 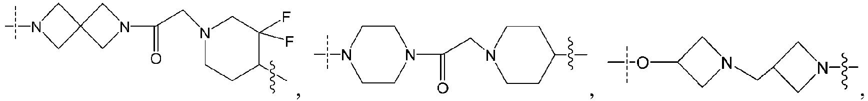 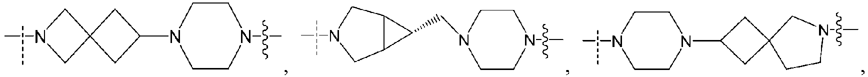 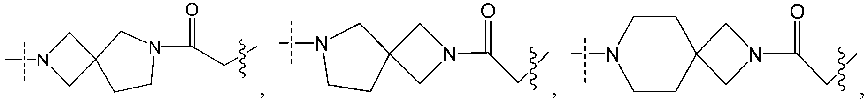 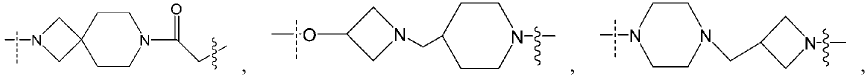 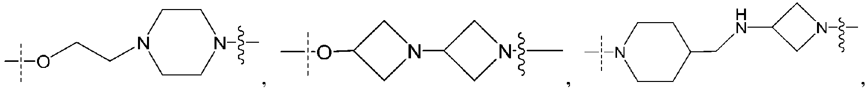 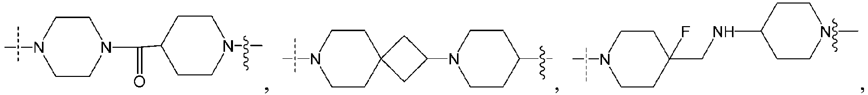 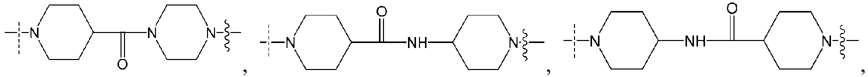 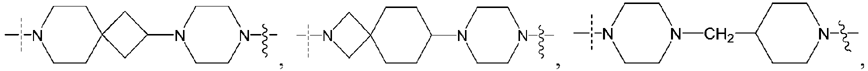 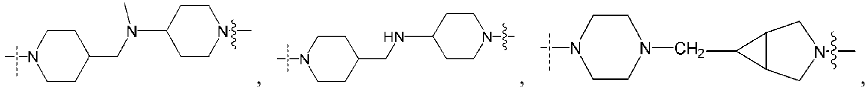 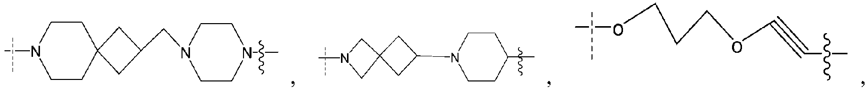 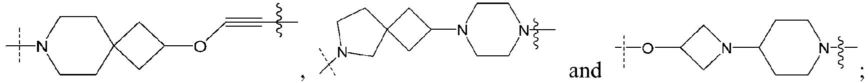
E3L is a ubiquitin ligase ligand.

2. The compound or a pharmaceutically acceptable salt, solvate, hydrate, or isomer thereof according to claim 1, wherein E3L is selected from:

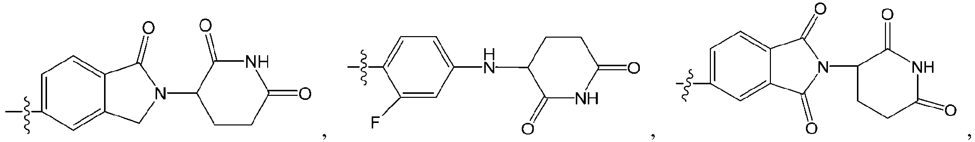

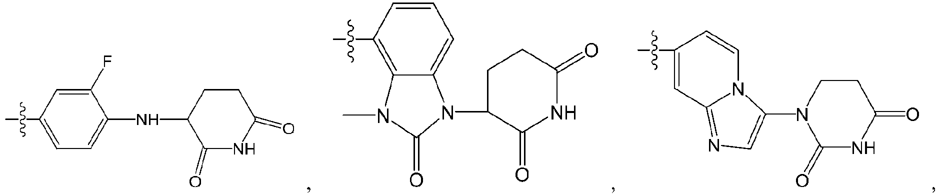

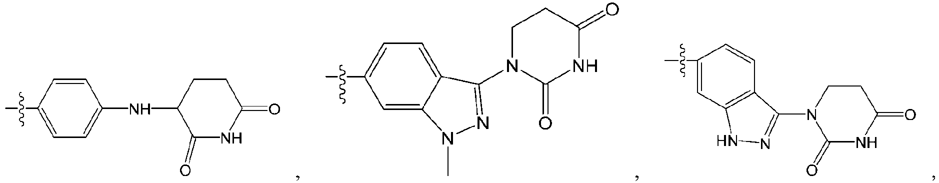

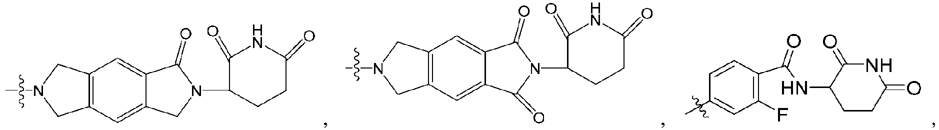

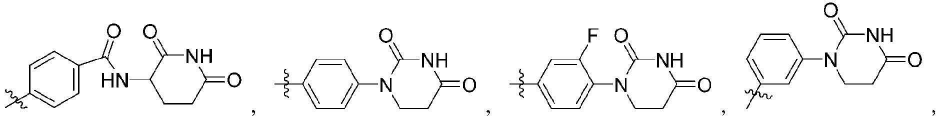

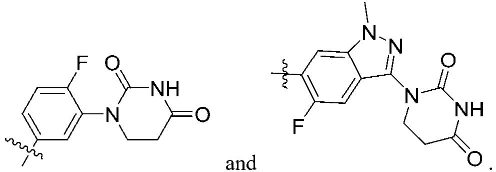

3. The compound or a pharmaceutically acceptable salt, solvate, hydrate, or isomer thereof according to claim 1 or 2, wherein the compound is represented by formula (II): 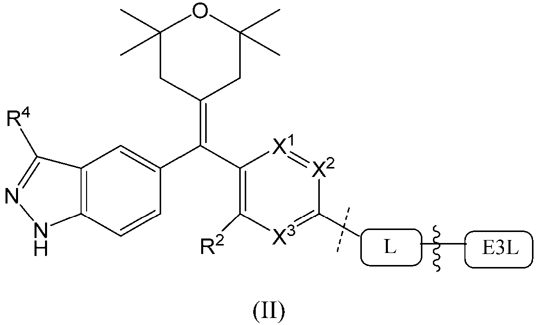 wherein R⁴ is selected from halogen, hydroxyl, cyano, oxy, benzyl, substituted or unsubstituted amino, substituted or unsubstituted C₁-C₆ alkyl, substituted or unsubstituted C₁-C₆ alkoxy, and C₁-C₆ acyl.

4. The compound or a pharmaceutically acceptable salt, solvate, hydrate, or isomer thereof according to claim 3, wherein R⁴ is halogen.

5. The compound or a pharmaceutically acceptable salt, solvate, hydrate, or isomer thereof according to claim 3, wherein the compound is represented by formula (III)

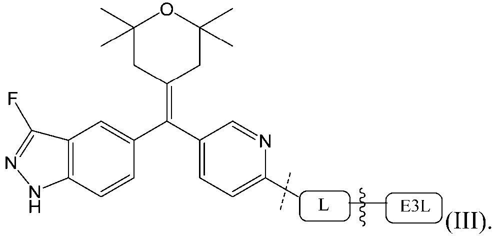

6. The compound or a pharmaceutically acceptable salt, solvate, hydrate, or isomer thereof according to claim 5, wherein the compound is represented by formula (IV)

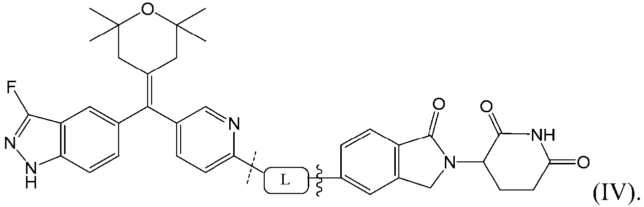

7. The compound or a pharmaceutically acceptable salt, solvate, hydrate, or isomer thereof according to claim 1 to 6, wherein L is any one selected from the group consisting of: 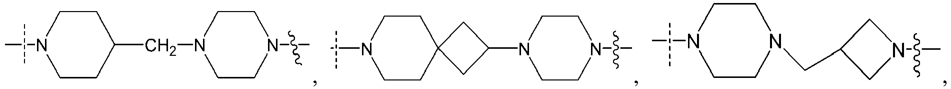 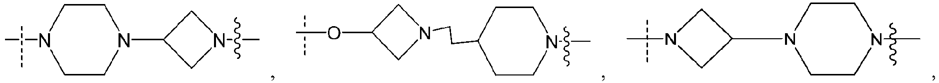 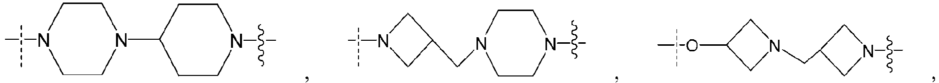 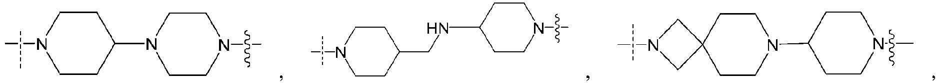 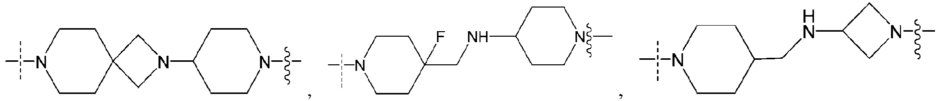 and

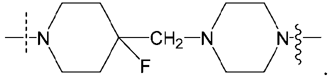

8. The compound or a pharmaceutically acceptable salt, solvate, hydrate, or isomer thereof according to claim 1, wherein X⁴ is N.

9. The compound or a pharmaceutically acceptable salt, solvate, hydrate, or isomer thereof according to claim 1, wherein X⁴ is CRa, Ra is selected from H, halogen and hydroxyl.

10. The compound or a pharmaceutically acceptable salt, solvate, hydrate, or isomer thereof according to claim 9, wherein Ra is H.

11. The compound or a pharmaceutically acceptable salt, solvate, hydrate, or isomer thereof according to claim 1, wherein X¹ is CR³, and R³ is H, CH₃O or F.

12. The compound or a pharmaceutically acceptable salt, solvate, hydrate, or isomer thereof according to claim 11, wherein R³ is H.

13. The compound or a pharmaceutically acceptable salt, solvate, hydrate, or isomer thereof according to claim 1, wherein X² is CH.

14. The compound or a pharmaceutically acceptable salt, solvate, hydrate, or isomer thereof according to claim 1, wherein X² is N.

15. The compound or a pharmaceutically acceptable salt, solvate, hydrate, or isomer thereof according to claim 1, wherein X³ is CH.

16. The compound or a pharmaceutically acceptable salt, solvate, hydrate, or isomer thereof according to claim 1, wherein R² is H.

17. The compound or a pharmaceutically acceptable salt, solvate, hydrate, or isomer thereof according to claim 2, wherein E3L is

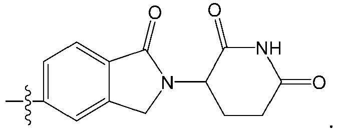

18. The compound or a pharmaceutically acceptable salt, solvate, hydrate, or isomer thereof according to claim 1 to 17, wherein the compound is selected from the group consisting of:

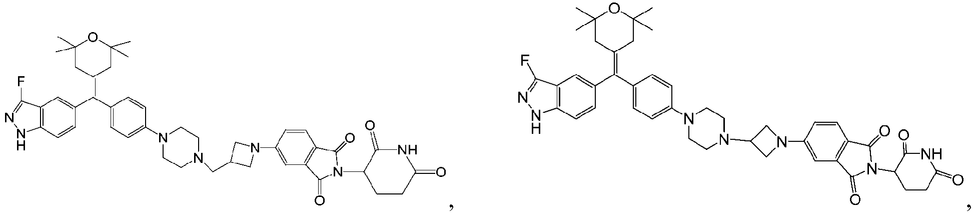

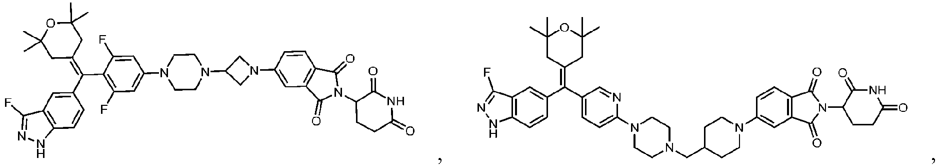

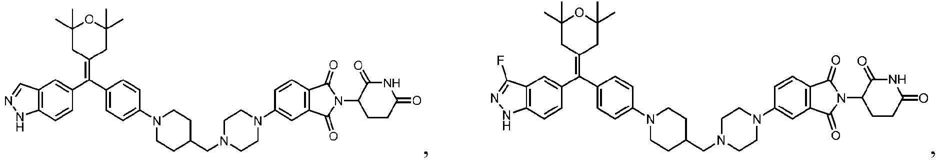

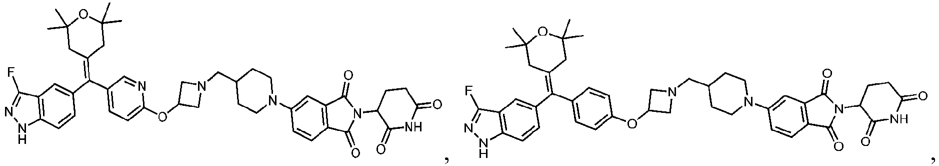

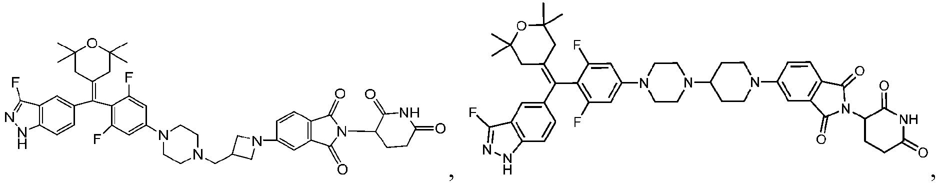

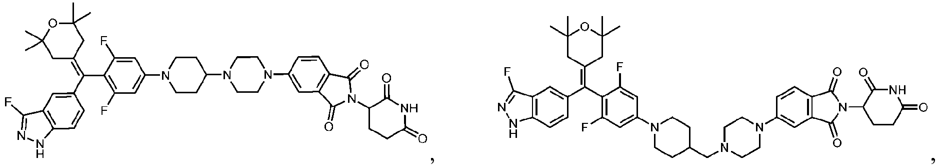

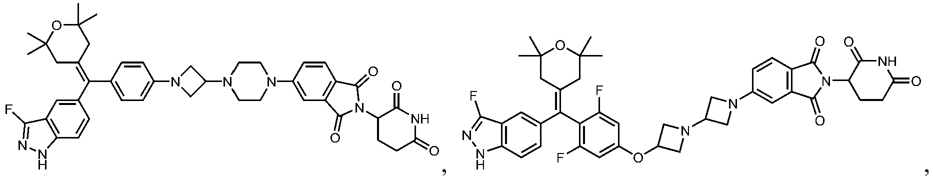

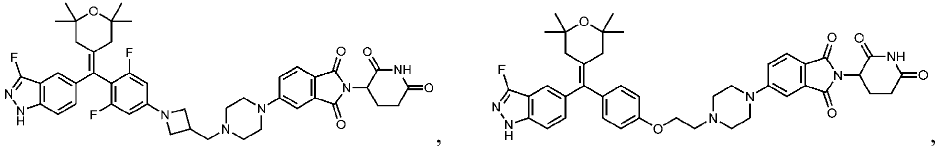

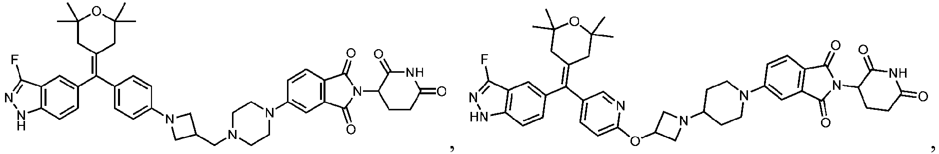

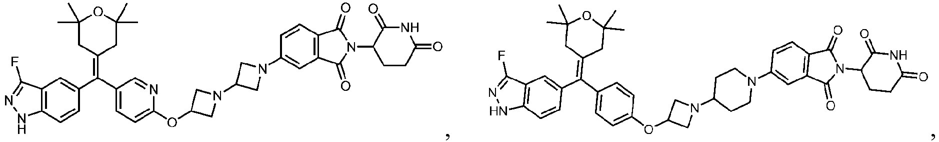

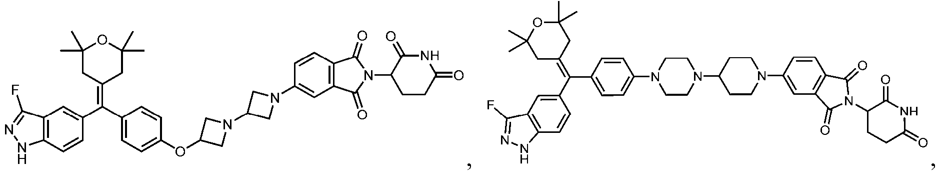

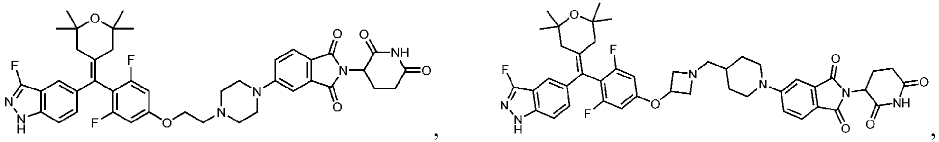

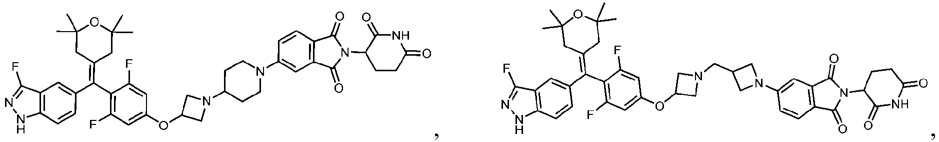

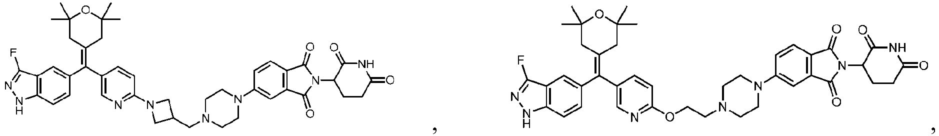

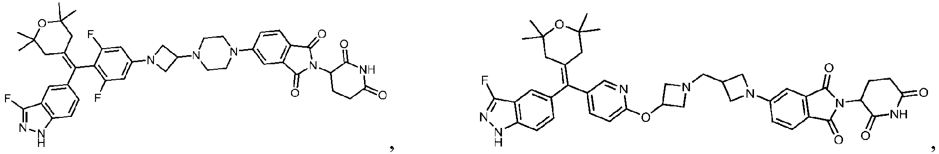

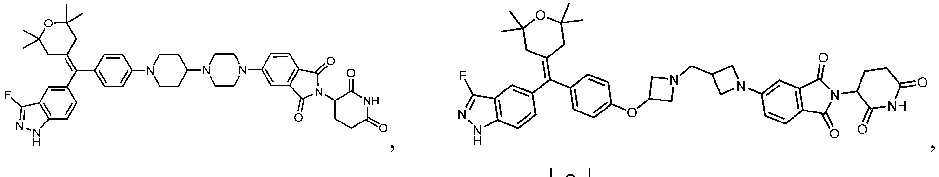

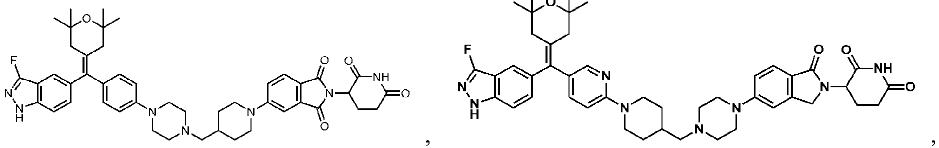

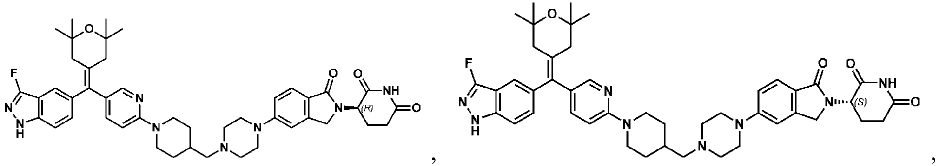

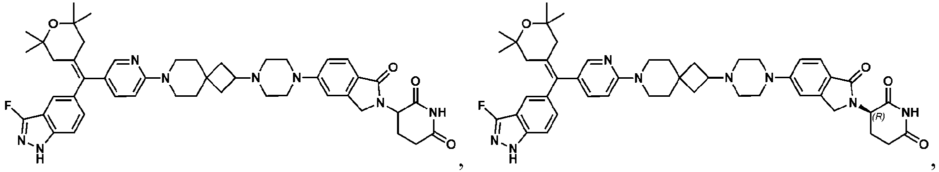

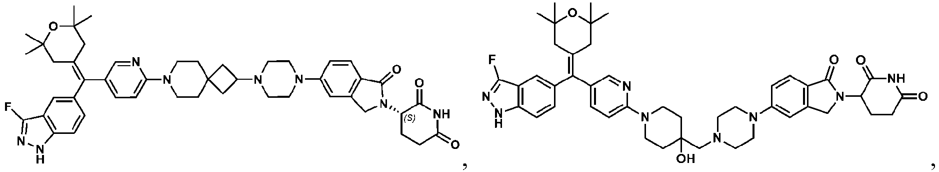

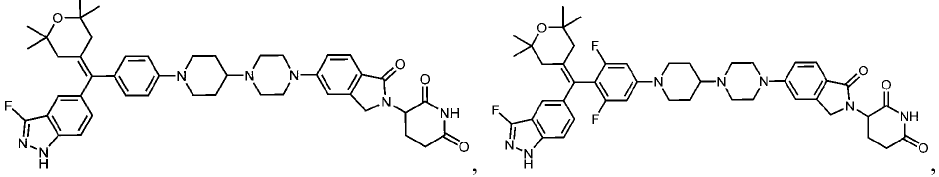

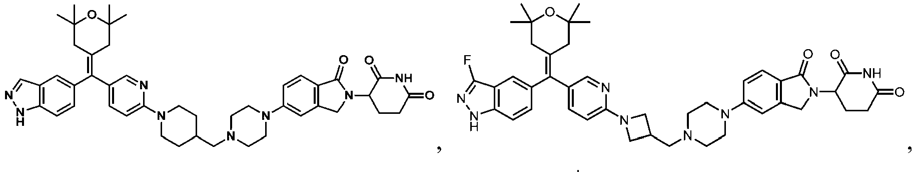

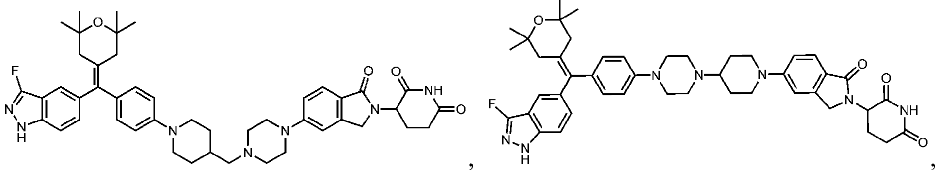

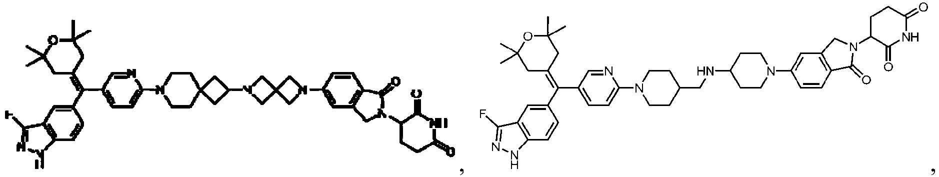

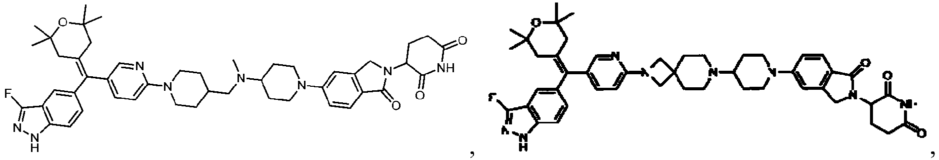

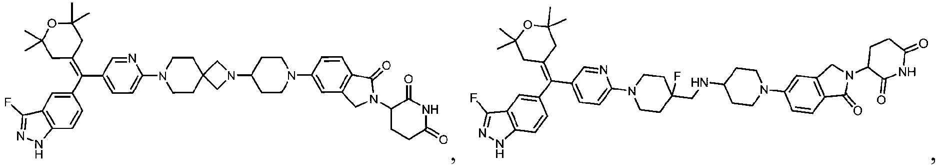

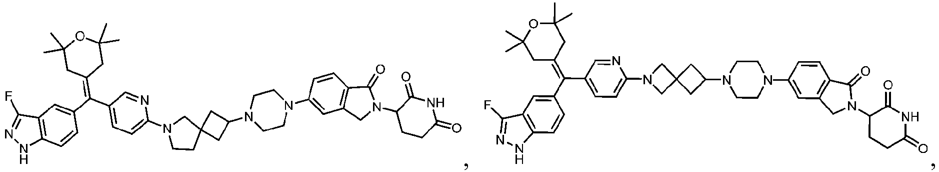

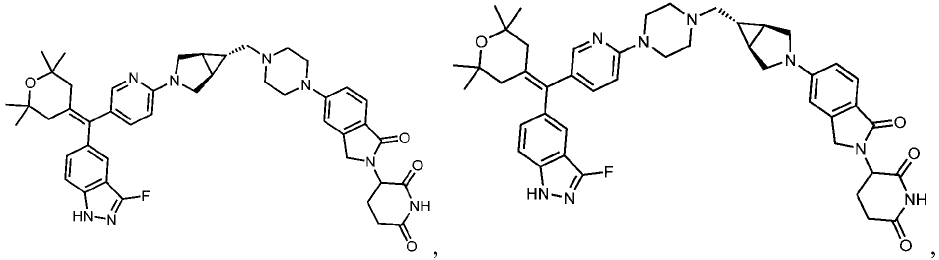

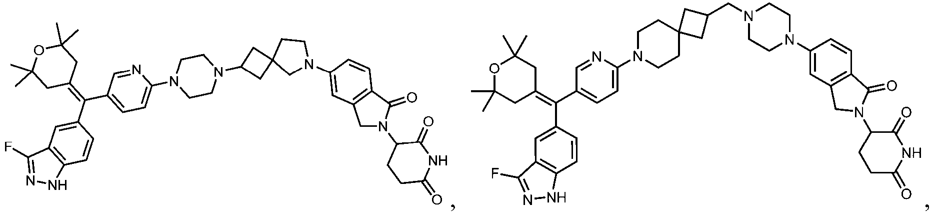

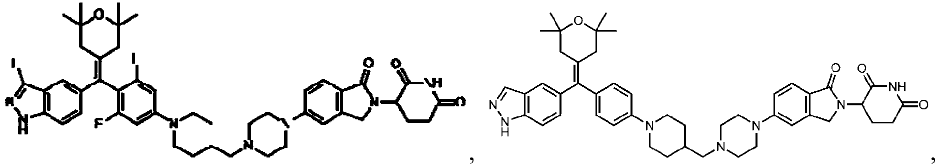

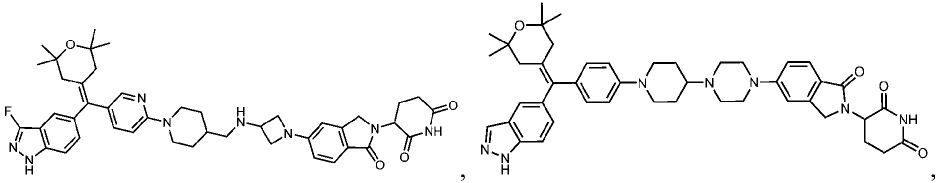

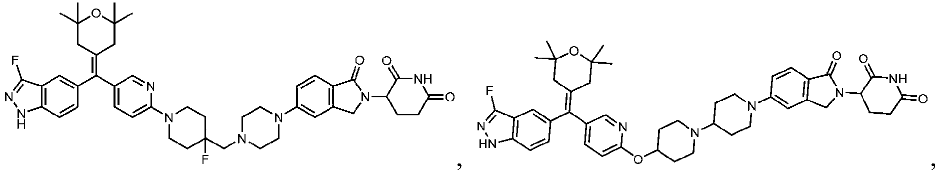

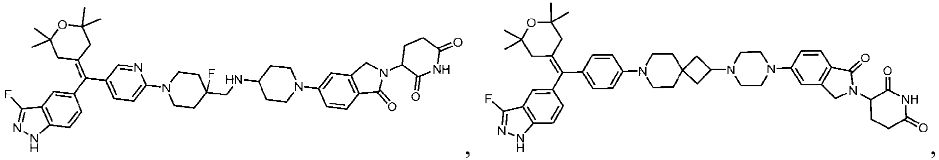

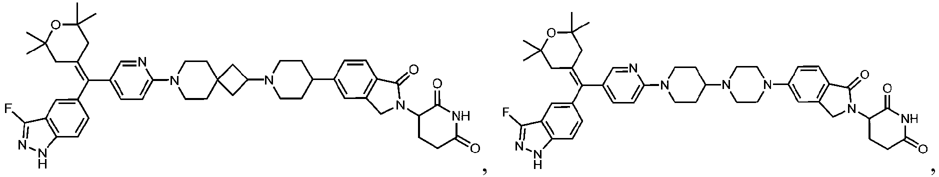

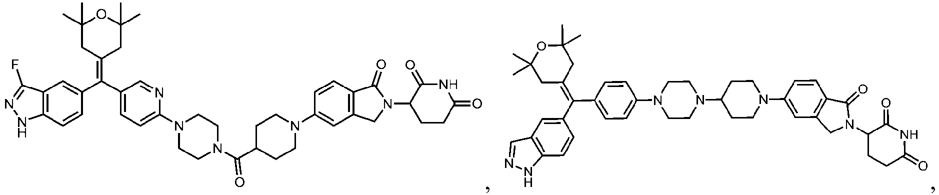

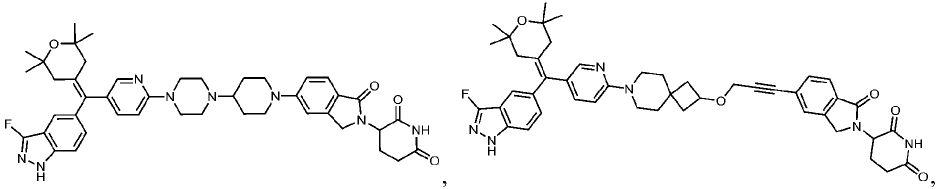

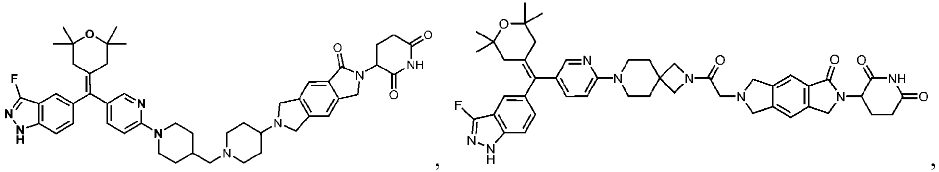

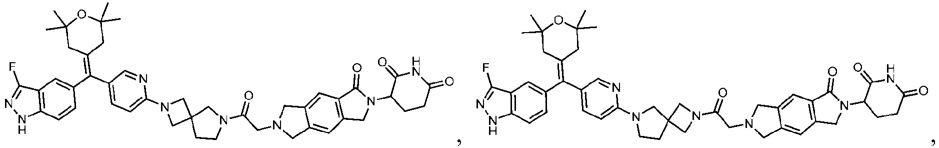

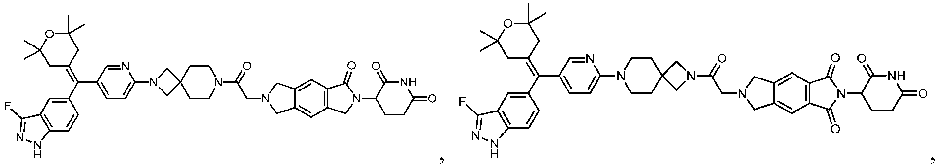

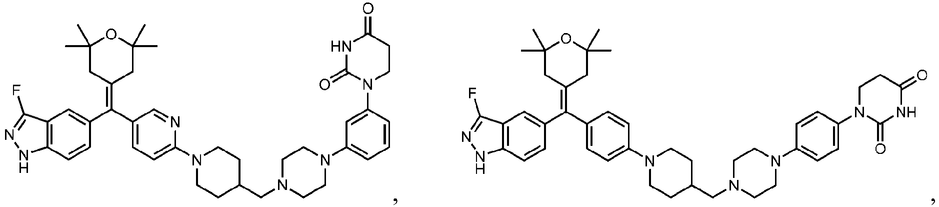

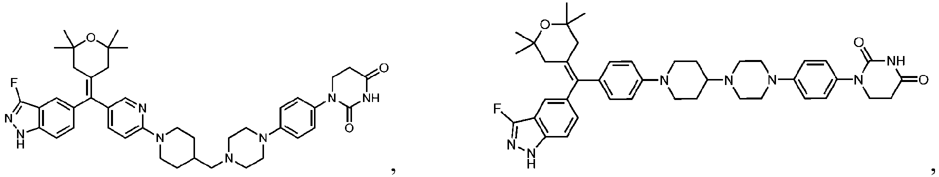

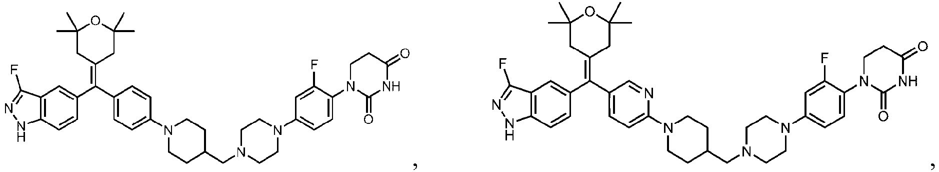

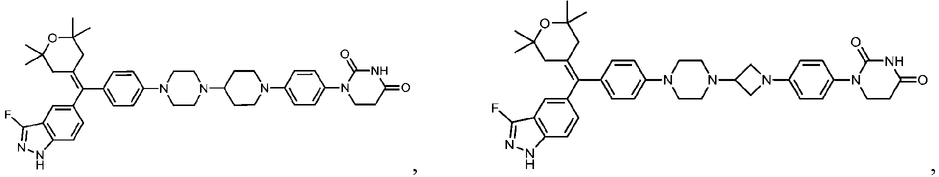

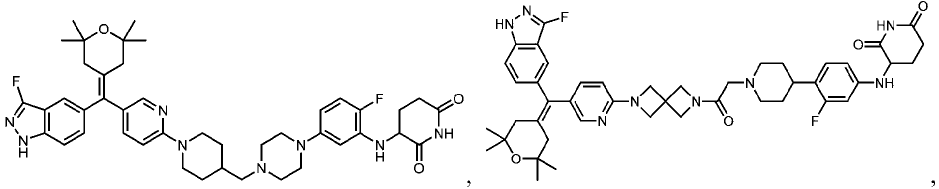

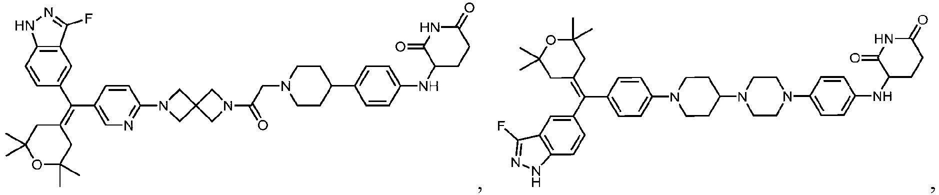

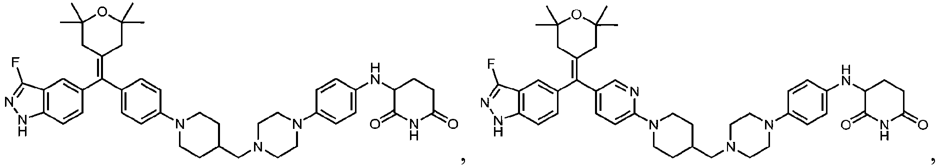

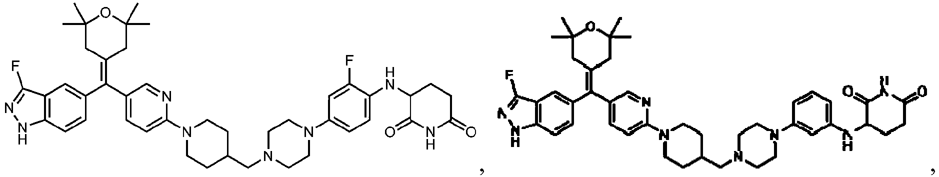

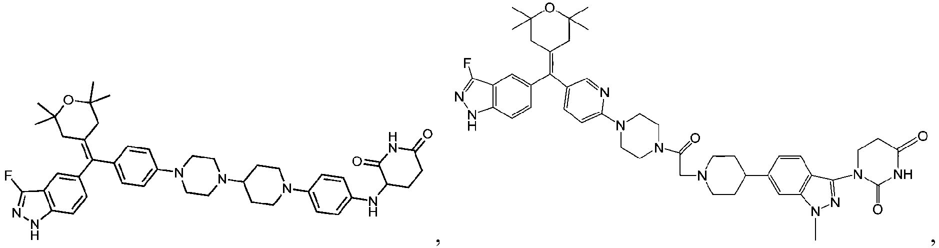

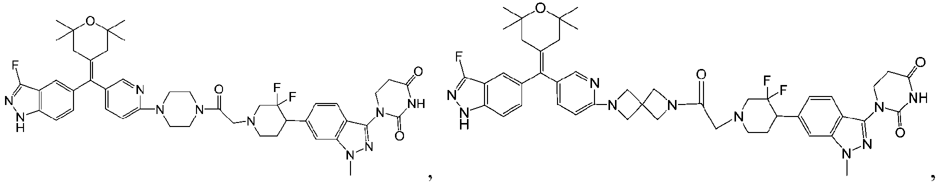

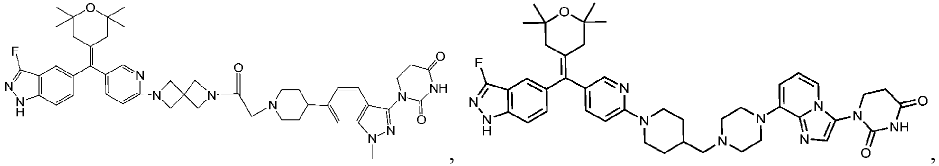

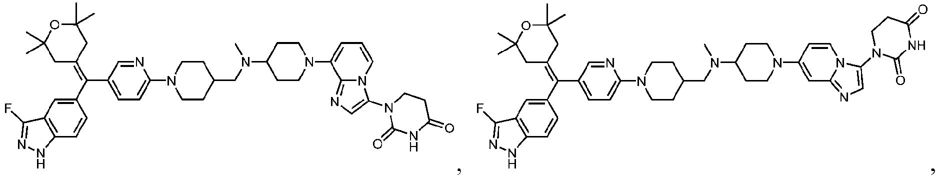

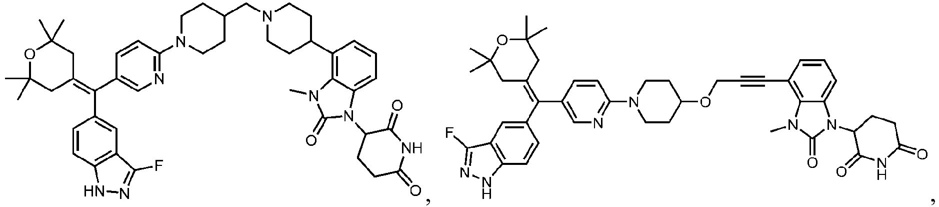

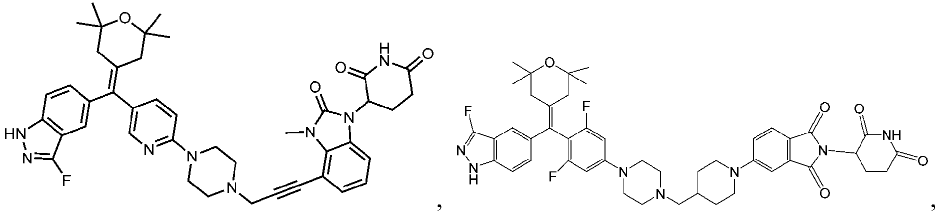

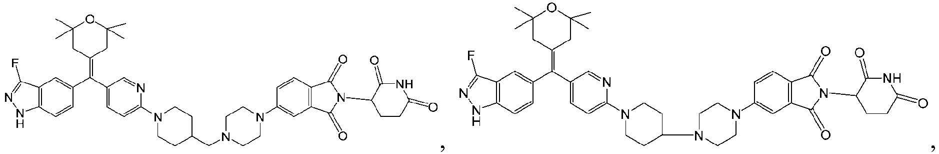

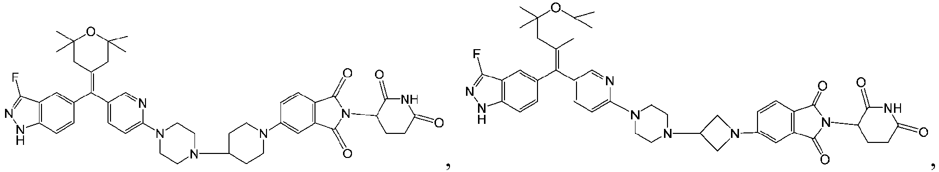

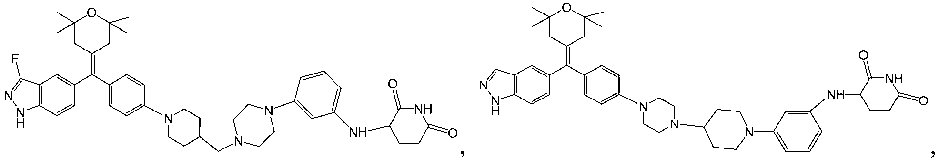

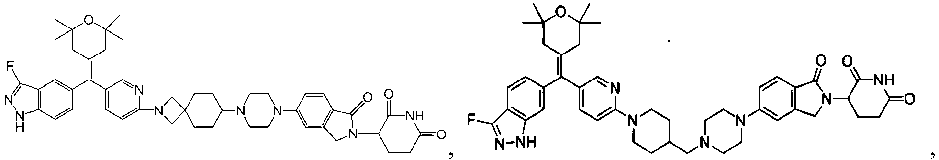

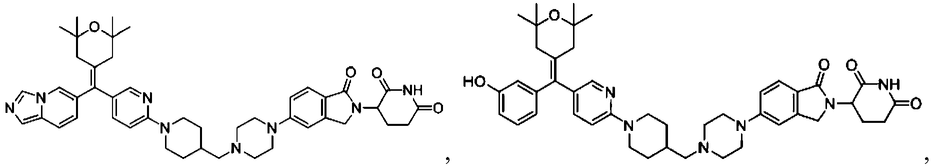

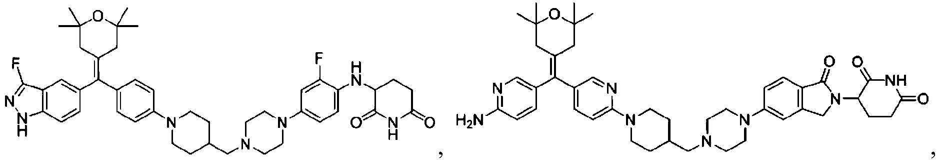

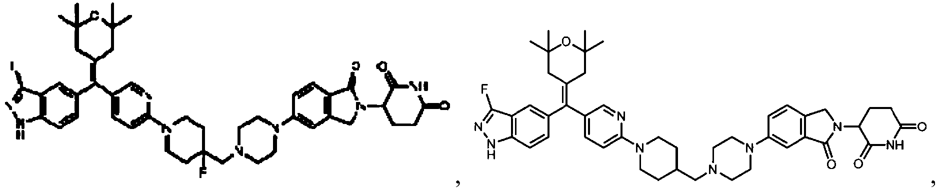

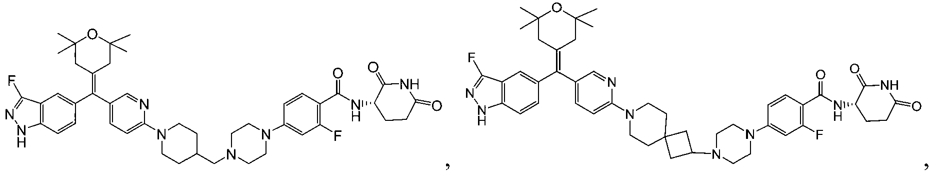

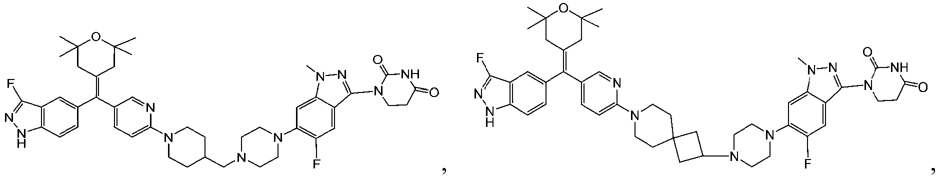

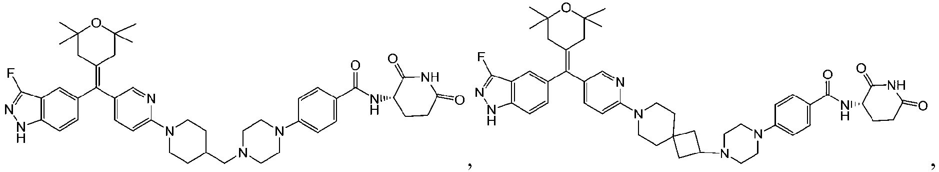

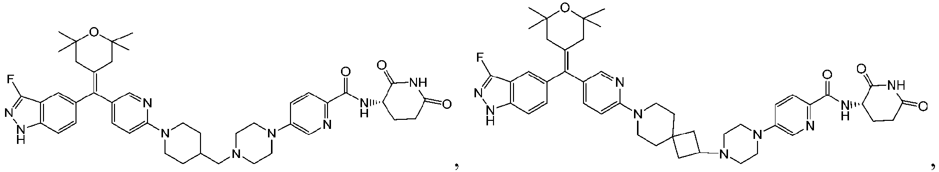

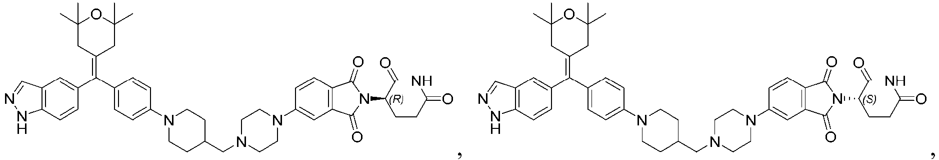

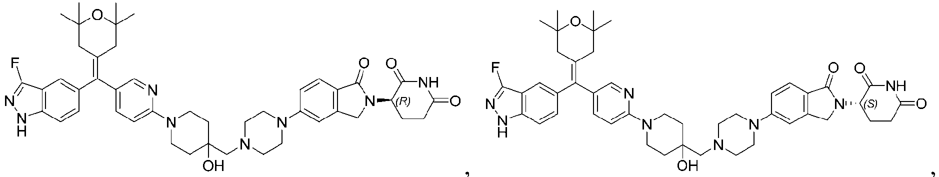

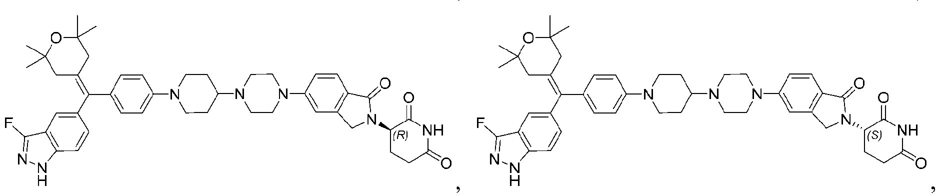

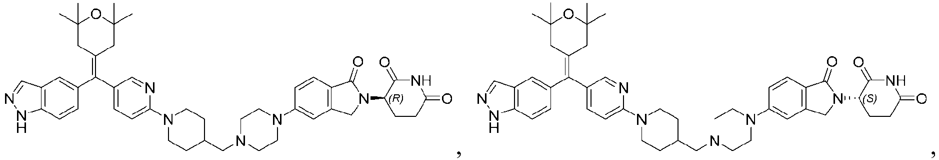

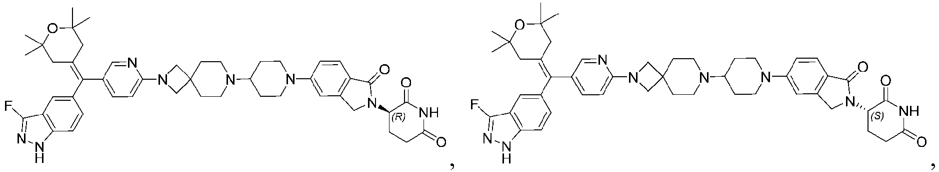

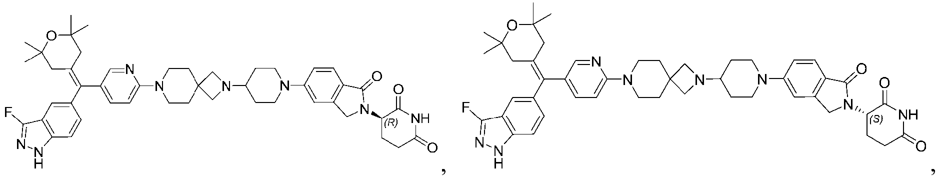

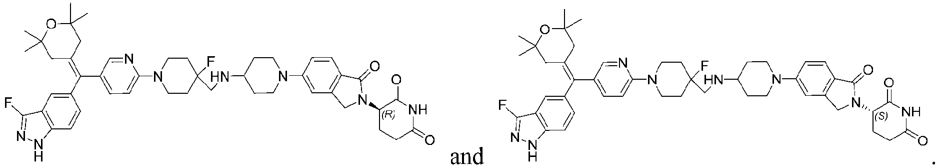

19. A pharmaceutical composition comprising the compound or a pharmaceutically acceptable salt, solvate, hydrate, or isomer thereof according to any one of claims 1 to 18, and a pharmaceutically acceptable carrier.

20. Use of the compound or a pharmaceutically acceptable salt, solvate, hydrate, or isomer thereof according to any one of claims 1 to 18, or the pharmaceutical composition according to claim 19, in the treatment of a disease treated by degrading a target protein that binds to a targeting ligand.

21. The use according to claim 20, wherein the disease is selected from abnormal cell proliferation, cancer, immune diseases, diabetes, cardiovascular diseases, infectious diseases, and inflammatory diseases.

22. The use according to claim 21, wherein the cancer is selected from breast cancer, endometrial cancer, uterine cancer, testicular cancer, cervical cancer, prostate cancer, ovarian cancer, endometriosis, fallopian tube tumors, ovarian tumors, leukemia, skin cancer, squamous cell carcinoma, basal cell carcinoma, adenocarcinoma, renal cell carcinoma, bladder cancer, intestinal cancer, colon cancer, esophageal cancer, head cancer, kidney cancer, liver cancer, lung cancer, neck cancer, pancreatic cancer, stomach cancer, lymphoma, non Hodgkin's lymphoma, melanoma, myeloproliferative disorders, sarcoma, angiosarcoma, peripheral neuroepithelioma, glioma, astrocytoma, oligodendroglioma, ependymoma, glioblastoma, neuroblastoma, gangliocytoma ganglioglioma, medulloblastoma, pineocytoma, meningioma, meningeal sarcoma, neurofibroma, schwannoma, thyroid carcinoma, Hodgkin's lymphoma, Wilms tumor, and teratoma; more preferably, the cancer is selected from breast cancer, endometrial cancer, uterine cancer, testicular cancer, cervical cancer, prostate cancer, ovarian cancer, fallopian tube tumor and ovarian tumor.

23. The use according to claim 22, wherein the cancer is selected from breast cancer, endometrial cancer, ovarian cancer, cervical cancer, prostate cancer, or endometriosis.

24. The use according to claim 21, wherein the infectious disease is selected from viral pneumonia, avian influenza, meningitis, gonorrhea, or diseases caused by infections with HIV, HBV, HCV, HSV, HPV, RSV, CMV, Ebola virus, flavivirus, rhabdovirus, rotavirus, influenza, coronavirus EBV, Diseases caused by drug-resistant viruses, RNA viruses, DNA viruses, adenoviruses, poxviruses, small ribonucleic acid viruses, Togaviridae, orthomyxoviruses, retroviruses, Hepadnavirus, Gram negative bacteria, Gram positive bacteria, atypical bacteria, Staphylococcus, Streptococcus, Escherichia coli, Salmonella, Helicobacter pylori, Chlamydiaceae, Mycoplasmataceae, fungi, protozoa, helminths, worms, prions or parasites.

25. Use of the compound or a pharmaceutically acceptable salt, solvate, hydrate, or isomer thereof according to any one of claims 1 to 18, or the pharmaceutical composition according to claim 19, in the manufacture of a medicament for treating a disease treated by degrading a target protein that binds to a targeting ligand.

26. The use according to claim 25, wherein the disease is selected from abnormal cell proliferation, cancer, immune diseases, diabetes, cardiovascular diseases, infectious diseases, and inflammatory diseases.

27. The use according to claim 26, wherein the cancer is selected from breast cancer, endometrial cancer, uterine cancer, testicular cancer, cervical cancer, prostate cancer, ovarian cancer, endometriosis, fallopian tube tumors, ovarian tumors, leukemia, skin cancer, squamous cell carcinoma, basal cell carcinoma, adenocarcinoma, renal cell carcinoma, bladder cancer, intestinal cancer, colon cancer, esophageal cancer, head cancer, kidney cancer, liver cancer, lung cancer, neck cancer, pancreatic cancer, stomach cancer, lymphoma, non Hodgkin's lymphoma, melanoma, myeloproliferative disorders, sarcoma, angiosarcoma, peripheral neuroepithelioma, glioma, astrocytoma, oligodendroglioma, ependymoma, glioblastoma, neuroblastoma, gangliocytoma ganglioglioma, medulloblastoma, pineocytoma, meningioma, meningeal sarcoma, neurofibroma, schwannoma, thyroid carcinoma, Hodgkin's lymphoma, Wilms tumor, and teratoma; more preferably, the cancer is selected from breast cancer, endometrial cancer, uterine cancer, testicular cancer, cervical cancer, prostate cancer, ovarian cancer, fallopian tube tumor and ovarian tumor.

28. The use according to claim 27, wherein the cancer is selected from breast cancer, endometrial cancer, ovarian cancer, cervical cancer, prostate cancer, or endometriosis.

29. The use according to claim 26, wherein the infectious disease is selected from viral pneumonia, avian influenza, meningitis, gonorrhea, or diseases caused by infections with HIV, HBV, HCV, HSV, HPV, RSV, CMV, Ebola virus, flavivirus, rhabdovirus, rotavirus, influenza, coronavirus EBV, drug-resistant viruses, RNA viruses, DNA viruses, adenoviruses, poxviruses, small ribonucleic acid viruses, Togaviridae, orthomyxoviruses, retroviruses, Hepadnavirus, Gram negative bacteria, Gram positive bacteria, atypical bacteria, Staphylococcus, Streptococcus, Escherichia coli, Salmonella, Helicobacter pylori, Chlamydiaceae, Mycoplasmataceae, fungi, protozoa, helminths, worms, prions or parasites.

30. A method for treating a disease in a subject by degrading a target protein that binds to a targeting ligand, comprising administering to the subject an effective amount of the compound or a pharmaceutically acceptable salt, solvate, hydrate, or isomer thereof according to any one of claims 1 to 18, or the pharmaceutical composition according to claim 19.

31. The method according to claim 30, wherein the disease is selected from abnormal cell proliferation, cancer, immune diseases, diabetes, cardiovascular diseases, infectious diseases, and inflammatory diseases.

32. The method according to claim 31, wherein the cancer is selected from breast cancer, endometrial cancer, uterine cancer, testicular cancer, cervical cancer, prostate cancer, ovarian cancer, endometriosis, fallopian tube tumors, ovarian tumors, leukemia, skin cancer, squamous cell carcinoma, basal cell carcinoma, adenocarcinoma, renal cell carcinoma, bladder cancer, intestinal cancer, colon cancer, esophageal cancer, head cancer, kidney cancer, liver cancer, lung cancer, neck cancer, pancreatic cancer, stomach cancer, lymphoma, non Hodgkin's lymphoma, melanoma, myeloproliferative disorders, sarcoma, angiosarcoma, peripheral neuroepithelioma, glioma, astrocytoma, oligodendroglioma, ependymoma, glioblastoma, neuroblastoma, gangliocytoma ganglioglioma, medulloblastoma, pineocytoma, meningioma, meningeal sarcoma, neurofibroma, schwannoma, thyroid carcinoma, Hodgkin's lymphoma, Wilms tumor, and teratoma; more preferably, the cancer is selected from breast cancer, endometrial cancer, uterine cancer, testicular cancer, cervical cancer, prostate cancer, ovarian cancer, fallopian tube tumor and ovarian tumor.

33. The method according to claim 32, wherein the cancer is selected from breast cancer, endometrial cancer, ovarian cancer, cervical cancer, prostate cancer, or endometriosis.

34. The method according to claim 31, wherein the infectious disease is selected from viral pneumonia, avian influenza, meningitis, gonorrhea, or diseases caused by infections with HIV, HBV, HCV, HSV, HPV, RSV, CMV, Ebola virus, flavivirus, rhabdovirus, rotavirus, influenza, coronavirus EBV, drug-resistant viruses, RNA viruses, DNA viruses, adenoviruses, poxviruses, small ribonucleic acid viruses, Togaviridae, orthomyxoviruses, retroviruses, Hepadnavirus, Gram negative bacteria, Gram positive bacteria, atypical bacteria, Staphylococcus, Streptococcus, Escherichia coli, Salmonella, Helicobacter pylori, Chlamydiaceae, Mycoplasmataceae, fungi, protozoa, helminths, worms, prions or parasites.
